(19) Europäisches Patentamt
European Patent Office
Office européen des brevets

(11) EP 2 446 062 B1

(12) **EUROPEAN PATENT SPECIFICATION**

(45) Date of publication and mention of the grant of the patent:
**13.08.2014 Bulletin 2014/33**

(21) Application number: **10792771.7**

(22) Date of filing: **25.06.2010**

(51) Int Cl.:
***C12Q 1/68*** *(2006.01)*

(86) International application number:
**PCT/US2010/040106**

(87) International publication number:
**WO 2010/151842 (29.12.2010 Gazette 2010/52)**

(54) **METHODS AND SYSTEMS FOR PHYLOGENETIC ANALYSIS**

VERFAHREN UND SYSTEM ZUR PHYLOGENETISCHEN ANALYSE

PROCÉDÉS ET SYSTÈMES D'ANALYSE PHYLOGÉNÉTIQUE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **26.06.2009 US 220937 P**
**09.11.2009 US 259565 P**
**25.03.2010 US 317644 P**
**24.05.2010 US 347817 P**

(43) Date of publication of application:
**02.05.2012 Bulletin 2012/18**

(73) Proprietor: **The Regents of the University of California**
**Oakland, CA 94607-5200 (US)**

(72) Inventors:
- **ANDERSEN, Gary, L.**
  **Berkeley, CA 94708 (US)**
- **DESANTIS, Todd, Z.**
  **Livermore, CA 94551 (US)**
- **BRODIE, Eoin**
  **San Francisco, CA 94114 (US)**

(74) Representative: **Roques, Sarah Elizabeth et al**
**J A Kemp**
**14 South Square**
**Gray's Inn**
**London WC1R 5JJ (GB)**

(56) References cited:
**WO-A2-99/22023 WO-A2-2007/018563**
**WO-A2-2007/039319 WO-A2-2008/130394**
**US-A1- 2002 187 490 US-A1- 2006 051 769**
**US-A1- 2007 122 831 US-A1- 2007 269 813**
**US-A1- 2008 139 398 US-A1- 2008 242 555**
**US-A1- 2008 254 472 US-B1- 7 115 364**

- **WILSON KENNETH H ET AL: "High-density microarray of small-subunit ribosomal DNA probes", APPLIED AND ENVIRONMENTAL MICROBIOLOGY, AMERICAN SOCIETY FOR MICROBIOLOGY, US, vol. 68, no. 5, 1 May 2002 (2002-05-01), pages 2535-2541, XP002289520, ISSN: 0099-2240, DOI: 10.1128/AEM. 68.5.2535-2541.2002**
- **T. Z. DESANTIS ET AL: "Comprehensive aligned sequence construction for automated design of effective probes (CASCADE-P) using 16S rDNA", BIOINFORMATICS, vol. 19, no. 12, 12 August 2003 (2003-08-12), pages 1461-1468, XP055050744, ISSN: 1367-4803, DOI: 10.1093/bioinformatics/btg200**
- **G. W. KLAU ET AL: "Optimal robust non-unique probe selection using Integer Linear Programming", BIOINFORMATICS, vol. 20, no. Suppl 1, 4 August 2004 (2004-08-04), pages i186-i193, XP055051261, ISSN: 1367-4803, DOI: 10.1093/bioinformatics/bth936**
- **MICHAEL WAGNER ET AL: "Unravelling Microbial Communities with DNA-Microarrays: Challenges and Future Directions", MICROBIAL ECOLOGY, SPRINGER-VERLAG, NE, vol. 53, no. 3, 8 March 2007 (2007-03-08), pages 498-506, XP019513473, ISSN: 1432-184X, DOI: 10.1007/S00248-006-9197-7**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- MEI R ET AL: "Probe selection for high-density oligonucleotide arrays", PROCEEDINGS OF THE NATIONAL ACADEMY OF SCIENCES, NATIONAL ACADEMY OF SCIENCES, US, vol. 100, no. 20, 30 September 2003 (2003-09-30), pages 11237-11242, XP002456743, ISSN: 0027-8424, DOI: 10.1073/PNAS.1534744100
- MIRJANA RAJILIC-STOJANOVIC ET AL: "Development and application of the human intestinal tract chip, a phylogenetic microarray: analysis of universally conserved phylotypes in the abundant microbiota of young and elderly adults", ENVIRONMENTAL MICROBIOLOGY, vol. 11, no. 7, 1 July 2009 (2009-07-01), pages 1736-1751, XP055050787, ISSN: 1462-2912, DOI: 10.1111/j.1462-2920.2009.01900.x
- C. PALMER: "Rapid quantitative profiling of complex microbial populations", NUCLEIC ACIDS RESEARCH, vol. 34, no. 1, 8 January 2006 (2006-01-08), pages e5-1-e5-10, XP055051215, ISSN: 0305-1048, DOI: 10.1093/nar/gnj007
- D. N. FRANK ET AL: "Molecular-phylogenetic characterization of microbial community imbalances in human inflammatory bowel diseases", PROCEEDINGS OF THE NATIONAL ACADEMY OF SCIENCES, vol. 104, no. 34, 21 August 2007 (2007-08-21), pages 13780-13785, XP055050956, ISSN: 0027-8424, DOI: 10.1073/pnas.0706625104
- HATTORI M ET AL: "The human intestinal microbiome: A new frontier of human biology", DNA RESEARCH, UNIVERSAL ACADEMY PRESS, JP, vol. 16, no. 1, 1 February 2009 (2009-02-01), pages 1-12, XP009156954, ISSN: 1340-2838, DOI: 10.1093/DNARES/DSN033
- TODD Z DESANTIS ET AL: "High-Density Universal 16S rRNA Microarray Analysis Reveals Broader Diversity than Typical Clone Library When Sampling the Environment", MICROBIAL ECOLOGY, SPRINGER-VERLAG, NE, vol. 53, no. 3, 2 March 2007 (2007-03-02), pages 371-383, XP019513458, ISSN: 1432-184X, DOI: 10.1007/S00248-006-9134-9
- EOIN L BRODIE ET AL: "Application of a High-Density Oligonucleotide Microarray Approach To Study Bacterial Population Dynamics during Uranium Reduction and Reoxidation", APPLIED AND ENVIRONMENTAL MICROBIOLOGY, AMERICAN SOCIETY FOR MICROBIOLOGY, US, vol. 72, no. 9, 1 September 2006 (2006-09-01), pages 6288-6298, XP008111078, ISSN: 0099-2240, DOI: 10.1128/AEM.00246-06
- GENTRY T J ET AL: "Microarray Applications in Microbial Ecology Research", MICROBIAL ECOLOGY, SPRINGER-VERLAG, NE, vol. 52, no. 2, 8 August 2006 (2006-08-08) , pages 159-175, XP019421797, ISSN: 1432-184X, DOI: 10.1007/S00248-006-9072-6
- A. LOY ET AL: "Oligonucleotide Microarray for 16S rRNA Gene-Based Detection of All Recognized Lineages of Sulfate-Reducing Prokaryotes in the Environment", APPLIED AND ENVIRONMENTAL MICROBIOLOGY, vol. 68, no. 10, 1 October 2002 (2002-10-01), pages 5064-5081, XP055051547, ISSN: 0099-2240, DOI: 10.1128/AEM.68.10.5064-5081.2002
- WU YI-BO ET AL: "Design of 16 S rRNA-based Oligonucleotide Array Using Group-specific Non-unique Probes in Large Scale Bacteria Detection", PROGRESS IN BIOCHEMISTRY AND BIOPHYSICS, vol. 36, no. 8, 16 October 2009 (2009-10-16), pages 1025-1034, XP0055051547, ISSN: 1000-3282
- HAZEN T C ET AL: "Deep-Sea Oil Plume Enriches Indigenous Oil-Degrading Bacteria", SCIENCE AMERICAN ASSOCIATION FOR THE ADVANCEMENT OF SCIENCE USA, [Online] vol. 330, no. 6001, 8 October 2010 (2010-10-08), pages 204-208, XP002691109, ISSN: 0036-8075 Retrieved from the Internet: URL:http: //www.sciencemag.org/content/supp l/ 2010/08/23/science. 1195979.DC1/Hazen.SOM .pdf> [retrieved on 2013-01-23]
- HORNER-DEVINE ET AL.: 'A taxa-area relationship for bacteria.' NATURE vol. 432, 2004, pages 750 - 753, XP008149633
- SCHLOSS ET AL.: 'Introducing DOTUR, a Computer Program for Defining Operational Taxonomic Units and Estimating Species Richness.' APPL ENVIRON MICROBIOL. vol. 71, no. 3, 2005, pages 1501 - 1506, XP008149627

## Description

### BACKGROUND OF THE INVENTION

With as many as $10^{30}$ microbial genomes globally, across multiple different environmental and host conditions, variety both within and between microbiomes is well recognized (Huse et al. (2008), PLoS Genetics 4(11): e1000255). As a result of this variety, characterizing the contents of a microbiome is a challenge for current approaches. Firstly, standard culturing techniques are successful in maintaining only a small fraction of the microorganisms in nature. Means of more direct profiling, such as sequencing, face two additional challenges. Both the sheer number of different genomes in a given sample and the degree of homology between members present a complex problem for already laborious procedures.

Biopolymers such as nucleic acids and proteins are often identified in the search for useful genes, to diagnose diseases or to identify organisms. Frequently, hybridization or another binding reaction is used as part of the identification step. As the number of possible targets increases in a sample, the design of systems to detect the different hybridization reactions increases in difficulty along with the analysis of the binding or hybridization data. The design and analysis problems become acute when there are many similar targets in a sample as is the case when the individual species or groups that comprise a microbiome are detected or quantified in a single assay based on a highly conserved polynucleotide. For example, while approximately 98% of bacteria found in the human gut belong to only four bacterial divisions, this includes approximately 36,000 different phylotypes at the strain level, having ≥99% sequence identity (Hattori et al. (2009), DNA Res. 16: 1-12). While possibly containing certain overlapping taxa, the different environments presented by the guts of other hosts are expected to support different microbiomes. In situations where contributions from multiple sub-enviroments are combined, such as a water source potentially contaminated by a variety of sources, just identifying the thousands of taxa is a significant challenge to current methods of detection. WO 2008/130394 provides a system for detecting a plurality of operational taxon units (OTUs). The present disclosure provides improvements to capacity and accuracy of detection. For example, the disclosure provides a system that comprises, in one embodiment, computer executable logic for deconvoluting signal intensities of the plurality of probe pairs into probability estimates by: (i) comparing signal intensities for perfect match probes, mismatch probes, positive control probes, and negative control probes; (ii) calculating a probability that an individual OTU is present based on the signal intensities of probes for that OTU; and (iii) penalizing the probability based on potential for cross-hybridization of probes with sequences from other OTUs. Further improvements and advantages are provided herein.

Since the study of microbiomes can offer new insight into origins of environmental change, disease, immunological functions, and physiological functions, improved methods for designing nucleic acids, proteins, or other probes that can recognize specific organisms, or taxa are needed. Similarly, improved methods for data analysis that allow detection and quantification of the members of a microbial community at high confidence levels are also needed.

### SUMMARY OF THE INVENTION

The invention provides a system for detecting in a single assay a plurality of operational taxonomic units (OTU) in a sample, wherein each of a plurality of first nucleic acid sequences comprise less than 0.01 percent of the total nucleic acids in a population of nucleic acid sequences from the sample; each of the plurality of first nucleic acid sequences are at least 95% homologous to all of the other first nucleic acid sequences in the population of nucleic acid sequences; the system comprising

(a) a plurality of probe pairs for each of at least 10,000 OTUs, each probe pair comprising a perfect match (PM) probe and a mismatch (MM) probe;
(b) a plurality of negative and positive control probes; and
(c) computer executable logic for deconvoluting signal intensities of the plurality of probe pairs into probability estimates by:

(i) comparing signal intensities for PM probes, MM probes, positive control probes, and negative control probes;
(ii) calculating a probability that an individual OTU is present based on the signal intensities of probes for that OTU; and
(iii) penalizing the probability based on potential for cross-hybridization of probes with sequences from other OTUs.

The invention also provides a method for measuring an increase of a particular microorganism's percentage of gut microbiome in an individual comprising applying a sample to the system of the invention and identifying the increase.

The invention also provides a method for determining the probability of the presence or quantity of a unique sequence or microorganism in a sample comprising:

(a) contacting said sample with a plurality of different probes;
(b) determining hybridization signal strength for sample sequences to each of said probes; and
(c) removing or attenuating from analysis an operational taxonomic unit (OTU) from the possible list based on hybridization signal strength data, thereby increasing the confidence in the remaining hybridization signal strength data;

wherein removing or attenuating is performed by penalizing OTUs present in the sample based on potential cross-hybridization of probes from the OTU with polynucleotides from the other OTUs.

The invention also provides a method for identifying a microbiome signature indicative of a condition comprising:

(a) comparing the presence and optionally abundance of one of more different OTUs in a control sample without said condition and a reference sample with said condition using the system of the invention; and
(b) identifying one or more OTUs that associate with said condition.

Some embodiments provide a system comprising a plurality of probes capable of determining the presence, absence, relative abundance, and/or quantity of at least 10,000 different OTUs in a single assay. In some embodiments, the system is configured to produce a biosignature that is indicative of fecal contamination. In some embodiments, the probes selectively hybridize to one or more highly conserved polynucleotides, which can include 16S rRNA gene, 23S rRNA gene, 5S rRNA gene, 5.8S rRNA gene, 12S rRNA gene, 18S rRNA gene, 28S rRNA gene, gyrB gene, rpoB gene, fusA gene, recA gene, coxl gene, nif13 gene, RNA molecules derived therefrom, or a combination thereof. In some embodiments, the conserved polynucleotides are amplicons. In some embodiments, the probes can be attached to a substrate. In some embodiments, the probes can form an array. In some embodiments, the substrate comprises a bead, microsphere, glass, plastic, or silicon. In some embodiments, the system is capable of performing sequencing reactions on the same highly conserved region of each of the OTUs. In some embodiments, the system further comprises one or more species-specific probes. In some embodiments, each of the OTUs is bacterial, archaeal, or fungal.

In some embodiments, the system further comprises a plurality of positive control probes. In some embodiments, the system further comprises a plurality of negative control probes. In some embodiments, the negative control probes comprise sequences that are not complementary to sequence found in the highly conserved polynucleotide. In some embodiments, the positive control probes comprise sequences that are complementary to a polynucleotide selected from SEQ ID NOs:51-100. In some embodiments, the positive control probes comprise one or more sequences selected from SEQ ID NOs: 51-100.

In some embodiments, the system removes data from at least a subset of said interrogation probes before making a final call on the presence, absence, relative abundance, and/or quantity of said OTUs. In some embodiments, the data is removed based on interrogation probe cross-hybridization potential.

Some embodiments provide a system capable of detecting one or more first nucleic acid sequences comprising $1x10^{-3}$ or less of the total nucleic acids present in a single assay with a confidence level greater than 95% and sensitivity level greater than 95%, wherein the one or more first nucleic acid sequences and set of remaining target nucleic acids are at least 95% homologous. In some embodiments, the system is configured to produce a biosignature that is indicative of fecal contamination. In some embodiments, one or more of the nucleic acid sequences are 16S rRNA gene, 23S rRNA gene, 5S rRNA gene, 5.8S rRNA gene, 12S rRNA gene, 18S rRNA gene, 28S rRNA gene, gyrB gene, rpoB gene, fusA gene, recA gene, coxl gene, nif13 gene, RNA molecules derived therefrom, or a combination thereof. In some embodiments, the nucleic acids comprise amplicons.

Some embodiments provide a system for determining the presence, absence, relative abundance, and/or quantity of a plurality of different OTUs in a single assay, said system comprising a plurality of polynucleotide interrogation probes, a plurality of polynucleotide positive control probes, and a plurality of polynucleotide negative control probes. In some embodiments, the system is configured to produce a biosignature that is indicative of fecal contamination. In some embodiments, the system removes data from at least a subset of said interrogation probes before making a final call on the presence, absence, relative abundance, and/or quantity of said microorganisms. In some embodiments, data is removed based on interrogation probe cross hybridization potential.

Some embodiments provide a system capable of detecting the presence, absence, relative abundance, and/or quantity of more than 10,000 different OTUs of a single domain (e.g. bacterial, archaeal, or fungal) in a single assay with confidence greater than 95%. In some embodiments, the system is configured to produce a biosignature that is indicative of fecal contamination. In some embodiments, the system comprises a plurality of probes that selectively hybridize to the same highly conserved region in each of said OTUs. In some embodiments, the system is capable of performing sequencing reactions on the same highly conserved region of each of said OTUs. In some embodiments, the system further comprises species-specific probes, wherein the probes do not hybridize to said highly conserved sequence. In some embodiments, the system comprises 100 species-specific probes.

Some embodiments provide a system for determining the presence, absence, relative abundance, and/or quantity of one or more microorganisms from a sample, said system comprising a plurality of OTUs, wherein the median number of probes per OTU is less than 26. Some embodiments provide a system for determining the presence, absence, relative abundance, and/or quantity of one or more microorganisms from a sample, said system comprising a plurality of OTUs, wherein the median number of cross-hybridizations per probe is less than 20. In some embodiments, the system is configured to produce a biosignature that is indicative of fecal contamination.

Some embodiments provide a method for determining a condition of a sample comprising: a) contacting said sample with a plurality of different probes; b) determining hybridization signal strength for each of said probes, wherein said determination establishes a biosignature for said sample; and c) comparing the biosignature of said sample to a biosignature for fecal contamination.

In one aspect of the invention, a method is .provided for determining the probability of the presence, relative abundance, and/or quantity of a microorganism in a sample comprising a) determining hybridization signal strength distributions of negative control probes that do not specifically hybridize to a highly conserved polynucleotide in the microorganism; b) determining hybridization signal strength distributions of positive control probes; c) determining hybridization signal strengths for a plurality of different interrogation probes, each of which is complementary to a section within the highly conserved polynucleotide; and d) using the hybridization signal strengths of the negative and positive probes to determine the probability that the hybridization signal for the different interrogation probes represents the presence, relative abundance, and/or quantity of the microorganism. In some embodiments, the hybridization signal strengths of the negative and positive probes are used to normalize or fit the interrogation probes hybridization data. In further embodiments, the normalization or fitting of interrogation probes hybridization data utilizes A+T content or normal and gamma distributions of the negative and positive control probes. In other embodiments, the negative control probes and/or the positive control probes comprise perfect match and mismatch probes. In further embodiments, the normal and gamma distribution of the negative and positive control probes involves calculating a pair difference score for said probes. In other embodiments, the hybridization signal strengths for the plurality of different interrogation probes are attenuated based on the G+C content of each probe.

In one aspect, a method is provided for determining the probability of the presence or quantity of a unique polynucleotide or microorganism in a sample comprising a) contacting the sample with a plurality of different probes; b) determining hybridization signal strength for sample polynucleotides to each of the probes; c) removing or attenuating from analysis an OTU/taxa from the possible list based on hybridization signal strength data, thereby increasing the confidence level of the remaining hybridization signal strength data. In some embodiments, the removing or attenuating is performed only on OTUs having a percentage of probes that pass a certain threshold intensity within such OTU. In some embodiments, only OTUs that pass a certain threshold are further analyzed. In still further embodiments, the removing or attenuating is performed by penalizing OTUs present in the sample based on potential cross hybridization of probes from the OTU with polynucleotides from other OTUs. In some embodiments, the penalization positively correlates with potential for cross hybridization with other OTUs. In other embodiments, penalization based on cross hybridization is performed at each level of a phylogenic tree starting with the lowest level. In further embodiments, only penalized OTUs scoring above a hybridization signal strength threshold are further analyzed. In still other embodiments, only parts of phylogenic tree that include an OTU are analyzed.

In a further aspect of the invention, a method is provided for determining presence or quantity of a plurality of different organisms in a sample comprising determining GC content of each probe and comparing each probe intensity to a positive control probe intensity and negative probe intensity to determine quantity of said probes.

In another aspect of the invention, computer executable logic is provided for determining a probability that one or more organisms from a set of different organisms are present in a sample said logic comprising: a) an algorithm for determining likelihood that individual interrogation probe intensities are accurate based on comparison with intensities of negative control probes and positive control probes; b) an algorithm for determining likelihood that an individual OTU is present based on intensities of interrogation probes from said OTU passing a first quantile threshold; and c) an algorithm for penalizing one or more OTUs that have passed the first quantile threshold based on potential for cross-hybridization of probes analyzing said OTUs sequences with sequences from other OTUs.

In a further aspect, computer executable logic is provided for determining the presence and optionally quantity of one or more microorganisms in a sample comprising: logic for analyzing intensities from a set of probes that selectively binds each of at least 10,000, 20,000, 30,000, 40,000, 50,000, 60,000, 70,000, 80,000, 90,000 or 100,000 highly conserved polynucleotides, and determining the presence of at least 90%, 95%, 97%, or more of all species present in said sample. The determination can be made with at least a 90%, 95%, 98%, 99%, or 99.5% confidence level.

In another aspect, computer executable logic is provided for determining the presence of one or more microorganisms in a sample comprising: logic for analyzing a set of at least 1000 different interrogation perfect probes, and logic for discarding information from at least 10% of said interrogation perfect probes in the process of making said determination.

In one aspect, a method is provided for probe selection comprising: a) selecting a set of highly conserved polynucleotides; b) comparing said plurality of polynucleotides against a plurality of standard polynucleotides to identify chimeric sequences; c) removing chimeric sequences identified in the comparison step; and d) selecting probes that are complementary to the remaining polynucleotides. In some embodiments, at least 500,000 highly conserved polynucleotides are selected. In other embodiments, a member of the plurality of polynucleotides is considered not a chimeric sequence if it shares greater than 95% similarity with a member of the plurality of standard polynucleotides. In still other embodiments, the plurality of polynucleotides are compared against themselves to identify chimeric sequences. In other embodiments, highly conserved polynucleotides comprise sequences from a 16S RNA gene, 23S RNA gene, 5S RNA gene, 5.8S rRNA gene, 12S rRNA gene, 18S rRNA gene, 28S rRNA gene, gyrB gene, rpoB gene, fusA gene, recA gene, cox1 gene, nifD gene, or combinations thereof.

In another aspect, a method of probe selection is provided comprising: a) selecting a plurality of nucleic acid sequences; b) aligning the plurality of nucleic acid sequences with a plurality of standard nucleic acid sequences to identify insertion points in each of the plurality of nucleic acid sequences; c) removing sequences with at least 10, 20, 30, 40, 50, or more insertion points or with insertions that are at least 100 nucleic acids in length; and d) selecting probes that are complementary to the remaining nucleic acids.

In a further aspect, a method of probe selection is provided comprising: a) selecting a plurality of nucleic acid sequences; b) filtering the plurality of nucleic acid sequences; c) performing hierarchical clustering on remaining nucleic acid sequences to generate a guide tree; and d) selecting probes that are complementary to each node in said guide tree. In some embodiments, filtering the plurality of nucleic acid sequences comprises removing sequences that are identified to comprise PCR primer artifacts, removing sequences that are identified to comprise insertions, removing sequences that are identified as chimeric, or any combination thereof.

In one aspect, a method is provided for identifying a microbiome signature indicative of a condition, the method comprising a) comparing the presence and optionally abundance of at least 1,000 different OTUs in a control sample without said condition and a reference sample with said condition; and b) identifying one or more OTUs that associate with said condition. In some embodiments, the condition is an oil spill. In some embodiments, an increase in the similarity in the presence and optionally abundance of said OTUs in said reference sample with respect to said control sample is indicative of remediation of said condition. In some embodiments, changes in the degree of similarity in the presence and optionally abundance of said OTUs in said reference sample with respect to said control sample are provided as a measure of remediation of said condition. In some embodiments, the method further comprises projecting a time to reaching a predetermined level of remediation of said condition.

In one aspect, a method is provided for selecting probes for assaying a condition in a sample comprising: a) applying one or more test samples having said condition to a detection system that simultaneously assays for the probability of the presence or absence of at least 10,000 OTUs of a single domain, such as bacteria, archea, fungus, or each known OTU of a single domain; b) applying one or more control samples not having said condition to said detection system to determine the probability of the presence or absence of said OTUs in said control samples; c) determining a pattern of OTUs associated with the test samples that is not associated with the control samples; and d) identifying probes that selectively detect the OTUs associated with the test sample. In some embodiments, one or more of the identified probes are selected for use in a low-density probe system. In some embodiments, the pattern consists of up to 200 different OTUs. In other embodiments, the sample is a water sample and the condition is fecal contamination, toxic alga-bloom contamination, presence of fish pathogens, a point source contamination, a non-point source contamination, or a combination thereof. In some embodiments, a unique biosignature of a type of contamination is used to determine the source of the contamination. In some embodiments, the sample is a human or animal sample. In some embodiments, the sample is obtained from the gut, respiratory system, oral cavity, sinuses, nares, urogenital tract, skin, feces, udders, or a combination thereof. In some embodiments, the condition being characterized (e.g., diagnosed or prognosed) in that sample is Crohn's Disease, irritable bowel syndrome, cancer, rhinitis, stomach ulcers, colitis, atopy, asthma, neonatal necrotizing enterocolitis, acne, food allergy, Gastroesophageal reflux disease, obesity or periodontal disease. In some embodiments, the sample is a food, water, soil, or air sample. In some embodiments, the sample is from a forest, industrial crop, or other plant.

In one aspect, a method is provided to identify at least one new indicator species for a condition comprising: a) assaying in a single experiment a control sample without said condition to determine the presence or absence of each OTU of all known bacteria, archaea, or fungi; b) assaying in a single experiment a test sample with said condition to determine the presence or absence of each OTU of all known bacteria, archaea, or fungi; c) comparing results from (a) and (b) to identify at least one microorganism whose abundance changes by a predetermined measure in response to the change in the condition, wherein the identified microorganism species represents said new indicator species for said condition. In some embodiments, the identified microorganism decreases in abundance in the presence of the condition while in others the identified microorganism increases in abundance. In some embodiments, the predetermined measure is at least a 2-fold change in abundance. In some embodiments, the predetermined measure is a statistically significant change in abundance.

In another aspect, a system is provided for determining the probability that a microorganism or a select group of microorganisms are present in a sample, the system comprising two or more probes identified by the disclosed algorithms. In some embodiments, the system determines the probability with a confidence level greater than 95%, 99% or 99.5%. In other embodiments, the determination is performed simultaneously or using a single assay.

In one aspect, a system is provided that is capable in a single assay of distinguishing between two OTUs on a phylogenetic tree with an accuracy/confidence of greater than or equal to 95%, 99% or 99.5% based on the selective hybridization of a plurality of probes to highly conserved nucleic acids isolated from each organism to be distinguished.

In another aspect, a system is provided that is capable of generating a microbiome signature comprising at least 10,000 OTUs from an environment in a single assay with an accuracy and/or confidence level greater than 95%. In some embodiments the probes selectively hybridize to nucleic acids from each organism being detected.

In one aspect a method is provided for detecting a source of microorganism contamination, the method comprising in a single assay, determining the present and quantity of at least 20, 50, 100, or more microorganism OTUs not naturally occurring in said sample and identifying the source of the contamination using a pattern of the presence and quantity of the OTUs.

In another aspect, a system is provided that is capable of detecting the presence and quantity of at least 50 different fecal taxa in a single assay. In some embodiments, the detection is based on the selective hybridization of a plurality of probes to highly conserved nucleic acids isolated from each organism to be detected. In some embodiments, detection is based on the selective hybridization of a plurality of probes that identify the organisms or taxa listed in Table 4. In some embodiments, detection comprises detecting hybridization of one or more probes that selectively hybridize to nucleic acids indicative of clean water taxa, wherein said probes are selected from that a plurality of probes that identify the organisms or taxa listed in Table 11.

In a further aspect, a method is provided for detecting fecal contamination in water comprising: detecting the presence or absence in the water sample of one or more polynucleotides which detect the taxa listed in Table 4. In some embodiments, the method further comprises detecting hybridization of one or more probes that selectively hybridize to polynucleotides indicative of clean water taxa listed in Table 11.

In another aspect, a method is provided for testing a water sample, the method comprising calculating a ratio of Bacilli, Bacteroidetes and Clostridia (BBC) species and $\alpha$-proteobacteria (A) species in said water, wherein a value greater than 1.0 is indicative of fecal contamination. In some embodiments, calculating the ratio does not rely on culturing, directly counting, PCR cloning, sequencing or use of a gene expression array. In some embodiments, the Bacilli, Bacteroidetes, Clostridia and $\alpha$-proteobacteria species comprise the species listed in Table 4. In some embodiments, calculating the ratio of BBC species to A species comprises contacting the water sample with a plurality of probes. In some embodiments, the plurality of probes are complimentary to a highly conserved gene.

In a further aspect, a method is provided for predicting the likelihood of a toxic alga bloom, the method comprising: a) contacting a water sample with a plurality of probes that selectively bind to nucleic acids derived from cyanobacteria selected from Table 6; b) using hybridization data derived to determine the quantity and composition of cyanobacteria in the water sample; c) measuring environmental conditions; and d) predicting the likelihood of a toxic alga bloom based on cyanobacteria quantity and composition and environmental conditions. In some embodiments, the probes to cyanobacteria nucleic acids are selected using the present methods and detect the genera listed in Table 6. In some embodiments, the environmental conditions comprise water temperature, turbidity, nitrogen concentration, oxygen concentration, carbon concentration, phosphate concentration and/or sunlight level. In further embodiments, a water management decision is made based on the likelihood of a toxic alga bloom.

In one aspect, a method is provided for determining a condition of a subject or a therapy for a subject, the method comprising performing a single nucleic acid assay on a sample from said subject to determine the presence and/or amount of at least 1000 OTUs.

In another aspect, a method is provided for predicting a condition of a sample, the method comprising a) determining microorganism population data as the probability of the presence or absence of at least 1,000 OTUs of microorganisms in the sample; b) determining gene expression data of one or more genes of said microorganisms in the sample; and c) using the expression data and population data to predict the condition of the sample. In some embodiments, the sample is a water or soil sample.

In another aspect, the invention provides a method for assessing damage caused by an oil spill. In some embodiments, the method comprises (a) determining the presence, absence, and/or abundance of at least 1,000 OTUs in one or more samples from one or more locations unaffected by said oil spill, thereby establishing an unaffected biosignature; (b) determining the presence, absence, and/or abundance of at least 1,000 OTUs in one or more samples from a location affected by said oil spill, thereby establishing an oil-spill-affected biosignature; and (c) comparing said unaffected biosignature to said oil-spill-affected biosignature, wherein differences in said biosignatures are indicative of affects on the microbiome of said location affected by said oil spill. In some embodiments, step (b) is performed at a first time and a second time. In some embodiments, a change in said differences in said biosignatures between said first time and said second time are used to track the progress of remediation of oil spill damage.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

Figure 1 illustrates an example of a suitable computer system environment.
Figure 2 illustrates a networked system for the remote acquisition or analysis of data obtained through a method of the invention.
Figure 3 illustrates a flow chart of the probe selection process.
Figures 4A-B demonstrate the distribution of observed pair difference score, d, from quantitative standards (QS) probes and negative controls (NC) probes.
Figure 5 is a graph showing variations of gamma scale across 79 arrays.
Figure 6 illustrates the pre-partition process for computational load balancing.
Figure 7 is a vector plot comparing the microbial community composition in polluted water samples compared to three potential pollution sources: sewage, septage and cattle waste to determine the source of the pollution.
Figure 8 is a logarithmic bar graph showing the number of OTUs detected (y-axis) by the PhyloChip for each pooled clean room sample (x-axis). The number of spores detected by the spore count are shown.
Figure 9 is a graphical representation showing the network of common and unique families detected in each pooled clean room sample.
Figure 10A shows a graph of the pair diffusion score frequencies of probes on the PhyloChip for the pooled clean room samples.
Figure 10B is a graphical representation showing the commonly detected phyla detected by the PhyloChip in PCR negative pooled clean room samples as a relationship network.
Figure 11 is a graphical representation comparing the probe responses to Faecalibacterium OTU 36742 observed on two different PhyloChip experiments.
Figure 12 is a graphical representation comparing the probe responses to Ruminococcus OTU 38712 observed on two different PhyloChip experiments.
Figure 13 is a density plots demonstrating the d observation of the Negative Control probes.
Figure 14 is a chart showing the concentration of 16S amplicon versus PhyloChip response.
Figure 15 is boxplot comparison of the detection algorithm based on pair "response score",r, distribution (novel) versus the positive fraction calculation (previously used with the G2 PhyloChip.
Figure 16 is two graphs that show the comparison of the r score metric versus the pf by receiver operator characteristic (R.O.C) plots.
Figure 17 is a chart showing PhyloChip results from similar biological communities form ordination clusters.
Figure 18 is a chart showing PhyloChip results from similar biological communities form ordination clusters.
Figure 19 shows an NMS analysis demonstrating that the four sampling sites are quite distinct, and that the biological replicates show quite high levels of similarity.
Figure 20 is a heatplot summary of an analysis called the Method of Shrunken Centroids to identify the -50 or so microbial OTUs that most significantly define the observed differences in overall community structure between sampling locations.
Figure 21 is a representation of differing degrees of change in community composition in response to a change in climate.
Figure 22 is two charts showing NMS ordinations of PhyloChip bacteria OTUs of: a) Fresh samples collected from the North, Mid and South-lat. sites in August 2005 and b) fresh samples and transplant-control samples from the same sited at the same time (1 year after transplanting). The fresh samples depicted in both graphs are the same samples. The bars represent 1 s.d. of 3 replicates.
Figure 23 is four charts showing NMS ordinations of PhyloChip bacteria OTUs of PhyloChip bacteria OTUs of reciprocally transplanted samples and transplanted controls collected 1 year after they were transplanted. Arrows show the trajectory of the change in composition of transplanted samples away from that of their site-of-origin controls.
Figure 24 shows 2 charts showing the NMS ordinations of PhyloChip bacteria OTUs of: a) Fresh samples collected from the North, Mid and South-lat. sites in September 2007 and b) fresh samples and transplant-control samples from the same sites at the same time (3 years after transplanting). The fresh samples depicted in both graphs are the same samples. The bars represent 1 s.d. of 3 replicates.
Figure 25 is four charts showing NMS ordinations of PhyloChip bacteria OTUs of reciprocally transplanted samples and transplanted controls collected 3 years after they were transplanted. Arrows show the trajectory of the change in composition of transplanted samples away from that of their site-of-origin controls.

Figure 26 is a schematic showing cluster analysis of detected bacterial taxa in fecal samples by species and type of animal (ruminants and grazers, pinnipeds, birds).
Figure 27 is a bar chart showing the number of indicator OTUs for each type of species.
Figure 28 is an ordination chart showing indicator communities were compared to polluted water samples for source identification.
Figure 29 is a bar chart showing sewage taxa with strong correlations to FIB.
Figure 30 is schematic showing results of cluster analysis which showed the comparison of community composition. Communities can be clustered according to the time in the receiving waters, source, and type of receiving waters.
Figure 31 is a bar chart showing the effect of time in receiving waters on fecal microbial communities.
Figure 32 is a bar chart showing the effect of creek versus bay water on waste microbial communities.
Figure 33 illustrates enrichment of bacterial taxa by an oil plume.

## DETAILED DESCRIPTION OF THE INVENTION

### DEFINITIONS

As used herein, the term "oligonucleotide" refers to a polynucleotide, usually single stranded, that is either a synthetic polynucleotide or a naturally occurring polynucleotide. The length of an oligonucleotide is generally governed by the particular role thereof, such as, for example, probe, primer and the like. Various techniques can be employed for preparing an oligonucleotide, for instance, biological synthesis or chemical synthesis. A nucleic acid of the present invention will generally contain phosphodiester bonds, although in some cases, as outlined below, nucleic acid analogs are included that may have alternate backbones, comprising, for example, phosphoramide (Beaucage, et al., Tetrahedron, 49(10):1925 (1993) and references therein; Letsinger, J. Org. Chem., 35:3800 (1970); Sprinzl, et al., Eur. J. Biochem., 81:579 (1977); Letsinger, et al., Nucl. Acids Res., 14:3487 (1986); Sawai, et al., Chem. Lett., 805 (1984), Letsinger, et al., J. Am. Chem. Soc., 110:4470 (1988); and Pauwels, et al., Chemica Scripta, 26:141 (1986)); phosphorothioate (Mag, et al, Nucleic Acids Res., 19:1437 (1991); and U.S. Pat. No. 5,644,048); phosphorodithioate (Briu, et al., J. Am. Chem. Soc., 111:2321 (1989)); 0-methylphophoroamidite linkages (see Eckstein, Oligonucleotides and Analogues: A Practical Approach, Oxford University Press); and peptide nucleic acid backbones and linkages (see Egholm, J. Am. Chem. Soc., 114:1895 (1992); Meier, et al., Chem. Int. Ed. Engl., 31:1008 (1992); Nielsen, Nature, 365:566 (1993); Carlsson, et al., Nature, 380:207 (1996)). Other analog nucleic acids include those with positive backbones (Denpcy, et al., Proc. Natl. Acad. Sci. USA, 92:6097 (1995)); non-ionic backbones (U.S. Pat. Nos. 5,386,023; 5,637,684; 5,602,240; 5,216,141; and 4,469,863; Kiedrowshi, et al., Angew. Chem. Intl. Ed. English, 30:423 (1991); Letsinger, et al., J. Am. Chem. Soc., 110:4470 (1988); Letsinger, et al., Nucleosides & Nucleotides, 13:1597 (1994); Chapters 2 and 3, ASC Symposium Series 580, "Carbohydrate Modifications in Antisense Research", Ed. Y. S. Sanghui and P. Dan Cook; Mesmaeker, et al., Bioorganic & Medicinal Chem. Lett., 4:395 (1994); Jeffs, et al., J. Biomolecular NMR, 34:17 (1994); Tetrahedron Lett., 37:743 (1996)); and non-ribose backbones, including those described in U.S. Pat. Nos. 5,235,033 and 5,034,506, and Chapters 6 and 7, ASC Symposium Series 580, "Carbohydrate Modifications in Antisense Research", Ed. Y. S. Sanghui and P. Dan Cook. Nucleic acids containing one or more carbocyclic sugars are also included within the definition of nucleic acids (see Jenkins, et al., Chem. Soc. Rev., (1995) pp. 169-176). Several nucleic acid analogs are described in Rawls, C & E News, Jun. 2, 1997, page 35.

The nucleic acid may be DNA, RNA, or a hybrid and may contain any combinationof deoxyribo- and ribonucleotides, and any combination of bases, including uracil, adenine, thymine, cytosine, guanine, inosine, xanthanine, hypoxanthanine, isocytosine, isoguanine, and base analogs such as nitropyrrole and nitroindole, etc. Oligonucleotides can be synthesized by standard methods such as those used in commercial automated nucleic acid synthesizers and later attached to an array, bead or other suitable surface. Alternatively, the oligonucleotides can be synthesized directly on the assay surface using photolithographic or other techniques. In some embodiments, linkers are used to attach the oligonucleotides to an array surface or to beads.

As used herein, the term "nucleic acid molecule" or "polynucleotide" refers to a compound or composition that is a polymeric nucleotide or nucleic acid polymer. The nucleic acid molecule may be a natural compound or a synthetic compound. The nucleic acid molecule can have from about 2 to 5,000,000 or more nucleotides. The larger nucleic acid molecules are generally found in the natural state. In an isolated state, the nucleic acid molecule can have about 10 to 50,000 or more nucleotides, usually about 100 to 20,000 nucleotides. It is thus obvious that isolation of a nucleic acid molecule from the natural state often results in fragmentation. It may be useful to fragment longer target nucleic acid molecules, particularly RNA, prior to hybridization to reduce competing intramolecular structures. Fragmentation can be achieved chemically or enzymatically. Typically, when the sample contains DNA, a nuclease such as deoxyribonuclease (DNase) is employed cleave the phosphodiester linkages. Nucleic acid molecules, and fragments thereof, include, but are not limited to, purified or unpurified forms of DNA (dsDNA and ssDNA) and RNA, including tRNA, mRNA, rRNA, mitochondrial DNA and RNA, chloroplast DNA and RNA, DNA/RNA hybrids, biological material or mixtures thereof, genes, chromosomes, plasmids, cosmids, the genomes of microorganisms, e.g., bacteria, yeasts, phage, chromosomes, viruses, viroids, molds, fungi, or other higher organisms such as plants, fish, birds, animals, humans, and the like. The polynucleotide can be only a minor fraction of a complex mixture such as a biological sample.

As used herein, the term "hybridize" refers to the process by which single strands of polynucleotides form a double-stranded structure through hydrogen bonding between the constituent bases. The ability of two polynucleotides to hybridize with each other is based on the degree of complementarity of the two polynucleotides, which in turn is based on the fraction of matched complementary nucleotide pairs. The more nucleotides in a given polynucleotide that are complementary to another polynucleotide, the more stringent the conditions can be for hybridization and the more specific will be the binding between the two polynucleotides. Increased stringency may be achieved by elevating the temperature, increasing the ratio of co-solvents, lowering the salt concentration, and combinations thereof.

As used herein, the terms "complementary," "complement," and "complementary nucleic acid sequence" refer to the nucleic acid strand that is related to the base sequence in another nucleic acid strand by the Watson-Crick base-pairing rules. In general, two polynucleotides are complementary when one polynucleotide can bind another polynucleotide in an anti-parallel sense wherein the 3'-end of each polynucleotide binds to the 5'-end of the other polynucleotide and each A, T(U), G, and C of one polynucleotide is then aligned with a T(U), A, C, and G, respectively, of the other polynucleotide. Polynucleotides that comprise RNA bases can also include complementary G/U or U/G basepairs.

As used herein, the term "clustering tree" refers to a hierarchical tree structure in which observations, such as organisms, genes, and polynucleotides, are separated into one or more clusters. The root node of a clustering tree consists of a single cluster containing all observations, and the leaf nodes correspond to individual observations. A clustering tree can be constructed on the basis of a variety of characteristics of the observations, such as sequences of the genes and morphological traits of the organisms. Many techniques known in the art, e.g. hierarchical clustering analysis, can be used to construct a clustering tree. A non- limiting example of the clustering tree is a phylogenetic, taxonomic or evolutionary tree.

As used herein, the terms "operational taxon unit," "OTU," "taxon," "hierarchical cluster," and "cluster" are used interchangeably. An operational taxon unit (OTU) refers to a group of one or more organisms that comprises a node in a clustering tree. The level of a cluster is determined by its hierarchical order. In one embodiment, an OTU is a group tentatively assumed to be a valid taxon for purposes of phylogenetic analysis. In another embodiment, an OTU is any of the extant taxonomic units under study. In yet another embodiment, an OTU is given a name and a rank. For example, an OTU can represent a domain, a sub-domain, a kingdom, a sub-kingdom, a phylum, a sub-phylum, a class, a sub¬class, an order, a sub-order, a family, a subfamily, a genus, a subgenus, or a species. In some embodiments, OTUs can represent one or more organisms from the kingdoms eubacteria, protista, or fungi at any level of a hierarchal order. In some embodiments, an OTU represents a prokaryotic or fungal order.

As used herein, the term "kmer" refers to a polynucleotide of length k. In some embodiments, k is an integer from 1 to 1000. In some embodiments, k is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 125, 150, 175, 200, 250, 300, 400, 500, 600, 700, 800, 900, or 1000.

As used herein, the term "perfect match probe" (PM probe) refers to a kmer which is 100% complementary to at least a portion of a highly conserved target gene or polynucleotide. The perfect complementarity usually exists throughout the length of the probe. Perfect probes, however, may have a segment or segments of perfect complementarity that is/are flanked by leading or trailing sequences lacking complementarity to the target gene or polynucleotide.

As used herein, the term "mismatch probe" (MM probe) refers a control probe that is identical to a corresponding PM probe at all positions except for one, 2, 3, 4, 5, 6, 7, 8, 9 or 10 nucleotides of the PM probe. Typically, the non-identical position or positions are located at or near the center of the PM probe. In some embodiments, the mismatch probes are universal mismatch probes, e.g., a collection of mismatch probes that have no more than a set number of nucleotide variations or substitutions compared to positive probes. For example, the universal mismatch probes may differ in nucleotide sequence by no more than five nucleotides compared to any one PM probe in the PM probe set. In some embodiments, a MM probe is used adjacent to each test probe, e.g., a PM probe targeting a bacterial 16S rRNA sequence, in the array.

As used herein, the term "probe pair" refers to a PM probe and its corresponding MM probe. In some embodiments, the PM probes and the MM probes are scored in relation to each other during data processing and statistic analysis. As used herein, the term "a probe pair associated with an OTU" is defined as a pair of probes consisting of an OTU-specific PM probe and its corresponding MM probe.

As used herein, a "sample" is from any source, including, but not limited to, a gas sample, a fluid sample, a solid sample, or any mixture thereof

As used herein, a "microorganism" or "organism" includes, but is not limited to, a virus, viroids, bacteria, archaea, fungi, protozoa and the like.

The term "sensitivity" refers to a measure of the proportion of actual positives which are correctly identified as such.

The term "specificity" refers to a measure of the proportion of actual negatives which are correctly identified as such

The term "confidence level" refers to the likelihood, expressed as a percentage, that the results of a test are real and repeatable, and not random. Confidence levels are used to indicate the reliability of an estimate and can be calculated by a variety of methods.

The present invention relates to systems and methods for detecting contamination broadly, and more specifically in water. "Contamination," as used herein, refers to the presence of any undesirable element or substance (a "contaminant") in an analyzed composition. In some embodiments, the analyzed composition is water. In further embodiments, the contaminant is a microorganism. Contamination may result from the presence of one or more contaminants above a threshold level.

In one aspect, the invention utilizes a biosignature of OTUs. As used herein, the term "biosignature" refers to an association of the level of one or more members of one or more OTUs with a particular condition. In one embodiment, the biosignature comprises a determination of the presence, absence, and/or quantity of at least 5, 10, 20, 50, 100, 250, 500, 1000, 5000, 10,000, 20,000, 30,000, 40,000, 50,000, 60,000, 70,000, 80,000, 90,000, 100,000, 250,000, 500,000 or 1,000,000 OTUs in a sample using a single assay. In some embodiments, the biosignature comprises the presence of or changes in the level of at least 1, 2, 3, 4, 5, 6, 7, 8, 9, 10, 15, 20, 30, 40, 50, 75, 100, 125, 150, 175, 200, 250, 300, or more OTUs.

In one embodiment, the biosignature is associated with a single condition, for example contamination by a single source. In another embodiment, the biosignature is associated with a combination of conditions, for example contamination by two or more sources, such as contamination by 2, 3, 4, 5, 6, 7, 8, 9, 10 or more sources. A biosignature can be obtained for any sample, including but not limited to, fresh water, drinking water, marine water, reclaimed water, treated water, desalinated water, sewage, lakes, rivers, streams, oceans, surface water, groundwater, runoff, waste water, aquifers, other natural or non-natural bodies of water, and known contaminants. A biosignature can be determined for a pure sample, a known contaminant, or a combination thereof. In some embodiments, a biosignature of a test sample is compared to a known biosignature, and a determination is made as to likelihood that the signatures are the same. In further embodiments, a biosignature of a sample is compared to a biosignature from a contamination source. The biosignature to which the biosignature of the test sample is compared can be determined before, after, or at substantially the same time as that of the test sample. Biosignatures can be the result of one or more analyses of one or more samples from a particular source. Examples of contamination sources whose signatures can be analyzed include, but are not limited to, fecal matter from humans; fecal matter from avian sources, including migratory and non-migratory birds; fecal matter from cattle and livestock, including elk, cows, deer, sheep, horses, pigs, and goats; and fecal matter from aquatic animals, including sea lions, seals, and otters. Water contamination detected herein can also be from decaying matter (e.g. plant or animal decay), oil spills, industrial waste or byproducts, and any other contaminant to which an OTU biosignature can be correlated.

In some embodiments, the biosignature of a test sample is a combination of two or more independent signatures, such as 2, 3, 4, 5, 6, 7, 8, 9, 10 or more independent signatures. In a preferred embodiment, each of the two or more biosignatures contained in a sample are assayed simultaneously. In a further embodiment, a subset of biosignatures can be evaluated through the use of low-density detection systems, comprising the determination of the presence, absence, and/or level of no more than 10, 25, 50, 100, 250, 500, 1000, 2000, or 5000 OTUs.

In one aspect, the invention provides methods, systems, and compositions for detecting and identifying a plurality of biomolecules and organisms in a sample. The invention utilizes the ability to differentiate between individual organisms or OTUs. In one aspect, the individual organisms or OTUs are identified using organism-specific and/or OTU-specific probes, e.g., oligonucleotide probes. More specifically, some embodiments relate to selecting organism-specific and/or OTU-specific oligonucleotide probes useful in detecting and identifying biomolecules and organisms in a sample. In some embodiments, an oligonucleotide probe is selected on the basis of the cross-hybridization pattern of the oligonucleotide probe to regions within a target oligonucleotide and its homologs in a plurality of organisms. The homologs can have nucleotide sequences that are at least 80%, 85%, 90%, 91%, 92%, 93%, 94%, 95%, 96%, 97%, 98%, 99% or 99.5% identical. Such oligonucleotides can be gene, or intergenetic sequences, in whole or a portion thereof. The oligonucleotides can range from 10 to over 10,000 nucleotides in length. In some other embodiments, a method is provided for detecting the presence of an OTU in a sample based at least partly on the cross-hybridization of the OTU-specific oligonucleotide probes to probes specific for other organisms or OTUs. In some embodiments, the biosignature to which a sample biosignature is compared comprises a positive result for the presence of the targets for one or more probes.

In one aspect, the invention provides a diagnostic system for the determination or evaluation of a biosignature of a sample. In one embodiment, the diagnostic system comprises at least 1, 2, 3, 4, 5, 6, 7, 8, 9, 10, 15, 20, 30, 40, 50, 75, 100, 125, 150, 175, 200, 250, 300, or more probes. In another embodiment, the diagnostic system comprises up to 1, 2, 3, 4, 5, 6, 7, 8, 9, 10, 15, 20, 30, 40, 50, 75, 100, 125, 150, 175, 200, 250, 300, or more probes.

## High Capacity Systems

In one aspect of the invention, a high capacity system is provided for determining a biosignature of a sample by assessing the total microorganism population of a sample in terms of the microorganisms present and their percent composition of the total population. The system comprises of a plurality of probes that are capable of determining the presence or quantity of at least 10,000, 20,000, 30,000, 40,000, 50,000, 60,000, or more different OTUs in a single assay. Typically, the probes selectively hybridize to a highly conserved polynucleotide. Usually, the probes hybridize to the same highly conserved polynucleotide or within a portion thereof. Generally, the highly conserved polynucleotide or fragment thereof comprises a gene or fragment thereof. Exemplary highly conserved polynucleotides comprise nucleotide sequences found in the 16S rRNA gene, 23S rRNA gene, 5S rRNA gene, 5.8S rRNA gene, 12S rRNA gene, 18S rRNA gene, 28S rRNA gene, gyrB gene, rpoB gene, fusA gene, recA gene, coxl gene and nifD gene. In other embodiments, two or more, three or more, four or more, five or more, six or more, seven or more, eight or more, nine or more, ten or more, 15 or more, 20 or more, 25 or more, or 50 or more collections of probes are employed, each of which specifically hybridizes to a different highly conserved polynucleotides. For example, one collection of probes binds to the same region of the 16S rRNA gene, while a second collection of probes binds to the same region of the 23S rRNA gene. The use of two or more collections of probes where each collection recognizes distinct and separate highly conserved polynucleotides allows for the generation and testing of more probes the use of which can provide greater discrimination between species or OTUs.

Highly conserved polynucleotides usually show at least 80%, 85%, 90%, 92%, 94%, 95%, or 97% homology across a domain, kingdom, phylum, class, order, family or genus, respectively. The sequences of these polynucleotides can be used for determining evolutionary lineage or making a phylogenetic determination and are also known as phylogenetic markers. In some embodiments, a biosignature comprises the presence, absence, and/or abundance of a combination of phylogenetice markers. The OTUs detected by the probes disclosed herein can be bacterial, archeal, fungal, or eukaryotic in origin. Additionally, the methodologies disclosed herein can be used to quantify OTUs that are bacterial, archaeal, fungal, or eukaryotic. By combining the various probes sets, a system for the detection of bacteria, archaea, fungi, eukaryotes, or combinations thereof can be designed. Such a universal microorganism test that is conducted as a single assay can provide great benefit for assessing and understanding the composition and ecology of numerous environments, including characterization of biosignatures for various samples, environments, conditions, and contaminants.

In another aspect of the invention, a system is provided that is capable of determining the probability of presence and optionally quantity of at least 10,000, 20,000, 30,000, 40,000, 50,000 or 60,000 different OTUs of a single domain in a single assay. Such a system makes a probability determination with a confidence level greater than 90%, 91%, 92%, 93%, 94%, 95%, 99% or 99.5%. In some embodiments, a biosignature can comprise the combined result of each probability determination.

Some embodiments provide a method of selecting an oligonucleotide probe that is specific for a node in a clustering tree. In some embodiments, the method comprises selecting a highly conserved target polynucleotide and its homologs for a plurality of organisms; clustering the polynucleotides and homologs of the plurality of organisms into a clustering tree; and determining a cross-hybridization pattern of a candidate oligonucleotide probe that hybridizes to a first polynucleotide to each node on the clustering tree. This determination is performed (e.g., in silico) to determine the likelihood that the probe would cross hybridize with homologs of its target complementary sequence. The candidate oligonucleotide probe can be complementary to a highly conserved target polynucleotide, a fragment of the highly conserved target or one of its homologs in one of the plurality of organisms. In some embodiments, a method is provided for the determination of the cross-hybridization pattern of a variant of the candidate oligonucleotide probe to each node on the clustering tree, wherein the variant corresponds to the candidate oligonucleotide probe but comprises at least 1 nucleotide mismatch; and selecting or rejecting the candidate oligonucleotide probe on the basis of the cross-hybridization pattern of the candidate oligonucleotide probe and the cross-hybridization pattern of the variant. In some embodiments, the node is an operational taxon unit (OTU). In some embodiments, the node is a single organism.

Some embodiments provide a method of selecting an OTU-specific oligonucleotide probe for use in detecting a plurality of organisms in a sample. In some embodiments, the method comprises: selecting a highly conserved target polynucleotide and its homologs from the plurality of organisms; clustering the polynucleotides of the target gene and its homologs from the plurality of organisms into one or more operational taxonomic units (OTUs), wherein each OTU comprises one or more groups of similar nucleotide sequence; determining the cross-hybridization pattern of a candidate OTU-specific oligonucleotide probe to the OTUs, wherein the candidate OTU-specific oligonucleotide probe corresponds to a fragment of the target gene or its homolog from one of the plurality of organisms; determining the cross-hybridization pattern of a variant of the candidate OTU-specific oligonucleotide probe to the OTUs, wherein the variant comprises at least 1 nucleotide mismatch from the candidate OTU-specific oligonucleotide probe; and selecting or rejecting the candidate OTU-specific oligonucleotide probe on the basis of the cross-hybridization pattern of the candidate OTU-specific oligonucleotide probe and the cross-hybridization pattern of the variant. In some embodiments, the candidate OTU-specific oligonucleotide probe is selected if the candidate OTU-specific oligonucleotide probe does not cross-hybridize with any polynucleotide that is complementary to probes from other OTUs. In further embodiments, the candidate OTU-specific oligonucleotide probe is selected if the candidate OTU-specific oligonucleotide probe cross-hybridizes with the polynucleotide in no more than 1, 2, 3, 4, 5, 6, 7, 8, 9, 10, 11, 12, 13, 14, 15, 20, 30, 40, 50, 100, 200, 500, or 1000 other OTU groups.

Some embodiments provide a method of selecting a set of organism-specific oligonucleotide probes for use in detecting a plurality of organisms in a sample. In some embodiments, the method comprises: identifying a highly conserved target polynucleotide and its homologs in the plurality of organisms; determining the cross-hybridization pattern of a candidate organism-specific oligonucleotide probe to the sequences of the highly conserved target polynucleotide and its homologs in the plurality of organisms, wherein the candidate oligonucleotide probe corresponds to a fragment of the target sequence or its homolog from one of the plurality of organisms; determining the cross-hybridization pattern of a variant of the candidate organism-specific oligonucleotide probe to the sequences of the highly conserved target sequence and its homologs in the plurality of organisms, wherein the variant comprises at least 1 nucleotide mismatch from the candidate organism-specific oligonucleotide probe; and selecting or rejecting the candidate organism-specific oligonucleotide probe on the basis of the cross-hybridization pattern of the candidate organism-specific oligonucleotide probe and the cross-hybridization pattern of the variant of the candidate organism-specific oligonucleotide probe.

In some embodiments, an OTU-specific oligonucleotide probe does not cross-hybridize with any polynucleotide that is complementary to probes from other OTUs. In other embodiments, an OTU-specific oligonucleotide probe cross-hybridizes with the polynucleotide in no more than 1, 2, 3, 4, 5, 6, 7, 8, 9, 10, 11, 12, 13, 14, 15, 20, 30, 40, 50, 100, 200, 500, or 1000 other OTU groups. Some embodiments utilize a set of organism-specific oligonucleotide probes for use in detecting a plurality of organisms in a sample. In further embodiments, the candidate organism-specific oligonucleotide probe is selected if the candidate organism-specific oligonucleotide probe only hybridizes with the target nucleic acid molecule of no more than 1, 2, 3, 4, 5, 6, 7, 8, 9, 10, 11, 12, 13, 14, 15, 20, 30, 40, 50 unique organisms in the plurality of organisms. In other embodiments, the process is iterative with multiple candidate specific-specific oligonucleotide probes selected. Frequently, the selected organism-specific oligonucleotide probes are clustered and aligned into groups of similar sequences that allow for the detection of an organism with high confidence based on no more than 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 50, or 60 organism-specific oligonucleotide probe matches per OTU. Generally, the candidate organism that the organism-specific oligonucleotide probes detect corresponds to a leaf or node of at least one phylogenetic, genealogic, evolutionary, or taxonomic tree. Knowledge of the position that a candidate organism detected by the organism-specific oligonucleotide probe occupies on a tree provides relational information of the organism to other members of its domain, phylum, class, subclass, order, family, subfamily, or genus.

In some embodiments, the method disclosed herein selects and/or utilizes a set of organism-specific oligonucleotide probes that are a hierarchical set of oligonucleotide probes that can be used to detect and differentiate a plurality of organisms. In some embodiments, the method selects and/or utilizes organism-specific or OTU-specific oligonucleotide probes that allow a comprehensive screen for at least 80%, 85%, 90%, 95%, 99% or 100% of all known bacterial or archaeal taxa in a single analysis, and thus provides an enhanced detection of different desired taxonomic groups. In some embodiments, the identity of all known bacterial or archaeal taxa comprises taxa that were previously identified by the use of oligonucleotide specific probes, PCR cloning, and sequencing methods. Some embodiments provide methods of selecting and/or utilizing a set of oligonucleotide probes capable of correctly categorizing mixed target nucleic acid molecules into their proper operational taxonomic unit (OTU) designations. Such methods can provide comprehensive prokaryotic or eukaryotic identification, and thus comprehensive biosignature characterization.

In some embodiments, the selected OTU-specific oligonucleotide probe is used to calculate the relative abundance of one or more organisms that belong to a specific OTU at differing levels of taxonomic identification. In some embodiments, an array or collection of microparticles comprising at least one organism-specific or OTU-specific oligonucleotide probe selected by the method disclosed herein is provided to infer specific microbial community activities. For example, the identity of individual taxa in a microbial consortium from an anaerobic environment for instance, a marsh, can be determined along with their relative abundance. If the consortium is suspected of harboring microorganisms capable of butanol fermentation, then after providing a suitable feedstock in an anaerobic environment if the production of butanol is noted, then those taxa responsible for butanol fermentation can be inferred by the microorganisms that have abundant quantities of 16S rRNA. The invention provides methods to measure taxa abundance based on the detection of directly labeled 16S rRNA capable of the anaerobic fermentation of butanol can be identified from a sample obtained from a marsh or other anaerobic environment.

Some embodiments select multiple probes for increasing the confidence level and/or sensitivity level of identification of a particular organism or OTU. The use of multiple probes can greatly increase the confidence level of a match to a particular organism. In some embodiments, the selected organism-specific oligonucleotide probes are clustered and aligned into groups of similar sequence such that detection of an organism is based on 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35 or more oligonucleotide probe matches. In some embodiments, the oligonucleotide probes are specific for a species. In other embodiments, the oligonucleotide probe recognizes related organisms such as organisms in the same subgenus, genus, subfamily, family, suborder, order, sub-class, class, sub-phylum, phylum, sub-kingdom, or kingdom.

Perfect match (PM) probes are perfectly complementary to the target polynucleotide, e.g., a sequence that identifies a particular organism. In some embodiments, a system of the invention comprises mismatch (MM) control probes. Usually, MM probes are otherwise identical to PM probes, but differ by one or more nucleotides. Probes with one or more mismatch can be used to indicate non-specific binding and a possible non-match to the target sequence. In some embodiments, the MM probes have one mismatch located in the center of the probe, e.g., in position 13 for a 25mer probe. The MM probe is scored in relation to its corresponding PM probe as a "probe pair." MM probes can be used to estimate the background hybridization, thereby reducing the occurrence of false positive results due to non-specific hybridization, a significant problem with many current detection systems. If an array is used, such as an Affymetrix high density probe array or Illumina bead array, ideally, the MM probe is positioned adj acent or close to its corresponding PM probe on the array.

Some embodiments relate to a method of selecting and/or utilizing a set of oligonucleotide probes that enable simultaneous identification of multiple prokaryotic taxa with a relatively high confidence level. Typically, the confidence level of identification is at least 90%, 91%, 92%, 93%, 94%, 95%, 96%, 97%, 98%, 99% or 99.5%. An OTU refers to an individual species or group of highly related species that share an average of at least 85%, 90%, 91%, 92%, 93%, 94%, 95%, 96%, 97%, 98%, 99% or 99.5% sequence homology in a highly conserved region. Multiple MM probes may be utilized to enhance the quantification and confidence of the measure. In some embodiments, each interrogation probe of a plurality of interrogation probes has from about 1 to about 20 corresponding mismatch control probes. In further embodiments, each interrogation probe has from about 1 to about 10, about 1 to about 5, about 1 to 4, 1 to 3, 2 or 1 corresponding mismatch probes. These interrogation probes target unique regions within a target nucleic acid sequence, e.g., a 16S rRNA gene, and provide the means for identifying at least about 10, 20, 50, 100, 500, 1,000, 2,000, 5,000, 10,000, 20,000, 30,000, 40,000, 50,000, 60,000, 70,000, 80,000, 90,000, 100,000, 250,000, 500,000 or 1,000,000 taxa. In some embodiments, multiple targets can be simultaneously assayed or detected in a single assay through a high-density oligonucleotide probe system. The sum of all target hybridizations is used to identify specific prokaryotic taxa. The result is a more efficient and less time consuming method of identifying unculturable or unknown organisms. The invention can also provide results that could not previously be achieved, e.g., providing results in hours where other methods would require days. In some embodiments, a microbiome (i.e., sample) can be assayed to determine the identity and abundance of its constituent microorganisms in less than 20, 19, 18, 17, 16, 15, 14, 13, 12, 11, 10, 9, 8, 7, 6, 5, 4, 3, 2, or 1 hour.

In some embodiments, the set of OTU-specific oligonucleotide probes comprises from about 1 to about 500 probes for each taxonomic group. In some embodiments, the probes are proteins including antibodies, or nucleic acid molecules including oligonucleotides or fragments thereof. In some embodiments, an oligonucleotide probe corresponds to a nucleotide fragment of the target nucleic acid molecule. In some embodiments, from about 1 to about 500, about 2 to about 200, about 5 to about 150, about 8 to about 100, about 10 to about 35, or about 12 to about 30 oligonucleotide probes can be designed for each taxonomic grouping. In other embodiments, a taxonomic group can have at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, or more probes. In some embodiments, various taxonomic groups can have different numbers of probes, while in other embodiments, all taxonomic groups have a fixed number of probes per group. Multiple probes in a taxonomic group can provide additional data that can be used to make a determination, also known as "making a call" as to whether an OTU is present or not. Multiple probes also allow for the removal of one or more probes from the analysis based on insufficient signal strength, cross hybridization or other anomalies. Removing probes can increase the confidence level of results and further allow for the detection of low abundant microorganisms. The oligonucleotide probes can each be from about 5 to about 100 nucleotides, from about 10 to about 50 nucleotides, from about 15 to about 35 nucleotides, or from about 20 to about 30 nucleotides. In some embodiments, the probes are at least 5-mers, 6-mers, 7-mers, 8-mers, 9-mers, 10-mers, 11-mers, 12-mers, 13-mers, 14-mers, 15-mers, 16-mers, 17-mers, 18-mers, 19-mers, 20-mers, 21-mers, 22-mers, 23-mers, 24-mers, 25-mers, 26-mers, 27-mers, 28-mers, 29-mers, 30-mers, 31-mers, 32-mers, 33-mers, 34-mers, 35-mers, 36-mers, 37-mers, 38-mers, 39-mers, 40-mers, 41-mers, 42-mers, 43-mers, 44-mers, 45-mers, 46-mers, 47-mers, 48-mers, 49-mers, 50-mers, 51-mers, 52-mers, 53-mers, 54-mers, 55-mers, 56-mers, 57-mers, 58-mers, 59-mers, 60-mers, 61-mers, 62-mers, 63-mers, 64-mers, 65-mers, 66-mers, 67-mers, 68-mers, 69-mers, 70-mers, 71-mers, 72-mers, 73-mers, 74-mers, 75-mers, 76-mers, 77-mers, 78-mers, 79-mers, 80-mers, 81-mers, 82-mers, 83-mers, 84-mers, 85-mers, 86-mers, 87-mers, 88-mers, 89-mers, 90-mers, 91-mers, 92-mers, 93-mers, 94-mers, 95-mers, 96-mers, 97-mers, 98-mers, 99-mers, 100-mers or combinations thereof

Some embodiments provide methods of selecting multiple, confirmatory, organism-specific or OTU-specific probes to increase the confidence of detection. In some embodiments, the methods also select one or more mismatch (MM) probes for every perfect match (PM) probe to minimize the effect of cross-hybridization by non-target regions. The organism-specific and OTU-specific oligonucleotide probes selected by the methods disclosed herein can simultaneously identify thousands of taxa present in an environmental sample and allow accurate identification of microorganisms and their phylogenetic relationships in a community of interest. Systems that use the organism-specific and OTU-specific oligonucleotide probes selected by the methods disclosed herein and the computational analysis disclosed herein have numerous advantages over rRNA gene sequencing techniques. Such advantages include reduced cost per microbiome analysis, and increased processing speed per sample or microbiome from both the physical analysis and the computational analysis point of view the analysis procedures are not adversely affected by chimeras, are not subject to creating artificial phylotypes and are not subject to barcode PCR bias. Additionally, quantitative standards can be run with a microbiome sample of the invention, something that is not possible with pyrosequencing.

Some embodiments provide a method for selecting and/or utilizing a set of OTU- or organism-specific oligonucleotide probes for use in an analysis system or bead multiplex system for simultaneously detecting a plurality of organisms in a sample. The method targets known diversity within target nucleic acid molecules to determine microbial community composition and establish a biosignature. The target nucleic acid molecule is typically a highly conserved polynucleotide. In some embodiments, the highly conserved polynucleotide is from a highly conserved gene, whereas in other embodiments the polynucleotide is from a highly conserved region of a gene with moderate or large sequence variation. In further embodiments, the highly conserved region may be an intron, exon, or a linking section of nucleic acid that separates two genes. In some embodiments, the highly conserved polynucleotide is from a "phylogenetic" gene. Phylogenetic genes include, but are not limited to, the 5.8S rRNA gene, 12S rRNA gene, 16S rRNA gene-prokaryotic, 16S rRNA gene-mitochondrial, 18S rRNA gene, 23S rRNA gene, 28S rRNA gene, gyrB gene, rpoB gene, fusA gene, recA gene, coxl gene, and the nifD gene. With eukaryotes, the rRNA gene can be nuclear, mitochondrial, or both. In some embodiments, the 16S-23S rRNA gene internal transcribed spacer (ITS) can be used for differentiation of closely related taxa with or without the use of other rRNA genes. For example, rRNA, e.g., 16S or 23S rRNA, acts directly in the protein assembly machinery as a functional molecule rather than having its genetic code translated into protein. Due to structural constraints of 16S rRNA, specific regions throughout the gene have a highly conserved polynucleotide seqeuence although non-structural segments may have a high degree of variability. Probing the regions of high variability can be used to identify OTUs that represent a single species level, while regions of less variability can be used to identify OTUs that represent a subgenus, a genus, a subfamily, a family, a sub-order, an order, a sub-class, a class, a subphylum, a phylum, a sub-kingdom, or a kingdom. The methods disclosed herein can be used to select organism-specific and OTU-specific oligonucleotide probes that offer high level of specificity for the identification of specific organisms, OTUs representing specific organisms, or OTUs representing specific taxonomic group of organisms. The systems and methods disclosed herein are particularly useful in identifying closely related microorganisms and OTUs from a background or pool of closely related organisms.

The probes selected and/or utilized by the methodologies of the invention can be organized into OTUs that provide an assay with a sensitivity and/or specificity of more than 80%, 85%, 90%, 91%, 92%, 93%, 94%, 95%, 96%, 97%, 98% or 99%. In some embodiments, sensitivity and specificity depends on the hybridization signal strength, number of probes in the OTU, the number of potential cross hybridization reactions, the signal strength of the mismatch probes, if present, background noise, or combinations thereof. In some embodiments, an OTU containing one probe may provide an assay with a sensitivity and specificity of at least 90%, while another OTU may require at least 20 probes to provide an assay with sensitivity and specificity of at least 90%.

Some embodiments relate to methods for phylogenetic analysis system design and signal processing and interpretation for use in detecting and identifying a plurality of biomolecules and organisms in a sample. More specifically, some embodiments relate to a method of selecting a set of organism-specific oligonucleotide probes for use in detecting a plurality of organisms in a sample with a high confidence level. Some embodiments relate to a method of selecting a set of OTU-specific oligonucleotide probes for use in detecting a plurality of organisms in a sample with a high confidence level.

In the case of highly conserved polynucleotides like 16S rRNA that may have only one to a few nucleotides of sequence variability over any 15- to 30-bp region targeted by probes for discrimination between related microbial species, it is advantageous to maximize the probe-target sequence specificity in an assay system. Some embodiments of the present invention provide methods of selecting organism-specific oligonucleotide probes that effectively minimize the influence of cross-hybridization. In one embodiment, the method comprises: (a) identifying sequences of a target nucleic acid molecule corresponding to the plurality of organisms; (b) determining the cross-hybridization pattern of a candidate organism-specific oligonucleotide probe to the target nucleic acid molecule from the plurality of organisms, wherein the candidate oligonucleotide probe corresponds to a sequence fragment of the target nucleic acid molecule from the plurality of organisms; (c) determining the cross-hybridization pattern of a variant of the candidate organism-specific oligonucleotide probe to the target nucleic acid molecule from the plurality of organisms, wherein the variant of the candidate organism-specific oligonucleotide probe comprises at least 1 nucleotide mismatch compared to the candidate organism-specific oligonucleotide probe ; and (d) selecting or rejecting the candidate organism-specific oligonucleotide probe on the basis of the cross-hybridization pattern of the candidate organism-specific oligonucleotide probe and the cross-hybridization pattern of the variant of the candidate organism-specific oligonucleotide probe. In some embodiments, a method of selecting a set of OTU-specific oligonucleotide probes for use in detecting a plurality of organisms in a sample is provided. In some embodiments, the method comprises: (a) identifying sequences of a target nucleic acid molecule corresponding to the plurality of organisms; (b) clustering the sequences of the target nucleic acid molecule from the plurality of organisms into one or more Operational Taxonomic Units (OTUs), wherein each OTU comprises one or more groups of similar sequences; (c) determining the cross-hybridization pattern of a candidate OTU-specific oligonucleotide probe to the OTUs, wherein the candidate OTU-specific oligonucleotide probe corresponds to a sequence fragment of the target nucleic acid molecule from one of the plurality of organisms; (d) determining the cross-hybridization pattern of a variant of the candidate OTU-specific oligonucleotide probe to the OTUs, wherein the variant of the candidate OTU-specific oligonucleotide probe comprises at least 1 nucleotide mismatch compared to the candidate OTU-specific oligonucleotide probe ; and (e) selecting or rejecting the candidate OTU-specific oligonucleotide probe on the basis of the cross-hybridization pattern of the candidate OTU-specific oligonucleotide probe to the OTUs and the cross-hybridization pattern of the variant of the candidate OTU-specific oligonucleotide probe to the OTUs. In some embodiments, candidate OTU-specific oligonucleotide probe are rejected when the candidate OTU-specific oligonucleotide probe or its variant are predicted to cross-hybridize with other target sequences. In some embodiments, a predetermined amount of predicted cross-hybridization is allowed.

In some embodiments, selected oligonucleotide probes are synthesized by any relevant method known in the art. Some examples of suitable methods include printing with fine-pointed pins onto glass slides, photolithography using pre-made masks, photolithography using dynamic micromirror devices, ink-jet printing, or electrochemistry. In one example, a photolithographic method can be used to directly synthesize the chosen oligonucleotide probes onto a surface. Suitable examples for the surface include glass, plastic, silicon and any other surface available in the art. In certain examples, the oligonucleotide probes can be synthesized on a glass surface at an approximate density from about 1,000 probes per $\mu m^2$ to about 100,000 probes per $\mu m^2$, preferably from about 2000 probes per $\mu m^2$ to about 50,000 probes per $\mu m^2$, more preferably from about 5000 probes per $\mu m^2$ to about 20,000 probes per $\mu m^2$. In one example, the density of the probes is about 10,000 probes per $\mu m^2$. The number of probes on the array can be quite large e.g., at least $10^5$, $10^6$, $10^7$, $10^8$ or $10^9$ probes per array. Usually, for large arrays only a relatively small proportion (i.e., less than about 1%, 0.1% 0.01%, 0.001%, 0.00001%, 0.000001% or 0.0000001 %) of the total number of probes of a given length target an individual OTU. Frequently, lower limit arrays have no more than 10, 25, 50, 100, 500, 1,000, 5,000, or 10,000, 25,000, 50,000, 100,000 or 250,000 probes.

Typically, the arrays or microparticles have probes to one or more highly conserved polynucleotides. The arrays or microparticles may have further probes (e.g. confirmatory probes) that hybridize to functionally expressed genes, thereby providing an alternate or confirmatory signal upon which to base the identification of a taxon. For example, an array may contain probes to 16S rRNA gene sequences from *Yersinia pestis* and *Vibrio cholerae* and also confirmatory probes to *Y. pestis* cafl virulence gene or *V. cholerae* zonula occludens toxin (zot) gene. The detection of hybridization signals based on probes binding to 16S rRNA polynucleotides associated with a particular OTU coupled with the detection of a hybridization signal based on a confirmatory probe can provide a higher level of confidence that the OTU is present. For instance, if hybridization signals are detected for the probes associated *Y. pestis* OTU and the confirmatory probe also displays a hybridization signal for the expression of *Y. pestis* cafl then the confidence level subscribed to the presence or quantity of *Y. pestis* will be higher than the confidence level obtained from the use of OTU probes alone.

A range of lengths of probes can be employed on the arrays or microparticles. As noted above, a probe may consist exclusively of a complementary segments, or may have one or more complementary segments juxtaposed by flanking, trailing and/or intervening segments. In the latter situation, the total length of complementary segment(s) can be more important that the length of the probe. In functional terms, the complementary segment(s) of the PM probes should be sufficiently long to allow the PM probes to hybridize more strongly to a target polynucleotide e.g., 16S rRNA, compared with a MM probe. A PM probe usually has a single complementary segment having a length of at least 15 nucleotides, and more usually at least 16, 17, 18, 19, 20, 21, 22, 23, 24, 25 or 30 bases exhibiting perfect complementarity.

In some arrays or lots of microparticles, all probes are the same length. In other arrays or lots of microparticles, probe length varies between quantification standard (QS) probes, negative control (NC) probes, probe pairs, probe sets (OTUs) and combinations thereof. For example, some arrays may have groups of OTUs that comprise probe pairs that are all 23 mers, together with other groups of OTUs or probe sets that comprise probe pairs that are all 25 mers. Additional groups of probes pairs of other lengths can be added. Thus, some arrays may contain probe pairs having sizes of 15 mers, 16mers, 17mers, 18mers, 19mers, 20mers, 21mers, 22mers, 23mers, 24mers, 25 mers, 26mers, 27 mers, 28mers, 29 mers, 30mers, 31mers, 32mers, 33mers, 34mers, 35mers, 36mers, 37mers, 38mers, 39mers, 40mers or combinations thereof. Other arrays may have different size probes within the same group, OTU, or probe set. In these arrays, the probes in a given OTU or probe set can vary in length independently of each other. Having different length probes can be used to equalize hybridization signals from probes depending on the hybridization stability of the oligonucleotide probe at the pH, temperature, and ionic conditions of the reaction.

In another aspect of the invention, a system is provided for determining the presence or quantity of a plurality of different OTUs in a single assay where the system comprises a plurality of polynucleotide interrogation probes, a plurality of polynucleotide positive control probes, and a plurality of polynucleotide negative control probes. In some embodiments, the system is capable of detecting the presence, absence, relative abundance, and/or quantity of at least 5, 10, 20, 50, 100, 250, 500, 1000, 5000, 10,000, 20,000, 30,000, 40,000, 50,000, 60,000, 70,000, 80,000, 90,000, 100,000, 250,000, 500,000 or 1,000,000 OTUs in a sample using a single assay. In some embodiments, the polynucleotide positive control probes include 1) probes that target sequences of prokaryotic or eukaryotic metabolic genes spiked into the target nucleic acid sequences in defined quantities prior to fragmentation, or 2) probes complimentary to a pre-labeled oligonucleotide added into the hybridization mix after fragmentation and labeling. The control added prior to fragmentation collectively tests the fragmentation, biotinylation, hybridization, staining and scanning efficiency of the system. It also allows the overall fluorescent intensity to be normalized across multiple analysis components used in a single or combined experiment, such as when two or more arrays are used in a single experiment or when data from two separate experiments is combined. The second control directly assays the hybridization, staining and scanning of the system. Both types of control can be used in a single experiment.

In some embodiments, the QS standards (positive controls) are PM probes. In other embodiments, the QS standards are PM and MM probe pairs. In further embodiments, the QS standards comprise a combination of PM and MM probe pairs and PM probes without corresponding MM probes. In another embodiment, the QS standards comprise at least one, two, three, four, five, six, seven, eight, nine, ten or more MM probes for each corresponding PM probe. In a further embodiment, the QS standards comprise at least one, two, three, four, five, six, seven, eight, nine, ten or more PM probes for each corresponding MM probe. A system can comprise at least 1 positive control probe for each 1, 10, 100, or 1000 different interrogation probes.

In some cases, the spiked-in oligonucleotides that are complementary to the positive control probes vary in G+C content, uracil content, concentration, or combinations thereof. In some embodiments, the G+C% ranges from about 30% to about 70%, about 35% to about 65% or about 40% to about 60%. QS standards can also be chosen based on the uracil incorporation frequency. The QS standards may incorporate uracil in a range from about 1 in 100 to about 60 in 100, about 4 in 100 to about 50 in 100, or about 10 in 100 to about 50 in 100. In some cases, the concentration of these added oligonucleotides will range over 1, 2, 3, 4, 5, 6, or 7 orders of magnitude. Concentration ranges of about $10^5$ to $10^{14}$, $10^6$ to $10^{13}$, $10^7$ to $10^{12}$, $10^7$ to $10^{11}$, $10^8$ to $10^{11}$, and $10^8$ to $10^{10}$ can be employed and generally feature a linear hybridization signal response across the range. In some embodiments, positive control probes for the conduction of the methods disclosed herein comprise polynucleotides that are complementary to the positive control sequences shown in Table 1. Other genes that can be used as targets for positive controls include genes encoding structural proteins, proteins that control growth, cell cycle or reproductive regulation, and house keeping genes. Additionally, synthetic genes based on highly conserved genes or other highly conserved polynucleotides can be added to the sample. Useful highly conserved genes from which synthetic genes can be designed include 16S rRNA genes, 18S rRNA genes, 23SrRNA genes. Exemplary control probes are provided as SEQ ID NOs:51-100.

Table 1 Positive Control Sequences

| Positive Control ID | Description |
|---|---|
| AFFX-BioB-5_at | *E. coli* biotin synthetase |
| AFFX-BioB-M_at | *E. coli* biotin synthetase |
| AFFX-BioC-5_at | *E. coli* bioC protein |
| AFFX-BioC-3_at | *E. coli* bioC protein |
| AFFX-BioDn-3_at | *E. coli* dethiobiotin synthetase |
| AFFX-CreX-5_at | Bacteriophage P1 cre recombinase protein |
| AFFX-DapX-5_at | *B. subtilis* dapB, dihydrodipicolinate reductase |
| AFFX-DapX-M_at | *B. subtilis* dapB, dihydrodipicolinate reductase |
| YFL039C | *Saccharomyces*, Gene for actin (Act 1p) protein |
| YER022W | *Saccharomyces*, RNA polymerase II mediator complex subunit (SRB4p) |
| YER 148 W | *Saccharomyces*, TATA-binding protein, general transcription factor (SPT15) |
| YEL002C | *Saccharomyces,* Beta subunit of the oligosaccharyl transferase (OST) glycoprotein complex (WBP1) |
| YEL024W | *Saccharomyces,* Ubiquinol-cytochrome-c reductase (RIP1) |
| Synthetic 16S rRNA controls | |

(continued)

| Positive Control ID | Description |
|---|---|
| SYNM neurolyt_st | Synthetic derivative of *Mycoplasma neurolyticum* 16S rRNA gene |
| SYNLc.oenos_st | Synthetic derivative of *Leuconostoc oenos* 16S rRNA gene |
| SYNCau.cres8_st | Synthetic derivative of *Caulobacter crescenius* 16S rRNA gene |
| SYNFer.nodosm_st | Synthetic derivative of *Fervidobacterium nodosum* 16S rRNA gene |
| SYNSap.grandi_st | Synthetic derivative of *Saprospira grandis* 16S rRNA gene |

In some embodiments, the negative controls comprise PM and MM probe pairs. In further embodiments, the negative controls comprise a combination of PM and MM probe pairs and PM probes without corresponding MM probes. In other embodiments, the negative control probes comprise at least one, two, three, four, five, six, seven, eight, nine, ten or more MM probes for each corresponding negative control PM probe. A system can comprise at least 1 negative control probe for each 1, 10, 100, or 1000 different interrogation probes (PMs).

Generally, the negative control probes hybridize weakly, if at all, to 16S rRNA gene or other highly conserved gene targets. The negative control probes can be complementary to metabolic genes of prokaryotic or eukaryotic origin. Generally, with negative control probes, no target material is spiked into the sample. In some embodiments, negative control probes are from the same collection of probes that are also used for positive controls, but no material complementary to the negative control probes are spiked into the sample, in contrast to the positive control probe methodology. In essence, the control probes are universal control probes and play the role of a positive or negative control probes depending on the system's design. One of skill in the art will appreciate that the universal control probes are not limited to highly conserved sequence analysis systems and have applications beyond the present embodiments disclosed herein.

In a further embodiment, probes to non-highly conserved polynucleotides are added to a system to provide species-specific identification or confirmation of results achieved with the probes to the highly conserved polynucleotides. Usually, these "confirmatory" probes cross hybridize very weakly, if at all, to highly conserved polynucleotides recognized by the perfect match probes. Useful species-specific genes include metabolic genes, genes encoding structural proteins, proteins that control growth, cell cycle or reproductive regulation, housekeeping genes or genes that encode virulence, toxins, or other pathogenic factors. In some embodiments, the system comprises at least 1, 5, 10, 20, 30, 40, 50 60, 70, 80, 90 100, 150, 200, 250, 300, 400, 500, 600, 700, 800, 900, 1000, 5,000 or 10,000 species- specific probes.

In some embodiments, a system of the invention comprises an array. Non-limiting examples of arrays include microarrays, bead arrays, through-hole arrays, well arrays, and other arrays known in the art suitable for use in hybridizing probes to targets. Arrays can be arranged in any appropriate configuration, such as, for example, a grid of rows and columns. Some areas of an array comprise the OTU detection probes whereas other areas can be used for image orientation, normalization controls, signal scaling, noise reduction processing, or other analyses. Control probes can be placed in any location in the array, including along the perimeter of the array, diagonally across the array, in alternating sections or randomly. In some embodiments, the control probes on the array comprise probe pairs of PM and MM probes. The number of control probes can vary, but typically the number of control probes on the array range from 1 to about 500,000. In some embodiments, at least 10, 100, 500, 1,000, 5,000, 10,000, 25,000, 50,000, 100,000, 250,000 or 500,000 control probes are present. When control probe pairs are used, the probe pairs will range from 1 to about 250,000 pairs. In some embodiments, at least 5, 50, 250, 500, 2,500, 5,000, 12,500, 25,000, 50,000, 125,000 or 250,000 control probe pairs are present. The arrays can have other components besides the probes, such as linkers attaching the probes to a support. In some embodiments, materials for fabricating the array can be obtained from Affymetrix (Santa Clara, California), GE Healthcare (Little Chalfont, Buckinghamshire, United Kingdom) or Agilent Technologies (Palo Alto, California.)

Besides arrays where probes are attached to the array substrate, numerous other technologies may be employed in the disclosed system for the practice of the methods of the invention. In one embodiment, the probes are attached to beads that are then placed on an array as disclosed by Ng et al. (Ng et al. A spatially addressable bead-based biosensor for simple and rapid DNA detection. Biosensors & Bioelectronics, 23:803-810, 2008).

In another embodiment, probes are attached to beads or microspheres, the hybridization reactions are performed in solution, and then the beads are analyzed by flow cytometry, as exemplified by the Luminex multiplexed assay system. In this analysis system, homogeneous bead subsets, each with beads that are tagged or labeled with a plurality of identical probes, are combined to produce a pooled bead set that is hybridized with a sample and then analyzed in real time with flow cytometry, as disclosed in US Patent 6,524,793. Bead subsets can be distinguished from each other by variations in the tags or labels, e.g., using variable in laser excitable dye content.

In a further embodiment, probes are attached to cylindrical glass microbeads as exemplified by the Illumina Veracode multiplexed assay system. Here, subsets of microbeads embedded with identical digital holographic elements are used to create unique subsets of probe-labeled microbeads. After hybridization, the microbeads are excited by laser light and the microbead code and probe label are read in real time multiplex assay.

In another embodiment, a solution based assay system is employed as exemplified by the NanoString nCounter Analysis System (Geiss G et al. Direct multiplexed measurement of gene expression with color-coded probe pairs. Nature Biotech. 26:317-325, 2008). With this methodology, a sample is mixed with a solution of reporter probes that recognize unique sequences and capture probes that allow the complexes formed between the nucleic acids in the sample and the reporter probes to be immobilized on a solid surface for data collection. Each reporter probe is color-coded and is detected through fluorescence.

In a further embodiment, branched DNA technology, as exemplified by Panomics QuantiGene Plex 2.0 assay system, is used. Branched DNA technology comprises a sandwich nucleic acid hybridization assay for RNA detection and quantification that amplifies the reporter signal rather than the sequence. By measuring the RNA at the sample source, the assay avoids variations or errors inherent to extraction and amplification of target polynucleotides. The QuantiGene Plex technology can be combined with multiplex bead based assay system such as the Luminex system described above to enable simultaneous quantification of multiple RNA targets directly from whole cells or purified RNA preparations.

**Probes and the Selection Thereof**

An exemplary process **300** for the design of target probes for use in the simultaneous detection of a plurality of microorganisms is illustrated in Fig. 3. Briefly, sequences are extracted from a database at a state **301.** Typically, the database contains phylogenetic sequences or other highly conserved or homologous sequences. The sequences are analyzed for chimeras at a state **302** that are removed from further consideration. Chimeric sequences result from the union of two or more unrelated sequences, typically from different genes. Optionally, sequences can be further analyzed for structural anomalies, such as propensity for hairpin loop formation, at a state **303** with the identified sequences subsequently removed from further consideration. Next, multiple sequence alignments are performed on the remaining sequences in the dataset at a state **304.** The aligned sequences are then checked for laboratory artifacts, such as PCR primer sequences, at a state **305,** with identified sequences removed from further consideration. The remaining sequences are clustered at a state **306** and perfect match (PM) probes are selected at a state **307** that have perfect complementarity to sections of the clustered sequences. Optionally, sequence coverage heuristics are performed at a state **308** prior to selecting the mismatch (MM) probes at a state **309** for the corresponding PM probes to create probe pairs. Finally, OTUs represented by probe sets comprising a plurality of probe pairs are assembled at a state **310** to construct a hierarchal taxonomy.

Generally, a database for extraction of sequences to be used for probe selection is chosen based on the particular conserved gene or highly homologous sequence of interest, the total number of sequences within the database, the length of the overall sequences or the length of highly conserved regions within the sequences listed in the database, and the quality of the sequences therein. Typically, between two databases of equal sequence number but of different sequence length, the database with longer target regions of highly conserved sequence will generally contain a larger total number of possible sequences that can be compared. In some embodiments, the sequences are at least 300, 400, 500, 600, 700, 800, 900, 1,000, 1,200, 1,400, 1,600, 1,800, 2,000, 4,000, 8,000, 16,000 or 24,000 nucleotides long. Generally, databases with larger number of total sequences provide more material to compare. In a further embodiment, the database contains at least 10,000, 20,000, 30,000, 40,000, 50,000, 60,000, 70,000, 80,000, 100,000, 200,000, 500,000, 1,000,000 or 2,000,000 sequence listings. A gene of particular interest for probe construction is 16S rDNA (16S rRNA gene). Other conserved genes include 18S rDNA, 23S rDNA, *gyrA, gyrB* gene, *groEL, rpoB gene, fusA* gene, *recA* gene, *sodA, cox1 gene,* and nifD gene. In a further embodiment, the spacer region between highly conserved segments of two genes can be used. For example, the spacer region between 16S and 23S rDNA genes can be used in conjunction with conserved sections of the 16S and 23S rDNA.

In some embodiments, the detection of a biosignature comprises the use of probes designed to hybridize with known or discovered targets within one or more OTUs. In some embodiments, targets are selected from a collection of known targets, such as in a database. In some embodiments of the invention, a database used for the selection of probes comprises at least 50%, 60%, 70%, 80%, 90%, 95%, 96%, 97%, 98%, 99% or up to 100% of the known sequences of the organisms of interest, e.g., of the bacteria, archaea, fungi, eukaryotes, microorganisms, or prokaryotes of interest. The sequences for each individual organism in the database can include more than 20%, 30%, 40%, 50%, 60%, 70%, 80%, 90%, or more than 95% of the genome of the organism, or of the non-redundant regions thereof. In some embodiments, the database includes up to 100% of the genome of the organisms whose sequenced are contained therein, or of the non-redundant sequences thereof. A listing of almost 40,000 aligned 16S rDNA sequences greater than 1250 nucleotides in length can be found on the Greengenes web application, a publicly accessible database run by Lawrence Berkeley National Laboratory. Other publicly accessible databases include GenBank, Michigan State University's ribosomal database project, the Max Planck Institute for Marine Microbiology's Silva database, and the National Institute of Health's NCBI. Proprietary sequence databases or combinations created by amalgamating the contents of two or more private and/or public databases can also be used to practice the methods of this invention. In some embodiments, a sample is assayed for all targets in one or more chosen databases simultaneously. In other embodiments, a sample is assayed for subsets of targets identified in one or more databases simultaneously. In some embodiments, a biosignature comprises the results of assaying a sample for some or all targets in one or more chosen databases. In other embodiments, a biosignature comprises a subset of the results of assaying a sample for some or all targets in one or more chosen databases.

The analysis of the selected sequences from the database for the detection and removal of chimeras at state **302** is typically performed by generating overlapping fragments and comparing these fragments against each other. Fragments may be retained if they have at least 60%, 70%, 80%, 90%, 95% or 99% sequence identity. It was realized that the above process potentially missed chimeras because the sequence diversity of the selected sequences may be low. By comparing the fragments against a core set of diverse chimera-free sequences, more chimeras can be identified and removed from the sequence set. In cases where one or more sequences are identified that as an ambiguous chimera, e.g., a chimera with a chimeric parent, the chimera is removed and the parent chimera is fragmented and a second comparison cycle is performed. Sequences from a dataset can also be screened for chimeras using a proprietary software program such as Bellerophon3 available from the Greengenes website at greengenes.lbl.gov.

The dataset of retained non-chimeric sequences can then be screened for structural anomalies at state **303** by aligning the retained sequences against the core set of known sequences. Sequences in the retained dataset that have at least 25, 30, 35, 40, 45, 50, 60, 70 or 80 gaps in their alignment when compared against a core set or have insertions of greater than 50, 60, 70, 80, 90, 100, 110, 120, 130, 140, 150, 160, 170, 180, 190, 200, 250, 300 or 400 basepairs when compared against the core set are tagged as having a sequence anomaly and are removed from the dataset.

The screened sequences are then aligned into a multiple sequence alignment (MSA) at state **304** for comparison against the known, chimeric free core set. One alignment tool for performing intensive alignment computations is NAST (Nearest Alignment Space Termination) web tool (DeSantis et al., Nucleic Acids Res. (2006) 34:W394-399). Any appropriate alignment tool can be used to compile the MSAs, for example, clustalw (Thompson et al., Nucleic Acids Res (1994) 22:4673-4680) and MUSCLE (Edgar, Nucleic Acids Res. (2004) 32:1792-1797).

The aligned sequences are searched for sequences harboring PCR primer sequences at state **305** and any so-identified sequences are removed from the dataset.

The aligned sequences can then be clustered at the state **306** to create what is termed a "guide tree." First, the sequences are converted to a list of kmers. A pair-wise comparison of the lists of kmers is performed and the percent of kmers in common is recorded in a sparse matrix only if a threshold similarity is found. The sparse matrix is clustered e.g., using complete linkage. Clustering includes agglomerative "bottom-up" or divisive "top-down" hierarchical clustering, distance "partition" clustering and alignment clustering. From each cluster, the sequence with the most information content is chosen as a representative. Usually, sequences derived from genome sequencing projects are given priority in cluster creation because they are less likely to be chimeras or have other sequence anomalies. The cyclic process is repeated using only the representatives from the previous cycle. For each new cycle, the threshold for recording in the sparse matrix is reduced. At the final stage, a root node is linked to the final representative sequences in a multifurcated tree. The representative sequences found in each cycle represent a node in the resulting guide tree. All nodes are linked based on their clustering results via a self-referential table allowing rapid access to any hierarchical point in the guide tree. In some embodiments, the results are stored in a database format, e.g., in a Structured Query Language (SQL) compliant format. In the resulting guide tree, each leaf node represents an individual organism and each node above the lowest level of the guide tree represents a candidate OTU.

Typical distance matrixes built from approximately 2 x $10^5$ sequences can require 40 billion intersections that would require about 40 gigabytes of data space if encoded to disk. Doubling the amount of sequences to 4 x $10^5$ requires a quadrupling of the file size (approximately 160GB). The clustering methodology illustrated here using a sparse matrix avoids the need for large files and the expected increase in computing time. Therefore the methodology can be performed more efficiently than conventional sequence clustering methods. Moreover, with distance matrices created from sequence alignments (e.g., DNA alignments), one misalignment can affect many distance values. In contrast, the clustering method illustrated herein is based on the alignment of kmers, and thus the effect of a misalignment on clustering values is significantly reduced.

Following guide tree construction, the dataset of remaining sequences, now termed the "filtered sequence dataset" is used to select candidate probes, e.g., PM probes. First, unsupported sequence polymorphisms are identified and removed from the filtered sequence dataset using a pre-clustering process that uses the guide tree generated above to create clusters over a minimum similarity and under a maximum size. Typically, clustered sequences are at least 80%, 85%, 90%, 95%, 97% or 99% similar. Usually, clusters have no more than 1,000, 500, 200, 100, 80, 60, 50, 40, 30, 20 or 10 sequences. This process allows sequence data outliers to be detected by comparison within near-neighbors and removed from the filtered sequence dataset.

Next, the remaining sequences are fragmented to the desired size to generate candidate target probes. Typically, the fragments range from about 10mer to 100mer, 15mer to about 50mer, about 20mer to about 40mer, about 20mer to about 30mer. Usually, the fragments are at least 15mer, 20mer, 25mer, 30mer, 40mer, 50mer or 100mer in size. Each candidate target probe is required to be found within a threshold fraction of at least one pre-cluster. Generally, threshold fractions of at least 80%, 90% or 95% are used.

All candidate PM probes that are within a threshold fraction of at least one pre-cluster are then evaluated for various biophysical parameters, such as melting temperature (61-80° C), G+C content (35-70%), hairpin energy over -4 kcal/mol, potential for self-dimerization (> 35° C). Candidate PM probes that fall outside of the setting boundaries of the biophysical parameters are eliminated from the dataset. Optionally, probes can be further filtered for ease of photolithographic synthesis.

The likelihood of cross-hybridization of each PM candidate probe to each non-target input 16s rRNA gene sequence is determined. The cross-hybridization pattern for each PM candidate probe is recorded.

Sequence coverage heuristics are performed at the state **308** are then applied to candidate PM probes with acceptable biophysical parameters.

For each candidate PM probe, corresponding MM probes can be generated at the state **309.** Each MM probe differs from its corresponding PM probe by at least one nucleotide. In some embodiments, the MM probe differs from its corresponding PM probe by 1, 2, 3, 4, 5, 6, 7, 8, 9 or 10 nucleotides. Within a MM probe, the mismatched nucleotide or nucleotides can include any of the 3 central bases that are not found in the same position or positions in the PM probe. For example, with a 25mer PM probe that has a guanine at the $13^{th}$ position, i.e., the central nucleotide, the MM probes comprise probes with adenine, thymine, uracil or cytosine at the $13^{th}$ position. Similarly, with a 25mer PM probe with an adenine at the $12^{th}$ nucleotide position and a guanine at the $13^{th}$ nucleotide position when read from the 3' direction, the possible MM probes comprise probes with guanine at the $12^{th}$ nucleotide and adenine, thymine or cytosine at the $13^{th}$ nucleotide position; cytosine at the $12^{th}$ nucleotide position and adenine, thymine or cytosine at the $13^{th}$ nucleotide position; and thymine at the $12^{th}$ nucleotide position and adenine, thymine or cytosine at the $13^{th}$ nucleotide position. In some embodiments, the mismatched nucleotide or nucleotides include any one or more of the nucleotides in a corresponding PM probe. Increasing the number of MM probes and/or the mis-match positions represented may be used to enhance quantification, accuracy, and confidence.

As describe above for the PM probes, each candidate MM probe is required to meet the set boundaries of one or more biophysical parameters, such as melting temperature, G+C content, hairpin energy, self-dimers and photolithography synthesis steps. Generally, these parameters are identical or substantially similar to the PM probe biophysical parameters.

Candidate MM probes that meet the biophysical parameters and optionally, photolithographic parameters above are then screened for the likelihood of cross-hybridization to a target sequence. Usually, a central kmer length is evaluated. For a 25mer candidate MM, a central kmer from the candidate MM, generally a 15mer, 16mer, 17mer, 18mer, or 19mer is compared against the target sequences. A candidate MM probe that contains a central kmer that is identical to a target sequence is eliminated. Next, candidate PM probes for which no suitable candidate MM probes can be identified are also eliminated.

Each candidate OTU may be evaluated to determine the number of PM probes that are incapable of hybridization to sequences outside the OTU.

In one embodiment, a pre-partition process is performed. A pre-partition is the largest possible clade (node_id) that does not exceed the max partition size. *See* Figure 6. Typically, useful partition sizes range from about 1,000 to about 8,000 nodes. Any pre-partition that is in a predetermined size range becomes a full-partition. Pre-partitions that are below the minimum partition size are combined into partitions by assembling sister nodes where possible. For example, assume that partitions are allowed to range in size from 1000 to 2000 members. If node A represents 1500 genes and its parent, node B, represents 2500 genes, then node A is considered a pre-partition. If node C is a sibling of node A, and node C represents only 50 genes, then node C is also a pre-partition because moving node C to its parent, node B, would encapsulate more than the maximum partition size of 2000 members.

To create candidate sequence clusters, transitive sequence clusters are identified using a sliding threshold of two distance matrixes based on either the count of pairwise unique candidate targets or the count of pairwise common candidate targets. Probes prevalent in a large fraction of the sequences in a candidate sequence cluster, e.g., >= 90% of the sequence in the cluster, are identified using the count of sequences containing the PM and the count of sequences with unambiguous data for given PM's locus. For each prevalent probe, a cross-hybridization potential outside the cluster is also tested. All information regarding cluster-PM sets is recorded. Futile clusters are defined as clusters for which only cross-hybridizing probes are identified are removed from the dataset.

Where necessary, probes that are expected to display some degree of cross-hybridization can be selected. Potentially hybridization-prone probes are constrained to reduce the probability that sequences outside the cluster could hybridize to many of the cluster-specific PM probes. A distribution algorithm can be used to examine a graph of probe-sequence interconnections (edges) and to favor sets of probes that minimize overlapping edges.

After solutions from all partitions are completed, a global reconciliation of set solutions across partitions is performed. The sequence clusters are locked as OTUs and each cluster's PM probe set is tested for global cross-hybridization against the other remaining PM probe sets. Probes are ranked for utility based on global cross-hybridization patterns.

The OTUs are assembled and annotated. Typically, each OTU is taxonomically annotated using one term for each rank from domain, kingdom, phylum, sub-phylum, class, sub-class, order, and family. As a result, all the 16S rRNA sequences presented without taxonomic nomenclature and annotated as "environmental samples" or "unclassified" are assigned with taxonomic annotation.

Each genus-level name recognized by NCBI is read and recorded. For each lineage of taxonomic terms, duplicate adjacent terms are removed; domain-level terms are found by direct pattern match; and phylum-level terms are found as rank immediately subordinate to domain. Order-level terms are found by -ales suffix and family-level terms are found by -eae suffix. If a family level-term is unavailable but a genus is identified (e.g., by match to an accepted list), the genus-level term is used to derive a family level-term. All unrecognized terms found between recognized terms are fit into available ranks (new ranks are not created for extra terms). Empty ranks are filled by deriving root terms from subordinate terms and adding pre-determined suffixes. Finally, the family of an OTU is determined by vote from the family assignment of the sequences. Ties are broken by priority sequences (e.g., sequences derived from genome sequencing projects can be given highest priority). All OTUs within a subfamily are compared by kmer distance among the sequences and OTUs are linked into a subfamily whenever a threshold similarity is observed. Each candidate OTU is evaluated to determine the count of targets which are prevalent across the sequences of the candidate OTU and are not expected to hybridize to sequences outside the OTU.

Exemplary PM and MM 25mer probes generated using the disclosed algorithms are provided as SEQ ID Nos. 1-50. It should be noted that the above process is applicable to the selection of probes ranging in size from at least 15 nucleotides to at least 200 nucleotides in length and includes probes that are flanked on one or both sides by common or irrelevant sequences, including linking sequences. Furthermore, probes selected by this process can be further processed to yield probes that are smaller than or larger than the original selected probes. For example, probes listed as SEQ ID Nos. 1-50 can be further processed by removing sequences from the 3'end, 5'end or both to produce smaller sequences that are identical to at least a portion of the sequence of the 25mers. In other embodiments, larger probes can be generated by incorporating the sequences of probes identified by the disclosed algorithms, i.e., a 25mer probe can be incorporated into a 30mer or larger, 35mer or larger, 40mer or larger, 45mer or larger, 50mer or larger, 55mer or larger, 60mer or larger, 65mer or larger, 70mer or larger, 75mer or larger, 80mer or larger, 85mer or larger or 90mer or larger probe. Additionally, probes listed as SEQ ID Nos. 1-50 can be shortened on one end and lengthened on the other end to yield probes that range from 10mer to 200mer.

Probes selected by the above process also include probes that comprise one or more base substitutions, for example uracil in the place of thymine; incorporate one or more base analogs such as nitropyrrole and nitroindole; comprise of one or more sugar substitutions, e.g., ribose in the place of deoxyribose, or any combination thereof. Similarly, probes selected by the process of the invention, may further comprise alternate backbone chemistry, for example, comprising of phosphoramide.

The size of the collection of putative probes generated by the methodologies of the invention is partially dependent on the length of the particular highly conserved sequence with longer sequences like that of 23S rRNA gene allowing for a greater number of homologous sequences than a smaller highly conserved sequence such as 16S rRNA gene. In some embodiments, the length of the highly conserved sequence is at least 100 bp, 250 bp, 500 bp, 1,000 bp, 2,000 bp, 4,000 bp, 8,000 bp, 10,000 bp, or 20,000 bp. Additionally, the size of the collection of putative probes generated by the methodologies of the invention is also dependent on the size of the collection of homologous sequences in one or more databases from which sequences are selected for the analysis and generation of probes. Larger collections of homologous sequences, by providing a larger pool of sequences that can be analyzed, allow for the generation of more putative probes. In some embodiments, the starting collection of homologous sequences in one or more databases contains at least 100,000, 250,000, 500,000, 1,000,000, 2,000,000, 5,000,000 or 10,000,000 sequences. The size of the collection of putative probes is further dependent on the length of the desired probe, because the probe length decreases, as the number of probes that bind to unique sequences increases. Depending on the particular highly conserved sequence, the size of the database and the length of the desired probe, collections of putative probes of at least 100, 1,000, 10,000, 25,000, 50,000, 100,000, 250,000, 500,000, 1,000,000, 2,000,000, 5,000,000 or 10,000,000 probes can be generated.

Detection systems can be constructed from the putative probes generated by the above methods. The detection system can have any number of probes and range from 1 probe to all the probes selected by the methodology. In some embodiments, the detection system comprises at least 1, 2, 3, 4, 5, 6, 7, 8, 9, 10, 15, 20, 25, 30, 35, 36, 40, 45, 50, 55, 60, 65, 70, 80, 90, 100, 125, 150, 200, 300, 400, 500, 1000, 2,000, 5,000, 10,000, 20,000, 40,000, 50,000, 100,000, 200,000, 500,000, 1,000,000 or 2,000,000 probes. Systems with large number of probes can be used to identify relevant microorganisms in a sample, e.g., an environment or clinical sample, and/or to generate a biosignature. In another embodiment, once relevant microorganisms are known, detection systems with low (e.g., 1-10,000) to medium (e.g., 10,000-100,000) numbers of probes can be designed for special purpose applications, such as determining one or more specific biosignatures. In some embodiments, knowledge of the identity of relevant microorganisms can be used to select further probes to these microorganisms. If, for instance, five 25mer probes in a first set of probes hybridize to a relevant microorganism, then variants of these five probes can be generated and tested (e.g. *in silico*) for their binding and biophysical characteristics. Alternately, identification of relevant microorganisms can lead to the generation of new probes that are unlike the probes first used to identify the microorganisms. For example, once novel microorganisms are identified, antibodies can be generated for specific applications.

To select OTU-specific probes, e.g., oligonucleotide probes specific for organisms that are included within a hierarchical node, additional PM probes can be chosen for each hierarchical node that has more than one child node. To qualify targets for selection to a certain node, a threshold fraction of sequences within a node matching a PM set are enforced. Examples of the threshold fractions included 0.2%, 0.5%, 1%, 2%, 3%, 4%, 5%, 6%, 7%, 8%, 9%, and 10%. Coverage of direct sub-nodes (children) is also enforced. For example, each target should be representative of at least 25% of at least one sub-node.

The specificity of the probes selected by the methods disclosed herein can be validated experimentally in a number of ways. For example, the hybridization signal of a probe in the presence of the target sequence can be measured and compared to the background signal. Target sequences can be derived from one or more pure cultures or from environmental or clinical samples that are known to contain the target sequence. A specific taxa can be identified as present in a sample if a majority (about 70% to about 100%, about 80% to about 100% or about 90% to about 100%) of the probes on the array have a hybridization signal at least about 50 times, 100 times, 150 times, 200 times, 250 times, 300 times, 350 times, 400 times, 450 times, 500 times, or 1,000 times greater than that of the background. Also, the hybridization signal of the probe can be compared to the hybridization signal of one or more of its mismatch probes. A PM:MM ratio of at least 1.05, 1.10, 1.15, 1.20, 1.25, 1.30, 1.40, 1.45, or 1.50 can indicate that the PM probe can selectively hybridize to its target sequence. An additional way to test the ability of a probe to selectively hybridize to its target is to calculate a pair difference score (d), further explained below. A pair difference score above 1.0 indicates that the probe can selectively hybridize to the target compared to one of its mismatch probes.

The methods disclosed herein can be used to select and/or utilize organism-specific and/or OTU-specific oligonucleotide probes for biomolecules, such as proteins, DNA, RNA, DNA or RNA amplicons, and native rRNA from a target nucleic acid molecule. In some embodiments, probes are designed to be antisense to the native rRNA so that rRNA from samples can be placed on the array to identify actively metabolizing organisms in a sample with no bias from PCR amplification. Actively metabolizing organisms have significantly higher numbers of ribosomes used for the production of proteins, compared to quiescent or dead organisms. Therefore, in some embodiments, the capacity of one or more organisms to make proteins at a particular point in time can be measured. In this way, the array system of the present embodiments can be used to directly identify the metabolizing organisms within diverse communities.

**Sample Preparation**

In some embodiments, the sample used can be an environmental sample from any source, for example, naturally occurring or artificial atmosphere, water systems and sources, soil or any other sample of interest. In some embodiments, the environmental sample may be obtained from, for example, indoor or outdoor air or atmospheric particle collection systems; indoor surfaces and surfaces of machines, devices or instruments. In some embodiments, ecosystems are sampled. Ecosystems can be terrestrial and include all known terrestrial environments including, but not limited to soil, surface and above surface environments. Ecosystems include those classified in the Land Cover Classification System (LCCS) of the Food and Agriculture Organization and the Forest-Range Environmental Study Ecosystems (FRES) developed by the United States Forest Service. Exemplary ecosystems include forests such as tropical rainforests, temperate rainforest, temperate hardwood forests, boreal forests, taiga and montane coniferous forests; grasslands including savannas and steppes; deserts; wetlands including marshes, swamps, bogs, estuaries, and sloughs; riparian ecosystems, alpine and tundra ecosystems. Ecosystems further include those associated with aquatic environments such as lakes, streams, springs, coral reefs, beaches, estuaries, sea mounts, trenches, and intertidal zones. Ecosystems also comprise soils, humus, mineral soils and aquifiers. Ecosystems further encompass underground environments, such as mines, oil fields, caves, faults and fracture zones, geothermal zones and aquifers. Ecosystems additionally include the microbiomes associated with plants, animals, and humans. Exemplary plant associated microbiomes include those found in or near roots, bark, trunks, leaves, and flowers. Animal and human associated microbiomes include those found in the gastrointestinal tract, respiratory system, nares, urogenital tract, mammary glands, oral cavity, auditory canal, feces, urine, and skin.

In other embodiments, the sample can be any kind of clinical or medical sample. For example, samples from blood, urine, feces, nares, the lungs or the gut of mammals may be assayed using the array system. Also, the probes selected by the methods disclosed herein and the array system of the present embodiments can be used to identify an infection in the blood of an animal. The probes selected by the methods disclosed herein and the array system of the present embodiments can also be used to assay medical samples that are directly or indirectly exposed to the outside of the body, such as the lungs, ear, nose, throat, the entirety of the digestive system or the skin of an animal. Hospitals currently lack the resources to identify the complex microbial communities that reside in these areas.

Techniques and systems to obtain genetic sequences from multiple organisms in a sample, such as an environmental or clinical sample, are well known by persons skilled in the art. For example, Zhou et al. (Appl. Environ. Microbiol. (1996) 62:316-322) provides a robust nucleic acid extraction and purification. This protocol may also be modified depending on the experimental goals and environmental sample type, such as soils, sediments, and groundwater. Many commercially available DNA extraction and purification kits can also be used. Samples with lower than 2 pg purified DNA may require amplification, which can be performed using conventional techniques known in the art, such as a whole community genome amplification (WCGA) method (Wu et al., Appl. Environ. Microbiol. (2006) 72, 4931-4941). In some embodiments, highly conserved sequences such as those found in the 16S RNA gene, 23S RNA gene, 5S RNA gene, 5.8S rRNA gene, 12S rRNA gene, 18S rRNA gene, 28S rRNA gene, gyrB gene, rpoB gene, fusA gene, recA gene, coxl gene and nifD gene are amplified. Usually, amplification is performed using PCR, but other types of nucleic acid amplification can be employed. Generally, amplification is performed using a single pair of universal primers specific to a highly conserved sequence. For redundancy or for increased amount of total amplicon concentration, two or more universal probe pairs each specific to a different highly conserved sequence can be used. Representative PCR primers include: bacterial primers 27F and 1492R.

Techniques and systems for obtaining purified RNA from environmental samples are also well known by persons skilled in the art. For example, the approach described by Hurt et al. (Appl. Environ. Microbiol. (2001) 67:4495-4503) can be used. This method can isolate DNA and RNA simultaneously within the same sample. A gel electrophoresis method can also be used to isolate community RNA (McGrath et al., J. Microbiol. Methods (2008) 75:172-176). Samples with lower than 5 pg purified RNA may require amplification, which can be performed using conventional techniques known in the art, such as a whole community RNA amplification approach (WCRA) (Gao et al., Appl. Environ. Microbiol. (2007) 73:563-571) to obtain cDNA. In some embodiments, environmental sampling and DNA extraction are conducted as previously described (DeSantis et al., Microbial Ecology, 53(3):371-383, 2007). In other embodiments, 16S rRNA or 23S rRNA is directly labeled and used without any amplification.

## Probe Preparation

Techniques and means for generating oligonucleotide probes to be used on analysis systems, beads or in other systems are well-known by persons skilled in the art. For example, the oligonucleotide probes can be generated by synthesis of synthetic polynucleotides or oligonucleotides, e.g., using N-phosphonate or phosphoramidite chemistries (Froehler et al., Nucleic Acid Res. 14:5399-5407 (1986); McBride et al., Tetrahedron Lett. 24:246-248 (1983)). Synthetic sequences are typically between about 10 and about 500 bases in length, more typically between about 15 and about 100 bases, and most preferably between about 20 and about 40 bases in length. In some embodiments, synthetic nucleic acids include non-natural bases, such as, but by no means limited to, inosine. An example of a suitable nucleic acid analogue is peptide nucleic acid (see, e.g., Egholm et al., Nature 363:566-568 (1993); U.S. Pat. No. 5,539,083). In some embodiments, at least 10, 25, 50, 100, 500, 1,000, 5,000, 10,000, 20,000, 40,000, 50,000, 60,000, 70,000, 80,000, 90,000 100,000, 200,000, 500,000, 1,000,000 or 2,000,000 probes are included on the array. In further embodiments, each PM probe has a corresponding MM probe present on the array. Typically, each probe pair is associated with an OTU. In some embodiments, at least 10, 25, 50, 100, 500, 1,000, 5,000, 10,000, 20,000, 40,000, 50,000, 60,000, 70,000, 80,000, 90,000 100,000, 200,000 or 500,000 probe pairs are placed on the array. Generally, sets of probe pairs have at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 probe pairs present.

In some embodiments, positive control probes that are complementary to particular sequences in the target sequences (e.g., 16S rRNA gene) are used as internal quantification standards (QS) and included in the system. In other embodiments, positive control probes, also known as internal DNA quantification standards (QS) probes are probes that hybridize to spiked-in nucleic acid sequence targets. Usually, the sequences are from metabolic genes. In some embodiments, negative control (NC) probes, e.g., probes that are not complementary or do not appreciably hybridize to sequences in the target sequences (e.g., 16S rRNA gene) are included on the array. Unlike the QS probes, no target material is spiked into the sample mix for the NC probes, prior to sample processing.

## Hybridization Platform Fabrication

In some embodiments, the probes are synthesized separately and then attached to a solid support or surface, which may be made, e.g., from glass, latex, plastic (e.g., polypropylene, nylon, polystyrene), polyacrylamide, nitrocellulose, gel, silicon, or other porous or nonporous material. In some embodiments, the surface is spherical or cylindrical as in the case of microbeads or rods. In other embodiments, the surface is planar, as in an array or microarray. For example, the method described generally by Schena et al, Science 270:467¬470 (1995) can be used for attaching the nucleic acids to a surface by printing on glass plates. In other embodiments, typically used for making high-density oligonucleotide arrays, thousands of oligonucleotides complementary to defined sequences are synthesized in situ at defined locations on a surface by photolithographic techniques (see e.g., Fodor et al., 1991, Science 251:767-773; Pease et al., 1994, Proc. Natl. Acad. Sci. U.S.A. 91:5022-5026; Lockhart et al., 1996, Nature Biotechnology 14:1675; U.S. Pat. Nos. 5,578,832; 5,556,752; and 5,510,270) or other methods for rapid synthesis and deposition of defined oligonucleotides (e.g., Blanchard et al., Biosensors & Bioelectronics 11:687-690). In some of these methods, oligonucleotides (e.g., 25-mers) of known sequence are synthesized directly on a surface such as a derivatized glass slide. Other methods for making analysis systems are also available, e.g., by masking (Maskos and Southern, 1992, Nuc. Acids. Res. 20:1679¬1684). Embodiments of the present invention are applicable to any type of array, for example, bead-based arrays, arrays on glass plates or derivatized glass slides as discussed above, and dot blots on nylon hybridization membranes.

Embodiments of the invention are applicable for use in any analysis system, including but not limited to bead or solution multiplex reaction platforms, or across multiple platforms, for example, Affymetrix GeneChip® Arrays, Illumina BeadChip® Arrays, Luminex xMAP® Technology, Agilent Two-Channel Arrays, MAGIChips (Analysis systems of Gel-immobilized Compounds) or the NanoString nCounter Analysis System. The Affymetrix (Santa Clara, CA, USA) platform DNA arrays can have the oligonucleotide probes (approximately 25mer) synthesized directly on the glass surface by a photolithography method at an approximate density of 10,000 molecules per $\mu m^2$ (Chee et al., Science (1996) 274:610-614). Spotted DNA arrays use oligonucleotides that are synthesized individually at a predefined concentration and are applied to a chemically activated glass surface. In general, oligonucleotide lengths can range from a few nucleotides to hundreds of bases in length, but are typically from about 10mer to 50mer, about 15mer to 40mer, or about 20mer to about 30mer in length.

### Microparticle Systems

Oligonucleotides produced using techniques known in the art can be built on and/or coupled to microspheres, beads, microbeads, rods, or other microscopic particles for use in arrays, flow cytometry and other multiplex assay systems. Numerous microparticles are commercially available from about 0.01 to 100 micrometers in diameter. Generally, microparticles from about 0.1-50 μm, about 1-20 μm, or about 3-10 μm are preferred. The size and shapes of microparticles can be uniform or they can vary. In some embodiments, sublots of different sizes, shapes or both are conjugated to probes before combining the sublots to make a final mixed lot of labeled microparticles. The individual sublots can therefore be distinguished and classified based on their size and shape. The size of the microparticles can be measured in practically any flow cytometry apparatus by so-called forward or small-angle scatter light. The shape of the particle can be also discriminated by flow cytometry, e.g., by high-resolution slit-scanning method.

Microparticles can be made out of any solid or semisolid material including glass, glass composites, metals, ceramics, or polymers. Frequently, the microparticles are polystyrene or latex material, but any type of polymeric material is acceptable including but not limited to brominated polystyrene, polyacrylic acid, polyacrylonitrile, polyacrylamide, polyacrolein, polybutadiene, polydimethylsiloxane, polyisoprene, polyurethane, polyvinylacetate, polyvinylchloride, polyvinylpyridine, polyvinylbenzylchloride, polyvinyltoluene, polyvinylidene chloride, polydivinylbenzene, polymethylmethacrylate, or combinations thereof. Microparticles, can be magnetic or non-magnetic and may also have a fluorescent dye, quantum dot, or other indicator material incorporated into the microparticle structure or attached to the surface of the microparticles. Frequently, microparticles may also contain 1 to 30% of a cross-linking agent, such as divinyl benzene, ethylene glycol dimethacrylate, trimethylol propane trimethacrylate, or N,N'methylene-bis-acrylamide or other functionally equivalent agents known in the art.

### Target Labeling

In one embodiment, the nucleic acid targets are labeled so that a laser scanner tuned to a specific wavelength of light can measure the number of fluorescent molecules that hybridized to a specific DNA probe. For arrays, the nucleic acid targets are typically fragmented to between 15 and 100 nucleotides in length and a biotinylated nucleotide is added to the end of the fragment by terminal DNA transferase. At a later stage, the biotinylated fragments that hybridize to the oligonucleotide probes are used as a substrate for the addition of multiple phycoerythrin fluorophores by a sandwich (Streptavidin) method. For some arrays, such as those made by AGILENT or NIMBLEGEN, the purified community DNA can be fluorescently labeled by random priming using the Klenow fragment of DNA polymerase and more than one fluorescent moiety can be used (e.g. controls could be labeled with Cy3, and experimental samples labeled with Cy5 for direct comparison by hybridization to a single analysis system). Some labeling methods incorporate the molecular label into the target during an amplification or enzymatic step to produce multiple labeled copies of the target.

In some embodiments, the detection system is able to measure the microbial diversity of complex communities without PCR amplification, and consequently, without the inherent biases associated with PCR amplification. Actively metabolizing cells typically have about 20,000 or more ribosomal copies within their cell for protein assembly compared to quiescent or dead cells that have few. In some embodiments, rRNA can be purified directly from environmental samples and processed with no amplification step, thereby avoiding any of the biases caused by the preferential amplification of some sequences over others. Thus, in some embodiments, the signal from the analysis system can reflect the true number of rRNA molecules that are present in the samples. This can be expressed as the number of cells multiplied by the number of rRNA copies within each cell. The number of cells in a sample can then be inferred by several different methods, such as, for example, quantitative real-time PCR, or FISH (fluorescence in situ hybridization.). Then the average number of ribosomes within each cell may be calculated.

### Hybridization

Hybridizations can be carried out under conditions well-known by persons skilled in the art. See Rhee et al. (Appl. Environ. Microbiol. (2004) 70:4303-4317) and Wu et al. (Appl. Environ. Microbiol. (2006) 72:4931-4941). The temperature can be varied to reduce or increase stringency and allow the detection of more or less divergent sequences. Robotic hybridization and stringency wash stations can be used to give more consistent results and reduce processing time. In some embodiments, the hybridization and washing process can be accomplished in less than about half an hour, 1 hour, 2 hours, 3 hours, 4 hours, 5 hours, 6 hours, 7 hours, 8 hours, 9 hours, 10 hours, 11 hours, 12 hours, 14 hours, 16 hours, 18 hours, 20 hours or 24 hours. Generally, hybridization and washing times are reduced for microparticle based detection systems owing to the greater accessibility of the probes to the target molecules. Generally, hybridization times may be reduced for low complexity assays and/or assays for which there is an excess of target analytes.

### Signal Quantification

After hybridization, arrays can be scanned using any suitable scanning device. Non-limiting examples of conventional microarray scanners include GeneChip Scanner 3000 or GeneArray Scanner, (Affymetrix, Santa Clara, CA); and ProScan Array (Perkin Elmer, Boston, MA); and can be equipped with lasers having resolutions of 10 pm or finer. The scanned image displays can be captured as a pixel image, saved, and analyzed by quantifying the pixel density (intensity) of each spot on the array using image quantification software (e.g., GeneChip Analysis system Analysis Suite, version 5.1 Affymetrix, Santa Clara, CA; and ImaGene 6.0, Biodiscovery Inc. Los Angeles, CA, USA). For each probe, an individual signal value can be obtained through imaging parsing and conversion to xy-coordinates. Intensity summaries for each feature can be created and variance estimations among the pixels comprising a feature can be calculated.

With flow cytometry based detection systems, a representative fraction of microparticles in each sublot of microparticles can be examined. The individual sublots, also known as subsets, can be prepared so that microparticles within a sublot are relatively homogeneous, but differ in at least one distinguishing characteristic from microparticles in any other sublot. Therefore, the sublot to which a microparticle belongs can readily be determined from different sublots using conventional flow cytometry techniques as described in U.S. Patent 6,449,562. Typically, a laser is shined on individual microparticles and at least three known classification parameter values measured: forward light scatter ($C_1$) which generally correlates with size and refractive index; side light scatter ($C_2$) which generally correlates with size; and fluorescent emission in at least one wavelength ($C_3$) which generally results from the presence of fluorochrome incorporated into the labeled target sequence. Because microparticles from different subsets differ in at least one of the above listed classification parameters, and the classification parameters for each subset are known, a microparticle's sublot identity can be verified during flow cytometric analysis of the pool of microparticles in a single assay step and in real-time. For each sublot of microparticles representing a particular probe, the intensity of the hybridization signal can be calculated along with signal variance estimations after performing background subtraction.

### Data Processing and Statistical Analysis

Simultaneous detection of at least 500, 1,000, 5,000, 10,000, 20,000, 30,000, 40,000, 50,000, 60,000, or more taxa with a high level of confidence can incorporate techniques to de-convolute the signal intensity of numerous probe sets into probability estimates. In some embodiments, the methods, compositions, and systems of the invention enable detection in one assay the presence or absence of a microorganism in a community of microorganisms, such as an environmental or clinical sample when the microorganism comprises less than 0.05% of the total population of microorganisms. In some embodiments, detection includes determining the quantity of the microorganism, e.g., the percentage of the microorganism in the total microorganism population. De-convolution techniques can include the incorporation of NC probe pairs into the analysis system and the use of the data to fit the hybridization signals from the QS probe pairs to the hybridization distribution of the NC probe pairs.

De-convolution techniques can allow the detection and quantification of nucleic acids in a sample and by inference, the detection and quantification of microorganisms in a sample. In one aspect of the invention, a system is provided for determining the presence or quantity of a microorganism in a sample comprising contacting a sample with a plurality of probes, detecting the hybridization signals of the sample nucleic acids with the probes and de- convoluting the signals to determine the presence, absence and/or quantity of a particular nucleic acid present in a population of nucleic acids where the particular nucleic acid is present at less than 0.01% of the total nucleic acid population. In some embodiments, the particular nucleic acid is at least 80%, 85%, 90%, 91%, 92%, 93%, 94%, 95%, 96% or 97% homologous to other nucleic acids in the population.

In some embodiments, the data output from an imaged or scanned sample is de-convoluted and analyzed using the following methods. Using an array as an illustrative example, the hybridization signals are converted to xy-coordinates with intensity summaries and variance estimates generated for the pixels using commercial software. The data is outputted using a standard data format like a CEL file (Affymetrix), or a Feature Report file (NimbleGen).

The hybridization signals undergo background subtraction. Typically, the background intensity is computed independently for each quadrant as the average signal intensity of the least intense 2% of the probes in the quadrant. Other threshold values may also be used, e.g., 0.5%, 1%, 3%, 4%, 5% or 10%. Background intensity is then subtracted from all probes in a quadrant before further computation is performed. This noise removal procedure can be done on a quadrant-by-quadrant basis or across a whole array.

In some embodiment, array signals are normalized to allow for the comparison of results achieved in different experiments or for the comparison of replicate experiments. Normalization can be achieved by a number of methods. In one embodiment, reproducibility between different probes for the same target are evaluated using a Position Dependent Nearest Neighbor (PDNN) model as described in Zhang L. et al., A model of molecular interactions on short oligonucleotide analysis systems, Nat. Biotechnol. 2003, 21(7):818-821. The PDNN model allows estimation of the sequence specific noise signal and a non-specific background signal, and thus enables estimation of the true intensity for the probes.

In other embodiments, per-array models of signal and background distributions using responses observed from comparison of the PM and MM probe pairs and the internal DNA quantification standards (QS) probe pairs are created. In one embodiment, the probability that each probe pair is "positive" is determined by calculating a difference score, *d*, for each probe pair. *d* may be defined as:

$$d = 1 - \left( \frac{PM - MM}{PM + MM} \right) \qquad \text{Eqn. 1}$$

wherein:

- $PM$ = scaled intensity of the perfect match probe;
- $MM$ = scaled intensity of the mismatch probe; and,
- $d$ = pair difference score.

The value of $d$ can range from 0 to 2. When $PM >> MM$, the value of $d$ approaches 0; when $PM = MM$, $d = 1$; and when $PM << MM$, the value of $d$ approaches 2.

In some embodiments, the internal DNA quantification standards (QS) and negative control (NC) probe pairs are binned and sorted by attributes of the probes. Examples of the attributes of the probes that can be used in the embodiments of the present invention include, but are not limited to binding energy; base composition, including A+T count, G+C count, and T count; sequence complexity; cross-hybridization binding energy; secondary structure; hair-pin forming potential; melting temperature; and length of the probe. These attributes of the probes may affect hybridization properties of the probes, for example, A+T count may affect hydrogen bonding of the probe, and T count may affect the length and base composition of the fragments produced by the use of DNase. Fragmentation with other enzyme systems may be influenced by the composition of other bases.

In one embodiment, QS and NC probe pairs are binned and sorted based on the individual probe's A+T count and T count. For each bin (A+T count by T count), the d values from the negative control probes are fit to a normal distribution to derive the scale (mean) and shape (standard deviation). Then, the *d* values from QS are fit to a gamma distribution to derive scale and shape. For each array, multiple density plots are produced by this process. Two examples of density plots generated from two different probe bins within the same array are shown in Figure 4A-B. The AT count is 14 for the probes represented both figures. The T count is 9 for the probes in Figure 4A, while the T count is 10 for the probes represented in Figure 4B. As these graphs demonstrate, even one extra T, as shown in Figure 4B, can result in appreciable difference in the probe gamma scale parameter. Variations of gamma scale across 79 arrays are shown in Figure 5.

The parameters derived from gamma and normal distributions are used to derive a pair response score, *r*, for each probe pair. *r* is an indicator of the probability that a probe pair is positive, i.e., the probability for a probe pair to be responsive to the target sequence. *r* may be defined as:

$$r = \left( \frac{pdf_{\gamma}(X = d)}{pdf_{\gamma}(X = d) + pdf_{norm}(X = d)} \right) \quad \text{Eqn. 2}$$

where:

*r* = response score to measure the potential that a specific probe pair is binding a target sequence and not a background signal, i.e. the probability of the probe pair being positive for the specific target sequence;
$pdf_{\gamma}$ (X = *d*) = probability that *d* could be drawn from the gamma distribution estimated for the target class ATx Ty;
$pdf_{norm}$ (X = *d*) = probability that *d* could be drawn from the normal distribution estimated for the target class ATx Ty.
*r* can range from 0 to 1. *r* approaches 1 when PM >> MM, and *r* approaches 0 when PM << MM.

Each set of interrogation probe pairs, e.g., an OTU, can be scored based on pair response scores, cross-hybridization relationships or both. In some embodiments, the system removes data from at least a subset of probe pair sets before making a final call on the presence or quantity of said microorganisms. In one embodiment, the data is removed based on interrogation probe cross hybridization potential. In one embodiment, the scoring of probe pairs is performed by a two-stage process as discussed below.

For example, a two stage analysis can be performed wherein only probe pairs that pass a first stage are analyzed in the next stage. In the first stage, the distribution of *r* across each set of probe pairs, *R*, is determined. For each set of probe pairs that is associated with an OTU, the *r* values of all probe pairs are ranked within the set, and percentage of probe pairs that meet one or more threshold *r* values are determined. Frequently, three threshold determinations are made at 25% increments across the total range of ranked probe pairs (interquartile Q1, Q2, and Q3); however, any number of threshold determinations or percentage increments can be used. For example, a determination may use one increment at 70% in which probe pairs must pass a threshold value of 80%.

Typically, to differentiate signal from noise, an OTU is considered to pass Stage 1 if Q1, Q2, and Q3 of the set of probe pairs that is associated with this OTU surpass the threshold of $Q1_{min}$, $Q2_{min}$, and $Q3_{min}$, respectively. That is, for an OTU to pass Stage 1, the *r* value of 75% of the probe pairs in the set of probe pairs that is associated with that OTU has to be at least $Q1_{min}$, the r value of 50% of the probe pairs in that set of probe pairs have to be at least $Q2_{min}$, and the *r* value of 25% of the probe pairs in that set of probe pairs have to be at least $Q3_{min}$. $Q1_{min}$ is at least about 0.5, about 0.55, about 0.6, about 0.65, about 0.7, about 0.75, about 0.8, about 0.82, about 0.84, about 0.86, about 0.88, about 0.90, about 0.91, about 0.92, about 0.93, about 0.94, about 0.95, about 0.96, about 0.97, about 0.98, or about 0.99. $Q2_{min}$ is at least about 0.5, about 0.55, about 0.6, about 0.65, about 0.7, about 0.75, about 0.8, about 0.82, about 0.84, about 0.86, about 0.88, about 0.90, about 0.91, about 0.92, about 0.93, about 0.94, about 0.95, about 0.96, about 0.97, about 0.98, or about 0.99. $Q3_{min}$ is at least about 0.5, about 0.55, about 0.6, about 0.65, about 0.7, about 0.75, about 0.8, about 0.82, about 0.84, about 0.86, about 0.88, about 0.90, about 0.91, about 0.92, about 0.93, about 0.94, about 0.95, about 0.96, about 0.97, about 0.98, about 0.99, about 0.992, about 0.994, about 0.996, about 0.998, or about 0.999. In some embodiments, $Q1_{min}$, $Q2_{min}$, and $Q3_{min}$ are determined empirically from spike-in experiments. For example, $Q1_{min}$, $Q2_{min}$, and $Q3_{min}$ are chosen to allow 2 pM amplicon concentration to pass. In one embodiment, $Q1_{min}$, $Q2_{min}$, and $Q3_{min}$ are 0.98, 0.97, and0.82, respectively. These threshold numbers were empirically derived using DNase to fragment the sample sequences. Since DNase has a T- bias, the use of other enzymes may require a shift in the threshold numbers and can be empirically derived.

In the second stage only the OTUs passing the first are considered as potential sources of cross-hybridization. In some embodiments, for each OTU, only probe-pairs with *r* > 0.5 (these are the probe pairs considered as to be likely responsive to the target sequence) are further analyzed. In other instances, only probe pairs with *r* > 0.6, 0.7, 0.8, or 0.9 are considered responsive and are further analyzed. Probe pairs that are unlikely to be responsive (i.e., *r* < 0.5) are not analyzed further even if their set *R*, was responsive overall. $R_{0.5}$ represents the subset of probe pairs in which all probe pairs have *r* > 0.5. Typically, based on the interquartile Q1, Q2 and Q3 values chosen at Stage 1, most of the probe pairs in the OTUs passing Stage 1 are analyzed. In other embodiments, only the probe-pairs with *r* > 0.55, 0.60, 0.65, 0.70, 0.75, 0.80, 0.85, or 0.90 are further analyzed.

For each probe pair in the $R_{0.5}$ subset, the count of putatively cross-hybridizing OTUs (i.e., the number of OTUs with which the probe pair can cross-hybridize) is determined. In this process, only the OTUs that have passed Stage 1 are considered as potential sources of cross-hybridization. Each probe pair in the $R_{0.5}$ subset is penalized by dividing its *r* value by the count of putatively cross-hybridizing OTUs to determine its modified possibility of being positive. The modified possibility of being positive for a probe pair may be represented by a $r_x$ value. $r_x$ may be defined as:

$$r_x = \frac{r}{\text{scalar}S_{1x}} \qquad \text{Eqn. 3}$$

where

$S_1$ = Set of OTUs passing Stage1; and,
$S_{1x}$ = Set of OTUs passing Stage 1 with cross hybridization potential to the given probe pair

$r_x$ is proportional to the response of the probe pair and the specificity of the probe pair given the community observed during the first stage. $r_x$ value can range from 0 to 1. For each set of probe pairs associated with an OTU, $r_x$ are calculated for each probe pair and ranked within the set. Interquartile Q1, Q2, Q3 values for the distribution of $r_x$ value in each set of probe pairs are determined. The taxon represented by the OTU is considered to be present if Q1 is greater than $Q_{x1}$, Q2 is greater than $Q_{x20}$ or Q3 is greater than $Q_{x3}$. $Q_{x1}$ is at least about 0.5, at least about 0.55, at least about 0.6, at least about 0.65, at least about 0.7 at least about 0.75, at least at least about 0.8, at least about 0.85, at least about 0.90, at least about 0.95, or at least about 0.97. $Q_{x2}$ is at least about 0.5, at least about 0.55, at least about 0.6, at least about 0.65, at least about 0.7 at least about 0.75, at least at least about 0.8, at least about 0.85, at least about 0.90, at least about 0.95, or at least about 0.97. $Q_{x3}$ is at least about 0.5, at least about 0.55, at least about 0.6, at least about 0.65, at least about 0.7 at least about 0.75, at least at least about 0.8, at least about 0.85, at least about 0.90, at least about 0.95, or at least about 0.97. In one embodiment, $Q_{x1}$ is at least 0.66, that is, 75% of the probe pairs in the set of the probe pairs have a $r_x$ value that is at least 0.66.

A two stage hybridization signal analysis procedure can be performed on hybridization signals from any array or microparticle generated data set, including data generated from the use of any combination of probes selected using the disclosed methodologies. In some embodiments, the second stage of the procedure penalizes probes based on the number of cross-hybridizations, the intensity of the cross-hybridization signals or a combination of the two.

The method disclosed herein is useful for hierarchical probe set scoring. An OTU may be present at a node at any hierarchical level on a clustering tree. As used herein, an OTU is a group of one or more organisms, such as a domain, a sub-domain, a kingdom, a sub¬kingdom, a phylum, a sub-phylum, a class, a sub-class, an order, a sub-order, a family, a subfamily, a genus, a subgenus, a species, or any cluster. In some embodiments, a $R_{0.5}$ set is collected for each node on the phylogenetic tree and consists of all unique probes from subordinate $R_{0.5}$sets. For example, for calculating $r_x$ values for probe pairs in a $R_{0.5}$ set for an OTU representing an "order," the count of putatively cross-hybridizing equally-ranked taxa (i.e., "order" node) containing at least one sequence with cross-hybridization potential is used as the denominator in Eqn. 3.

In some embodiments, the OTUs at the leaf level (e.g., species, sub-genus or genus) are first analyzed. Then each successive level of nodes in the clustering tree is analyzed. In one embodiment, the analysis is performed up to the domain level. In another embodiment, the analysis is performed up to the phylum level. In yet another embodiment, the analysis is performed up to the kingdom level. Penalization for cross-hybridization in Eqn. 3 is only performed for probes on the same taxonomy level. All present taxa are quantified using the mean scaled PM probe intensity after discarding the highest and lowest value of the set *R* (HybScore). In some embodiments, only taxa present at a first level are analyzed further.

In some embodiments, a summary abundance score is determined. Corrected abundance scores are created based on G+C content and uracil incorporation. Generally, probes with higher G+C content produce a higher hybridization signal that is typically compensated for correcting the abundance scores.

The probability of detection for each taxonomic node is determined by summarizing terminal node detection and the breadth of cross-hybridization relationships. Hierarchical probes are scored for evidence of novel organisms based on cluster analysis.

In some embodiments, the system is capable of analyzing other data in conjunction with that obtained from the analysis of probe hybridization signal strength. In some embodiments, the system can analyze sequencing reaction data including that obtained with high-through put sequencing techniques. In some embodiments, the sequencing data is from same regions of the same highly conserved sequence analyzed by the method disclosed herein using probes.

**High Capacity Analysis System Applications**

Numerous natural human created environments can be sampled and assayed to determine the environment's microbiome composition. By having an assay system capable of detecting in a single assay the presence or quantity of at least 10,000, 20,000, 30,000, 40,000, 50,000, 60,000, 70,000, 80,000, 90,000, 100,000, 200,000, 500,000 or 1,000,000 bacterial or archeal taxa, a complete picture of the prokaryotic ecosystem can be achieved quickly and at relatively low cost providing the ability to examine numerous environments of scientific, healthcare or regulatory interest.

The elucidation of a specific microbiome associated with an ecosystem, physical environment, crop, animal, human, organ system and the like allows for the generation of a "signature," "biosignature," or "fingerprint" of the particular environment sampled, terms used interchangeably herein. If the biosignature is from a normal or healthy system or individual, or is from a physical environment associated with the maintenance of healthy state of individuals that inhabit the physical environment or use items produced in the physical environment, then the biosignature of the normal or healthy place can be used as a reference for the comparison of later samples from the same environment to monitor for changes that are associated with an abnormal or unhealthy state or condition. For example, if a later biosignature of a water source shows that the microbiome has shifted away from that associated with potable water, then preemptive measures could be taken to prevent a continued shift, for example by identifying a contaminant and/or contamination source and taking steps to treat and/or remove it.. As a further example, if a later fingerprint of an orchard shows that the microbiome has shifted away from that associated with healthy trees and high productivity, then preemptive measures could be taken to apply nutrients that favor the growth and maintenance of the healthy microorganism or alternatively, a compost tea can be applied to boost the number of healthy microorganisms.

Similarly, a biosignature of an environment can be compared to a biosignature generated from a pool of samples that represent an average or normal biosignature for a population or collection of environments. For example, a sample from an unhealthy individual could be assayed and the microbial biosignature compared to the biosignature seen in a healthy population at large. If one or more microorganisms are detected in the unhealthy individual that are either not seen in the general population or not seen at the same prevalence then therapeutic measures can be taken to selectively eliminate or reduce in number the microorganisms associated with the unhealthy state. For instance, the microflora of the gastrointestinal tract can be compared between children that suffer from allergies and healthy children. If the allergy sufferers are shown to have one or more dominant microorganisms in their gastrointestinal tracks compared to the other children, then an available drug and/or dietary therapy that specifically targets the prevalent, abnormal microorganisms can be administered. Alternatively or additionally, the gastrointestinal population in the allergy sufferer can be shifted through the introduction of large numbers of the microorganisms associated with healthy children such as through probiotic foods or supplements. Similarly, the allergy sufferer could be given nutritional supplements that promote the growth of the health microorganisms, or the child’s parents can be directed to change the child’s diet to foods that favor the growth of the healthy microorganisms over that of the unhealthy ones. Once a relationship is known between the prevalence of a particular microorganism or group of microorganisms and a disease state, then disease progression or treatment response can also be monitored using the present systems and methods.

Numerous microbiomes of animals or humans can be analyzed with the present systems and methods including the gut, respiratory system, urogenital tract, mammary glands, skin, oral cavity, auditory canal, and skin. Clinical samples such as blood, sputum, nares, feces, and urine can be used with the method. From the analysis of normal individuals and those suffering from a disease or condition, a large database of fingerprints or biosignature can be assembled. By comparing the biosignatures between healthy and disease related states, associations can be made as to the influence and importance of individual components of the microbiome.

Once these associations are made, treatments can be designed and tested to alter the composition of the microbiota seen in the disease state. Additionally, by regularly monitoring the microbial composition of an affected organ system in a diseased individual, disease progress or response to therapy can be observed and if need, additional therapeutic measures taken to alter the microbiome composition to one that is more representative of that seen in a healthy population.

An interesting property of bacteria that has great importance in healthcare, water quality and food safety is quorum sensing. Many bacteria are able to sense the presence of other members of their species or related species and upon reaching a specific density the bacteria start producing various virulence or pathogenicity factors. In other words, the bacteria’s gene expression is coordinated as a group. For example, some bacteria produce exopolysaccharides that are known as "slime layers." The secretion of exopolysaccharidse can decrease the ability of white blood cells to phagocytize the microorganisms and make the microorganisms more resistant to therapeutics or cleaning agents. Traditional methodologies require the detection of specific gene expression in order to detect or study quorum sensing and other population induced effects. The present systems and methods can be used to understand the changes that occur in a microbiome that are associated with a given effect such as biofilm formation or toxicity production. One can develop protocols with the present systems and methods to look for and determine conditions that lead to quorum sensing. For example, testing samples at various timepoints and under varying conditions can lead to determining how and when to intervene or reverse population induced expression of virulence or pathogenicity factors.

For example, the clean rooms used to assemble components of satellites and other space craft can be surveyed with the present systems and methods to understand what microbial communities are present and to develop better decontamination and cleaning techniques to prevent the introduction of terrestrial microbes to other planets or samples thereof or to develop methodologies to distinguish data generated by putative extraterrestrial microorganisms from that generated by contaminating terrestrial microorganisms.

For example, food preparation sites, intensive care facilities, clean room environments such as operating theaters, drug manufacturing facilities, medical device manufacturing facilities and the like can be surveyed with the present systems and methods to ascertain the composition the local microbial communities and the quantity of the individual taxa that comprise the microbial communities. Such testing can be instrumental in preventing contamination in manufacturing processes and subsequent recalls of contaminated consumer products or the spread of infection and disease.

In one embodiment, a method is provided to identify a new indicator species for an environmental or health condition with the present systems and methods. The condition can be that of a normal or healthy state. Alternatively, the indicator species can be for an unhealthy or abnormal condition. To indentify a new indicator species, a normal sample is simultaneously assayed to determine the presence or quantity of each OTU associated with all known bacteria, archae, or fungi; this test result is compared to the results achieved in the simultaneous assay of sample from the environment of the condition where the presence or quantity of each OTU associated with all known bacteria, archae, or fungi was determined. Microorganisms that change in abundance at least 2-fold, 3-fold, 4-fold, 5-fold, 10-fold, 20-fold, 50-fold or 100-fold, either increasing in abundance or decreasing in abundance represent putative indicator species for a condition.

In some embodiments, methods are provided for identifying indicator species associated with environmental change including root growth and changes in soil composition such as increased availability of carbon substrates in soil or the presence of heavy metal or uranium, changes in soil pH, and changes in precipitation amounts and patterns. In other embodiments, methods using the present systems and methods are provided for identifying indicator species associated with coral stress and coral bleaching or changes in other marine and other aquatic environments.

In other embodiments, methods are provided for identifying indicators species associated with a disease state, disease progression, treatment regimen, probiotic administration including progression of disease in CF patients and exacerbations of COPD. In other embodiments, methods are provided for monitoring a change in the environment or health status associated with introducing one or more new microorganisms into a community. For example, measures to increase a particular microorganism's percentage of the gut microbiome in an individual, such as feeding a person yogurt or a food supplement containing L. casei, can be monitored using the present methods and systems.

**Combined Analysis**

The ability to identify and quantitate the microorganisms in a sample can be combined with a gene expression technology such as a functional gene array to correlate populations with observed gene expression. Similarly, microbiome composition analysis can be conelated with the presence of chemicals, proteins including enzymes, toxins, drugs, antibiotics or other sample constituents. For instance, nucleic acids isolated from a soil sample can be analyzed to elucidate the microbiome composition (e.g. biosignature) and also to identify expressed genes. In the bare, nutrient-poor soils on the Antarctic, this analysis associated chitinase and mannanase expression with *Bacteroidetes* and $CH_4$-related genes with *Alphaproteobacteria.* (Yergeau et al., Environmental microarray analyses of Antarctic soil microbial communities. ISME J. 3:340-351, 2009). Significant correlations were also found between taxon abundances and C- and N-cycle gene abundance. From this data, one can predict that certain organisms or groups of organisms are required or account for the majority of an expected or observed enzymatic or degradative process. For example, members of the *Bacteroidetes* phylum probably degrade the majority of environmental chitin, a major constituent of exoskeletons of insect and arthropods and also of fungi cell walls, at the sample locale.

This methodology can be used to identify new antibiotic producing organisms, even ones that are unculturable. For instance, soil extracts can be tested for antibiotic activity. If a positive extract is found, a sample of the soil from which a portion was extracted for antibiotic can be analyzed for microbial composition and perhaps gene expression. Major constituents of the microbiome could be correlated with antibiotic activity with the correlation strengthened through gene expression data allowing one to predict that a particular organism or group of organisms is responsible for the observed antibiotic activity.

In one aspect, the invention provides a method for determining a condition in a sample. In one embodiment, the method comprises a) contacting said sample with a plurality of different probes; b) determining hybridization signal strength for each of said probes, wherein said determination establishes a biosignature for said sample; and, c) comparing the biosignature of said sample to a biosignature for fecal contamination. In some embodiments, a method is provided for making a prediction about a sample comprising a) determining microorganism population data as the probability of the presence or absence of at least 100 OTUs of microorganisms in said sample; b) determining gene expression data of one or more genes by said microorganisms in said sample and c) using said expression data and population data to make a prediction about said sample. In some embodiments, the prediction entails the identity of a microorganism responsible for a characteristic or condition observed in the soil or local environment.

Other combined analysis methods include the use of a diffusion chamber to retain microorganisms in a water sample while one or more constituents or parameters of the water sample are changed. For instance, the salinity or pH of the water can be changed abruptly or gradually over time. Diffusion chambers are useful to mimic the conditions of a receiving water into which is placed, for example, raw sewage. Following specific time intervals, the microbiome of the water sample in the diffusion chamber can be determined. Microorganisms that cannot tolerate the new environment conditions will die, become reduced in number due to unfavorable conditions or predation, or remain static in their numbers. In contrast, microorganisms that can tolerate the new conditions will at least maintain their number or thrive, perhaps becoming a dominant population. Use of a diffusion chamber coupled with a system capable of detecting the presence or quantity of at least 10,000 OTUs can allow the identification of microorganisms that perish or fail to thrive when placed in a new environment. Such microorganisms are termed "transient", meaning that their percent composition of the microbiome changes quickly. The identification of transient microorganisms can be used to ascertain the time and/or place they were introduced into an environment. For example, the identification in a sample of water of an appreciable quantity of transient microorganisms associated with contaminated water that have a half-life of around 4 hours, would indicate that the microorganisms were likely introduced into the body of water within the past day (6 half-lives). Different transient microorganisms can have different half-lives for a particular condition. Armed with the knowledge of the half-lives in a receiving water of various transient microorganisms associated with contaminated water, a time course of a spill, for example a sewage discharge, can be constructed. Use of the time course can be used to pinpoint the source of the discharge and in the case of illegal discharges, for example by a cruise or cargo ship, allow the identification and citation of the violator.

Diffusion chambers can also take the form of a semi-permeable capsule, tube, rod, or sphere or other solid or semi-solid object. A microbiome or a select group of bacteria can be placed inside the capsule, that is then sealed and introduced into an environment for a specified period of time. Upon removal, the capsule is opened and the microbiome or select group of bacteria sampled to ascertain changes in the presence or quantity of the individual constituents. For example, rather than placing a sample of raw sewage into a diffusion chamber, the raw sewage could be placed into a semi-permeable capsule that is then placed into a quantity of the receiving water or into the actual receiving body of water. The capsule can be removed once or periodically from the quantity of receiving water or body of water to sample the microbiome. Alternatively, multiple single use capsules with identical quantities of the microbiome can be used, each one removed and sampled at a different time point. Microbiomes placed in capsules or other semi-permeable containers can be introduced into a living organism, usually through an orifice, to measure changes to the microbiome composition associated with a particular organ or system environment. For example, a semi¬permeable capsule or tube containing a microbiome can be introduced into the gastrointestinal system through the mouth or anus. A microbiome from a healthy individual can be introduced in this manner into an unhealthy individual, say a patient suffering from Crohn's disease or irritable bowel syndrome to ascertain the effect of the unhealthy condition on the normal, healthy individual associated microbiome. In this manner, the efficacy of drug effectiveness and treatment protocols could also be evaluated based on the effects of the gut ecology on a known microbiome.

**Low Density-Special Purpose Detection Systems**

In some embodiments, probes are selected for constructing special purpose systems including those with arrays or microparticles. Typically, special purpose "low density" systems, are designed for use in a specific environment or for a particular application and usually feature a reduced number of probes, "down-selected" probes, that are specific to organisms that are known or expected to be present in the particular environment, such as associated with a particular biosignature. In some cases the biosignature is fecal contamination. Typically, a low density system comprises no more than 10, 20, 50, 100, 200, 500, 1,000, 2,000, 5,000 or 10,000 down selected probes or 5, 10, 25, 50, 100, 250, 500, 1,000, 2,500 or 5,000 down selected probes probe pairs (PM and MM probes). In some embodiments, only 1, 2, 3, 4, 5, 6, 7, 8, 9, or 10 probes are used per OTU. In further embodiments, only PM probes are used. Generally, these down-selected probes have robust hybridization signals and few or no cross hybridizations. In some embodiments, the collection of down selected probes have a median cross hybridization potential number of less than 20, 15, 10, 8, 7, 6, 5, 4, 3, 2, or 1 per probe. Frequently the down selected probes belong to OTUs that have reduced numbers of probes. In some embodiments, the OTUs of a down select probe collection have a median number of less than 25, 20, 15, 14, 13, 12, 11, 10, 9, 8, 7, 6, 5, 4, 3 or 2 probes per OTU. Generally, low density systems feature probes that recognize no more than 10, 25, 50, 100, 250, 500, 1,000, 2,000, or 5,000 taxa. For a set number of probes, a number of design strategies can be employed for low density systems. One approach is to maximize the number of OTUs identified, e.g., use one probe per OTU with no mismatch probes. Another approach is to select probes based on the desired confidence level. Here, multiple probes for each OTU along with corresponding mismatch probes may be required to achieve at least 95% confidence level for the presence and quantity of each OTU. The probes for a particular low density application can be selected by applying a sample from an appropriate environment to a high density analysis system, e.g., a detection system that can in a single assay determine the probability of the presence or quantity of at least 10,000, 20,000, 30,000, 40,000, 50,000, 60,000, 70,000, 80,000, 90,000, 100,000, 250,000, 500,00 or 1,000,000 OTUs of a single domain, such as bacteria, archea, or fungi, or alternatively, for each known OTU of a single domain. Probes associated with prevalent OTUs can be selected for a low density system. Alternately, the OTUs seen in a sample of interest can be compared with a control sample and shared OTUs subtracted out with the probes associated with the remaining OTUs selected for the low density system. Additionally, probes can be selected based on a change in prevalence of OTUs between the environment of interest and a control environment. For example, OTUs that are at least 2-fold 5-fold, 10-fold, 100-fold or 1,000-fold more abundant in the sample of interest compared to the control sample are included in the down selected probe set. Using this information, a down selected array, bead multiplex system or other low density assay system is designed.

"Low density" assays systems can be used to identify select microorganisms and determine the percentage composition of various select microorganisms in relation to each other. Low density assay systems can be constructed using probes selected through the disclosed methodologies. These low density systems can identify at least 1, 2, 3, 4, 5, 6, 7, 8, 9, 10, 15, 20, 30, 40, 50, 60, 70, 80, 90, 100, 200, 500, 1000 or more microorganisms. Representative microorganisms to be identified or quantitated are listed in Table 2.

Table 2 Representative Microorganisms Recognized by Low Density Assay Systems

| Species | Application |
|---|---|
| Listeria monocytogenes | Food safety, environmental surveillance of food processing plants |
| Salmonella enterica subsp. enterica serovar Enteritidis | Food safety, environmental surveillance of food processing plants |
| Pseudomonas aeruginosa | Pulmonary health |

Low density assays systems are useful for numerous environmental and clinical applications. Exemplary applications are listed in Table 2. These applications include water quality testing for fecal or other contamination, testing for animal or human pathogens, pinpointing sources of water contamination, testing reclaimed or recycled water, testing sewage discharge streams including ocean discharge plumes, monitoring of aquaculture facilities for pathogens, monitoring beaches, swimming areas or other water related recreational facilities and predicting toxic alga blooms. Other applications include making water management or treatment decisions based on the testing or monitoring results.

Food monitoring applications include the periodic testing of production lines at food processing plants, surveying slaughter houses, inspecting the kitchens and food storage areas of restaurants, hospitals, schools, correctional facilities and other institutions for food borne pathogens such as *E. coli* strains O157:H7 or O111:B4, *Listeria monocytogenes,* or *Salmonella enterica subsp. enterica serovar Enteritidis.* Shellfish and shellfish producing waters can be surveyed for alga responsible for paralytic shellfish poisoning, neurotoxic shellfish poisoning, diarrhetic shellfish poisoning and amnesic shellfish poisoning. Additionally, imported foodstuffs can be screened while in customs before release to ensure food security.

Plant pathogen monitoring applications include horticulture and nursery monitoring for instance the monitoring for *Phytophthora ramorum,* the microorganism responsible for Sudden Oak Death, crop pathogen surveillance and disease management and forestry pathogen surveillance and disease management.

Medical conditions that can be identified, diagnosed, prognoses, track, or treated based on data obtained with a low density system include but are not limited to, cystic fibrosis, chronic obstructive pulmonary disease, Crohn's Disease, irritable bowel syndrome, cancer, rhinitis, stomach ulcers, colitis, atopy, asthma, neonatal necrotizing enterocolitis, obesity, periodontal disease and any disease or disorder caused by, aggravated by or related to the presence, absence or population change of a microorganism. Through the judicious selection of OTUs to be included in a system, the system becomes a diagnostic device capable of diagnosing one or more conditions or diseases with a high level of confidence producing very low rates of false positive or false negative readings.

Manufacturing environments for pharmaceuticals, medical devices, and other consumables or critical components where microbial contamination is a major safety concern can be surveyed for the presence of specific pathogens like Pseudomonas aeruginosa, or Staphylococcus aureus, the presence of more common microorganisms associated with humans, microorganisms associated with the presence of water or others that represent the bioburden that was previously identified in that particular environment or in similar ones.

Similarly, the construction and assembly areas for sensitive equipment including space craft can be monitored for previously identified microorganism that are known to inhabit or are most commonly introduced into such environments.

National security applications include monitoring of air, water and buildings for known bioterrorist threats such as *Francisella tularensis* or *Bacillus anthracis.* Other uses include the testing of suspicious packages or mail.

Energy security can be increased through improved gas and oil exploration methodologies and by microbial enhanced oil recovery (MEOR). Oil and gas reservoirs often leak low molecular weight components of the accumulated hydrocarbons including methane, ethane, propane and butane. These hydrocarbons can serve as food sources for a variety of microorganisms. By sampling microbial communities overlying hydrocarbon accumulations and comparing the microbiome with the microbiome observed in similar environments that are devoid of hydrocarbons, indicator species can be discovered that can then be used to identify new areas for oil and gas exploration. Soil samples can be collected from a grid array in the prospective oilfield and based on the abundance of each hydrocarbon indicator microorganism, contoured surface maps can be constructed delineating the locations of hydrocarbon plumes.

Most conventional oil recovery processes are only able to retrieve from 15 to 50% of the available oil in the reservoir. Tertiary oil recovery generally entails more expensive methods extraction techniques such as thermal recovery, chemical flooding, or miscible displacement (gas injection) to extract a last fraction of a reserve. MEOR offers a lower cost tertiary recovery method because microbes can produce biosurfactants or gases in situ using simple and cheap nutrients. Additionally, certain microorganisms can metabolize long chain hydrocarbons to create smaller, less viscous hydrocarbons (biocracking) that are easier to pump out. The ability to measure or monitor the whole microbiome of an oil field can allow for the identification and isolation of microorganisms that are associated with more productive fields. Additionally, a whole microbiome approach allows for the monitoring of a MEOR field to optimize production by observing the microbiome and adjusting nutrient levels to induce or maintain an optimal community composition for oil extraction.

Forensic science requires reliable systems for determining when events occurred, such as time of death in a murder investigation. The collection and classification of insects is currently used, but changes in microbial populations can offer another avenue to determining the time and circumstances of death.

Successful bioremediation can require active monitoring and management of microbial populations to ensure that desired species are present at the start of the bioremediation project and that their numbers are adequately maintained, perhaps through timely supplementation of essential or preferred nutrients.

In some embodiments, the low density systems also feature confirmatory probes that are specific (complimentary) for genes or sequences expressed in specific organisms. For example, the *cafl* virulence gene of *Yersinia pestis* and the zonula occludens toxin (*zot*) gene of *Vibrio cholerae* and also confirmatory probes to *Y. pestis* or *V. cholerae*.

**Kits**

As used herein a "kit" refers to any delivery system for delivering materials or reagents for carrying out a method of the invention. In the context of assays, such delivery systems include systems that allow for the storage, transport, or delivery of arrays or beads with probes, reaction reagents (e.g., probes, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials for assays of the invention.

In one aspect of the invention, kits for analysis of nucleic acid targets are provided. According to one embodiment, a kit includes a plurality of probes capable of determining the presence or quantity over 10, 20, 50, 100, 200, 500, 1,000, 2,000, 5,000, 10,000, 20,000, 30,000, 40,000 50,000 or 60,000 different OTUs in a single assay. Such probes can be coupled to, for example, an array or plurality of microbeads. In some aspects a kit comprises at least 5, 10, 15, 20, 50, 100, 200, 500, 1,000, 2,000, 5,000, 10,000, 20,000, 50,000, 100,000, 200,000, 500,000, 1,000,000 or 2,000,000 interrogation probes selected using the disclosed methodologies and/or for use in the identification and/or comparison of a biosignature of one or more samples.

The kit can also include reagents for sample processing. In some embodiments, the reagents comprise reagents for the PCR amplification of sample nucleic acids including primers to amplify regions of a highly conserved sequence such as regions of the 16S rRNA gene. In still other embodiments, the reagents comprise reagents for the direct labeling of rRNA. In further embodiments, the kit includes instructions for using the kit. In other embodiments, the kit includes a password or other permission for the electronic access to a remote data analysis and manipulation software program. Such kits will have a variety of uses, including environmental monitoring, diagnosing disease, monitoring disease progress or response to treatment, and identifying a contamination source and/or the presence, absence, or amount of one or more contaminants.

**Computer Implemented Methods**

FIG. 1 illustrates an example of a suitable computing system environment or architecture in which computing subsystems may provide processing functionality to execute software embodiments of the present invention, including probe selection, analysis of samples, and remote networking. The method or system disclosed herein may also operational with numerous other general purpose or special purpose computing system including personal computers, server computers, hand-held or laptop devices, multiprocessor systems, and the like.

The method or system may be described in the general context of computer-executable instructions, such as program modules, being executed by a computer. The method or system may also be practiced in distributed computing environments where tasks are performed by remote processing devices that are linked through a communications network.

With reference to FIG. 1, an exemplary system for implementing the method or system includes a general purpose computing device in the form of a computer **102.**

Components of computer **102** may include, but are not limited to, a processing unit **104,** a system memory **106,** and a system bus **108** that couples various system components including the system memory to the processing unit **104.**

Computer **102** typically includes a variety of computer readable media. Computer readable media includes both volatile and nonvolatile media, removable and non-removable media and a may comprise computer storage media. Computer storage media includes, but is not limited to, RAM, ROM, EEPROM, flash memory or other memory technology, CD- ROM, digital versatile disks (DVD) or other optical disk storage, magnetic cassettes, magnetic tape, magnetic disk storage or other magnetic storage devices.

The system memory **106** includes computer storage media in the form of volatile and/or nonvolatile memory such as read only memory (ROM) **110** and random access memory (RAM) **112.** A basic input/output system **114** (BIOS), containing the basic routines that help to transfer information between elements within computer **102,** such as during start-up, is typically stored in ROM **110.** RAM **112** typically contains data and/or program modules that are immediately accessible to and/or presently being operated on by processing unit **104.** FIG. 1 illustrates operating system **132,** application programs **134** such as sequence analysis, probe selection, signal analysis and cross-hybridization analysis programs, other program modules 136, and program data **138.**

The computer **102** may also include other removable/non-removable, volatile/nonvolatile computer storage media. By way of example only, FIG. 1 illustrates a hard disk drive **116** that reads from or writes to non-removable, nonvolatile magnetic media, a magnetic disk drive **118** that reads from or writes to a removable, nonvolatile magnetic disk **120,** and an optical disk drive **122** that reads from or writes to a removable, nonvolatile optical disk **124** such as a CD ROM or other optical media. Other removable/non-removable, volatile/nonvolatile computer storage media that can be used in the exemplary operating environment include magnetic tape cassettes, flash memory cards, digital versatile disks, digital video tape, solid state RAM, solid state ROM, and the like. The hard disk drive **116** is typically connected to the system bus **108** through a non-removable memory interface such as interface **126,** and magnetic disk drive **118** and optical disk drive **122** are typically connected to the system bus **108** by a removable memory interface, such as interface **128** or **130.**

The drives and their associated computer storage media discussed above and illustrated in FIG. 1, provide storage of computer readable instructions, data structures, program modules and other data for the computer **102.** In FIG. 1, for example, hard disk drive **116** is illustrated as storing operating system **132,** application programs **134,** other program modules **136,** and program data **138.** A user may enter commands and information into the computer **102** through input devices such as a keyboard **140** and a mouse, trackball or touch pad **142.** These and other input devices are often connected to the processing unit **104** through a user input interface **144** that is coupled to the system bus, but may be connected by other interface and bus structures, such as a parallel port or a universal serial bus (USB). A monitor **158** or other type of display device is also connected to the system bus **108** via an interface, such as a video interface or graphics display interface **156**. In addition to the monitor **158**, computers may also include other peripheral output devices such as speakers (not shown) and printer (not shown), which may be connected through an output peripheral interface (not shown).

The computer **102** can be integrated into an analysis system, such as a microarray or other probe system described herein. Alternatively, the data generated by an analysis system can be imported into the computer system using various means known in the art.

The computer **102** may operate in a networked environment using logical connections to one or more remote computers or analysis systems. The remote computer may be a personal computer, a server, a router, a network PC, a peer device or other common network node, and typically includes many or all of the elements described above relative to the computer **102**. The logical connections depicted in FIG. 1 include a local area network (LAN) **148** and a wide area network (WAN) **150,** but may also include other networks. Such networking environments are commonplace in offices, enterprise-wide computer networks, intranets and the Internet. When used in a LAN networking environment, the computer **102** is connected to the LAN **148** through a network interface or adapter **152.** When used in a WAN networking environment, the computer **102** typically includes a modem **154** or other means for establishing communications over the WAN **150,** such as the Internet. The modem **154,** which may be internal or external, may be connected to the system bus **108** via the user input interface 144, or other appropriate mechanism. In a networked environment, program modules depicted relative to the computer 102, or portions thereof, may be stored in the remote memory storage device.

In further aspects of the invention, computer-implemented methods are provided for analyzing the presence or quantity of over 20, 50, 100, 200, 500, 1,000, 2,000, 5,000, 10,000, 20,000, 30,000, 40,000 50,000 or 60,000 different OTUs in a single assay. In one embodiment, computer executable logic is provided for determining the presence or quantity of one or more microorganisms in a sample comprising: logic for analyzing intensities from a set of probes that selectively binds each of at least 20, 50, 100, 200, 500, 1,000, 2,000, 5,000, 10,000, 20,000, 30,000, 40,000 50,000 or 60,000 unique and highly conserved polynucleotides and determining the presence of at least 97% of all species present in said sample with at least 90%, 95%, 96%, 97%, 98%, 99% or 99.5% confidence level.

In one embodiment, computer executable logic is provided for determining probability that one or more organisms, from a set of different organisms, are present in a sample. The computer logic comprises processes or instructions for determining the likelihood that individual interrogation probe intensities are accurate based on comparison with intensities of negative control probes and positive control probes; a process or instructions for determining likelihood that an individual OTU is present based on intensities of interrogation probes from OTUs that pass a first quantile threshold; and a process or instructions for penalizing one or more OTUs that have passed the first quantile threshold based on their potential for cross-hybridizing with other probes that have also passed the first quantile threshold.

In a further embodiment, computer executable logic is provided for determining the presence of one or more microorganisms in a sample. The logic allows for the analysis of a set of at least 1000 different interrogation perfect probes. The logic further provides for the discarding of information from at least 10% of the interrogation perfect match probes in the process of making the determination. In some embodiments, the computer executable logic is stored on computer readable media and represents a computer software product.

In other embodiments, computer software products are provided wherein computer executable logic embodying aspects of the invention is stored on computer media like hard drives or optical drives. In one embodiment, the computer software products comprise instructions that when executed perform the methods described herein for determining candidate probes.

In further embodiments, computer systems are provided that can perform the methods of the inventions. In some embodiments, the computer system is integrated into and is part of an analysis system, like a flow cytometer or a microarray imaging device. In other embodiments, the computer system is connected to or ported to an analysis system. In some embodiments, the computer system is connected to an analysis system by a network connection. Figure 2 illustrates one embodiment of a networked system for remote data acquisition or analysis that utilizes a computer system illustrated in Figure 1. In this example, a sample is imaged using a commercially available imaging system and software. The data is outputted using a standard data format like a CEL file (AFFYMETRIX®), or a Feature Report file (NIMBLEGEN®). Then the data is sent to a remote or central location for analysis using a method of the invention. In some embodiments, a standardized analysis is performed providing signal normalization, OTU quantification, and visual analytics. In other embodiments, a customized analysis is performed using a fixed protocol designed for the user's particular needs. In still other embodiments, a user configurable analysis is used, include a protocol that allows for the user to adjust at least one variable before each analysis run.

After processing, the results are stored in an exchangeable binary format for later use or sharing. Additionally, hybridization scores and OTU probability values may be exported to a tab delimited file or in a format compatible with UniFrac (Lozupone, et al., UniFrac--an online tool for comparing microbial community diversity in a phylogenetic context, BMC Bioinformatics, 7, 371; 2006) for further statistical analysis of the detected sample communities.

In some embodiments, multiple, interactive views of the data are available, including taxonomic trees, heatmaps, hierarchical clustering, parallel coordinates (time series), bar plots, and multidimensional scaling scatterplots. In some embodiments, the taxonomy tree displays the mean intensities for each detected OTU and displays the leaves of the tree as a heatmap of samples. The tree may be dynamically pruned by filtering OTUs below a certain intensity or probability threshold. Additionally, the tree may be summarized at any level from phylum to subfamily. In other embodiments, the user can hierarchically cluster both OTUs and samples using any of the standard distance and linkage methods from the integrated C Clustering Library (de Hoon, et al., Open source clustering software, Bioinformatics, 20, 1453-1454; 2004), and the resulting dendrograms displayed in a secondary heatmap window. In some embodiments, a third window is provided that displays interactive bar plots of differential OTU intensities to facilitate pairwise comparison of samples. For any two samples, the height of the difference bars displays either the absolute or relative difference in mean intensity between OTUs. The bars may be grouped and sorted along the horizontal axis by any taxonomic rank for easy identification and comparison. Synchronized selection and filtering affords users the unique ability to seamlessly navigate between multiple views of the data. For example, users can select a cluster in the hierarchical clustering window and simultaneously view the selected organisms in the taxonomy tree, immediately revealing both their phylogenetic and environmental relationship. In further embodiments, the data from the analysis system, i.e., analysis system or flow cytometer, can be co-analyzed and displayed with high-throughput sequencing data. In some embodiments, for each organism identified as present in the sample, the user is able to view a list of other environments where the particular organism is found.

In some embodiments, the screen displays are dynamic and synchronized to allow the selection or filtration of OTUs with changes to any view simultaneously reflected in all other views. Additionally, OTUs confirmed by 16S rRNA gene, 18S rRNA gene, or 23S rRNA gene sequencing can be co-displayed in all views.

EXAMPLES

Example 1: PhyloChip Array Analysis

Following sample preparation, application, incubation and washing, using standard techniques, PhyloChip G3 arrays were scanned using a GeneArray Scanner from Affymetrix. The scan was captured as a pixel image using standard AFFYMETRIX® software (GCOS v1.6 using parameter: Percentile v6.0) that reduces the data to an individual row in a text-encoded table for each probe. See Table 3.

Table 3 Exemplary Display of Array Data

| | | |
|---|---|---|
| [INTENSITY] | | |
| NumberCells=506944 | | |
| CellHeader=XY | MEAN | STDV |
| NPIXELS | | |
| 0 | 0 | 167.0 |
| 47.9 | 25 | |
| 1 | 0 | 4293.0 |
| 1060.2 | 25 | |
| 2 | 0 | 179.3 |
| 43.7 | 36 | |
| 3 | 0 | 4437.0 |
| 681.5 | 25 | |

Each analysis system had approximately 1,016,000 cells, with 1 probe sequence per cell. The analysis system scanner recorded the signal intensity across the array, which ranges from 0 to 65,000 arbitrary units (a.u) in a regular grid with -30-45 pixels per cell. A 2 pixel margin was used between adjacent cells, leaving approximately 25-40 pixels per probe of usable signal. From these pixels, the AFFYMETRIX® software computed the 75th percentile average pixel intensity (denoted as the "MEAN"), the standard deviation of signal intensity among the about 25-40 pixels (denoted as the "STDV"), and the number of pixels used per cell (denoted as "NPIXELS"). Any cells that had pixels that were three standard deviations apart in signal intensity were classified as outliers.

The analysis systems were divided into a user-defined number of horizontal and vertical divisions. By default, four horizontal and four vertical divisions were created resulting in 16 regularly spaced sectors for independent background subtraction. The background intensity was computed independently for each quadrant, as the average signal intensity of the least intense 2% (by default) of probes in that quadrant. The background intensity was then subtracted from all probes before further computation.

The noise value was estimated according to recommendations in the AFFYMETRIX® GeneChip User Guide v3.3. Noise (N) was due to variations in pixel intensity signals observed by the scanner as it read the array surface and was calculated as the standard deviation of the pixel intensities within each of the identified background cells divided by the square root of the number of pixels comprising that cell. The average of the resulting quotients was used for N in the calculations described below:

$$N = \frac{\sum_{i \in B} \frac{S_i}{\sqrt{pix_i}}}{\text{scalarB}}$$

where

B is a background cell
$S_i$ is the standard deviation among the pixels in B
$pix_i$ is the count of pixels in B
scalarB is the count of all background cells, cumulative

The intensities of all probes were then scaled so that the average observed signal intensity of the spiked in probes had a pre-determined signal strength. This was accomplished by finding a scaling factor (Sf) in order to force the mean response of the corresponding PM probes to a target mean using the equation below:

$$S_f = \bar{e}_t \Big/ \frac{\sum_{i \in K_{pm}} e_i}{\text{scalarK}_{pm}}$$

where

$\bar{e}_t$ = targeted mean intensity (default: 2500)
$\text{scalarK}_{pm}$ = count of probes complementing any spike-in
$S_f$ = scaling factor

Typically, the pre-determined signal strengths ranged from about 0 to about 65,000. Once the scaling factor was derived, all cell intensities were multiplied by the scaling factor.

The noise (N) was scaled by the same factor: $N_s$ = N X $S_f$; where $N_s$ = scaled noise, N = unscaled noise, and $S_f$ = scaling factor.

As an alternative or optional step, MM probes with high hybridization signal responses were identified and the probe pair eliminated where:

$$\left[\left(\frac{MM}{PM} > srt_r\right) \wedge \left(MM - PM > N_s \times sdtm_r\right)\right] \vee \left[PM \in O\right] \vee \left[MM \in O\right]$$

where:

*PM* = scaled intensity of the perfect match probe
*MM* = scaled intensity of the perfect match probe
$srt_r$ = reverse standard ratio threshold (default : 1.3)
$sdtm_r$ = reverse standard difference threshold multiplier (default : 130)
$N_s$ = scaled noise
*O* = outlier set

The remaining probe pairs were scored by:

$$\left(\frac{PM}{MM} > srt\right) \wedge \left(PM - MM > N_s^2 \times sdtm\right)$$

where:

*PM* = scaled intensity of the perfect match probe
*MM* = scaled intensity of the perfect match probe
*srt* = standard ratio threshold (default : 1.3)
*sdtm* = standard difference threshold multiplier (default : 130)
$N_x$ = scaled noise

After classifying an OTU as "present", the present call was propagated upwards through the taxonomic hierarchy by considering any node (subfamily, family, order, etc.) as 'present' if at least one of its subordinate OTUs was present.

Hybridization intensity was the measure of OTU abundance and was calculated in arbitrary units for each probe set as the trimmed average (maximum and minimum values removed before averaging) of the PM minus MM intensity differences across the probe pairs in a given probe set.

Example 2: Water Quality Testing—Fecal Contamination Assay

The dry weather water flow in the lower Mission Creek and Laguna watersheds of Santa Barbara, California, a place associated with elevated fecal indicator bacteria concentrations and human fecal contamination will be sampled with an array of the present invention. The goal is to characterize whole bacterial community composition and biogeographic pattern in an urbanized creek, 2) compare taxa detected by molecular methods to conventional fecal indicator bacteria, and 3) elucidate reliable groups of bacterial taxa to be used in culture-independent community-based fecal contamination monitoring (indicator species for fecal contamination).

The watersheds flow through an urbanized area of downtown Santa Barbara. Places to be sampled include storm drains, sections of the flowing creek, lagoon (M2, M4) and ocean. Additionally sites include where Old Mission Creek tributary discharges into Mission Creek. The dry creek flow can have many sources including underground springs in the upstream reaches, urban runoff associated with irrigation and washing, groundwater seepage, sump or basement pumps, and potentially illicit sewer connections. Sampling will be done during a period when there will not have been rain for at least 48 hours prior to or during the sampling. Besides the watershed samples, human feces and sewage will be sampled.

Materials and Methods

*Sample description, collection and extraction.* Water samples are collected over 3-5 days from a watershed during a period of dry weather. Additionally, fecal samples including human feces sewage inflow are collected. Dissolved oxygen (DO), pH, temperature and salinity are measured along with each sampling. Water samples are filtered in the lab on 0.22 pm filters and extracted for DNA using the UltraClean Water DNA kit (MoBio Laboratories), and archived at -20 °C. Concentrations (by IDEXX) of Total Coliforms, *E coli,* and *Enterococcus spp.,* as well as quantitative PCR (qPCR) measurements of Human-specific *Bacteroides* Marker (HBM) are also performed.

*16S rRNA gene amplification for analysis system analysis.* The 16S rDNA is amplified from the gDNA using non-degenerate Bacterial primers 27F.jgi and 1492R. Polymerase chain reaction (PCR) is carried out using the TaKaRa Ex Taq system (Takara Bio Inc, Japan). The amplification protocol is previously described (Brodie et al., Application of a High Density Oligonucleotide Analysis system Approach to Study Bacterial Population Dynamics during Uranium Reduction and Reoxidation. Applied Environ Microbio. 72:6288¬6298, 2006).

*Analysis system processing, and image data analysis.* Analysis system analysis is performed using a high-density phylogenetic analysis system (PhyloChip). The protocols are previously reported (Brodie et al., 2006). Briefly, amplicons are concentrated to a volume less than 40μl by isopropanol precipitation. The DNA amplicons are fragmented with DNAse, biotin labeled, denatured, and hybridized to the DNA analysis system at 48 °C overnight (> 16 hr). The arrays are subsequently washed and stained. Arrays are scanned using a GeneArray Scanner (Affymetrix, Santa Clara, CA, USA). The CEL files obtained from the Affymetrix software that produces information about the fluorescence intensity of each probe (PM, MM, and control probes) are analyzed using the CELanalysis software designed by Todd DeSantis (LBNL, Berkeley, USA).

*PhyloChip data normalization.* All statistical analyses are carried out in R (Team RCD (2008) R: A language and environment for statistical computing)). To correct for variation associated with quantification of amplicon target (quantification variation), and downstream variation associated with target fragmentation, labeling, hybridization, washing, staining and scanning (analysis system technical variation) a two-step normalization procedure is developed: First, for each PhyloChip experiment, a scaling factor best explaining the intensities of the spiked control probes under a multiplicative error model is estimated using a maximum-likelihood procedure. The intensities in each experiment are multiplied with its corresponding optimal scaling factor. In addition, the intensities for each experiment are corrected for the variation in total array intensity by dividing the intensities by its corresponding total array intensity separately for bacteria and archea.

*Statistical Analysis.* All statistical analyses were carried out in R. Bray-Curtis distances were calculated using normalized fluorescence intensity with the *bcdist* function in the *ecodist* package (Goslee SC & Urban DL (2007) The ecodist package for dissimilarity- based analysis of ecological data. J Stat Softw 22(7):1-19). Mantel correlation between Bray- Curtis distance matrices of community data, geographical distance and environmental variables are calculated using the mantel function in the vegan package. Pearson's correlation is calculated with 1000 permutations of the Monte Carlo (randomization) test. Non-metric multidimensional scaling (NMDS) is performed using the *metaMDS* function of the *vegan* package. A relaxed neighbor-joining tree is generated using Clearcut ( Evans J, Sheneman L, & Foster JA (2006) Relaxed neighbor-joining: a fast distance-based phylogenetic tree. Construction method. J Mol Evol 62:785-792.). Separate clearcut trees are generated for the 'resident' and 'transient' communities for each site. Unweighted UniFrac distances (Lozupone C & Knight R (2005) UniFrac: a new phylogenetic method for comparing microbial communities. Applied and Environmental Microbiology 71(12):8228-8235) are calculated for each of the sites.

PhyloChip derived parameters

*Fecal Taxa*. Taxa that are present in all three fecal samples, and in all 27 water samples are tabulated separately. The list of 'Fecal Taxa' is derived by removing those taxa found in all water samples from the taxa that are present in all three fecal samples.

*Transient and resident subpopulations.* Taxa that are present in at least one sample from each site across the sampling period are tabulated and variances of the fluorescence intensities for those taxa are generated. The taxa in the top deciles are defined as the 'transient' subpopulation, and taxa in the bottom deciles were defined as the 'resident' subpopuation.

*BBC:A.* The number of taxa in the classes of Bacilli, Bacteroidetes, Clostridia, and a - proteobacteria are tallied. The ratio is calculated using the following formula:

$$BBC:A = \frac{Bac + Bct + Cls}{A}$$

The count for unique taxa in each of the class is normalized by dividing by the total taxa in each class detected by the analysis system.

Aligned sequences from published studies are downloaded from Greengenes (DeSantis TZ, et al. (2006) Greengenes, a chimera-checked 16S rRNA gene database and workbench compatible with ARB. Applied and Environmental Microbiology 72(7):5069¬5072) and re-classified using PhyloChip taxonomy. The counts of unique taxa are tallied for each Bacterial class. BBC:A are calculated using the formula above. If no taxon is detect for a class, the count for the class is set as 0.5.

Resolving community differences among habitats

Mission Creek samples are delineated into three habitat types: ocean, estuarine lagoon, and fresh water (creeks and storm drain effluent). Bray-Curtis distances of the watershed samples and three fecal samples (two sewage and one human feces) are calculated. Non-metric multidimensional scaling (NMDS) ordination and plotting of the first two axes are used to display the distances between samples. Bacterial communities are clearly separated by habitat types. The drain samples are most similar to the fecal samples. Lagoon samples are most similar to the ocean samples.

Signature taxa that account for the majority of differences in bacterial communities observed between habitats are identified by comparing the detected taxa at the class level among all habitat types. The number of taxas in each habitat type are divided by the total detected for each sample type to obtain a percent detection. Comparing the fecal samples to samples taken above the urban zone or those from the lagoon or ocean show that there are lower fractions of α-proteobacteria and higher fractions of Bacilli and Clostridia. Moreover, five classes are only detected in the fecal samples: Solibacteres, Unclassified Acidobacteria, Chloroflexi-4, Coprothermobacteria and Fusobacteria. Chloroflexi-3 are only detected in creek samples, and Thermomicrobia, Unclassified Termite group 1, and Unclassified Chloroflexi only in the ocean samples. The top 10 classes with the highest standard deviations across the four habitats are (in descending order): Clostridia, α-proteobacteria, Bacilli, γ-proteobacteria, β-proteobacteria, Actinobacteria, Flavobacteria, Bacteroidetes, Cyanobacteria, and c-proteobacteria. Of those classes, Clostridia, Bacilli, and Bacteroidetes fractions are higher, but a-proteobacteria fractions were lower. These four taxa can be used as indicators of fecal contamination.

"Transient" and "resident" subpopulations

Subpopulations of taxa are identified that fluctuate the most between samplings. These are term "transient" populations. Populations that remain stable the sampling period are term "resident" populations. A comparison of taxa found in the "transient" and "resident" subpopulations illustrate differences in community composition from site to site. The six major orders (Enterobacteriales, Lactobacillales, Actinomycetales, Bacteroidales, Clostridiales and Bacillales) of the Fecal Taxa are compared to further dissect the distribution of fecal bacteria over time. The number of transient Enterobacteriales in samples from some sites are extremely high compare to the rest of the sites. While others have high resident subpopulations of Bacillales. Bacteria are identified that are ubiquitous and not affected by changes in the environmental variables measured, as measured by PhyloChip. Bacteria classes that have similar numbers of taxa throughout the watershed and fecal samples included Verrucomicrobiae, Planctomycetacia, α-proteobacteria, Anaerolinaea, Acidobacteria, Sphingobacteria, and Spirochaetes

Bacilli, Bacteroidetes and Clostridia to α-proteobacteria ratio

Four bacterial classes: Bacilli, Bacteroidetes, Clostridia and α-Proteobacteria are identified as having the highest variance among the habitat types and are further developed as fecal indicators.

The combined percentage of Bacilli, Bacteroidetes and Clostridia represent about 20-35% of total classes detected in the fecal samples, whereas their percentages at sites with expected cleaner water such as creek, lagoon and ocean are less than 10-15%. At least 45% of the taxa detected in creek water, lagoon and ocean samples are α-Proteobacteria. These microorganisms were classified as Clean Water Taxa (Table 11) as the percentage of Proteobacteria found in fecal samples is significantly lower at about 35-45%. The ratio of Bacilli, Bacteroidetes and Clostridia to α-proteobacteria (BBC:A) for fecal samples is about 3-5-fold higher than the ratios found in other habitat types. The BBC:A ratios are calculated for each site, and exhibit the same pattern as Fecal Taxa counts across all sites with ocean water having the lowest BBC:A of about 0.75-0.90 with samples close to observed sites of fecal contamination at around 1.50 to about 1.90.

This ratio contains non-coliform associated bacteria, and avoids the potential of false positive fecal detection due to growth of coliforms in the environment. Bacteroidetes and Clostridia are well known fecal-associated anaerobic bacteria. Bacilli are not especially fecal-associated but have been found in aerobic thermophilic swine wastewater bioreactors ( Juteau P, Tremblay D, Villemur R, Bisaillon JG, & Beaudet R (2005) Analysis of the bacterial community inhabiting an aerobic thermophilic sequencing batch reactor (AT-SBR) treating swine waste Applied Microbiology and Biotechnology 66:115-122.). Therefore, the presence of Bacilli, Bacteroides and Clostridiales is a good indication of wastewater-, waste treatment-, and human-derived fecal pollution. α-proteobacteria are mostly phototrophic bacteria that are abundant in the environment, and play key roles in global carbon, sulfur and nitrogen cycles. Many α-proteobacteria thrive under low-nutrient conditions, and will be a good proxy for non-fecal bacteria found in non contaminated aquatic environments.

The results compare well to BBC:A found in other fecal-associated sources that are analyzed by the PhyloChip with mouse cecum, cow colon, sewage contaminated groundwater, human colon, and secondary sewage. These sources have BBC:A of above 1.2. In contrast, anaerobic groundwater has a BBC:A of 0.80-0.99.

To confirm the value of the BBC:A ratio for detecting fecal contamination, published studies of bacterial communities obtained by sequencing are analyzed. Ratios from mammalian guts, anaerobic digester sludge, ocean, Antarctic lake ice, and drinking water also demonstrate that there are differences between fecal and non-fecal samples. Mammalian gut samples have BBC:A ranging from about 10 to about 260. Anaerobic digester sludge samples have BBC:A of at least 1 to about 10. These results may reflect the highly-selected community in anaerobically-digested waste activated sludge in wastewater treatment. Non- fecal samples have BBC:A from 0 to 0.94. The sequencing results confirm that a BBC:A threshold of 1.0 can be used as a cutoff for identifying fecal pollution in water with values of 1 and above indicating polluted water. This method of calculating a BBC:A value offers numerous advantages including speed, as culturing is not required, greater detection ability as it can detect microorganisms that are currently unculturable and also avoids expense and technical problems associated with PCR cloning and high through-put sequencing.

The BBC:A ratio can be used to track the source of fecal pollution as the number usually increases in samples obtained from sites closer to a source of fecal pollution.

<u>Example 3: Fecal Sample Associated Taxa</u>

Three fecal samples (human feces, from Santa Barbara, and two raw sewage, from the influent at the El Estero Wastewater Treatment plant, Santa Barbara, CA) were collected. Water column samples from nine locations were also collected within the Mission Creek and Laguna watersheds in Santa Barbara County, California. Taxa were present, as indicated by analysis using the PhyloChip assay, in all three fecal samples, and in all 27 water samples. The results were tabulated separately.

The list of 503 taxa are shown in Table 4 and was derived by removing those taxa found in all 27 water samples from the taxa that were present in all three fecal samples. These 503 taxa could potentially represent bacteria that are common in the human feces and sewage samples analyzed, but not found in the background environment. The similarity of the whole bacterial community composition to the fecal-associated subpopulation is useful as an indication of fecal pollution.

**Table 4 Fecal Taxa**

| |
|---|
| Bacteria;OD1;OP11-5;Unclassified;Unclassified;sf_1;515 |
| Bacteria;NC10;NC10-1;Unclassified;Unclassified;sf_1;452 |
| Bacteria;Acidobacteria;Acidobacteria-6;Unclassified;Unclassified;sf_1;897 |
| Bacteria;Bacteroidetes;Bacteroidetes;Bacteroidales;Unclassified;sf_15;6233 |
| Bacteria;Bacteroidetes;Bacteroidetes;Bacteroidales;Prevotellaceae;sf_1;6011 |
| Bacteria;Bacteroidetes;Bacteroidetes;Bacteroidales;Porphyromonadaceae;sf_1;5460 |

(continued)

| |
|---|
| Bacteria;Bacteroidetes;Bacteroidetes;Bacteroidales;Prevotellaceae;sf_1;6047 |
| Bacteria;Bacteroidetes;Flavobacteria;Flavobacteriales;Flavobacteriaceae;sf_1;5942 |
| Bacteria;Bacteroidetes;Flavobacteria;Flavobacteriales;Flavobacteriaceae;sf_1;5589 |
| Bacteria;Bacteroidetes;Unclassified;Unclassified;Unclassified;sf_4;5703 |
| Bacteria;Bacteroidetes;Sphingobacteria;Sphingobacteriales;Sphingobacteriaceae;sf_1;5459 |
| Bacteria;Bacteroidetes;Sphingobacteria;Sphingobacteriales;Sphingobacteriaceae;sf_1;5492 |
| Bacteria;Bacteroidetes;Bacteroidetes;Bacteroidales;Bacteroidaceae;sf_6;5792 |
| Bacteria;Bacteroidetes;Sphingobacteria;Sphingobacteriales;Crenotrichaceae;sf_11;5619 |
| Bacteria;Bacteroidetes;Sphingobacteria;Sphingobacteriales;Crenotrichaceae;sf_11;6123 |
| Bacteria;Bacteroidetes;Sphingobacteria;Sphingobacteriales;Flexibacteraceae;sf_19;5667 |
| Bacteria;Chlamydiae;Chlamydiae;Chlamydiales;Chlamydiaceae;sf_1;4820 |
| Bacteria;Cyanobacteria;Cyanobacteria;Chloroplasts;Chloroplasts;sf11;5123 |
| Bacteria;marine group A;mgA-1;Unclassified;Unclassified;sf_1;6408 |
| Bacteria;Spirochaetes;Spirochaetes;Spirochaetales;Spirochaetaceae;sf_1;6502 |
| Bacteria;Spirochaetes;Spirochaetes;Spirochaetales;Spirochaetaceae;sf_1;6494 |
| Bacteria;Spirochaetes;Spirochaetes;Spirochaetales;Spirochaetaceae;sf_1;6583 |
| Bacteria;Spirochaetes;Spirochaetes;Spirochaetales;Spirochaetaceae;sf_1;6476 |
| Bacteria;Spirochaetes;Spirochaetes;Spirochaetales;Spirochaetaceae;sf_1;6490 |
| Bacteria;Spirochaetes;Spirochaetes;Spirochaetales;Spirochaetaceae;sf_1;6506 |
| Bacteria;Spirochaetes;Spirochaetes;Spirochaetales;Spirochaetaceae;sf_1;6571 |
| Bacteria;Proteobacteria;Alphaproteobacteria;Acetobacterales;Roseococcaceae;sf_1;7500 |
| Bacteria;Proteobacteria;Alphaproteobacteria;Acetobacterales;Acetobacteraceae;sf_1;7600 |
| Bacteria;Proteobacteria;Alphaproteobacteria;Azospirillales;Azospirillaceae;sf_1;6959 |
| Bacteria;Proteobacteria;Alphaproteobacteria;Unclassified;Unclassified;sf_6;7312 |
| Bacteria;Proteobacteria;Alphaproteobacteria;Unclassified;Unclassified;sf_2;6697 |
| Bacteria;Proteobacteria;Betaproteobacteria;Neisseriales;Neisseriaceae;sf_1;7675 |
| Bacteria;Proteobacteria;Betaproteobacteria;MND1 clone group;Unclassified;sf_1;7808 |
| Bacteria;Proteobacteria;Betaproteobacteria;Methylophilales;Methylophilaceae;sf_1;8137 |
| Bacteria;Proteobacteria;Betaproteobacteria;Rhodocyclales;Rhodocyclaceae;sf_1;7817 |
| Bacteria;Proteobacteria;Betaproteobacteria;Unclassified;Unclassified;sf_3;8036 |
| Bacteria;Proteobacteria;Betaproteobacteria;Burkholderiales;Alcaligenaceae;sf_1;7768 |
| Bacteria;Proteobacteria;Betaproteobacteria;Burkholderiales;Comamonadaceae;sf_1;7942 |
| Bacteria;Proteobacteria;Betaproteobacteria;Burkholderiales;Comamonadaceae;sf_1;7847 |
| Bacteria;Proteobacteria;Betaproteobacteria;Burkholderiales;Comamonadaceae;sf_1;7941 |
| Bacteria;Proteobacteria;Betaproteobacteria;Unclassified;Unclassified;sf_3;8045 |
| Bacteria;Proteobacteria;Betaproteobacteria;Burkholderiales;Comamonadaceae;sf_1;7745 |
| Bacteria;Proteobacteria;Betaproteobacteria;Burkholderiales;Ralstoniaceae;sf_1;7778 |
| Bacteria;Proteobacteria;Gammaproteobacteria;GAO cluster;Unclassified;sf_1;9059 |
| Bacteria;Proteobacteria;Gammaproteobacteria;Thiotrichales;Thiotrichaceae;sf_3;8741 |

(continued)

| |
|---|
| Bacteria;Proteobacteria;Gammaproteobacteria;uranium waste clones;Unclassified;sf_1;8231 |
| Bacteria;Proteobacteria;Gammaproteobacteria;Oceanospirillales;Oceanospirillaceae;sf_1;8596 |
| Bacteria;Proteobacteria;Gammaproteobacteria;Legionellales;Coxiellaceae;sf_3;9444 |
| Bacteria;Proteobacteria;Gammaproteobacteria;Oceanospirillales;Alcanivoraceae;sf_1;9658 |
| Bacteria;Proteobacteria;Gammaproteobacteria;Pseudomonadales;Pseudomonadaceae;sf_1;8601 |
| Bacteria;Proteobacteria;Gammaproteobacteria;Unclassified;Unclassified;sf_3;8959 |
| Bacteria;Proteobacteria;Gammaproteobacteria;Alteromonadales;Alteromonadaceae;sf_1;9486 |
| Bacteria;Proteobacteria;Gammaproteobacteria;Alteromonadales;Alteromonadaceae;sf_1;8863 |
| Bacteria;Proteobacteria;Gammaproteobacteria;Alteromonadales;Alteromonadaceae;sf_1;9501 |
| Bacteria;Proteobacteria;Gammaproteobacteria;Alteromonadales;Shewanellaceae;sf_1;8581 |
| Bacteria;Proteobacteria;Gammaproteobacteria;Pasteurellales;Pasteurellaceae;sf_1;9237 |
| Bacteria;Proteobacteria;Gammaproteobacteria;Enterobacteriales;Enterobacteriaceae;sf_1;8554 |
| Bacteria;Proteobacteria;Gammaproteobacteria;Enterobacteriales;Enterobacteriaceae;sf_1;8885 |
| Bacteria;Proteobacteria;Gammaproteobacteria;Enterobacteriales;Enterobacteriaceae;sf_1;8700 |
| Bacteria;Proteobacteria;Gammaproteobacteria;Enterobacteriales;Enterobacteriaceae;sf_1;8529 |
| Bacteria;Proteobacteria;Gammaproteobacteria;Enterobacteriales;Enterobacteriaceae;sf_1;8770 |
| Bacteria;Proteobacteria;Gammaproteobacteria;Enterobacteriales;Enterobacteriaceae;sf_1;8225 |
| Bacteria; Unctassified;Unclassified;Unclassified;Unclassified;sf_160;10012 |
| Bacteria;Proteobacteria;Deltaproteobacteria;Desulfovibrionales;Desulfovibrionaceae;sf_1;10189 |
| Bacteria;Proteobacteria;Deltaproteobacteria;Syntrophobacterales;Syntrophaceae;sf_3;9665 |
| Bacteria;Proteobacteria;Epsilonproteobacteria;Campylobacterales;Helicobacteraceae;sf_23;10443 |
| Bacteria;Proteobacteria;Epsilonproteobacteria;Campylobacterales;Helicobacteraceae;sf_3;10576 |
| Bacteria;Proteobacteria;Epsilonproteobacteria;Campylobacterales;Unclassified;sf_1;10407 |
| Bacteria;Gemmatimonadetes;Unclassified;Unclassified;Unclassified;sf_5;317 |
| Bacteria;Actinobacteria;Actinobacteria;Rubrobacterales;Rubrobacteraceae;sf_1;1551 |
| Bacteria;Actinobacteria;Actinobacteria;Acidimicrobiales;Unclassified;sf_1;1666 |
| Bacteria;Actinobacteria;BD2-10 group;Unclassified;Unclassified;sf_2;1652 |
| Bacteria;Actinobacteria;Actinobacteria;Actinomycetales;Unclassified;sf_3;2045 |
| Bacteria;Actinobacteria;Actinobacteria;Actinomycetales;Cellulomonadaceae;sf_1;1748 |
| Bacteria;Actinobacteria;Actinobacteria;Actinomycetales;Actinomycetaceae;sf_1;1684 |
| Bacteria;Actinobacteria;Actinobacteria;Bifidobacteriales;Bifidobacteriaceae;sf_1;1444 |
| Bacteria;Actinobacteria;Actinobacteria;Actinomycetales;Kineosporiaceae;sf_1;1598 |
| Bacteria;Actinobacteria;Actinobacteria;Actinomycetales;Kineosporiaceae;sf_1;1961 |
| Bacteria;Actinobacteria;Actinobacteria;Actinomycetales;Corynebacteriaceae;sf_1;1517 |
| Bacteria;Actinobacteria;Actinobacteria;Actinomycetales;Corynebacteriaceae;sf_1;1803 |
| Bacteria;Actinobacteria;Actinobacteria;Actinomycetales;Dietziaceae;sf_1;1970 |
| Bacteria;Firmicutes;Unclassified;Unclassified;Unclassified;sf_8;2433 |
| Bacteria;Firmicutes;Clostridia;Unclassified;Unclassified;sf_4;2398 |
| Bacteria;Chloroflexi;Dehalococcoidetes;Unclassified;Unclassified;sf_1;2339 |

(continued)

| |
|---|
| Bacteria;Chloroflexi;Dehalococcoidetes;Unclassified;Unclassified;sf_1;2497 |
| Bacteria;Firmicutes;Clostridia;Clostridiales;Peptococc/Acidaminococc;sf_11;709 |
| Bacteria;Firmicutes;Clostridia;Clostridiales;Peptococc/Acidaminococc;sf_11;242 |
| Bacteria;Firmicutes;Clostridia;Clostridiales;Lachnospiraceae;sf_5;3042 |
| Bacteria;Firmicutes;Clostridia;Clostridiales;Lachnospiraceae;sf_5;3076 |
| Bacteria;Firmicutes;Clostridia;Clostridiales;Lachnospiraceae;sf_5;3171 |
| Bacteria;Firmicutes;Clostridia;Clostridiales;Lachnospiraceae;sf_5;2681 |
| Bacteria;Firmicutes;Clostridia;Clostridiales;Peptostreptococcaceae;sf_5;2721 |
| Bacteria;Firmicutes;Clostridia;Clostridiales;Peptostreptococcaceae;sf_5;2796 |
| Bacteria;Firmicutes;Clostridia;Clostridiales;Clostridiaceae;sf_12;2915 |
| Bacteria;TM7;Unclassified;Unclassified;Unclassified;sf_1;3025 |
| Bacteria;Firmicutes;Bacilli;Bacillales;Paenibacillaceae;sf_1;3299 |
| Bacteria;Firmicutes;Bacilli;Bacillales;Halobacillaceae;sf_1;3344 |
| Bacteria;Firmicutes;Bacilli;Lactobacillales;Streptococcaceae;sf_1;3722 |
| Bacteria;Firmicutes;Mollicutes;Acholeplasmatales;Acholeplasmataceae;sf_1;3976 |
| Bacteria;Acidobacteria;Unclassified;Unclassified;Unclassified;sf_1;4222 |
| Bacteria;Firmicutes;Clostridia;Clostridiales;Clostridiaceae;sf_12;4406 |
| Bacteria;Firmicutes;Clostridia;Clostridiales;Lachnospiraceae;sf_5;4212 |
| Bacteria;Firmicutes;Clostridia;Clostridiales;Clostridiaceae;sf_12;4359 |
| Bacteria;Firmicutes;Clostridia;Clostridiales;Clostridiaceae;sf_12;4475 |
| Bacteria;Unclassified;Unclassified;Unclassified;Unclassified;sf_160;4410 |
| Bacteria;Firmicutes;Clostridia;Clostridiales;Clostridiaceae;sf_12;4306 |
| Bacteria;Firmicutes;Clostridia;Clostridiales;Clostridiaceae;sf_12;4427 |
| Bacteria;Firmicutes;Clostridia;Clostridiales;Clostridiaceae;sf_12;4296 |
| Bacteria;Verrucomicrobia;Verrucomicrobiae;Verrucomicrobiales;Verrucomicrobia subdivision 7;sf_1;559 |
| Bacteria;Bacteroidetes;Flavobacteria;Flavobacteriales;Flavobacteriaceae;sf_1;6200 |
| Bacteria;Proteobacteria;Betaproteobacteria;Burkholderiales;Comamonadaceae;sf_1;7971 |
| Bacteria;Verrucomicrobia;Verrucomicrobiae;Verrucomicrobiales;Verrucomicrobia subdivision 5;sf_1;533 |
| Bacteria;Verrucomicrobia;Unclassified;Unclassified;Unclassified;sf_4;288 |
| Bacteria;Bacteroidetes;Bacteroidetes;Bacteroidales;Bacteroidaceae;sf_12;5320 |
| Bacteria;Bacteroidetes;Bacteroidetes;Bacteroidales;Bacteroidaceae;sf_12;5950 |
| Bacteria;Bacteroidetes;Bacteroidetes;Bacteroidales;Prevotellaceae;sf_1;5905 |
| Bacteria;Cyanobacteria;Cyanobacteria;Nostocales;Unclassified;sf_1;5047 |
| Bacteria;Cyanobacteria;Cyanobacteria;Nostocales;Unclassified;sf_1;5072 |
| Bacteria;Cyanobacteria;Cyanobacteria;Nostocales;Unclassified;sf_1;5191 |
| Bacteria;Cyanobacteria;Cyanobacteria;Nostocales;Unclassified;sf_1;5199 |
| Bacteria;BRCI;Unclassified;Unclassified;Unclassified;sf_1;5051 |
| Bacteria;Cyanobacteria;Cyanobacteria;Chloroplasts;Chloroplasts;sf_5;5130 |
| Bacteria;Unclassified;Unclassified;Unclassified;Unclassified;sf_160;6337 |

(continued)

| |
|---|
| Bacteria;Unclassified;Unclassified;Unclassified;Unclassified;sf_160;6360 |
| Bacteria;Acidobacteria;Acidobacteria;Acidobacteriales;Acidobacteriaceae;sf_14;6425 |
| Bacteria;Spirochaetes;Spirochaetes;Spirochaetales;Spirochaetaceae;sf_1;6529 |
| Bacteria;Proteobacteria;Alphaproteobacteria;Acetobacterales;Acetobacteraceae;sf_1;7529 |
| Bacteria;Proteobacteria;Betaproteobacteria;Unclassified;Unclassified;sf_3;8007 |
| Bacteria;Proteobacteria;Betaproteobacteria;MND1 clone group;Unclassified;sf_1;7993 |
| Bacteria;Proteobacteria;Gammaproteobacteria;Alteromonadales;Alteromonadaceae;sf_1;9491 |
| Bacteria;Proteobacteria;Gammaproteobacteria;Alteromonadales;Shewanellaceae;sf_1;8201 |
| Bacteria;Proteobacteria;Gammaproteobacteria;Pasteurellales;Pasteurellaceae;sf_1;8409 |
| Bacteria;Proteobacteria;Gammaproteobacteria;Enterobacteriales;Enterobacteriaceae;sf_1;9363 |
| Bacteria;Proteobacteria;Gammaproteobacteria;Enterobacteriales;Enterobacteriaceae;sf_1;8934 |
| Bacteria;Proteobacteria;Gammaproteobacteria;Enterobacteriales;Enterobacteriaceae;sf_1;8467 |
| Bacteria;Proteobacteria;Gammaproteobacteria;Enterobacteriales;Enterobacteriaceae;sf_1;8530 |
| Bacteria;Proteobacteria;Gammaproteobacteria;Enterobacteriales;Enterobacteriaceae;sf_1;9390 |
| Bacteria;Proteobacteria;Gammaproteobacteria;Enterobacteriales;Enterobacteriaceae;sf_1;8251 |
| Bacteria;Proteobacteria;Gammaproteobacteria;Enterobacteriales;Enterobacteriaceae;sf_1;8890 |
| Bacteria;Proteobacteria;Gammaproteobacteria;Enterobacteriales;Enterobacteriaceae;sf_1;8362 |
| Bacteria;Proteobacteria;Gammaproteobacteria;Enterobacteriales;Enterobacteriaceae;sf_1;8510 |
| Bacteria;Proteobacteria;Gammaproteobacteria;Enterobacteriales;Enterobacteriaceae;sf_1;8711 |
| Bacteria;Proteobacteria;Gammaproteobacteria;Enterobacteriales;Enterobacteriaceae;sf_1;8712 |
| Bacteria;Proteobacteria;Gammaproteobacteria;Enterobacteriales;Enterobacteriaceae;sf_1;8739 |
| Bacteria;Proteobacteria;Gammaproteobacteria;Enterobacteriales;Enterobacteriaceae;sf_1;9417 |
| Bacteria;Proteobacteria;Gammaproteobacteria;Enterobacteriales;Enterobacteriaceae;sf_1;8473 |
| Bacteria;Proteobacteria;Deltaproteobacteria;Myxococcales;Polyangiaceae;sf_3;10082 |
| Bacteria;Proteobacteria;Deltaproteobacteria;Syntrophobacterales;Syntrophobacteraceae;sf_1;9864 |
| Bacteria;Proteobacteria;Deltaproteobacteria;Syntrophobacterales;Syntrophobacteraceae;sf_1;9731 |
| Bacteria;Actinobacteria;Actinobacteria;Actinomycetales;Frankiaceae;sf_1;1286 |
| Bacteria;Actinobacteria;Actinobacteria;Actinomycetales;Dietziaceae;sf_1;1872 |
| Bacteria;Chloroflexi;Dehalococcoidetes;Unclassified;Unclassifled;sf_1;2397 |
| Bacteria;Chloroflexi;Unclassified;Unclassified;Unclassified;sf_1;2534 |
| Bacteria;Firmicutes;Clostridia;Clostridiales;Peptococc/Acidaminococc;sf_11;710 |
| Bacteria;Firmicutes;Clostridia;Clostridiales;Peptococc/Acidaminococc;sf_11;300 |
| Bacteria;Firmicutes;Clostridia;Clostridiales;Lachnospiraceae;sf_5;3218 |
| Bacteria;Firmicutes;Catabacter;Unclassified;Unclassified;sf_4;2716 |
| Bacteria;Firmicutes;Clostridia;Clostridiales;Peptostreptococcaceae;sf_5;2679 |
| Bacteria;Firmicutes;Clostridia;Clostridiales;Peptostreptococcaceae;sf_5;2714 |
| Bacteria;Firmicutes;Clostridia;Clostridiales;Peptostreptococcaceae;sf_5;2722 |
| Bacteria;Firmicutes;Clostridia;Clostridiales;Peptostreptococcaceae;sf_5;2993 |
| Bacteria;Firmicutes;Clostridia;Clostridiales;Clostridiaceae;sf_12;3021 |

(continued)

| |
|---|
| Bacteria;Firmicutes;Bacilli;Lactobacillales;Carnobacteriaceae;sf_1;3536 |
| Bacteria;Firmicutes;Bacilli;Lactobacillales;Streptococcaceae;sf_1;3869 |
| Bacteria;Firmicutes;Bacilli;Lactobacillales;Streptococcaceae;sf_1;3588 |
| Bacteria;Firmicutes;Mollicutes;Anaeroplasmatales;Erysipelotrichaceae;sf_3;3981 |
| Bacteria;Firmicutes;Catabacter;Unclassified;Unclassified;sf_1;4261 |
| Bacteria;Firmicutes;Clostridia;Clostridiales;Lachnospiraceae;sf_5;4571 |
| Bacteria;Firmicutes;Clostridia;Clostridiales;Lachnospiraceae;sf_5;4623 |
| Bacteria;Firmicutes;Clostridia;Clostridiales;Clostridiaceae;sf_12;4589 |
| Bacteria;Bacteroidetes;Bacteroidetes;Bacteroidales;Unaassified;sf_15;5511 |
| Bacteria;Proteobacteria;Gammaproteobacteria;Enterobacteriales;Enterobacteriaceae;sf_1;8286 |
| Bacteria;Proteobacteria;Deltaproteobacteria;Desulfobacterales;Desulfobacteraceae;sf_5;10136 |
| Bacteria;Aquificae;Aquificae;Aquificales;Unclassified;sf_1;2364 |
| Bacteria;Verrucomicrobia;Verrucomicrobiae;Verrucomicrobiales;Verrucomicrobiaceae;sf_6;871 |
| Bacteria;Verrucomicrobia;Verrucomicrobiae;Verrucomicrobiales;Verrucomicrobiaceae;sf_1;1024 |
| Bacteria;Bacteroidetes;Sphingobacteria;Sphingobacteriales;Crenotrichaceae;sf_11;5334 |
| Bacteria;Chloroflexi;Anaerolineae;Unclassified;Unclassified;sf_9;72 |
| Bacteria;Cyanobacteria;Cyanobacteria;Nostocales;Unclassified;sf_1;5004 |
| Bacteria;Acidobacteria;Solibacteres;Unclassified;Unclassified;sf_1;6426 |
| Bacteria;Spirochaetes;Spirochaetes;Spirochaetales;Spirochaetaceae;sf_1;6507 |
| Bacteria;Spirochaetes;Spirochaetes;Spirochaetales;Spirochaetaceae;sf_1;6460 |
| Bacteria;Spirochaetes;Spirochaetes;Spirochaetales;Spirochaetaceae;sf_1;6579 |
| Bacteria;Spirochaetes;Spirochaetes;Spirochaetales;Leptospiraceae;sf_3;6470 |
| Bacteria;Proteobacteria;Alphaproteobacteria;Unclassified;Unclassified;sf_6;7647 |
| Bacteria;Proteobacteria;Betaproteobacteria;Nitrosomonadales;Nitrosomonadaceae;sf_1;8145 |
| Bacteria;Proteobacteria;Betaproteobacteria;Burkholderiales;Comamonadaceae;sf_1;7822 |
| Bacteria;Proteobacteria;Betaproteobacteria;Unclassified;Unclassified;sf_3;7954 |
| Bacteria;Proteobacteria;Gammaproteobacteria;Thiotrichales;Thiotrichaceae;sf_3;8321 |
| Bacteria;Proteobacteria;Gammaproteobacteria;Thiotrichales;Francisellaceae;sf_1;9554 |
| Bacteria;Proteobacteria;Gammaproteobacteria;Xanthomonadales;Xanthomonadaceae;sf_3;8983 |
| Bacteria;Proteobacteria;Gammaproteobacteria;Legionellales;Coxiellaceae;sf_3;8969 |
| Bacteria;Proteobacteria;Gammaproteobacteria;Oceanospirillales;Halomonadaceae;sf_1;8598 |
| Bacteria;Proteobacteria;Gammaproteobacteria;Alteromonadales;Alteromonadaceae;sf_1;9236 |
| Bacteria;Proteobacteria;Gammaproteobacteria;Enterobacteriales;Enterobacteriaceae;sf_1;8742 |
| Bacteria;Proteobacteria;Gammaproteobacteria;Enterobacteriales;Enterobacteriaceae;sf_1;9135 |
| Bacteria;Proteobacteria;Gammaproteobacteria;Enterobacteriales;Enterobacteriaceae;sf_1;9496 |
| Bacteria;Proteobacteria;Gammaproteobacteria;Enterobacteriales;Enterobacteriaceae;sf_1;8886 |
| Bacteria;Proteobacteria;Gammaproteobacteria;Enterobacteriales;Enterobacteriaceae;sf_1;9651 |
| Bacteria;Proteobacteria;Gammaproteobacteria;Enterobacteriales;Enterobacteriaceae;sf_1;8379 |
| Bacteria;Proteobacteria;Gammaproteobacteria;Enterobacteriales;Enterobacteriaceae;sf_1;9142 |

(continued)

| |
|---|
| Bacteria;Proteobacteria;Gammaproteobacteria;Enterobacteriales;Enterobacteriaceae;sf_1;9345 |
| Bacteria;Proteobacteria;Gammaproteobacteria;Enterobacteriales;Enterobacteriaceae;sf1;8282 |
| Bacteria;Proteobacteria;Gammaproteobacteria;Enterobacteriales;Unclassified;sf_1;8430 |
| Bacteria;Proteobacteria;Gammaproteobacteria;Enterobacteriales;Enterobacteriaceae;sf_1;8505 |
| Bacteria;Proteobacteria;Gammaproteobacteria;Enterobacteriales;Enterobacteriaceae;sf_1;8528 |
| Bacteria;Proteobacteria;Gammaproteobacteria;Enterobacteriales;Enterobacteriaceae;sf_1;8936 |
| Bacteria;Proteobacteria;Gammaproteobacteria;Enterobacteriales;Enterobacteriaceae;sf_1;9060 |
| Bacteria;Proteobacteria;Gammaproteobacteria;Enterobacteriales;Enterobacteriaceae;sf_1;9274 |
| Bacteria;Proteobacteria;Deltaproteobacteria;Desulfovibrionales;Desulfovibrionaceae;sf_1;10212 |
| Bacteria;Proteobacteria;Deltaproteobacteria;EB1021 group;Unclassified;sf_4;10024 |
| Bacteria;Proteobacteria;Epsilonproteobacteria;Campylobacterales;Campylobacteraceae;sf_3;10397 |
| Bacteria;Actinobacteria;Actinobacteria;Acidimicrobiales;Microthrixineae;sf_1;1576 |
| Bacteria;Actinobacteria;Actinobacteria;Actinomycetales;Pseudonocardiaceae;sf_1;1863 |
| Bacteria;Firmicutes;Clostridia;Clostridiales;Clostridiaceae;sf_12;252 |
| Bacteria;Firmicutes;Clostridia;Clostridiales;Lachnospiraceae;sf_5;2709 |
| Bacteria;Firmicutes;Clostridia;Clostridiales;Lachnospiraceae;sf_5;3060 |
| Bacteria;Firmicutes;Clostridia;Clostridiales;Peptostreptococcaceae;sf_5;2729 |
| Bacteria;Firmicutes;Bacilli;Bacillales;Bacillaceae;sf1;234 |
| Bacteria;Firmicutes;Bacilli;Bacillales;Bacillaceae;sf_1;3460 |
| Bacteria;Firmicutes;Bacilli;Bacillales;Halobacillaceae;sf_1;3769 |
| Bacteria;Firmicutes;Bacilli;Bacillales;Bacillaceae;sf_1;3900 |
| Bacteria;Firmicutes;Bacilli;Bacillales;Caryophanaceae;sf_1;3285 |
| Bacteria;Firmicutes;Bacilli;Lactobacillales;Lactobacillaceae;sf_1;3768 |
| Bacteria;Firmicutes;Mollicutes;Acholeplasmatales;Acholeplasmataceae;sf_1;4044 |
| Bacteria;Firmicutes;Mollicutes;Acholeplasmatales;Acholeplasmataceae;sf_1;4045 |
| Bacteria;Firmicutes;Mollicutes;Anaeroplasmatales;Erysipelotrichaceae;sf_3;3965 |
| Bacteria;Firmicutes;Clostridia;Clostridiales;Clostridiaceae;sf_12;4614 |
| Bacteria;Fimicutes;Clostridia;Clostridiales;Clostridiaceae;sf_12;4415 |
| Bacteria;Firmicutes;Clostridia;Clostridiales;Clostridiaceae;sf_12;4548 |
| Bacteria;Firmicutes;Clostridia;Clostridiales;Clostridiaceae;sf_12;4555 |
| Bacteria;Nitrospira;Nitrospira;Nitrospirales;Nitrospiraceae;sf_2;542 |
| Bacteria;Nitrospira;Nitrospira;Nitrospirales;Nitrospiraceae;sf_2;697 |
| Bacteria;Natronoanaerobium;Unclassified;Unclassified;Unclassified;sf_1;769 |
| Bacteria;Acidobacteria;Acidobacteria-4;Ellin6075/11-25;Unclassified;sf_1;435 |
| Bacteria;Bacteroidetes;Bacteroidetes;Bacteroidales;Prevotellaceae;sf_1;5484 |
| Bacteria;Cyanobacteria;Cyanobacteria;Pseudanabaena;Unclassified;sf_1;5008 |
| Bacteria;marine group A;mgA-1;Unclassified;Unclassified;sf 1;6454 |
| Bacteria;Proteobacteria;Alphaproteobacteria;Verorhodospirilla;Unclassified;sf_1;7109 |
| Bacteria;Proteobacteria;Alphaproteobacteria;Bradyrhizobiales;Beijerinck/Rhodoplan/Methylocyst;sf_3;7401 |

(continued)

| |
|---|
| Bacteria;Proteobacteria;Betaproteobacteria;Rhodocyclales;Rhodocyclaceae;sf_1;7951 |
| Bacteria;Proteobacteria;Gammaproteobacteria;Thiotrichales;Thiotrichaceae;sf_3;9321 |
| Bacteria;Proteobacteria;Gammaproteobacteria;Oceanospirillales;Halomonadaceae;sf_1;8317 |
| Bacteria;Proteobacteria;Gammaproteobacteria;Pseudomonadales;Moraxellaceae;sf_3;9359 |
| Bacteria;Proteobacteria;Gammaproteobacteria;Alteromonadales;Alteromonadaceae;sf_1;8533 |
| Bacteria;Proteobacteria;Gammaproteobacteria;Enterobacteriales;Enterobacteriaceae;sf_1;9358 |
| Bacteria;Proteobacteria;Gammaproteobacteria;Enterobacteriales;Enterobacteriaceae;sf_1;9302 |
| Bacteria;Proteobacteria;Gammaproteobacteria;Enterobacteriales;Enterobacteriaceae;sf_1;8603 |
| Bacteria;Proteobacteria;Gammaproteobacteria;Enterobacteriales;Enterobacteriaceae;sf_1;9265 |
| Bacteria;Proteobacteria;Deltaproteobacteria;Myxococcales;Unclassified;sf_1;10259 |
| Bacteria;Proteobacteria;Deltaproteobacteria;Unclassified;Unclassified;sf_7;10048 |
| Bacteria;Proteobacteria;Deltaproteobacteria;EB1021 group;Unclassified;sf4;9741 |
| Bacteria;Chloroflexi;Chloroflexi-4;Unclassified;Unclassified;sf2;2344 |
| Bacteria;Firmicutes;Clostridia;Clostridiales;Peptococc/Acidaminococc;sf_11;39 |
| Bacteria;Firmicutes;Clostridia;Clostridiales;Lachnospiraceae;sf_5;3036 |
| Bacteria;Firmicutes;Clostridia;Clostridiales;Lachnospiraceae;sf_5;2825 |
| Bacteria;Firmicutes;Clostridia;Clostridiales;Peptostreptococcaceae;sf_5;58 |
| Bacteria;Firmicutes;Bacilli;Lactobacillales;Lactobacillaceae;sf_1;3566 |
| Bacteria;Firmicutes;Bacilli;Lactobacillales;Streptococcaceae;sf_1;3251 |
| Bacteria;Firmicutes;Mollicutes;Anaeroplasmatales;Erysipelotrichaceae;sf_3;768 |
| Bacteria;Firmicutes;Clostridia;Clostridiales;Clostridiaceae;sf_12;4297 |
| Bacteria;Firmicutes;Clostridia;Clostridiales;Clostridiaceae;sf_12;4299 |
| Bacteria;Firmicutes;Clostridia;Clostridiales;Clostridiaceae;sf12;4502 |
| Bacteria;Firmicutes;Clostridia;Clostridiales;Clostridiaceae;sf12;4554 |
| Bacteria;Firmicutes;Clostridia;Clostridiales;Clostridiaceae;sf_12;4157 |
| Bacteria;Firmicutes;Clostridia;Clostridiales;Clostridiaceae;sf12;4267 |
| Bacteria;Bacteroidetes;Bacteroidetes;Bacteroidales;Porphyromonadaceae;sf_1;5961 |
| Bacteria;Bacteroidetes;Bacteroidetes;Bacteroidales;Prevotellaceae;sf_1;5916 |
| Bacteria;Bacteroidetes;Flavobacteria;Flavobacteriales;Flavobacteriaceae;sf_1;5473 |
| Bacteria;Cyanobacteria;Cyanobacteria;Nostocales;Unclassified;sf_1;5028 |
| Bacteria;Cyanobacteria;Cyanobacteria;Nostocales;Unclassified;sf_1;5174 |
| Bacteria;Cyanobacteria;Cyanobacteria;Nostocales;Unclassified;sf_1;5175 |
| Bacteria;TM7;Unclassified;Unclassified;Unclassified;sf_1;5061 |
| Bacteria;Proteobacteria;Betaproteobacteria;Burkholderiales;Burkholderiaceae;sf_1;7782 |
| Bacteria;Proteobacteria;Gammaproteobacteria;Chromatiales;Unclassified;sf_1;9282 |
| Bacteria;Proteobacteria;Gammaproteobacteria;Oceanospirillales;Halomonadaceae;sf_1;8854 |
| Bacteria;Proteobacteria;Gammaproteobacteria;Pseudomonadales;Pseudomonadaceae;sf_1;8209 |
| Bacteria;Proteobacteria;Gammaproteobacteria;Enterobacteriales;Enterobacteriaceae;sf_6;8783 |
| Bacteria;Firmicutes;Clostridia;Clostridiales;Peptococc/Acidaminococc;sf_11;304 |

(continued)

| |
|---|
| Bacteria;Firmicutes;Clostridia;Clostridiales;Peptococc/Acidaminococc;sf_11;131 |
| Bacteria;Firmicutes;Clostridia;Clostridiales;Clostridiaceae;sf_12;206 |
| Bacteria;Firmicutes;Clostridia;Clostridiales;Lachnospiraceae;sf_5;2834 |
| Bacteria;Firmicutes;Clostridia;Clostridiales;Lachnospiraceae;sf_5;2844 |
| Bacteria;Firmicutes;Clostridia;Clostridiales;Peptostreptococcaceae;sf_5;2694 |
| Bacteria;Firrmicutes;Clostridia;Clostridiales;Peptostreptococcaceae;sf_5;3080 |
| Bacteria;Firmicutes;Clostridia;Clostridiales;Peptostreptococcaceae;sf_5;3182 |
| Bacteria;Firmicutes;Clostridia;Clostridiales;Peptostreptococcaceae;sf_5;619 |
| Bacteria;Firmicutes;Bacilli;Bacillales;Bacillaceae;sf_1;305 |
| Bacteria;Firmicutes;Bacilli;Bacillales;Bacillaceae;sf_1;3836 |
| Bacteria;Firmicutes;Bacilli;Bacillales;Bacillaceae;sf_1;462 |
| Bacteria;Firmicutes;Bacilli;Bacillales;Bacillaceae;sf_1;3831 |
| Bacteria;Firmicutes;Bacilli;Lactobacillales;Enterococcaceae;sf_1;3288 |
| Bacteria;Firmicutes;Bacilli;Lactobaciliales;Enterococcaceae;sf_1;3598 |
| Bacteria;Firmicutes;Mollicutes;Acholeplasmatales;Acholeplasmataceae;sf_1;3961 |
| Bacteria;Firmicutes;Mollicutes;Acholeplasmatales;Acholeplasmataceae;sf_1;3975 |
| Bacteria;Firmicutes;Mollicutes;Anaeroplasmatales;Erysipelotrichaceae;sf_3;3952 |
| Bacteria;Firmicutes;Clostridia;Clostridiales;Clostridiaceae;sf_12;4584 |
| Bacteria;Firmicutes;Clostridia;Clostridiales;Clostridiaceae;sf_12;4459 |
| Bacteria;Firmicutes;Clostridia;Clostridiales;Lachnospiraceae;sf_5;4533 |
| Bacteria;Firmicutes;Clostridia;Clostridiales;Lachnospiraceae;sf_5;4539 |
| Bacteria;Firmicutes;Clostridia;Clostridiales;Clostridiaceae;sf_12;4637 |
| Bacteria;Firmicutes;Catabacter;Unclassified;Unclassified;sf_4;4526 |
| Bacteria;Firmicutes;Clostridia;Clostridiales;Clostridiaceae;sf_12;4560 |
| Bacteria;Firmicutes;Clostridia;Clostridiales;Clostridiaceae;sf_12;4310 |
| Bacteria;Proteobacteria;Betaproteobacteria;Burkholderiales;Unclassified;sf_1;7879 |
| Bacteria;Proteobacteria;Gammaproteobacteria;Xanthomonadales;Xanthomonadaceae;sf_3;921 |
| Bacteria;Proteobacteria;Gammaproteobacteria;Alteromonadales;Pseudoalteromonadaceae;sf_1;9339 |
| Bacteria;Proteobacteria;Deltaproteobacteria;Myxococcales;Polyangiaceae;sf_3;10065 |
| Bacteria;Proteobacteria;Deltaproteobacteria;Unclassified;Unclassified;sf_9;9738 |
| Bacteria;Proteobacteria;Epsilonproteobacteria;Campylobacterales;Helicobacteraceae;sf_3;10572 |
| Bacteria;Actinobacteria;Actinobacteria;Actinomycetales;Nocardiopsaceae;sf_1;1385 |
| Bacteria;Firmicutes;Clostridia;Clostridiales;Peptococc/Acidaminococc;sf_11;71 |
| Bacteria;Firmicutes;Clostridia;Clostridiales;Lachnospiraceae;sf_5;3059 |
| Bacteria;TM7;TM7-3;Unclassified;Unclassified;sf_1;2697 |
| Bacteria;Firmicutes;Bacilli;Bacillales;Paenibacillaceae;sf_1;3630 |
| Bacteria;Firmicutes;Bacilli;Bacillales;Bacillaceae;sf_1;3424 |
| Bacteria;Firmicutes;Bacilli;Bacillales;Bacillaceae;sf_1;3661 |
| Bacteria;Firmicutes;Bacilli;Bacillales;Bacillaceae;sf_1;283 |

(continued)

| |
|---|
| Bacteria;Firmicutes;Bacilli;Bacillales;Bacillaceae;sf_1;829 |
| Bacteria;Firmicutes;Bacilli;Bacillales;Bacillaceae;sf_1;3675 |
| Bacteria;Firmicutes;Mollicutes;Entomoplasmatales;Entomoplasmataceae;sf_1;4074 |
| Bacteria;Firmicutes;Clostridia;Clostridiales;Clostridiaceae;sf_12;4156 |
| Bacteria;Firmicutes;Clostridia;Clostridiales;Clostridiaceae;sf_12;4575 |
| Bacteria;Proteobacteria;Gammaproteobacteria;Enterobacteriales;Enterobacteriaceae;sf_1;8631 |
| Bacteria;Proteobacteria;Epsilonproteobacteria;Campylobacterales;Helicobacteraceae;sf_3;10534 |
| Bacteria;Nitrospira;Nitrospira;Nitrospirales;Nitrospiraceae;sf_1;179 |
| Bacteria;Verrucomicrobia;Verrucomicrobiae;Verrucomicrobiales;Verrucomicrobiasubdivision7;sf_1;446 |
| Bacteria;Bacteroidetes;Sphingobacteria;Sphingobacteriales;Sphingobacteriaceae;sf_1;6272 |
| Bacteria;Spirochaetes;Spirochaetes;Spirochaetales;Spirochaetaceae;sf_1;6487 |
| Bacteria;Spirochaetes;Spirochaetes;Spirochaetales;Spirochaetaceae;sf_1;6554 |
| Bacteria;Proteobacteria;Betaproteobacteria;Nitrosomonadales;Nitrosomonadaceae;sf_1;7931 |
| Bacteria;Firmicutes;Clostridia;Clostridiales;Lachnospiraceae;sf_5;3111 |
| Bacteria;Firmicutes;Clostridia;Clostridiales;Lachnospiraceae;sf_5;2693 |
| Bacteria;Firmicutes;Clostridia;Clostridiales;Peptostreptococcaceae;sf_5;2913 |
| Bacteria;Firmicutes;Clostridia;Clostridiales;Peptostreptococcaceae;sf_5;1037 |
| Bacteria;Firmicutes;Bacilli;Bacillales;Sporolactobacillaceae;sf_1;3365 |
| Bacteria;Firmicutes;Bacilli;Bacillales;Bacillaceae;sf_1;3419 |
| Bacteria;Firmicutes;Bacilli;Bacillales;Halobacillaceae;sf_1;3756 |
| Bacteria;Firmicutes;Bacilli;Bacillales;Halobacillaceae;sf_1;3849 |
| Bacteria;Firmicutes;Bacilli;Lactobacillales;Enterococcaceae;sf_1;3881 |
| Bacteria;Firmicutes;Bacilli;Lactobacillales;Streptococcaceae;sf_1;3629 |
| Bacteria;Firmicutes;Mollicutes;Anaeroplasmatales;Erysipelotrichaceae;sf_3;144 |
| Bacteria;Firmicutes;Clostridia;Clostridiales;Clostridiaceae;sf_12;4632 |
| Bacteria;Bacteroidetes;Flavobacteria;Flavobacteriales;Flavobacteriaceae;sf_1;5509 |
| Bacteria;Proteobacteria;Deltaproteobacteria;Myxococcales;Polyangiaceae;sf_3;9912 |
| Bacteria;NC10;NC10-1;Unclassified;Unclassified;sf_1;536 |
| Bacteria;Proteobacteria;Gammaproteobacteria;Enterobacteriales;Enterobacteriaceae;sf_1;8640 |
| Bacteria;Actinobacteria;Actinobacteria;Actinomycetales;Unclassified;sf_4;1337 |
| Bacteria;Actinobacteria;Actinobacteria;Actinomycetales;Kineosporiaceae;sf_1;1087 |
| Bacteria;Firmicutes;Clostridia;Clostridiales;Clostridiaceae;sf_12;2786 |
| Bacteria;Firmicutes;Clostridia;Clostridiales;Eubacteriaceae;sf_1;28 |
| Bacteria;Firmicutes;Bacilli;Bacillales;Bacillaceae;sf_1;3540 |
| Bacteria;Firmicutes;Bacilli;Bacillales;Bacillaceae;sf_1;3827 |
| Bacteria;Firmicutes;Bacilli;Lactobacillales;Lactobacillaceae;sf_1;3703 |
| Bacteria;Firmicutes;gutclonegroup;Unclassified;Unclassified;sf_1;4298 |
| Bacteria;Firmicutes;Catabacter;Unclassified;Unclassified;sf_4;4325 |
| Bacteria;Unclassified;Unclassified;Unclassified;Unclassified;sf_92;9999 |

(continued)

| |
|---|
| Bacteria;Bacteroidetes;Bacteroidetes;Bacteroidales;Bacteroidaceae;sf_12;5621 |
| Bacteria;BRC1;Unaassified;Unclassified;Unclassified;sf_1;5143 |
| Bacteria;Proteobacteria;Betaproteobacteria;Rhodocyclales;Rhodocyclaceae;sf_1;8052 |
| Bacteria;Proteobacteria;Gammaproteobacteria;Alteromonadales;Alteromonadaceae;sf_1;8904 |
| Bacteria;Proteobacteria;Deltaproteobacteria;Myxococcales;Polyangiaceae;sf_3;10353 |
| Bacteria;Firmicutes;Bacilli;Bacillales;Bacillaceae;sf_1;3283 |
| Bacteria;Firmicutes;Bacilli;Bacillales;Staphylococcaceae;sf_1;3258 |
| Bacteria;Firmicutes;Bacilli;Bacillales;Staphylococcaceae;sf_1;3605 |
| Bacteria;Firmicutes;Bacilli;Lactobacillales;Leuconostocaceae;sf_1;3497 |
| Bacteria;Firmicutes;Bacilli;Lactobacillales;Streptococcaceae;sf_1;3290 |
| Bacteria;Firmicutes;Unclassified;Unclassified;Unclassified;sf_8;4536 |
| Bacteria;Firmicutes;Clostridia;Clostridiales;Lachnospiraceae;sf_5;4155 |
| Bacteria;Firmicutes;Clostridia;Clostridiales;Clostridiaceae;sf_12;4378 |
| Bacteria;Verrucomicrobia;Verrucomicrobiae;Verrucomicrobiales;Unclassified;sf_3;1 |
| Bacteria;Acidobacteria;Acidobacteria;Acidobacteriales;Acidobacteriaceae;sf_14;208 |
| Bacteria;Bacteroidetes;Bacteroidetes;Bacteroidales;Bacteroidaceae;sf_12;5275 |
| Bacteria;Bacteroidetes;Flavobacteria;Flavobacteriales;Flavobacteriaceae;sf1;5423 |
| Bacteria;Proteobacteria;Betaproteobacteria;Nitrosomonadales;Nitrosomonadaceae;sf_1;7805 |
| Bacteria;Proteobacteria;Betaproteobacteria;Nitrosomonadales;Nitrosomonadaceae;sf_1;7858 |
| Bacteria;Actinobacteria;Actinobacteria;Actinomycetales;Frankiaceae;sf_1;1105 |
| Bacteria;Gemmatimonadetes;Unclassified;Unclassified;Unclassified;sf_5;1565 |
| Bacteria;Actinobacteria;Actinobacteria;Actinomycetales;Micrococcaceae;sf_1;1213 |
| Bacteria;Firmicutes;Clostridia;Clostridiales;Lachnospiraceae;sf_5;2804 |
| Bacteria;Firmicutes;Bacilli;Bacillales;Staphylococcaceae;sf_1;3284 |
| Bacteria;Firmicutes;Bacilli;Bacillales;Staphylococcaceae;sf_1;3628 |
| Bacteria;Firmicutes;Bacilli;Lactobacillales;Lactobacillaceae;sf_1;3547 |
| Bacteria;Firmicutes;Bacilli;Lactobacillales;Lactobacillaceae;sf_1;3634 |
| Bacteria;Firmicutes;Bacilli;Lactobacillales;Enterococcaceae;sf_1;3261 |
| Bacteria;Firmicutes;Clostridia;Clostridiales;Clostridiaceae;sf_12;4638 |
| Bacteria;Firmicutes;Clostridia;Clostridiales;Clostridiaceae;sf_12;4275 |
| Bacteria;Firmicutes;Clostridia;Clostridiales;Peptococc/Acidaminococc;sf_11;489 |
| Bacteria;Proteobacteria;Betaproteobacteria;Unclassified;Unclassified;sf_3;7765 |
| Bactena;NC10;NC10-2;Unclassified;Unclassified;sf_1;10254 |
| Bacteria;Actinobacteria;Actinobacteria;Actinomycetales;Mycobacteriaceae;sf_1;1365 |
| Bacteria;Firmicutes;Clostridia;Clostridiales;Peptostreptococcaceae;sf_5;3112 |
| Bacteria;Firmicutes;Clostridia;Clostridiales;Clostridiaceae;sf_12;3219 |
| Bacteria;Firmicutes;Bacilli;Bacillales;Bacillaceae;sf_1;385 |
| Bacteria;Firmicutes;Bacilli;Bacillales;Bacillaceae;sf_1;571 |
| Bacteria;Firmicutes;Bacilli;Bacillales;Staphylococcaceae;sf_1;3684 |

(continued)

| |
|---|
| Bacteria;Firmicutes;Bacilli;Lactobacillales;Enterococcaceae;sf_1;3433 |
| Bacteria;Proteobacteria;Betaproteobacteria;Nitrosomonadales;Nitrosomonadaceae;sf_1;7831 |
| Bacteria;Firmicutes;Bacilli;Lactobacillales;Lactobacillaceae;sf_1;3330 |
| Bacteria;Proteobacteria;Gammaproteobacteria;GAO custer;Unclassified;sf_1;8980 |
| Bacteria;Firmicutes;Clostridia;Clostridiales;Lachnospiraceae;sf_5;2756 |
| Bacteria;Firmicutes;Bacilli;Bacillales;Staphylococcaceae;sf_1;3545 |
| Bacteria;Firmicutes;Bacilli;Lactobacillales;Enterococcaceae;sf_1;3298 |
| Bacteria;Nitrospira;Nitrospira;Nitrospirales;Nitrospiraceae;sf_3;833 |
| Bacteria;Bacteroidetes;Bacteroidetes;Bacteroidales;Bacteroidaceae;sf_12;5474 |
| Bacteria;Bacteroidetes;Unclassified;Unclassified;Unclassified;sf_1;5745 |
| Bacteria;Bacteroidetes;Bacteroidetes;Bacteroidales;Prevotellaceae;sf_1;5769 |
| Bacteria;Cyanobacteria;Cyanobacteria;Oscillatoriales;Unclassified;sf_1;5184 |
| Bacteria;Proteobacteria;Gammaproteobacteria;GAO cluster;Unclassified;sf_1;9468 |
| Bacteria;Proteobacteria;Deltaproteobacteria;Desulfuromonadales;Geobacteraceae;sf_1;9956 |
| Bacteria;Firmicutes;Clostridia;Clostridiales;Lachnospiraceae;sf_5;3066 |
| Bacteria;Firmicutes;Clostridia;Clostridiales;Lachnospiraceae;sf_5;3088 |
| Bacteria;Firmicutes;Clostridia;Clostridiales;Lachnospiraceae;sf_5;3075 |
| Bacteria;Firmicutes;Bacilli;Bacillales;Bacillaceae;sf_1;3688 |
| Bacteria;Firmicutes;Bacilli;Bacillales;Staphylococcaceae;sf_1;3822 |
| Bacteria;Firmicutes;Clostridia;Clostridiales;Lachnospiraceae;sf_5;4167 |
| Bacteria;Firmicutes;Bacilli;Bacillales;Thermoactinomycetaceae;sf_1;3539 |
| Bacteria;Bacteroidetes;Bacteroidetes;Bacteroidales;Rikenellaceae;sf_5;5889 |
| Bacteria;Bacteroidetes;Bacteroidetes;Bacteroidales;Porphyromonadaceae;sf_1;5932 |
| Bacteria;Bacteroidetes;Bacteroidetes;Bacteroidales;Prevotellaceae;sf_1;5437 |
| Bacteria;Firmicutes;Clostridia;Clostridiales;Lachnospiraceae;sf_5;3089 |
| Bacteria;Firmicutes;Bacilli;Bacillales;Staphylococcaceae;sf_1;3569 |
| Bacteria;Firmicutes;Bacilli;Lactobacillales;Lactobacillaceae;sf_1;3767 |
| Bacteria;Firmicutes;Bacilli;Lactobacillales;Enterococcaceae;sf_1;3713 |
| Bacteria;Firmicutes;Mollicutes;Unclassified;Unclassified;sf_6;149 |
| Bacteria;Chloroflexi;Dehalococcoidetes;Unclassified;Unclassified;sf_1;2487 |
| Bacteria;Firmicutes;Clostridia;Clostridiales;Lachnospiraceae;sf_5;2784, |
| Bacteria;Firmicutes;Clostridia;Clostridiales;Lachnospiraceae;sf_5;2937 |
| Bacteria;Firmicutes;Bacilli;Bacillales;Staphylococcaceae;sf_1;3794 |
| Bacteria;Firmicutes;Bacilli;Lactobacillales;Enterococcaceae;sf_1;3382 |
| Bacteria;Firmicutes;Bacilli;Lactobacillales;Enterococcaceae;sf_1;3318 |
| Bacteria;Firmicutes;Bacilli;Lactobacillales;Streptococcaceae;sf_1;3397 |
| Bacteria;Firmicutes;Bacilli;Lactobacillales;Streptococcaceae;sf_1;3446 |
| Bacteria;Bacteroidetes;Bacteroidetes;Bacteroidales;Prevotellaceae;sf_1;5946 |
| Bacteria;Actinobacteria;Actinobacteria;Actinomycetales;Thermomonosporaceae;sf_1;1406 |

(continued)

| |
|---|
| Bacteria;Actinobacteria;Actinobacteria;Actinomycetales;Corynebacteriaceae;sf_1;1428 |
| Bacteria;Firmicutes;Bacilli;Lactobacillales;Enterococcaceae;sf_1;3392 |
| Bacteria;Firmicutes;Bacilli;Lactobacillales;Enterococcaceae;sf_1;3680 |
| Bacteria;Firmicutes;Mollicutes;Anaeroplasmatales;Erysipelotrichaceae;sf_3;3943 |
| Bacteria;Bacteroidetes;Flavobacteria;Flavobacteriales;Flavobacteriaceae;sf_1;5339 |
| Bacteria;Cyanobacteria;Cyanobacteria;Oscillatoriales;Unclassified;sf_1;5215 |
| Bacteria;Actinobacteria;Actinobacteria;Actinomycetales;Pseudonocardiaceae;sf_1;1402 |
| Bacteria;Firmicutes;Bacilli;Lactobacillales;Lactobacillaceae;sf_1;3521 |
| Bacteria;Firmicutes;Bacilli;Lactobacillales;Lactobacillaceae;sf_1;3885 |
| Bacteria;Firmicutes;Bacilli;Lactobacillales;Streptococcaceae;sf_1;3250 |
| Bacteria;Firmicutes;Bacilli;Lactobacillales;Streptococcaceae;sf_1;3906 |
| Bacteria;Firmicutes;Bacilli;Lactobacillales;Streptococcaceae;sf_1;3287 |
| Bacteria;Firmicutes;Bacilli;Lactobacillales;Unaassified;sf_1;3481 |
| Bacteria;Firmicutes;Clostridia;Clostridiales;Clostridiaceae;sf_12;4173 |
| Bacteria;Proteobacteria;Deltaproteobacteria;Desulfobacterales;Desulfobacteraceae;sf_5;10275 |
| Bacteria;Bacteroidetes;Bacteroidetes;Bacteroidales;Prevotellaceae;sf_1;5940 |
| Bacteria;Firmicutes;Clostridia;Clostridiales;Lachnospiraceae;sf_5;3087 |
| Bacteria;Firmicutes;Clostridia;Clostridiales;Lachnospiraceae;sf_5;2991 |
| Bacteria;Firmicutes;Clostridia;Clostridiales;Lachnospiraceae;sf_5;4381 |
| Bacteria;Firmicutes;Bacilli;Lactobacillales;Aerococcaceae;sf_1;3504 |
| Bacteria;Firmicutes;Clostridia;Clostridiales;Lachnospiraceae;sf_5;4443 |
| Bacteria;Bacteroidetes;Bacteroidetes;Bacteroidales;Prevotellaceae;sf_1;5398 |
| Bacteria;Firmicutes;Clostridia;Clostridiales;Lachnospiraceae;sf_5;2849 |
| Bacteria;Proteobacteria;Betaproteobacteria;Burkholderiales;Comamonadaceae;sf_1;7834 |
| Bacteria;Proteobacteria;Gammaproteobacteria;Pasteurellales;Pasteurellaceae;sf_1;9263 |
| Bacteria;Firmicutes;Clostridia;Clostridiales;Lachnospiraceae;sf_5;2726 |
| Bacteria;Firmicutes;Clostridia;Clostridiales;Unclassified;sf_17;2683 |
| Bacteria;Firmicutes;Clostridia;Clostridiales;Lachnospiraceae;sf_5;3107 |
| Bacteria;Firmicutes;Clostridia;Clostridiales;Lachnospiraceae;sf_5;3033 |
| Bacteria;Firmicutes;Clostridia;Clostridiales;Clostridiaceae;sf_12;2736 |
| Bacteria;Firmicutes;Clostridia;Clostridiales;Lachnospiraceae;sf_5;4538 |
| Bacteria;Firmicutes;Clostridia;Clostridiales;Lachnospiraceae;sf_5;2808 |
| Bacteria;Firmicutes;Clostridia;Clostridiales;Lachnospiraceae;sf_5;2733 |
| Bacteria;Firmicutes;Clostridia;Clostridiales;Clostridiaceae;sf_12;3019 |
| Bacteria;Firmicutes;Clostridia;Clostridiales;Lachnospiraceae;sf_5;2747 |
| Bacteria;Firmicutes;Clostridia;Clostridiales;Lachnospiraceae;sf_5;2793 |
| Bacteria;Firmicutes;Clostridia;Clostridiales;Lachnospiraceae;sf_5;4563 |
| Bacteria;Fusobacteria;Fusobacteria;Fusobacterales;Fusobacteriaceae;sf_1;488 |
| Bacteria;Firmicutes;Clostridia;Clostridiales;Lachnospiraceae;sf_5;3149 |

(continued)

| |
|---|
| Bacteria;Firmicutes;Mollicutes;Anaeroplasmatales;Erysipelotrichaceae;sf_3;3956 |
| Bacteria;Bacteroidetes;Bacteroidetes;Bacteroidales;Rikenellaceae;sf_5;6032 |
| Bacteria;Bacteroidetes;Bacteroidetes;Bacteroidales;Bacteroidaceae;sf_12;5285 |
| Bacteria;Bacteroidetes;Bacteroidetes;Bacteroidales;Unclassified;sf_15;5299 |
| Bacteria;Bacteroidetes;Bacteroidetes;Bacteroidales;Bacteroidaceae;sf_12;5424 |
| Bacteria;Bacteroidetes;Bacteroidetes;Bacteroidales;Bacteroidaceae;sf_12;5551 |
| Bacteria;Bacteroidetes;Bacteroidetes;Bacteroidales;Bacteroidaceae;sf_12;5979 |
| Bacteria;Bacteroidetes;Bacteroidetes;Bacteroidales;Bacteroidaceae;sf_12;6064 |
| Bacteria;Proteobacteria;Gammaproteobacteria;Pasteurellales;Pasteurellaceae;sf_1;9360 |
| Bacteria;Proteobacteria;Gammaproteobacteria;Pasteurellales;Pasteurellaceae;sf_1;8228 |
| Bacteria;Proteobacteria;Gammaproteobacteria;Pasteurellales;Pasteurellaceae;sf_1;8861 |
| Bacteria;Fusobacteria;Fusobacteria;Fusobacterales;Fusobacteriaceae;sf_3;558 |
| Bacteria;Firniicutes;Clostridia;Clostridiales;Peptococc/Acidaniinococc;sf_11;181 |
| Bacteria;Firmicutes;Clostridia;Clostridiales;Lachnospiraceae;sf_5;2731 |
| Bacteria;Firmicutes;Clostridia;Clostridiales;Lachnospiraceae;sf_5;3032 |
| Bacteria;Firmicutes;Clostridia;Clostridiales;Unclassified;sf_17;2730 |
| Bacteria;Firmicutes;Clostridia;Clostridiales;Lachnospiraceae;sf_5;2769 |
| Bacteria;Firmicutes;Clostridia;Clostridiales;Lachnospiraceae;sf_5;2928 |
| Bacteria;Firmicutes;Clostridia;Clostridiales;Lachnospiraceae;sf_5;2753 |
| Bacteria;Firmicutes;Clostridia;Clostridiales;Clostridiaceae;sf_12;2898 |
| Bacteria;Firmicutes;Clostridia;Clostridiales;Lachnospiraceae;sf_5;2965 |
| Bacteria;Firmicutes;Clostridia;Clostridiales;Lachnospiraceae;sf_5;2737 |
| Bacteria;Firmicutes;Clostridia;Clostridiales;Lachnospiraceae;sf_5;3016 |
| Bacteria;Firmicutes;Clostridia;Clostridiales;Lachnospiraceae;sf_5;3185 |
| Bacteria;Firmicutes;Clostridia;Clostridiales;Unclassified;sf_17;2912 |
| Bacteria;Firmicutes;Bacilli;Lactobacillales;Streptococcaceae;sf_1;3253 |
| Bacteria;Firmicutes;Mollicutes;Unclassified;Unclassified;sf_6;196 |
| Bacteria;Firmicutes;Clostridia;Clostridiales;Lachnospiraceae;sf_5;4500 |
| Bacteria;Firmicutes;Clostridia;Clostridiales;Clostridiaceae;sf_12;4570 |

Fecal Taxa were found consisting of Firmicutes, Proteobacteria, Bacteroidetes and Actinobacteria. Of the Firmicutes most are from the order Clostridiales including the families Lachnospiraceae, Peptostreptococcaceae, Acidaminococci, and Clostridiaceae; a smaller percentage of bacteria from the order Bacillales are present including Bacillaceae, Halobacillaceae, Staphylococcaceae; as well as a similar precentage of Lactobacillales including the families of Lactobacillaceae, Enterococcaceae and Streptococcaceae. In the Proteobacteria phylum about a third are from Enterobacteriales including Enterobacteriaceae; with small percentatges of Alteromonadales including Alteromonadaceae, and Shewanellaceae. Other smaller constituent populations include taxa from the Order Burkholderiales including Burkholderiaceae, Comamonadaceae, Alcaligenaceae, Oxalobacteraceae, and Ralstoniaceae.

In some embodiments, a system is provided for detecting the presence or quantity of at least 10, 25, 50, 100, 200, 300, 400, or 500 different fecal taxa selected from Table 4 in a single assay. In further embodiments, the system comprises probes that selectively hybridize to each of the at least 10, 25, 50, 100, 200, 300, 400, or 500 different fecal taxa. In other embodiments, a method is provided for detecting fecal contamination in water comprising detecting the presence or quantity of one or more nucleic acid sequence selected from the group consisting of the 16S RNA sequences for fecal taxa listed in Table 4 in a water sample. In further embodiments, the detection method relies on detecting one or more 16S RNA sequences for clean water taxa listed in Table 11. In still further embodiments, the water sample is contacted with a plurality of probes that selectively hybridize to the one or more clean water taxa. Useful probes include those that can be used to identify organisms or taxa listed in Table 11.

Example 4: Water Quality Testing, Fecal Contamination, and Flow Cytometry

Water quality is tested using a microparticle based multiplex system. A plurality of probes that recognize a collection of core microrganisms (Bacilli, Bacteroides and Clostridiales) that are associated with fecal contaminated water are selected from Table 4. An additional plurality of probes that recognize a collection of core microorganisms ($\alpha$-proteobacteria) associated with clean water are also selected from a plurality of probes that identify the organisms or taxa listed in Table 11. A sublot of labeled microparticles is made for each probe within the two collections of plurality of probes. The probes are coupled to 3.0 micrometers latex microspheres (manufactured by Interfacial Dynamics) by carbodiimide coupling. After coupling the sublots are combined. Next negative control probe-coupled microparticles and positive control probe-coupled microparticles are added to make a finished lot of labeled microparticles.

Water samples are filtered on 0.22 $\mu$m filters and extracted for DNA using the UltraClean Water DNA kit (MoBio Laboratories). 16S rRNA genes are PCR amplified using universal bacterial primers 27F and 1492R. Eight replicate reactions across a temperature gradient (48-58°C) are performed for each sample to minimize potential PCR amplification bias. The pooled amplicon of each sample (250 ng) is spiked with internal QS standards to permit normalization of assay hybridization signals. This mix is fragmented, biotin labeled and hybridized to the microparticles by combining approximately 40 picomoles of the bead-attached oligos with approximately 2-fold higher amount of biotin labeled amplicon in 2.3X SSC buffer at approximately 25°C. This mixture is incubated for two hours at room temperature followed by washing, dilution with 300 microlitres of saline pH 7.3, and analysis on the "FAGSCAN" (manufactured by Becton-Dickinson Immunocytometry Systems). The results demonstrate a ratio of BBC:A of 1.05 indicating that the water sample is contaminated with fecal matter.

Example 5: Fecal Contamination Associated Taxa

A biosignature can be determined for fecal contamination by analyzing a sample suspected of fecal contamination using the systems and methods of the invention. DNA is extracted from the sample using standard techniques. 16S rDNA can then be amplified, processed, and analyzed as described in Example 2. Analysis by probe hybridization can be conducted using an array, as described in Example 2, or by using a flow cytometry method similar to that in Example 4, with probes bound to beads. The presence, absence, and/or level can be scored for each probe evaluated, and/or for each OTU represented by the probes evaluated. This collection of data, or a subset thereof, can then serve as a biosignature for contamination by fecal contamination, to which the biosignatures of test samples can be compared.

A water sample taken near a recreational beach is identified as having an unacceptably high level of fecal contamination. A series of water samples are collected near the beach and up the watershed of a nearby creek. The water samples are processed and then assayed on low density water quality arrays. After imaging and signal processing, the BBC:A ratios are calculated for each sample. The BBC:A signal is about 1.05-1.10 near the beach and increases up the watershed and then abruptly drops below 0.95 signifying clean water. The site surrounding location that has the highest BBC:A reading is searched and a ruptured sewer line is found. Repair of the sewer line increases the water quality in the creek watershed.

Fecal Taxa are found consisting of Firmicutes, Proteobacteria, Bacteroidetes and Actinobacteria. Of the Firmicutes most are from the order Clostridiales including the families Lachnospiraceae, Peptostreptococcaceae, Acidaminococci, and Clostridiaceae; a smaller percentage of bacteria from the order Bacillales are present including Bacillaceae, Halobacillaceae, Staphylococcaceae; as well as a similar precentage of Lactobacillales including the families of Lactobacillaceae, Enterococcaceae and Streptococcaceae. In the Proteobacteria phylum about a third are from Enterobacteriales including Enterobacteriaceae; with small percentatges of Alteromonadales including Alteromonadaceae, and Shewanellaceae. Other smaller constituent populations include taxa from the Order Burkholderiales including Burkholderiaceae, Comamonadaceae, Alcaligenaceae, Oxalobacteraceae, and Ralstoniaceae.

In some embodiments, a system is provided for detecting the presence or quantity of at least 10, 25, 50, 100, 200, 300, 400, or 500 different fecal taxa selected from Table 4 in a single water quality test assay. In further embodiments, the system comprises probes that selectively hybridize to each of the at least 10, 25, 50, 100, 200, 300, 400, or 500 different fetal taxa. In other embodiments, a method is provided for detecting fecal contamination in water comprising detecting the presence or quantity of one or more nucleic acid sequence selected from the group consisting of the 16S RNA sequences for fecal taxa listed in Table 4 in a water sample. In further embodiments, the detection method relies on detecting one or more 16S RNA sequences for clean water taxa listed in Table 11. In still further embodiments, the water sample is contacted with a plurality of probes that selectively hybridize to the one or more clean water taxa. Useful probes include those can be used to identify the organisms or taxa listed in Table 11.

Example 6: Toxic Alga Bloom

Cyanobacteria, also known as blue-green algae, represent a major constituent of aquatic microbiomes. Under appropriate conditions, usually plentiful availability of nutrients, their numbers can increase rapidly resulting in an alga bloom. Once the nutrients are used up, the blooms die and then undergo bacterial decomposition that can consume all of the available dissolved oxygen leading to dead zones that are devoid of macroscopic life. Also worrisome is the ability of these cyanobacteria to sense the presence of others cyanobacteria or bacteria (quorum sensing) and at the specific density produce neurotoxins. Ingestion of water containing the cyanobacteria or their neurotoxins or seafood, particularly shellfish from areas with toxic alga blooms can cause serious injury or death. Methods to predict the probability of alga blooms, including toxic alga blooms are needed to protect the public health and ensure the safety of drinking water and seafood.

In some embodiments, a method is provided for predicting the likelihood of a toxic alga bloom comprising a) contacting a water sample with a plurality of probes that selectively bind to nucleic acids derived from cyanobacteria selected from Table 6; b) using hybridization data to determine the quantity and composition of cyanobacteria in the water sample; c) measuring environmental conditions; and d) predicting the likelihood of a toxic alga bloom based on cyanobacteria quantity and composition and environmental conditions. In further embodiments, the probes are selected by the methods discussed above to detect the genera listed in Table 6. Useful environmental conditions to monitor include water temperature, turbidity, nitrogen, phosphate, or iron concentration or sunlight intensity. In further embodiments, the presence or quantity of other microorganisms, particularly bacterial organisms is determined. Frequently, toxic bloom producing cyanobacteria live symbiotically with certain bacteria that use quorum sensing. Cyanobacteria may be able to read or hijack the bacterial quorum sensing, therefore knowledge of quantities of the symbiotic bacteria may be important for toxin expression (e.g. may influence, catalyze, or control toxin levels). Knowledge of the relationships of the populations present in an aquatic microbiome, include knowledge of the bacteria and cyanobacteria that are capable of quorum sensing and the densities at which this phenomena occurs can allow one to predict when a toxic alga bloom may occur. Armed with this predictive power, water management decisions can be made based on the likelihood of a toxic alga bloom, including banning swimming or shellfish collecting in areas likely to experience a bloom, or switching a municipal water supply over to an alternate water source like well water.

Table 6: Toxic Alga Bloom Cyanobacteria Genera

| **Genera** |
|---|
| *Microcystis* |
| *Anabaena* |
| *Planktothrix (Oscillatoria)* |
| *Nostoc* |
| *Hapalosiphon* |
| *Anabaenopsis* |
| *Nodularia* |
| *Aphanizomenon* |
| *Lyngbya* |
| *Schizothrix* |
| *Cylindrospermopsis* |
| *Aphanizomemon* |
| *Umezakia* |

A water sample from a recreational area at a local lake is applied to a down-selected phylogenetic array with probes selected as discussed above to detect nucleic acids from 100 OTUs of cyanobacteria associated with toxic alga blooms. Three cyanobacteria OTUs are detected and quantified that correlate to cyanobacteria densities above 50,000 cyanobacteriums per ml of water. The water temperature is 70° F, clarity is poor with a Secchi disk visible until 14 inches of depth, with bright sunshine predicted for the next 5 days with ambient outdoor daytime temperatures expected to climb into the nineties. The probability of a toxic alga bloom is over 90%. Preparations are made to close the swimming area at the recreational area and the managers of the municipal water supply are notified to switch over from surface water to well water in two days based on detection of cyanobacteria in a water sample.

Example 7: PhyloChip Array

An array system, "PhyloChip", was fabricated with some of the organism-specific and OTU-specific 16s rRNA probes selected by the methods described herein. The PhyloChip array consisted of 1,016,064 probe features, arranged as a grid of 1,008 rows and columns. Of these features, -90% were oligonucleotide PM or MM probes with exact or inexact complementarity, respectively, to 16s rRNA genes. Each probe is paired with a mismatch control probe to distinguish target-specific hybridization from background and non-target cross-hybridization. The remaining probes were used for image orientation, normalization controls, or for pathogen-specific signature amplicon detection using additional targeted regions of the chromosome. Each high-density 16s rRNA gene microarray was designed with additional probes that (1) targeted amplicons of prokaryotic metabolic genes spiked into the 16s rRNA gene amplicon mix in defined quantities just prior to fragmentation and (2) were complementary to pre-labelled oligonucleotides added into the hybridization mix. The first control collectively tested the target fragmentation, labeling by biotinylation, array hybridization, and staining/scanning efficiency. It also allowed the overall fluorescent intensity to be normalized across all the arrays in an experiment. The second control directly assayed the hybridization, staining and scanning.

Complementary targets to the probe sequences hybridize to the array and fluorescent signals were captured as pixel images using standard AFFYMETRIX® software (GeneChip Microarray Analysis Suite, version 5.1) that reduced the data to an individual signal value for each probe and was typically exported as a human readable CEL' file. Background probes were identified from the CEL file as those producing intensities in the lowest 2% of all intensities. The average intensity of the background probes was subtracted from the fluorescence intensity of all probes. The noise value (N) was the variation in pixel intensity signals observed by the scanner as it reads the array surface. The standard deviation of the pixel intensities within each of the identified background probe intensities was divided by the square root of the number of pixels comprising that feature. The average of the resulting quotients was used for N in the calculations described below.

Using previous methods, probe pairs scored as positive are those that meet two criteria: (i) the fluorescence intensity from the perfectly matched probe (PM) was at least 1.3 times greater than the intensity from the mismatched control (MM), and (ii) the difference in intensity, PM minus MM, was at least 130 times greater than the squared noise value (>130 N2). The positive fraction (PosFrac) was calculated for each probe set as the number of positive probe pairs divided by the total number of probe pairs in a probe set. An OTU was considered 'present' when its PosFrac for the corresponding probe set was > 0.92 (based on empirical data from clone library analyses). Replicate arrays cuold be used collectively in determining the presence of each OTU by requiring each to exceed a PosFrac threshold. Present calls were propagated upwards through the taxonomic hierarchy by considering any node (subfamily, family, order, etc.) as 'present' if at least one of its subordinate OTUs was present.

Hybridization intensity was the measure of OTU abundance and was calculated in arbitrary units for each probe set as the trimmed average (maximum and minimum values removed before averaging) of the PM minus MM intensity differences across the probe pairs in a given probe set. All intensities < 1 were shifted to 1 to avoid errors in subsequent logarithmic transformations.

The analysis methods described in Example 1 can also be applied to a sample that has been applied to the presently described PhyloChip G3 array.

A Latin Square Validation was carried out on the PhyloChip G3 array. The novel PhyloChip microarray (G3) was manufactured containing multiple probes for each known Bacterial and Archaeal taxon. The array was challenged with triplicate mixtures of 26 organisms combined in known but randomly assigned concentrations spanning over several orders of magnitude using a Latin Square experimental design. Probe-target complexes were quantified by flourescence intensity. To monitor community dynamics within the environment, water samples were taken from the San Francisco Bay (CA) at two time points following a point-source sewage spill. Entire 16S rRNA gene amplicon pools (~100 billion molecules/time point) were evaluated with the array. Three replicates were tested on different days with 78 Latin Square chips and 1 Quantitative Standards only control. The amplicon concentration range was > 4.5 $\log_{10}$. The target concentration was from 0.25 pM to 477.79 pM, increasing 37% per step plus a 0 pM (26 different concentrations). Each chip contained all 26 targets, each with a different concentration 0-66 ng each for 243 ng total spike. The Latin Square matrix is not shown.

Figure 14 is a chart showing the concentration of 16S amplicon versus PhyloChip response. Concentration is displayed as the log base 2 picomolar concentration within the PhyloChip hybridization chamber. The y-axis is the average of the multiple perfect match probes in the probe set. The vertical error bars denote the standard deviation of 3 replicate trials. The r-squared value over 0.98 indicates that the PhyloChip G3 array is quantitative in its ability to track changes in concentration.

Figure 15 and 16 shows that model-based detection is an improvement over positive fraction detection of probe sets. Low concentrations (down to 2pM) are differentiated from background in Latin Square.

Figure 15 is boxplot comparison of the detection algorithm based on pair "response score",r, distribution (novel) versus the positive fraction calculation (previously used with the G2 PhyloChip). In both plots the x-axis is the concentration of the spiked-in 16S amplicon (The arrow begins at 2 picomolar and extends through 500 picomolar). The y-axis ranges between 0 and 1 in both plots. The top plot's y-axis displays the median r score of all the probes within a probe set whereas the bottom plot's y-axis displays the positive fraction from the same data set. At low concentrations, 0.25 pM, both plots show a wide distribution of scores (see long whiskers), at 2pM the top boxplots have short whiskers indicating that multiple measurements using a variety of bacterial and archaeal species all have very similar median r scores. The corresponding concentration on the positive fraction graph has a wide range of positive fraction scores. At nearly all concentrations, the r score outperforms the positive fraction.

Figure 16 is two graphs that show the comparison of the r score metric versus the pf by receiver operator characteristic (R.O.C) plots. The steeper slope of the top curve compared to the bottom curve demonstrates that the r score metric can differentiate true positives from false positives more efficiently than the pf metric. The grayscale bar indicates the cutoff values (for either r scores or pf) at each point along the curve.

The validation shows that the novel PhyloChip G3 array is capable of excellent organism detection and quantification in a sample over the prior G2 array.

Example 8: Water Quality Testing-Contamination Source Identified

A water sample can be assayed for contamination by fecal contamination by obtaining a biosignature for the water sample and comparing it to a biosignature for fecal contamination, such as the biosignature described in Example 5, using the systems and methods of the invention. DNA can be extracted from the sample, amplified, processed, and analyzed as in Example 2. Analysis by probe hybridization can be conducted using an array, as described in Example 2, or by using a flow cytometry method similar to that in Example 4, with probes bound to beads. The presence, absence, and/or level can be scored for each probe evaluated, and/or for each OTU represented by the probes evaluated. This data can then be compared to one or more biosignatures for one or more contaminants, including fecal contamination. If the degree of similarity between the biosignature of the test sample and the biosignature of fecal contamination is high, the sample is determined to contain fecal contamination. If the degree of similarity between the biosignatures is low, the sample is determined not to contain the fecal contamination.

In a real-world scenario, the PhyloChip was used to compare the microbial community composition in polluted water samples compared to three potential pollution sources: sewage, septage and cattle waste, to determine which of the three sources most likely contributed to the pollution. Figure 7 plots each PhyloChip result in 2D space. The plot revealed that the contaminated water samples (High Enterococus) fall along a vector toward the source community, sewage in this case.

This example illustrates the power of community analysis using the PhyloChip to identify the cause of *Enterococcus exceedences* in public waterways when the source is otherwise unknown.

In another example, two water samples were collected in Richardson Bay near the site of a 764,000 gallon sewage spill of primary-treated sewage from the Sausalito-Marin City Sanitary District in February 2009. One sample (#3) was collected directly adjacent to the plant 24 hours after the spill began and greatly exceeded water quality criteria for culture-based fecal indicator tests (IDEXX) for enterococcus, total coliforms and *E. coli*. The second sample (#26) was collected 150 m offshore 72 hours after the spill began and contained negligible (below detection limit) numbers of all fecal indicator bacteria. Samples of surface water were collected with 1 liter sterile bottles and stored at 4° C until filtration (within 5 hours of collection) at Lawrence Berkeley National Laboratory. 750 ml of sample were vacuum filtered through Whatman Anodisc membrane filters (47 mm dia., 0.2 μm pore size) and immediately stored at -80° C until DNA extraction.

Genomic DNA was extracted from filters using a bead beating and phenol/chloroform extraction method. 16S ribosomal RNA genes were amplified by PCR using universal primers 27F (5'-AGAGTTTGATCCTGGCTCAG-3') and 1492R (5'-GGTTACCTTGTTACGACTT-3') for bacteria, and 4Fa (5'- TCCGGTTGATCCTGCCRG-3') and 1492R for archaea. Each PCR reaction contained 1× Ex Taq buffer (Takara Bio Inc., Japan), 0.125 units/μl Ex Taq polymerase, 0.8 mM dNTP mixture, 1.0 μg/μl BSA, and 300 nM each primer and 0.5 μl template. PCR conditions were 95°C (3 min), followed by 30 cycles 95°C (30 s), 48-58°C (25 s), 72°C (2 min), followed by a final extension 72°C (10 min). Each DNA extract was amplified in 8 replicate 25 μl reactions spanning a range of annealing temperatures between 48-58°C. PCR products from different annealing temperature were combined for each sample and concentrated using Microcon YM-100 filters (Millipore).

Following gel quantification, 500 ng of bacterial 16S rRNA gene amplicons and 50 ng of archaeal amplicons were processed for PhyloChip analysis. PCR products were spiked with control amplicons derived from prokaryotic and eukaryotic metabolic genes and also synthetic 16S-like genes. This mix was fragmented to 50-200 bp using DNase I

(0.02 U/μg DNA; Invitrogen) and One-Phor-All buffer by incubating at 20° C for 10 min and 98° C for 10 min. Terminal labeling of fragments was accomplished using GeneChip WT Double Stranded DNA Terminal Labeling kit (Affymetrix # 900812) per manufacturer's instructions. Fragmented sample was labeled using terminal deoxynucleotidyl transferase and Affymetrix DNA Labeling Reagent by incubating at 37° C for 60 min, followed by a 10 min 70° C step. Hybridization to the array was carried out using the GeneChip Hybridization, Wash, and Stain Kit (Affymetrix #900720). Labeled DNA (42 μl) was combined with Control Oligonucleotide B2 (Affymetrix #900301), DMSO (final concentration 15.7%) and MES buffer to a final volume of 130 μl and denatured at 99°C for 5 minutes followed by 48°C for 5 minutes. The entire reaction mixture was then added to the PhyloChip and incubated at 48° C overnight (>16 h) at 60 rpm. The PhyloChips were subsequently washed and stained per the Affymetrix protocol using a GeneChip Fluidics Station 450 and then scanned using a GeneArray Scanner. The scan was captured as a pixel image using standard Affymetrix software (GeneChip Operating Software, version 1.0) that reduced the data to an individual signal value for each probe. Using analysis algorithms described here, a large number of taxa were identified as being present in either samples. In addition, many distinctive taxa were found to be unique to the water sample directly adjacent to the sewage spill within the first 24 hours of the spill and many different distinctive taxa were identified in the putatively non-polluted sample taken 150 meters offshore 72 hours after the spill. The taxa identified from the sewage spill site (sample 3), as well as their associated probes, can be used as a basis for the identification of fecal contamination in associated receiving waters.

A Fecalbacterium probe set and the individual probes of this probe set were analyzed at every step of the process using the methods of Example 1. A summary statistic of all probe sets identified as positive in each of the 2 samples and what was different was determined (not shown)

The use of the PhyloChip with diffusion chamber tests can give important information on the fate of a given microbiome such as the gut bacteria of animals etc. in a given receiving water. By using diffusion chambers to look at the survival rates of the members of the microbiome in a second environment such as different receiving waters to drive the selection of appropriate indicator organisms. There is a big difference in microbiome survival profiles between salt and fresh water. Also, it may be possible to ascertain the age of a spill, e.g., ongoing vs. several days old, by comparing the different survival rates of selected organisms. While use of a few organisms in a diffusion chamber test has been well known, the ability of the PhyloChip to perform a whole microbiome analysis will lead to previously unattainable results.

The sewage samples above were also submitted to diffusion tests using a diffusion chamber. The sewage microbiome along with the sewage microbiome mixed with the receiving waters were each tested so that effects of predation from organisms in the receiving waters could be accounted for.

<u>Example 9: Evaluating Sets of Probe Pair Responses to Determine the Presence or Absence of an OTU</u>

Two bay water samples were taken at two time points after a water sewage leak. DNA from each sample was extracted, PCR amplified, digested, labeled and hybridized to PhyloChips. The response patterns from the probe sets for two selected human fecal OTUs were carefully examined as an illustrative example.

Spill 3 - 24 hours after start of spill, ankle deep directly in front of plant
Spi1126 - 72 hours after start of the spill, 500 ft offshore

OTU:36742
ss_id:2036742 Bacteria; Firmicutes; Clostridia_SP; Clostridiales_CL; Clostridiales;
Faecalibacterium_FM; sfA; OTU:36742

One sequence in this OTU:
DQ805677.1 gg_id:185502 human fecal clone RL306aa189f12

**OTU:38712**
ss_id: 2038712 Bacteria; Firmicutes; Clostridia_SP; Clostridiales_CL; Clostridiales;
Ruminococcus_FM; sfA; OTU:38712;
One sequence in this OTU:
DQ797288.1 gg_id:188731 human fecal clone RL248_aai97d06

In Figures 11 and 12, the probe responses are presented and the uses of thresholds are demonstrated for both these OTUs. The PhyloChip is designed to contain *multiple* DNA probes complementary to specific DNA targets within the OTU. Each of these targets may have different A+T content, different T content, and may have putative cross-hybridization potential to other OTUs. These three factors are utilized for de-convolution of probe intensity measurements into presence or absence calls for an OUT.

After the scans were collected, probe intensities were background-subtracted and scaled to the spike-ins.

Figure 11 compares the probe responses to Faecalibacterium OTU 36742 observed on two different PhyloChip experiments. The "Intensity" bar charts display the intensity from each PM and MM probe in blue and red, respectively, grouped as pairs. OTU 36742 has 30 probe pairs. The intensity measurements range from 5.7 to 30334.3 a.u. (arbitrary units). Next we calculate the pair difference score, *d*, for each probe pair by comparing the PM and MM intensities. For example, pair #6 reported a PM intensity of 9941.4 and a MM intensity of 903.4 for Spill 3.

$$d = 1 - \left( \frac{PM - MM}{PM + MM} \right) = 1 - \left( \frac{9941.4 - 903.4}{9941.4 + 903.4} \right) = 0.166$$

Performing this transformation allows the difference between PM and MM probes to be expressed with a single number. The possible range of *d* is 0 to 2 and *d* approaches 0 when PM >> MM, *d* = 1 when PM = MM and *d* approaches 2 when PM << MM. Thus pair #6 displayed a sequence-specific interaction in Spill 3 since 0.166 is close to 0. The *d* values are plotted on the bar graphs labeled 'd', directly below their respective probe pairs. Notice that the same probe pair (#6) in Spill 26 produced a *d* value of 0.870. This is indicative of less separation between PM and MM values since 0.870 is further from zero than 0.166. Comparing *d* scores from the same probe pair across different chips is equitable since the probe composition is exactly the same (it is the same probe pair viewed under different experimental conditions).

In the next step, the *d* scores are normalized to enable comparison of probe pairs with various nucleotide compositions. The goal in this transformation is to determine if the *d* value for a pair is more similar to *d* values derived from negative controls (NC, probe pairs with no potential cross-hybridization to any 16S rRNA sequence) or from positive controls which are the Quantitative Standards (QS, probe pairs with PM's matching the non-16S rRNA genes which are spiked into the experiment). Because the $d_{QS}$ values are dependent on their target's A+T count and T count, the QS pairs are grouped by these attributes into classes and a separate distribution of $d_{QS}$ values are found for each. The $d_{NC}$ values are grouped in the same way. Because there is variation in the responses within each class, a distribution is estimated from the observations. Examples are shown below for Spill 3. Notice the different shape of the orange density plots which demonstrate the *d* observation of the Negative Control probes which are normally distributed. As shown in Figure 13, in class "9T 14AT," the mean $d_{NC}$ is greater than class "4T 11AT", also the variance is greater for class "9T 14AT." Comparing the green density plots (estimated to follow a gamma distribution), quantitative standards for class "4T 11AT" nearly always produce *d* scores close to zero whereas class "9T 14AT" contains more observations of higher *d* scores (less distinction between PM and MM). In this example it can be seen that class "9T 14AT" has a larger range of *d* scores shared by both NC and QS (Figure 13).

Next, each *d* value from an OTU probe set is compared to the distributions of $d_{QS}$ and $d_{NC}$ from the same class. For example, in OTU 36742 probe #6 has 9 thymine bases and 14 bases that are either thymine or adenine (Table 7). In Spill 3 this pair achieved a *d* value of 0.166.

**Table 7 PM targets and the their counts of T and A+T for OTU 36742**

| pair # | PM target seq | T count | A+T count |
|---|---|---|---|
| 1 | TGATTACCTAGGTGTTGGAGGATTG | 9 | 14 |
| 2 | CAATCCTCCAACACCTAGGTAATCA | 5 | 14 |
| 3 | ACGCCGCGTAGAGGAAGAAGGTCTT | 4 | 11 |
| 4 | AAGACCTTCTTCCTCTACGCGGCGT | 7 | 11 |
| 5 | ATCCTGCGACGCACATAGAAATATG | 5 | 14 |
| 6 | CATATTTCTATGTGCGTCGCAGGAT | 9 | 14 |
| 7 | GACACGGCCCAGATTCTTACGGGAG | 4 | 10 |
| 8 | CTCCCGTAAGAATCTGGGCCGTGTC | 6 | 10 |
| 9 | TTTTCCTGGTAGTGCAGAGGTAGGC | 8 | 12 |
| 10 | GCCTACCTCTGCACTACCAGGAAAA | 4 | 12 |
| 11 | ACCAACTGACGCTGAGGCTTGAAAG | 4 | 12 |
| 12 | CTTTCAAGCCTCAGCGTCAGTTGGT | 8 | 12 |

(continued)

| pair # | PM target seq | T count | A+T count |
|---|---|---|---|
| 13 | TTGCTTCCTCCATCTAGTGGACAAC | 8 | 13 |
| 14 | GTTGTCCACTAGATGGAGGAAGCAA | 5 | 13 |
| 15 | GAAACAACGTCCCAGTTTGGACTGC | 5 | 12 |
| 16 | GCAGTCCAAACTGGGACGTTGTTTC | 7 | 12 |
| 17 | TGTTTCTTTCGGGACGCAGAGACAG | 7 | 12 |
| 18 | CTGTCTCTGCGTCCCGAAAGAAACA | 5 | 12 |
| 19 | GGCCCAGATTCTTACGGGAGGCAGC | 4 | 9 |
| 20 | GCTGCCTCCCGTAAGAATCTGGGCC | 5 | 9 |
| 21 | CTAATACCGCATTAGAGCCCACAGG | 4 | 12 |
| 22 | CCTGTGGGCTCTAATGCGGTATTAG | 8 | 12 |
| 23 | AGGCTTGAAAGTGTGGGTAGCAAAC | 5 | 13 |
| 24 | GTTTGCTACCCACACTTTCAAGCCT | 8 | 13 |
| 25 | AGTGGACAACGGGTGAGTAACACAT | 4 | 13 |
| 26 | ATGTGTTACTCACCCGTTGTCCACT | 9 | 13 |
| 27 | GATTACCTAGGTGTTGGAGGATTGA | 8 | 14 |
| 28 | TCAATCCTCCAACACCTAGGTAATC | 6 | 14 |
| 29 | ACATGAGGAACCTGCCACATACAGG | 3 | 12 |
| 30 | CCTGTATGTGGCAGGTTCCTCATGT | 9 | 12 |

To determine the response score, *r* for probe #6, we find the probability that a probe with d=0.166 would be found among the normal distribution of NC (orange in density plots below) then find the probability that a probe pair of d=0.166 would be found among the gamma distribution of the QS, then ultimately record a ratio as the response score *r* according to the following equation:

$$r = \left( \frac{pdf_{\gamma}(X = d)}{pdf_{\gamma}(X = d) + pdf_{norm}(X = d)} \right)$$

where :

$r$ = response score to measure the potential that the probe pair is responding to a target and not the background

pdf $_{\gamma}(X = d)_{\gamma}$ = probability that $d$ could be drawn from the gamma distribution estimated for the target class ATx Ty

$pdf_{norm}\ (X = d)\gamma$ = probability that $d$ could be drawn from the normal distribution estimated for the target class ATx Ty

The response score, *r*, ranges from 0..1 where 1 indicates that a probe pair was observed to have an unambiguous positive response. When *r* = 0.5, the probe pair response resembles the NC and the QS equally and thus we can consider the response ambiguous. Continuing with our example probe #6 from OTU 36742:

$$pdf_{\gamma}(X = 0.166) = 0.951$$

$$pdf_{norm}(X = 0.166) = 0.058$$

$$r = \left(\frac{0.951}{0.951+0.058}\right) = 0.943$$

The r value for OTU 36742 #6 is plotted in Figure 11 according to its response in both experiments. In Spill 26, this probe pair was less "positive" then in Spill 3.

There is an option in *r* scoring for certain probe pairs. The first, more-stringent option, calculates *r* only if sufficient observations (user defined threshold) from the QS or the NC are recorded to estimate the distributions described above. This first option is demonstrated in Figure 12 OTU 38717 on the plots for *r*. The probe pairs circled in red were not used in finding $rQ_1$, $rQ_2$ and $rQ_3$ as described below. The second option calculates *r* scores for all probe pairs, using the general $d_{QS}$ and $d_{NC}$ model (using all QS and NC pairs irrespective of their class), whenever the class-specific model is not determined. This option is not shown in Figure 12. The advantage of the second option is to increase the number of probe-pairs used in the analysis. A third option allows the nearest-class model to be used when a pair's specific class model is not determined for a given array. For example, if an experimental scan of a PhyloChip resulted in masking "outlier" probe pairs and this resulted in an insufficient pair count for the QS or NC for class "4T 12AT", pairs of this class could be compared to the "5T 12AT" model. This hybrid of the two options allows both a high number of pairs to be observed and allows near class-specific response scoring. This option is also not shown in Figure 12.

Next, all the *r* scores for a probe set are considered collectively in "Stage 1" probe set Presence/Absence scoring. Of the 30 probe pairs for OTU 36742, notice many of the *r* scores are near 1 in Spill 3 but few are near 1 in Spill 26 (Figure 11). To quantitatively differentiate these distributions, the *r* scores are ranked and the breakpoints (quartiles), $rQ_1$, $rQ_2$ and $rQ_3$ are found by dividing the ranked observations into 4 equally-sized bins. The calculated quartiles for two OTUs across 2 experiments are shown in Table 8. This table describes the probe set performance. Spill3 OTU 36742 *r*Q2 = 0.934 can be read as "Of the set of probe pairs targeting OTU 36742, half produced *r* scores over 0.934".

**Table 8 "Stage 1" results for 2 OTUs compared across 2 experiments.**

| Experiment | OTU | $rQ_1$ | $rQ_2$ | $rQ_3$ |
|---|---|---|---|---|
| Spill 3 | 36742 | 0.207 | 0.934 | 0.983 |
| Spill 26 | 36742 | 0.015 | 0.172 | 0.763 |
| Spill 3 | 38712 | 0.738 | 0.953 | 0.991 |
| Spill 26 | 38712 | 0.789 | 0.985 | 0.996 |

The quartiles are illustrated as green lines in Figures 11 and 12 on each plot of the response scores (*r*). For an OTU to pass "Stage 1", all three of the following criteria must be met: $rQ_1 \geq 0.200$, $rQ_2 \geq 0.920$, and $rQ_3 \geq 0.977$. These criteria were learned from the Latin Square Data (not shown in this document). From Table 8, all four OTUs pass Stage 1 except OTU 36742 in Spill 26.

Only the OTUs which pass Stage 1 are considered in Stage 2 scoring. The objective in Stage 2 is to estimate the specificity of each responsive probe pair (where $r > 0.5$) in consideration of the community of OTUs that pass Stage 1 on the same array. This is accomplished by penalizing each *r* score according to its putative cross-hybridization potential. Probe pairs that have putative cross-hybridization potential to many *OTUs passing Stage 1* will be penalized by a greater factor than those with putative cross-hybridization to few *OTUs passing Stage 1.* The penalized score, $r_x$, is calculated as

$$r_{xi} = \frac{r_i}{scalar(O_{S1} \cap O_{hi})}$$

where :

$O_{S1}$ = the set of OTUs passing Stage 1
$O_{hi}$ = the set of OTUs with putative ability to hybridize to PM probe
$scalar(Osi \cap O_{hi})$ = the count of OTUs with hybridization potential and passing Stage 1

Probe pair 10 (pp10) in Figure 12 exemplifies this effect. In Spill 3 pp10 achieved a high *r* score (0.997). The PM of pp10 can potentially hybridize to sequences in 11 different OTUs, 7 of these 11 passed Stage 1 (see row of numbers labeled "Penalties"). Thus *r* score is divided by 7 to yield $r_x = 0.142$. The downward pointing arrows on Figures 11 and 12 demonstrate the magnitude of the penalty for each probe pair. After all penalties are considered, the $r_x$ values

are ranked and quartiles found as above ($r_xQ_1$, $r_xQ_2$, $r_xQ_3$). Examples are shown in Table 9.

**Table 9 "Stage 1" and "Stage 2" results for 2 OTUs compared across 2 experiments.**

| Experiment | OTU | $rQ_1$ | $rQ_2$ | $rQ_3$ | $r_xQ_1$ | $r_xQ_2$ | $r_xQ_3$ |
|---|---|---|---|---|---|---|---|
| Spill 3 | 36742 | 0.207 | 0.934 | 0.983 | 0.200 | 0.529 | 0.947 |
| Spill 26 | 36742 | 0.015 | 0.172 | 0.763 | NA | NA | NA |
| Spill 3 | 38712 | 0.738 | 0.953 | 0.991 | 0.080 | 0.142 | 0.214 |
| Spill 26 | 38712 | 0.789 | 0.985 | 0.996 | 0.158 | 0.496 | 0.864 |

In the specific example described here we can conclude that Faecalibacterium OTU 36742 was present in Spill 3 but not Spill 26 based on responsiveness alone. *Only* in Spill 3 did Faecalibacterium OTU 36742 pass Stage 1. Conversely, the probe set for Ruminococcus OTU 38712 was responsive in Stage 1 analysis for both Spills but after further automated analysis refinement in Stage 2, it was determined as present in only Spill 26. Cutoff values for Stage 2: $r_xQ_1 \geq 0.100$, $r_xQ_2 \geq 0.200$, and $r_xQ_3 \geq 0.300$, as empirically determined from the Latin Square Data (not shown in this document). As shown in Table 9, OTU 38712 did not meet these cutoff values in Spill 3.

Example 10: Microbiome Signatures of Clean Ocean Water and Treated Wastewater Provide Effluent and Ocean Associated Taxa

Samples of dechlorinated effluent collected from the Montecito Sanitary District Wastewater Treatment Plant (Santa Barbara, California) and samples of clean ocean water (1000m offshore, Santa Barbara, California) were collected over a period of a year. The dechlorinated effluent samples were combined before processing and analysis as were the clean ocean water samples. Sample processing and analysis was performed as described in Example 2. The microbiome signatures for the dechlorinated effluent and the clean ocean water were compared. The effluent microbiome comprised of 266 taxa (Table 10) that were not found in the clean ocean water microbiome. The clean ocean water microbiome comprised of 231 taxa (Table 11) that were not found in the effluent samples.

The identified taxa represent "signature taxa" for treated effluent and clean ocean water respectively. Signature taxa can be identified from numerous environments, such as raw sewage, healthy, sick or diseased patients, food processing plants that repeatedly pass food safety inspections and those that routinely receive citations. Signature taxa have many uses. For instance, the presence or a specific abundance of different raw sewage signature taxa in the microbiome generated from a fresh water sample can signify insufficient processing at an upstream water treatment plant, something that can occur when large volumes of water are sent to a water treatment facility via storm drains. The presence or abundance of raw sewage taxa in a fresh water microbiome can also signify a leaking sewer pipe, seepage from an improperly maintained septic field or an illegal discharge.

**Table 10. Effluent Microbiome**

| Taxa |
|---|
| Bacteria;Proteobacteria;Alphaproteobacteria;Rhodobacterales;Rhodobacteraceae;sf_1;6848 |
| Bacteria;Proteobacteria;Alphaproteobacteria;Rhodobacterales;Rhodobacteraceae;sf_1;7602 |
| Bacteria;Proteobacteria;Alphaproteobacteria;Rhodobacterales;Rhodobacteraceae;sf_1;6883 |
| Bacteria;Bacteroidetes;Flavobacteria;Flavobacteriales;Flavobacteriaceae;sf_1;5671 |
| Bacteria;Bacteroidetes;Flavobacteria;Flavobacteriales;Flavobacteriaceae;sf_1;5695 |
| Bacteria;Bacteroidetes;Flavobacteria;Flavobacteriales;Flavobacteriaceae;sf_1;5896 |
| Bacteria;Proteobacteria;Alphaproteobacteria;Rhodobacterales;Rhodobacteraceae;sf_1;7596 |
| Bacteria;Proteobacteria;Alphaproteobacteria;Rhodobacterales;Rhodobacteraceae;sf_1;6982 |
| Bacteria;Proteobacteria;Alphaproteobacteria;Unclassified;Unclassified;sf_6;7252 |
| Bacteria;Proteobacteria;Alphaproteobacteria;Rhodobacterales;Rhodobacteraceae;sf_1;7050 |
| Bacteria;Bacteroidetes;Flavobacteria;Flavobacteriales;Flavobacteriaceae;sf_1;5919 |
| Bacteria;Proteobacteria;Alphaproteobacteria;Rhodobacterales;Rhodobacteraceae;sf_1;7288 |
| Bacteria;Proteobacteria;Alphaproteobacteria;Rhodobacterales;Rhodobacteraceae;sf_1;7432 |

(continued)

| Taxa |
| --- |
| Bacteria;Proteobacteria;Alphaproteobacteria;Rhodobacterales;Rhodobacteraceae;sf_1;6664 |
| Bacteria;Cyanobacteria;Cyanobacteria;Prochlorales;Unclassified;sf_1;5076 |
| Bacteria;Proteobacteria;Alphaproteobacteria;Unclassified;Unclassified;sf_6;7196 |
| Bacteria;Proteobacteria;Gammaproteobacteria;Alteromonadales;Alteromonadaceae;sf_1;8517 |
| Bacteria;Proteobacteria;Gammaproteobacteria;SAR86;Unclassified;sf_1;9648 |
| Bacteria;Proteobacteria;Unclassified;Unclassified;Unclassified;sf_20;7365 |
| Bacteria;Actinobacteria;BD2-10group;Unclassified;Unclassified;sf_1;1675 |
| Bacteria;Cyanobacteria;Cyanobacteria;Chloroplasts;Chloroplasts;sf_5;5007 |
| Bacteria;Proteobacteria;Alphaproteobacteria;Unclassified;Unclassified;sf_6;7510 |
| Bacteria;Proteobacteria;Gammaproteobacteria;SAR86;Unclassified;sf_1;9620 |
| Bacteria;Unclassified;Unclassified;Unclassified;Unclassified;sf_148;5235 |
| Bacteria;Cyanobacteria;Cyanobacteria;Thermosynechococcus;Unclassified;sf_1;5012 |
| Bacteria;Proteobacteria;Gammaproteobacteria;Chromatiales;Ectothiorhodospiraceae;sf_1;9387 |
| Bacteria;Proteobacteria;Gammaproteobacteria;Unclassified;Unclassified;sf_3;8647 |
| Bacteria;Proteobacteria;Alphaproteobacteria;Rhodobacterales;Rhodobacteraceae;sf_1;7054 |
| Bacteria;Proteobacteria;Alphaproteobacteria;Rhodobacterales;Rhodobacteraceae;sf_1;7233 |
| Bacteria;Proteobacteria;Alphaproteobacteria;Rhodobacterales;Rhodobacteraceae;sf_1;7045 |
| Bacteria;Proteobacteria;Alphaproteobacteria;Rhodobacterales;Rhodobacteraceae;sf_1;6960 |
| Bacteria;Proteobacteria;Alphaproteobacteria;Rhodobacterales;Rhodobacteraceae;sf_1;7405 |
| Bacteria;Proteobacteria;Alphaproteobacteria;Rhodobacterales;Rhodobacteraceae;sf_1;7329 |
| Bacteria;Proteobacteria;Gammaproteobacteria;Oceanospirillales;Alcanivoraceae;sf_1;9043 |
| Bacteria;Proteobacteria;Alphaproteobacteria;Unclassified;Unclassified;sf_6;7520 |
| Bacteria;Proteobacteria;Alphaproteobacteria;Rhodobacterales;Rhodobacteraceae;sf_1;7499 |
| Bacteria;Proteobacteria;Gammaproteobacteria;SUP05;Unclassified;sf_1;8953 |
| Bacteria;Proteobacteria;Alphaproteobacteria;Rhodobacterales;Rhodobacteraceae;sf_1;7649 |
| Bacteria;Proteobacteria;Alphaproteobacteria;Bradyrhizobiales;Unclassified;sf_1;7143 |
| Bacteria;Actinobacteria;BD2-10group;Unclassified;Unclassified;sf_1;1732 |
| Bacteria;Proteobacteria;Gammaproteobacteria;Unclassified;Unclassified;sf_3;9016 |
| Bacteria;Planctomycetes;Planctomycetacia;Planctomycetales;Planctomycetaceae;sf_3;4654 |
| Bacteria;Unclassified;Unclassified;Unclassified;Unclassified;sf_148;4970 |
| Bacteria;Proteobacteria;Alphaproteobacteria;Rhodobacterales;Rhodobacteraceae;sf_1;7429 |
| Bacteria;Actinobacteria;Actinobacteria;Actinomycetales;Acidothermaceae;sf_1;1399 |
| Bacteria;Proteobacteria;Alphaproteobacteria;Rhodobacterales;Rhodobacteraceae;sf_1;6894 |
| Bacteria;Actinobacteria;Actinobacteria;Acidimicrobiales;Acidimicrobiaceae;sf_1;1282 |
| Bacteria;Proteobacteria;Alphaproteobacteria;Rhodobacterales;Rhodobacteraceae;sf_1;7033 |
| Bacteria;Proteobacteria;Alphaproteobacteria;Rhodobacterales;Rhodobacteraceae;sf_1;7140 |
| Bacteria;Proteobacteria;Alphaproteobacteria;Rhodobacterales;Rhodobacteraceae;sf_1;7085 |
| Bacteria;Proteobacteria;Alphaproteobacteria;Unclassified;Unclassified;sf_6;7421 |

(continued)

| Taxa |
|---|
| Bacteria;Proteobacteria;Alphaproteobacteria;Unclassified;Unclassified;sf_6;6858 |
| Bacteria;Proteobacteria;Gammaproteobacteria;Unclassified;Unclassified;sf_3;8333 |
| Bacteria;Proteobacteria;Unclassified;Unclassified;Unclassified;sf_20;7541 |
| Bacteria;Proteobacteria;Gammaproteobacteria;Unclassified;Unclassified;sf_3;9061 |
| Bacteria;Proteobacteria;Alphaproteobacteria;Rhodobacterales;Rhodobacteraceae;sf_1;6796 |
| Bacteria;Firmicutes;Clostridia;Halanaerobiales;Halobacteroidaceae;sf_1;887 |
| Bacteria;Proteobacteria;Alphaproteobacteria;Rhodobacterales;Rhodobacteraceae;sf_1;6714 |
| Bacteria;Bacteroidetes;Sphingobacteria;Sphingobacteriales;Unclassified;sf_3;5799 |
| Bacteria;Planctomycetes;Planctomycetacia;Planctomycetales;Pirellulae;sf_3;4801 |
| Bacteria;Bacteroidetes;Bacteroidetes;Bacteroidales;Rikenellaceae;sf_5;5889 |
| Bacteria;Bacteroidetes;Flavobacteria;Flavobacteriales;Unclassified;sf_3;5900 |
| Bacteria;Cyanobacteria;Cyanobacteria;Chloroplasts;Chloroplasts;sf_5;4983 |
| Bacteria;Cyanobacteria;Cyanobacteria;Chloroplasts;Chloroplasts;sf_5;5111 |
| Bacteria;Cyanobacteria;Cyanobacteria;Chloroplasts;Chloroplasts;sf_5;5156 |
| Bacteria;Proteobacteria;Gammaproteobacteria;Unclassified;Unclassified;sf_3;8805 |
| Bacteria;Bacteroidetes;Sphingobacteria;Sphingobacteriales;Flexibacteraceae;sf_19;5404 |
| Bacteria;Aquificae;Aquificae;Aquificales;Hydrogenothermaceae;sf_1;737 |
| Bacteria;Proteobacteria;Alphaproteobacteria;Consistiales;Unclassified;sf_5;7504 |
| Bacteria;Bacteroidetes;Bacteroidetes;Bacteroidales;Rikenellaceae;sf_5;5945 |
| Bacteria;Proteobacteria;Alphaproteobacteria;Rhodobacterales;Rhodobacteraceae;sf_1;7224 |
| Bacteria;Proteobacteria;Betaproteobacteria;Unclassified;Unclassified;sf_3;7923 |
| Bacteria;Bacteroidetes;Unclassified;Unclassified;Unclassified;sf_4;6190 |
| Bacteria;Proteobacteria;Alphaproteobacteria;Consistiales;Unclassified;sf_5;7203 |
| Bacteria;Proteobacteria;Alphaproteobacteria;Consistiales;SAR11;sf_1;7376 |
| Bacteria;Proteobacteria;Alphaproteobacteria;Rhodobacterales;Rhodobacteraceae;sf_1;7590 |
| Bacteria;Proteobacteria;Alphaproteobacteria;Rhizobiales;Unclassified;sf_1;7012 |
| Bacteria;Proteobacteria;Gammaproteobacteria;Unclassified;Unclassified;sf_3;8933 |
| Bacteria;Proteobacteria;Alphaproteobacteria;Rhodobacterales;Rhodobacteraceae;sf_1;6866 |
| Bacteria;Cyanobacteria;Cyanobacteria;Chloroplasts;Chloroplasts;sf_5;5166 |
| Bacteria;Bacteroidetes;Flavobacteria;Flavobacteriales;Flavobacteriaceae;sf_1;6104 |
| Bacteria;Cyanobacteria;Cyanobacteria;Chloroplasts;Chloroplasts;sf_5;5221 |
| Bacteria;Cyanobacteria;Cyanobacteria;Chloroplasts;Chloroplasts;sf_5;5120 |
| Bacteria;Bacteroidetes;Bacteroidetes;Bacteroidales;Rikenellaceae;sf_5;5947 |
| Bacteria;Bacteroidetes;Bacteroidetes;Bacteroidales;Unclassified;sf_15;6078 |
| Bacteria;Proteobacteria;Gammaproteobacteria;Oceanospirillales;Unclassified;sf_3;8961 |
| Bacteria;Bacteroidetes;Flavobacteria;Flavobacteriales;Flavobacteriaceae;sf_1;5641 |
| Bacteria;Proteobacteria;Gammaproteobacteria;Methylococcales;Methylococcaceae;sf_1;8821 |
| Bacteria;Proteobacteria;Gammaproteobacteria;Acidithiobacillales;Acidithiobacillaceae;sf_1;8913 |

(continued)

| Taxa |
|---|
| Bacteria;Proteobacteria;Gammaproteobacteria;Unclassified;Unclassified;sf_3;9456 |
| Bacteria;Proteobacteria;Alphaproteobacteria;Rhodobacterales;Hyphomonadaceae;sf_1;7584 |
| Bacteria;Cyanobacteria;Unclassified;Unclassified;Unclassified;sf_5;4993 |
| Bacteria;Proteobacteria;Gammaproteobacteria;Oceanospirillales;Halomonadaceae;sf_1;9141 |
| Bacteria;Cyanobacteria;Cyanobacteria;Geitlerinema;Unclassified;sf_1;4999 |
| Bacteria;Proteobacteria;Alphaproteobacteria;Rhodobacterales;Rhodobacteraceae;sf_1;6771 |
| Bacteria;Proteobacteria;Gammaproteobacteria;Oceanospirillales;Unclassified;sf_3;9010 |
| Bacteria;Acidobacteria;Acidobacteria-9;Unclassified;Unclassified;sf_1;704 |
| Bacteria;OP1;Unclassified;Unclassified;Unclassified;sf_4;728 |
| Bacteria;Proteobacteria;Alphaproteobacteria;Rhodobacterales;Rhodobacteraceae;sf_1;7508 |
| Bacteria;Bacteroidetes;Flavobacteria;Flavobacteriales;Flavobacteriaceae;sf_1;5559 |
| Bacteria;Bacteroidetes;Bacteroidetes;Bacteroidales;Unclassified;sf_15;5998 |
| Bacteria;Proteobacteria;Gammaproteobacteria;Chromatiales;Chromatiaceae;sf_1;8407 |
| Bacteria;Proteobacteria;Gammaproteobacteria;Alteromonadales;Alteromonadaceae;sf_1;9442 |
| Bacteria;Gemmatimonadetes;Unclassified;Unclassified;Unclassified;sf_6;2554 |
| Bacteria;Proteobacteria;Alphaproteobacteria;Bradyrhizobiales;Unclassified;sf_1;7255 |
| Bacteria;Bacteroidetes;Bacteroidetes;Bacteroidales;Rikenellaceae;sf_5;6317 |
| Bacteria;Actinobacteria;Actinobacteria;Actinomycetales;Micrococcaceae;sf_1;1266 |
| Bacteria;Proteobacteria;Alphaproteobacteria;Rhodobacterales;Rhodobacteraceae;sf_1;7049 |
| Bacteria;Proteobacteria;Epsilonproteobacteria;Campylobacterales;Helicobacteraceae;sf_3;10534 |
| Bacteria;Proteobacteria;Alphaproteobacteria;Rhodobacterales;Rhodobacteraceae;sf_1;7362 |
| Bacteria;Bacteroidetes;Flavobacteria;Flavobacteriales;Flavobacteriaceae;sf_1;5955 |
| Bacteria;Proteobacteria;Unclassified;Unclassified;Unclassified;sf_21;8509 |
| Bacteria;Proteobacteria;Alphaproteobacteria;Rhodobacterales;Rhodobacteraceae;sf_1;7373 |
| Bacteria;Proteobacteria;Gammaproteobacteria;GAOcluster;Unclassified;sf_1;9008 |
| Bacteria;Proteobacteria;Alphaproteobacteria;Rhodobacterales;Rhodobacteraceae;sf_1;7032 |
| Bacteria;Proteobacteria;Alphaproteobacteria;Rhodobacterales;Rhodobacteraceae;sf_1;6661 |
| Bacteria;Bacteroidetes;Unclassified;Unclassified;Unclassified;sf_4;5637 |
| Bacteria;Proteobacteria;Gammaproteobacteria;Enterobacteriales;Enterobacteriaceae;sf_1;9309 |
| Bacteria;Proteobacteria;Alphaproteobacteria;Rhodobacterales;Rhodobacteraceae;sf_1;6979 |
| Bacteria;Proteobacteria;Gammaproteobacteria;Alteromonadales;Alteromonadaceae;sf_1;9236 |
| Bacteria;Proteobacteria;Alphaproteobacteria;Rhizobiales;Phyllobacteriaceae;sf_1;7009 |
| Bacteria;Proteobacteria;Gammaproteobacteria;Alteromonadales;Alteromonadaceae;sf_1;9486 |
| Bacteria;Cyanobacteria;Cyanobacteria;Nostocales;Unclassified;sf_1;5174 |
| Bacteria;Cyanobacteria;Cyanobacteria;Nostocales;Unclassified;sf_1;5028 |
| Bacteria;Proteobacteria;Gammaproteobacteria;Unclassified;Unclassified;sf_3;8883 |
| Bacteria;Chloroflexi;Anaerolineae;Unclassified;Unclassified;sf_9;94 |
| Bacteria;Proteobacteria;Alphaproteobacteria;Rhodobacterales;Rhodobacteraceae;sf_1;7523 |

(continued)

| Taxa |
|---|
| Bacteria;Bacteroidetes;Flavobacteria;Flavobacteriales;Flavobacteriaceae;sf_1;5490 |
| Bacteria;Cyanobacteria;Cyanobacteria;Nostocales;Unclassified;sf_1;5175 |
| Bacteria;Verrucomicrobia;Verrucomicrobiae;Verrucomicrobiales;Verrucomicrobiasubdivision7;sf_1;760 |
| Bacteria;Proteobacteria;Alphaproteobacteria;Consistiales;SAR11;sf_2;7043 |
| Bacteria;Chloroflexi;Anaerolineae;Chloroflexi-1f;Unclassified;sf_1;765 |
| Bacteria;Proteobacteria;Unclassified;Unclassified;Unclassified;sf_28;10091 |
| Bacteria;Proteobacteria;Gammaproteobacteria;GAOcluster;Unclassified;sf_1;8980 |
| Bacteria;Aquificae;Aquificae;Aquificales;Hydrogenothermaceae;sf_1;220 |
| Bacteria;Bacteroidetes;Sphingobacteria;Sphingobacteriales;Sphingobacteriaceae;sf_1;5492 |
| Bacteria;Proteobacteria;Gammaproteobacteria;Alteromonadales;Alteromonadaceae;sf_1;8863 |
| Bacteria;Cyanobacteria;Cyanobacteria;Spirulina;Unclassified;sf_1;5034 |
| Bacteria;Bacteroidetes;Flavobacteria;Flavobacteriales;Flavobacteriaceae;sf_1;5499 |
| Bacteria;Gemmatimonadetes;Unclassified;Unclassified;Unclassified;sf_5;227 |
| Bacteria;Proteobacteria;Alphaproteobacteria;Sphingomonadales;Sphingomonadaceae;sf_1;7110 |
| Bacteria;Proteobacteria;Alphaproteobacteria;Rhodobacterales;Rhodobacteraceae;sf_1;7125 |
| Bacteria;Cyanobacteria;Cyanobacteria;Chloroplasts;Chloroplasts;sf_5;5130 |
| Bacteria;Proteobacteria;Alphaproteobacteria;Rhodobacterales;Rhodobacteraceae;sf_1;7536 |
| Bacteria;Unclassified;Unclassified;Unclassified;Unclassified;sf_92;9999 |
| Bacteria;Proteobacteria;Deltaproteobacteria;Unclassified;Unclassified;sf_9;9993 |
| Bacteria;Proteobacteria;Alphaproteobacteria;Rhodobacterales;Rhodobacteraceae;sf_1;6805 |
| Bacteria;Unclassified;Unclassified;Unclassified;Unclassified;sf_148;5022 |
| Bacteria;Bacteroidetes;Bacteroidetes;Bacteroidales;Bacteroidaceae;sf_12;5950 |
| Bacteria;Proteobacteria;Alphaproteobacteria;Rhodobacterales;Rhodobacteraceae;sf_1;7493 |
| Bacteria;Verrucomicrobia;Verrucomicrobiae;Verrucomicrobiales;Verrucomicrobiasubdivision5;sf_1;533 |
| Bacteria;Proteobacteria;Deltaproteobacteria;Desulfobacterales;Desulfobacteraceae;sf_5;9777 |
| Bacteria;Proteobacteria;Alphaproteobacteria;Unclassified;Unclassified;sf_6;6986 |
| Bacteria;Proteobacteria;Alphaproteobacteria;Rhodobacterales;Rhodobacteraceae;sf_1;6679 |
| Bacteria;Cyanobacteria;Cyanobacteria;Nostocales;Unclassified;sf_1;5072 |
| Bacteria;Cyanobacteria;Cyanobacteria;Nostocales;Unclassified;sf_1;5199 |
| Bacteria;Cyanobacteria;Cyanobacteria;Nostocales;Unclassified;sf_1;5191 |
| Bacteria;Cyanobacteria;Cyanobacteria;Nostocales;Unclassified;sf_1;5047 |
| Bacteria;Bacteroidetes;Flavobacteria;Flavobacteriales;Flavobacteriaceae;sf_1;5509 |
| Bacteria;Bacteroidetes;Flavobacteria;Flavobacteriales;Cryomorphaceae;sf_1;5400 |
| Bacteria;Bacteroidetes;Flavobacteria;Flavobacteriales;Flavobacteriaceae;sf_1;5301 |
| Bacteria;Proteobacteria;Alphaproteobacteria;Fulvimarina;Unclassified;sf_1;7281 |
| Bacteria;Proteobacteria;Epsilonproteobacteria;Campylobacterales;Helicobacteraceae;sf_3;10614 |
| Bacteria;Firmicutes;Mollicutes;Mycoplasmatales;Mycoplasmataceae;sf_1;4102 |
| Bacteria;Dictyoglomi;Dictyoglomi;Dictyoglomales;Dictyoglomaceae;sf_9;7579 |

(continued)

| Taxa |
|---|
| Bacteria;Proteobacteria;Gammaproteobacteria;Alteromonadales;Alteromonadaceae;sf_1;9586 |
| Bacteria;Cyanobacteria;Cyanobacteria;Nostocales;Unclassified;sf_1;5004 |
| Bacteria;Proteobacteria;Alphaproteobacteria;Rhodobacterales;Rhodobacteraceae;sf_1;7383 |
| Bacteria;Proteobacteria;Gammaproteobacteria;Alteromonadales;Alteromonadaceae;sf_1;8533 |
| Bacteria;Proteobacteria;Gammaproteobacteria;Alteromonadales;Alteromonadaceae;sf_1;9247 |
| Bacteria;Proteobacteria;Gammaproteobacteria;Alteromonadales;Alteromonadaceae;sf_1;8600 |
| Bacteria;Proteobacteria;Alphaproteobacteria;Rhodobacterales;Rhodobacteraceae;sf_1;312 |
| Bacteria;Verrucomicrobia;Verrucomicrobiae;Verrucomicrobiales;Verrucomicrobiaceae;sf_6;203 |
| Bacteria;Actinobacteria;Actinobacteria;Actinomycetales;Microbacteriaceae;sf_1;1135 |
| Bacteria;Firmicutes;Clostridia;Clostridiales;Eubacteriaceae;sf_1;28 |
| Bacteria;Cyanobacteria;Cyanobacteria;Pseudanabaena;Unclassified;sf_1;5008 |
| Bacteria;Proteobacteria;Alphaproteobacteria;Rhodobacterales;Rhodobacteraceae;sf_1;6955 |
| Bacteria;Proteobacteria;Alphaproteobacteria;Rhodobacterales;Rhodobacteraceae;sf_1;7084 |
| Bacteria;Bacteroidetes;Sphingobacteria;Sphingobacteriales;Sphingobacteriaceae;sf_1;6250 |
| Bacteria;Proteobacteria;Alphaproteobacteria;Rhodobacterales;Rhodobacteraceae;sf_1;7560 |
| Bacteria;Proteobacteria;Alphaproteobacteria;Rhodobacterales;Rhodobacteraceae;sf_1;7211 |
| Bacteria;Proteobacteria;Alphaproteobacteria;Rhodobacterales;Rhodobacteraceae;sf_1;6784 |
| Bacteria;Bacteroidetes;Sphingobacteria;Sphingobacteriales;Flexibacteraceae;sf_19;6261 |
| Bacteria;Proteobacteria;Alphaproteobacteria;Rhodobacterales;Rhodobacteraceae;sf_1;6827 |
| Bacteria;Cyanobacteria;Cyanobacteria;Chloroplasts;Chloroplasts;sf_5;5060 |
| Bacteria;OD1;OP11-5;Unclassified;Unclassified;sf_1;515 |
| Bacteria;Proteobacteria;Alphaproteobacteria;Unclassified;Unclassified;sf_6;7107 |
| Bacteria;Proteobacteria;Deltaproteobacteria;Myxococcales;Polyangiaceae;sf_3;10298 |
| Bacteria;Actinobacteria;Actinobacteria;Unclassified;Unclassified;sf_1;1370 |
| Bacteria;Chloroflexi;Thermomicrobia;Unclassified;Unclassified;sf_2;652 |
| Bacteria;Bacteroidetes;Bacteroidetes;Bacteroidales;Prevotellaceae;sf_1;6152 |
| Bacteria;Spirochaetes;Spirochaetes;Spirochaetales;Spirochaetaceae;sf_1;6458 |
| Bacteria;Proteobacteria;Alphaproteobacteria;Rhodobacterales;Rhodobacteraceae;sf_1;7262 |
| Bacteria;Verrucomicrobia;Verrucomicrobiae;Verrucomicrobiales;Verrucomicrobiaceae;sf_6;871 |
| Bacteria;Proteobacteria;Gammaproteobacteria;Alteromonadales;Alteromonadaceae;sf_1;9491 |
| Bacteria;Bacteroidetes;Sphingobacteria;Sphingobacteriales;Flexibacteraceae;sf_19;5728 |
| Bacteria;Proteobacteria;Alphaproteobacteria;Sphingomonadales;Sphingomonadaceae;sf_1;7576 |
| Bacteria;Verrucomicrobia;Verrucomicrobiae;Verrucomicrobiales;Verrucomicrobiaceae;sf_7;29 |
| Bacteria;Chlorobi;Unclassified;Unclassified;Unclassified;sf_6;5294 |
| Bacteria;Cyanobacteria;Cyanobacteria;Chloroplasts;Chloroplasts;sf_5;5039 |
| Bacteria;Bacteroidetes;Flavobacteria;Flavobacteriales;Flavobacteriaceae;sf_1;5758 |
| Bacteria;Proteobacteria;Gammaproteobacteria;Oceanospirillales;Halomonadaceae;sf_1;9446 |
| Bacteria;Gemmatimonadetes;Unclassified;Unclassified;Unclassified;sf_5;1127 |

(continued)

| Taxa |
|---|
| Bacteria;Firmicutes;Clostridia;Clostridiales;Clostridiaceae;sf_12;4156 |
| Bacteria;Bacteroidetes;Sphingobacteria;Sphingobacteriales;Crenotrichaceae;sf_11;5463 |
| Bacteria;Cyanobacteria;Cyanobacteria;Plectonema;Unclassified;sf_1;5010 |
| Bacteria;Bacteroidetes;Sphingobacteria;Sphingobacteriales;Flexibacteraceae;sf_19;5994 |
| Bacteria;Proteobacteria;Gammaproteobacteria;Enterobacteriales;Enterobacteriaceae;sf_1;8173 |
| Bacteria;TM7;Unclassified;Unclassified;Unclassified;sf_1;3025 |
| Bacteria;Proteobacteria;Alphaproteobacteria;Rhizobiales;Unclassified;sf_1;7339 |
| Bacteria;Proteobacteria;Gammaproteobacteria;Oceanospirillales;Halomonadaceae;sf_1;8598 |
| Bacteria;Proteobacteria;Alphaproteobacteria;Rhizobiales;Bradyrhizobiaceae;sf_1;7096 |
| Bacteria;Cyanobacteria;Cyanobacteria;Chloroplasts;Chloroplasts;sf_5;5006 |
| Bacteria;Unclassified;Unclassified;Unclassified;Unclassified;sf_132;9820 |
| Bacteria;Proteobacteria;Alphaproteobacteria;Rhodobacterales;Rhodobacteraceae;sf_1;6862 |
| Bacteria;Cyanobacteria;Cyanobacteria;Plectonema;Unclassified;sf_1;5210 |
| Bacteria;Gemmatimonadetes;Unclassified;Unclassified;Unclassified;sf_5;2047 |
| Bacteria;Actinobacteria;Actinobacteria;Actinomycetales;Microbacteriaceae;sf_1;1186 |
| Bacteria;Proteobacteria;Alphaproteobacteria;Rhodobacterales;Rhodobacteraceae;sf_1;7364 |
| Bacteria;Proteobacteria;Alphaproteobacteria;Rhodobacterales;Rhodobacteraceae;sf_1;7453 |
| Bacteria;Proteobacteria;Alphaproteobacteria;Rhizobiales;Brucellaceae;sf_1;6757 |
| Bacteria;Proteobacteria;Deltaproteobacteria;Desulfuromonadales;Geobacteraceae;sf_1;482 |
| Bacteria;Proteobacteria;Deltaproteobacteria;Desulfobacterales;Desulfobacteraceae;sf_5;10136 |
| Bacteria;Cyanobacteria;Cyanobacteria;Chroococcales;Unclassified;sf_1;5219 |
| Bacteria;Chlorobi;Chlorobia;Chlorobiales;Chlorobiaceae;sf_1;995 |
| Bacteria;Acidobacteria;Acidobacteria;Acidobacteriales;Acidobacteriaceae;sf_16;6414 |
| Bacteria;Verrucomicrobia;Verrucomicrobiae;Verrucomicrobiales;Verrucomicrobiaceae;sf_6;613 |
| Bacteria;Proteobacteria;Alphaproteobacteria;Consistiales;Unclassified;sf_5;7592 |
| Bacteria;Proteobacteria;Alphaproteobacteria;Rhizobiales;Unclassified;sf_1;6726 |
| Bacteria;Cyanobacteria;Cyanobacteria;Chloroplasts;Chloroplasts;sf_5;5182 |
| Bacteria;Actinobacteria;Actinobacteria;Actinomycetales;Kineosporiaceae;sf_1;1598 |

**Table 11**. Clean Ocean Water Microbiome

| Taxa |
|---|
| Bacteria;Proteobacteria;Alphaproteobacteria;Rhodobacterales;Rhodobacteraceae;sf_1;6848 |
| Bacteria;Proteobacteria;Alphaproteobacteria;Rhodobacterales;Rhodobacteraceae;sf_1;7602 |
| Bacteria;Proteobacteria;Alphaproteobacteria;Rhodobacterales;Rhodobacteraceae;sf_1;6883 |
| Bacteria;Bacteroidetes;Flavobacteria;Flavobacteriales;Flavobacteriaceae;sf_1;5671 |
| Bacteria;Bacteroidetes;Flavobacteria;Flavobacteriales;Flavobacteriaceae;sf_1;5695 |
| Bacteria;Bacteroidetes;Flavobacteria;Flavobacteriales;Flavobacteriaceae;sf_1;5896 |

(continued)

| Taxa |
|---|
| Bacteria;Proteobacteria;Alphaproteobacteria;Rhodobacterales;Rhodobacteraceae;sf_1;7596 |
| Bacteria;Proteobacteria;Alphaproteobacteria;Rhodobacterales;Rhodobacteraceae;sf_1;6982 |
| Bacteria;Proteobacteria;Alphaproteobacteria;Unclassified;Unclassified;sf_6;7252 |
| Bacteria;Proteobacteria;Alphaproteobacteria;Rhodobacterales;Rhodobacteraceae;sf_1;7050 |
| Bacteria;Bacteroidetes;Flavobacteria;Flavobacteriales;Flavobacteriaceae;sf_1;5919 |
| Bacteria;Proteobacteria;Alphaproteobacteria;Rhodobacterales;Rhodobacteraceae;sf_1;7288 |
| Bacteria;Proteobacteria;Alphaproteobacteria;Rhodobacterales;Rhodobacteraceae;sf_1;7432 |
| Bacteria;Proteobacteria;Alphaproteobacteria;Rhodobacterales;Rhodobacteraceae;sf_1;6664 |
| Bacteria;Cyanobacteria;Cyanobacteria;Prochlorales;Unclassified;sf_1;5076 |
| Bacteria;Proteobacteria;Alphaproteobacteria;Unclassified;Unclassified;sf_6;7196 |
| Bacteria;Proteobacteria;Gammaproteobacteria;Alteromonadales;Alteromonadaceae;sf_1;8517 |
| Bacteria;Proteobacteria;Gammaproteobacteria;SAR86;Unclassified;sf_1;9648 |
| Bacteria;Proteobacteria;Unclassified;Unclassified;Unclassified;sf_20;7365 |
| Bacteria;Actinobacteria;BD2-10group;Unclassified;Unclassified;sf_1;1675 |
| Bacteria;Cyanobacteria;Cyanobacteria;Chloroplasts;Chloroplasts;sf_5;5007 |
| Bacteria;Proteobacteria;Alphaproteobacteria;Unclassified;Unclassified;sf_6;7510 |
| Bacteria;Proteobacteria;Gammaproteobacteria;SAR86;Unclassified;sf_1;9620 |
| Bacteria;Unclassified;Unclassified;Unclassified;Unclassified;sf_148;5235 |
| Bacteria;Cyanobacteria;Cyanobacteria;Thermosynechococcus;Unclassified;sf_1;5012 |
| Bacteria;Proteobacteria;Gammaproteobacteria;Chromatiales;Ectothiorhodospiraceae;sf_1;9387 |
| Bacteria;Proteobacteria;Gammaproteobacteria;Unclassified;Unclassified;sf_3;8647 |
| Bacteria;Proteobacteria;Alphaproteobacteria;Rhodobacterales;Rhodobacteraceae;sf_1;7054 |
| Bacteria;Proteobacteria;Alphaproteobacteria;Rhodobacterales;Rhodobacteraceae;sf_1;7233 |
| Bacteria;Proteobacteria;Alphaproteobacteria;Rhodobacterales;Rhodobacteraceae;sf_1;7045 |
| Bacteria;Proteobacteria;Alphaproteobacteria;Rhodobacterales;Rhodobacteraceae;sf_1;6960 |
| Bacteria;Proteobacteria;Alphaproteobacteria;Rhodobacterales;Rhodobacteraceae;sf_1;7405 |
| Bacteria;Proteobacteria;Alphaproteobacteria;Rhodobacterales;Rhodobacteraceae;sf_1;7329 |
| Bacteria;Proteobacteria;Gammaproteobacteria;Oceanospirillales;Alcanivoraceae;sf_1;9043 |
| Bacteria;Proteobacteria;Alphaproteobacteria;Unclassified;Unclassified;sf_6;7520 |
| Bacteria;Proteobacteria;Alphaproteobacteria;Rhodobacterales;Rhodobacteraceae;sf_1;7499 |
| Bacteria;Proteobacteria;Gammaproteobacteria;SUP05;Unclassified;sf_1;8953 |
| Bacteria;Proteobacteria;Alphaproteobacteria;Rhodobacterales;Rhodobacteraceae;sf_1;7649 |
| Bacteria;Proteobacteria;Alphaproteobacteria;Bradyrhizobiales;Unclassified;sf_1;7143 |
| Bacteria;Actinobacteria;BD2-10group;Unclassified;Unclassified;sf_1;1732 |
| Bacteria;Proteobacteria;Gammaproteobacteria;Unclassified;Unclassified;sf_3;9016 |
| Bacteria;Planctomycetes;Planctomycetacia;Planctomycetales;Planctomycetaceae;sf_3;4654 |
| Bacteria;Unclassified;Unclassified;Unclassified;Unclassified;sf_148;4970 |
| Bacteria;Proteobacteria;Alphaproteobacteria;Rhodobacterales;Rhodobacteraceae;sf_1;7429 |

(continued)

| Taxa |
|---|
| Bacteria;Actinobacteria;Actinobacteria;Actinomycetales;Acidothermaceae;sf_1;1399 |
| Bacteria;Proteobacteria;Alphaproteobacteria;Rhodobacterales;Rhodobacteraceae;sf_1;6894 |
| Bacteria;Actinobacteria;Actinobacteria;Acidimicrobiales;Acidimicrobiaceae;sf_1;1282 |
| Bacteria;Proteobacteria;Alphaproteobacteria;Rhodobacterales;Rhodobacteraceae;sf_1;7033 |
| Bacteria;Proteobacteria;Alphaproteobacteria;Rhodobacterales;Rhodobacteraceae;sf_1;7140 |
| Bacteria;Proteobacteria;Alphaproteobacteria;Rhodobacterales;Rhodobacteraceae;sf_1;7085 |
| Bacteria;Proteobacteria;Alphaproteobacteria;Unclassified;Unclassified;sf_6;7421 |
| Bacteria;Proteobacteria;Alphaproteobacteria;Unclassified;Unclassified;sf_6;6858 |
| Bacteria;Proteobacteria;Gammaproteobacteria;Unclassified;Unclassified;sf_3;8333 |
| Bacteria;Proteobacteria;Unclassified;Unclassified;Unclassified;sf_20;7541 |
| Bacteria;Proteobacteria;Gammaproteobacteria;Unclassified;Unclassified;sf_3;9061 |
| Bacteria;Proteobacteria;Alphaproteobacteria;Rhodobacterales;Rhodobacteraceae;sf_1;6796 |
| Bacteria;Firmicutes;Clostridia;Halanaerobiales;Halobacteroidaceae;sf_1;887 |
| Bacteria;Proteobacteria;Alphaproteobacteria;Rhodobacterales;Rhodobacteraceae;sf_1;6714 |
| Bacteria;Bacteroidetes;Sphingobacteria;Sphingobacteriales;Unclassified;sf_3;5799 |
| Bacteria;Planctomycetes;Planctomycetacia;Planctomycetales;Pirellulae;sf_3;4801 |
| Bacteria;Bacteroidetes;Bacteroidetes;Bacteroidales;Rikenellaceae;sf_5;5889 |
| Bacteria;Bacteroidetes;Flavobacteria;Flavobacteriales;Unclassified;sf_3;5900 |
| Bacteria;Cyanobacteria;Cyanobacteria;Chloroplasts;Chloroplasts;sf_5;4983 |
| Bacteria;Cyanobacteria;Cyanobacteria;Chloroplasts;Chloroplasts;sf_5;5111 |
| Bacteria;Cyanobacteria;Cyanobacteria;Chloroplasts;Chloroplasts;sf_5;5156 |
| Bacteria;Proteobacteria;Gammaproteobacteria;Unclassified;Unclassified;sf_3;8805 |
| Bacteria;Bacteroidetes;Sphingobacteria;Sphingobacteriales;Flexibacteraceae;sf_19;5404 |
| Bacteria;Aquificae;Aquificae;Aquificales;Hydrogenothermaceae;sf_1;737 |
| Bacteria;Proteobacteria;Alphaproteobacteria;Consistiales;Unclassified;sf_5;7504 |
| Bacteria;Bacteroidetes;Bacteroidetes;Bacteroidales;Rikenellaceae;sf_5;5945 |
| Bacteria;Proteobacteria;Alphaproteobacteria;Rhodobacterales;Rhodobacteraceae;sf_1;7224 |
| Bacteria;Proteobacteria;Betaproteobacteria;Unclassified;Unclassified;sf_3;7923 |
| Bacteria;Bacteroidetes;Unclassified;Unclassified;Unclassified;sf_4;6190 |
| Bacteria;Proteobacteria;Alphaproteobacteria;Consistiales;Unclassified;sf_5;7203 |
| Bacteria;Proteobacteria;Alphaproteobactena;Consistiales;SAR11;sf_1;7376 |
| Bacteria;Proteobacteria;Alphaproteobacteria;Rhodobacterales;Rhodobacteraceae;sf_1;7590 |
| Bacteria;Proteobacteria;Alphaproteobacteria;Rhizobiales;Unclassified;sf_1;7012 |
| Bacteria;Proteobacteria;Gammaproteobacteria;Unclassified;Unclassified;sf_3;8933 |
| Bacteria;Proteobacteria;Alphaproteobacteria;Rhodobacterales;Rhodobacteraceae;sf_1;6866 |
| Bacteria;Cyanobacteria;Cyanobacteria;Chloroplasts;Chloroplasts;sf_5;5166 |
| Bacteria;Bacteroidetes;Flavobacteria;Flavobacteriales;Flavobacteriaceae;sf_1;6104 |
| Bacteria;Cyanobacteria;Cyanobacteria;Chloroplasts;Cluoroplasts;sf_5;5221 |

(continued)

| Taxa |
|---|
| Bacteria;Cyanobacteria;Cyanobacteria;Chloroplasts;Chloroplasts;sf_5;5120 |
| Bacteria;Bacteroidetes;Bacteroidetes;Bacteroidales;Rikenellaceae;sf_5;5947 |
| Bacteria;Bacteroidetes;Bacteroidetes;Bacteroidales;Unclassified;sf_15;6078 |
| Bacteria;Proteobacteria;Gammaproteobacteria;Oceanospirillales;Unclassified;sf_3;8961 |
| Bacteria;Bacteroidetes;Flavobacteria;Flavobacteriales;Flavobacteriaceae;sf_1;5641 |
| Bacteria;Proteobacteria;Gammaproteobacteria;Methylococcales;Methylococcaceae;sf_1;8821 |
| Bacteria;Proteobacteria;Gammaproteobacteria;Acidithiobacillales;Acidithiobacillaceae;sf_1;8913 |
| Bacteria;Proteobacteria;Gammaproteobacteria;Unclassified;Unclassified;sf_3;9456 |
| Bacteria;Proteobacteria;Alphaproteobacteria;Rhodobacterales;Hyphomonadaceae;sf_1;7584 |
| Bacteria;Cyanobacteria;Unclassified;Unclassified;Unclassified;sf_5;4993 |
| Bacteria;Proteobacteria;Gammaproteobacteria;Oceanospirillales;Halomonadaceae;sf_1;9141 |
| Bacteria;Cyanobacteria;Cyanobacteria;Geitlerinema;Unclassified;sf_1;4999 |
| Bacteria;Proteobacteria;Alphaproteobacteria;Rhodobacterales;Rhodobacteraceae;sf_1;6771 |
| Bacteria;Proteobacteria;Gammaproteobacteria;Oceanospirillales;Unclassified;sf_3;9010 |
| Bacteria;Acidobacteria;Acidobacteria-9;Unclassified;Unclassified;sf_1;704 |
| Bacteria;OP10;Unclassified;Unclassified;Unclassified;sf_4;728 |
| Bacteria;Proteobacteria;Alphaproteobacteria;Rhodobacterales;Rhodobacteraceae;sf_1;7508 |
| Bacteria;Bacteroidetes;Flavobacteria;Flavobacteriales;Flavobacteriaceae;sf_1;5559 |
| Bacteria;Bacteroidetes;Bacteroidetes;Bacteroidales;Unclassified;sf_15;5998 |
| Bacteria;Proteobacteria;Gammaproteobacteria;Chromatiales;Chromatiaceae;sf_1;8407 |
| Bacteria;Proteobacteria;Gammaproteobacteria;Alteromonadales;Alteromonadaceae;sf_1;9442 |
| Bacteria;Gemmatimonadetes;Unclassified;Unclassified;Unclassified;sf_6;2554 |
| Bacteria;Proteobacteria;Alphaproteobacteria;Bradyrhizobiales;Unclassified;sf_1;7255 |
| Bacteria;Bacteroidetes;Bacteroidetes;Bacteroidales;Rikenellaceae;sf_5;6317 |
| Bacteria;Actinobacteria;Actinobacteria;Actinomycetales;Micrococcaceae;sf_1;1266 |
| Bacteria;Proteobacteria;Alphaproteobacteria;Rhodobacterales;Rhodobacteraceae;sf_1;7049 |
| Bacteria;Proteobacteria;Epsilonproteobacteria;Campylobacterales;Helicobacteraceae;sf_3;10534 |
| Bacteria;Proteobacteria;Alphaproteobacteria;Rhodobacterales;Rhodobacteraceae;sf_1;7362 |
| Bacteria;Bacteroidetes;Flavobacteria;Flavobacteriales;Flavobacteriaceae;sf_1;5955 |
| Bacteria;Proteobacteria;Unclassified;Unclassified;Unclassified;sf_21;8509 |
| Bacteria;Proteobacteria;Alphaproteobacteria;Rhodobacterales;Rhodobacteraceae;sf_1;7373 |
| Bacteria;Proteobacteria;Gammaproteobacteria;GAOcluster;Unclassified;sf_1;9008 |
| Bacteria;Proteobacteria;Alphaproteobacteria;Rhodobacterales;Rhodobacteraceae;sf_1;7032 |
| Bacteria;Proteobacteria;Alphaproteobacteria;Rhodobacterales;Rhodobacteraceae;sf_1;6661 |
| Bacteria;Bacteroidetes;Unclassified;Unclassified;Unclassified;sf_4;5637 |
| Bacteria;Proteobacteria;Gammaproteobacteria;Enterobacteriales;Enterobacteriaceae;sf_1;9309 |
| Bacteria;Proteobacteria;Alphaproteobacteria;Rhodobacterales;Rhodobacteraceae;sf_1;6979 |
| Bacteria;Proteobacteria;Gammaproteobacteria;Alteromonadales;Alteromonadaceae;sf_1;9236 |

(continued)

| Taxa |
|---|
| Bacteria;Proteobacteria;Alphaproteobacteria;Rhizobiales;Phyllobacteriaceae;sf_1;7009 |
| Bacteria;Proteobacteria;Gammaproteobacteria;Alteromonadales;Alteromonadaceae;sf_1;9486 |
| Bacteria;Cyanobacteria;Cyanobacteria;Nostocales;Unclassified;sf_1;5174 |
| Bacteria;Cyanobacteria;Cyanobacteria;Nostocales;Unclassified;sf_1;5028 |
| Bacteria;Proteobacteria;Gammaproteobacteria;Unclassified;Unclassified;sf_3;8883 |
| Bacteria;Chloroflexi;Anaerolineae;Unclassified;Unclassified;sf_9;94 |
| Bacteria;Proteobacteria;Alphaproteobacteria;Rhodobacterales;Rhodobacteraceae;sf_1;7523 |
| Bacteria;Bacteroidetes;Flavobacteria;Flavobacteriales;Flavobacteriaceae;sf_1;5490 |
| Bacteria;Cyanobacteria;Cyanobacteria;Nostocales;Unclassified;sf_1;5175 |
| Bacteria;Verrucomicrobia;Verrucomicrobiae;Verrucomicrobiales;Verrucomicrobiasubdivision7;sf_1;760 |
| Bacteria;Proteobacteria;Alphaproteobacteria;Consistiales;SAR11;sf_2;7043 |
| Bacteria;Chloroflexi;Anaerolineae;Chloroflexi-1f;Unclassified;sf_1;765 |
| Bacteria;Proteobacteria;Unclassified;Unclassified;Unclassified;sf_28;10091 |
| Bacteria;Proteobacteria;Gammaproteobacteria;GAOcluster;Unclassified;sf_1;8980 |
| Bacteria;Aquificae;Aquificae;Aquificales;Hydrogenothermaceae;sf_1;220 |
| Bacteria;Bacteroidetes;Sphingobacteria;Sphingobacteriales;Sphingobacteriaceae;sf_1;5492 |
| Bacteria;Proteobacteria;Gammaproteobacteria;Alteromonadales;Alteromonadaceae;sf_1;8863 |
| Bacteria;Cyanobacteria;Cyanobacteria;Spirulina;Unclassified;sf_1;5034 |
| Bacteria;Bacteroidetes;Flavobacteria;Flavobacteriales;Flavobacteriaceae;sf_1;5499 |
| Bacteria;Gemmatimonadetes;Unclassified;Unclassified;Unclassified;sf_5;227 |
| Bacteria;Proteobacteria;Alphaproteobacteria;Sphingomonadales;Sphingomonadaceae;sf_1;7110 |
| Bacteria;Proteobacteria;Alphaproteobacteria;Rhodobacterales;Rhodobacteraceae;sf_1;7125 |
| Bacteria;Cyanobacteria;Cyanobacteria;Chloroplasts;Chloroplasts;sf_5;5130 |
| Bacteria;Proteobacteria;Alphaproteobacteria;Rhodobacterales;Rhodobacteraceae;sf_1;7536 |
| Bacteria;Unclassified;Unclassified;Unclassified;Unclassified;sf_92;9999 |
| Bacteria;Proteobacteria;Deltaproteobacteria;Unclassified;Unclassified;sf_9;9993 |
| Bacteria;Proteobacteria;Alphaproteobacteria;Rhodobacterales;Rhodobacteraceae;sf_1;6805 |
| Bacteria;Unclassified;Unclassified;Unclassified;Unclassified;sf_148;5022 |
| Bacteria;Bacteroidetes;Bacteroidetes;Bacteroidales;Bacteroidaceae;sf_12;5950 |
| Bacteria;Proteobacteria;Alphaproteobacteria;Rhodobacterales;Rhodobacteraceae;sf_1;7493 |
| Bacteria;Verrucomicrobia;Verrucomicrobiae;Verrucomicrobiales;Verrucomicrobiasubdivision5;sf_1;533 |
| Bacteria;Proteobacteria;Deltaproteobacteria;Desulfobacterales;Desulfobacteraceae;sf_5;9777 |
| Bacteria;Proteobacteria;Alphaproteobacteria;Unclassified;Unclassified;sf_6;6986 |
| Bacteria;Proteobacteria;Alphaproteobacteria;Rhodobacterales;Rhodobacteraceae;sf_1;6679 |
| Bacteria;Cyanobacteria;Cyanobacteria;Nostocales;Unclassified;sf_1;5072 |
| Bacteria;Cyanobacteria;Cyanobacteria;Nostocales;Unclassified;sf_1;5199 |
| Bacteria;Cyanobacteria;Cyanobacteria;Nostocales;Unclassified;sf_1;5191 |
| Bacteria;Cyanobacteria;Cyanobacteria;Nostocales;Unclassified;sf_1;5047 |

(continued)

| Taxa |
|---|
| Bacteria;Bacteroidetes;Flavobacteria;Flavobacteriales;Flavobacteriaceae;sf_1;5509 |
| Bacteria;Bacteroidetes;Flavobacteria;Flavobacteriales;Cryomorphaceae;sf_1;5400 |
| Bacteria;Bacteroidetes;Flavobacteria;Flavobacteriales;Flavobacteriaceae;sf_1;5301 |
| Bacteria;Proteobacteria;Alphaproteobacteria;Fulvimarina;Unclassified;sf_1;7281 |
| Bacteria;Proteobacteria;Epsilonproteobacteria;Campylobacterales;Helicobacteraceae;sf_3;10614 |
| Bacteria;Firmicutes;Mollicutes;Mycoplasmatales;Mycoplasmataceae;sf_1;4102 |
| Bacteria;Dictyoglomi;Dictyoglonli;Dictyoglomales;Dictyoglomaceae;sf_9;7579 |
| Bacteria;Proteobacteria;Gammaproteobacteria;Alteromonadales;Alteromonadaceae;sf_1;9586 |
| Bacteria;Cyanobacteria;Cyanobacteria;Nostocales;Unclassified;sf_1;5004 |
| Bacteria;Proteobacteria;Alphaproteobacteria;Rhodobacterales;Rhodobacteraceae;sf_1;7383 |
| Bacteria;Proteobacteria;Gammaproteobacteria;Alteromonadales;Alteromonadaceae;sf_1;8533 |
| Bacteria;Proteobacteria;Gammaproteobacteria;Alteromonadales;Alteromonadaceae;sf_1;9247 |
| Bacteria;Proteobacteria;Gammaproteobacteria;Alteromonadales;Alteromonadaceae;sf_1;8600 |
| Bacteria;Proteobacteria;Alphaproteobacteria;Rhodobacterales;Rhodobacteraceae;sf_1;312 |
| Bacteria;Verrucomicrobia;Verrucomicrobiae;Verrucomicrobiales;Verrucomicrobiaceae;sf_6;203 |
| Bacteria;Actinobacteria;Actinobacteria;Actinomycetales;Microbacteriaceae;sf_1;1135 |
| Bacteria;Firmicutes;Clostridia;Clostridiales;Eubacteriaceae;sf_1;28 |
| Bacteria;Cyanobacteria;Cyanobacteria;Pseudanabaena;Unclassified;sf_1;5008 |
| Bacteria;Proteobacteria;Alphaproteobacteria;Rhodobacterales;Rhodobacteraceae;sf_1;6955 |
| Bacteria;Proteobacteria;Alphaproteobacteria;Rhodobacterales;Rhodobacteraceae;sf_1;7084 |
| Bacteria;Bacteroidetes;Sphingobacteria;Sphingobacteriales;Sphingobacteriaceae;sf_1;6250 |
| Bacteria;Proteobacteria;Alphaproteobacteria;Rhodobacterales;Rhodobacteraceae;sf_1;7560 |
| Bacteria;Proteobacteria;Alphaproteobacteria;Rhodobacterales;Rhodobacteraceae;sf_1;7211 |
| Bacteria;Proteobacteria;Alphaproteobacteria;Rhodobacterales;Rhodobacteraceae;sf_1;6784 |
| Bacteria;Bacteroidetes;Sphingobacteria;Sphingobacteriales;Flexibacteraceae;sf_19;6261 |
| Bacteria;Proteobacteria;Alphaproteobacteria;Rhodobacterales;Rhodobacteraceae;sf_1;6827 |
| Bacteria;Cyanobacteria;Cyanobacteria;Chloroplasts;Chloroplasts;sf_5;5060 |
| Bacteria;OD1;OP11-5;Unclassified;Unclassified;sf_1;515 |
| Bacteria;Proteobacteria;Alphaproteobacteria;Unclassified;Unclassified;sf_6;7107 |
| Bacteria;Proteobacteria;Deltaproteobacteria;Myxococcales;Polyangiaceae;sf_3;10298 |
| Bacteria;Actinobacteria;Actinobacteria;Unclassified;Unclassified;sf_1;1370 |
| Bacteria;Chloroflexi;Thermomicrobia;Unclassified;Unclassified;sf_2;652 |
| Bacteria;Bacteroidetes;Bacteroidetes;Bacteroidales;Prevotellaceae;sf_1;6152 |
| Bacteria;Spirochaetes;Spirochaetes;Spirochaetales;Spirochaetaceae;sf_1;6458 |
| Bacteria;Proteobacteria;Alphaproteobacteria;Rhodobacterales;Rhodobacteraceae;sf_1;7262 |
| Bacteria;Verrucomicrobia;Verrucomicrobiae;Verrucomicrobiales;Verrucomicrobiaceae;sf_6;871 |
| Bacteria;Proteobacteria;Gammaproteobacteria;Alteromonadales;Alteromonadaceae;sf_1;9491 |
| Bacteria;Bacteroidetes;Sphingobacteria;Sphingobacteriales;Flexibacteraceae;sf_19;5728 |

(continued)

| Taxa |
|---|
| Bacteria;Proteobacteria;Alphaproteobacteria;Sphingomonadales;Sphingomonadaceae;sf_1;7576 |
| Bacteria;Verrucomicrobia;Verrucomicrobiae;Verrucomicrobiales;Verrucomicrobiaceae;sf_7;29 |
| Bacteria;Chlorobi;Unclassified;Unclassified;Unclassified;sf_6;5294 |
| Bacteria;Cyanobacteria;Cyanobacteria;Chloroplasts;Chloroplasts;sf_5;5039 |
| Bacteria;Bacteroidetes;Flavobacteria;Flavobacteriales;Flavobacteriaceae;sf_1;5758 |
| Bacteria;Proteobacteria;Gammaproteobacteria;Oceanospirillales;Halomonadaceae;sf_1;9446 |
| Bacteria;Gemmatimonadetes;Unclassified;Unclassified;Unclassified;sf_5;1127 |
| Bacteria;Firmicutes;Clostridia;Clostridiales;Clostridiaceae;sf_12;4156 |
| Bacteria;Bacteroidetes;Sphingobacteria;Sphingobacteriales;Crenotrichaceae;sf_11;5463 |
| Bacteria;Cyanobacteria;Cyanobacteria;Plectonema;Unclassified;sf_1;5010 |
| Bacteria;Bacteroidetes;Sphingobacteria;Sphingobacteriales;Flexibacteraceae;sf_19;5994 |
| Bacteria;Proteobacteria;Gammaproteobacteria;Enterobacteriales;Enterobacteriaceae;sf_1;8173 |
| Bacteria;TM7;Unclassified;Unclassified;Unclassified;sf_1;3025 |
| Bacteria;Proteobacteria;Alphaproteobacteria;Rhizobiales;Unclassified;sf_1;7339 |
| Bacteria;Proteobacteria;Gammaproteobacteria;Oceanospirillales;Halomonadaceae;sf_1;8598 |
| Bacteria;Proteobacteria;Alphaproteobacteria;Rhizobiales;Bradyrhizobiaceae;sf_1;7096 |
| Bacteria;Cyanobacteria;Cyanobacteria;Chloroplasts;Chloroplasts;sf_5;5006 |
| Bacteria;Unclassified;Unclassified;Unclassified;Unclassified;sf_132;9820 |
| Bacteria;Proteobacteria;Alphaproteobacteria;Rhodobacterales;Rhodobacteraceae;sf_1;6862 |
| Bacteria;Cyanobacteria;Cyanobacteria;Plectonema;Unclassified;sf_1;5210 |
| Bacteria;Gemniatimonadetes;Unclassified;Unclassified;Unclassified;sf_5;2047 |
| Bacteria;Actinobacteria;Actinobacteria;Actinomycetales;Microbacteriaceae;sf_1;1186 |
| Bacteria;Proteobacteria;Alphaproteobacteria;Rhodobacterales;Rhodobacteraceae;sf_1;7364 |
| Bacteria;Proteobacteria;Alphaproteobacteria;Rhodobacterales;Rhodobacteraceae;sf_1;7453 |
| Bacteria;Proteobacteria;Alphaproteobacteria;Rhizobiales;Brucellaceae;sf_1;6757 |
| Bacteria;Proteobacteria;Deltaproteobacteria;Desulfuromonadales;Geobacteraceae;sf_1;482 |
| Bacteria;Proteobacteria;Deltaproteobacteria;Desulfobacterales;Desulfobacteraceae;sf_5;10136 |
| Bacteria;Cyanobacteria;Cyanobacteria;Chroococcales;Unclassified;sf_1;5219 |
| Bacteria;Chlorobi;Chlorobia;Chlorobiales;Chlorobiaceae;sf_1;995 |
| Bacteria;Acidobacteria;Acidobacteria;Acidobacteriales;Acidobacteriaceae;sf_16;6414 |
| Bacteria;Verrucomicrobia;Verrucomicrobiae;Verrucomicrobiales;Verrucomicrobiaceae;sf_6;613 |
| Bacteria;Proteobacteria;Alphaproteobacteria;Consistiales;Unclassified;sf_5;7592 |
| Bacteria;Proteobacteria;Alphaproteobacteria;Rhizobiales;Unclassified;sf_1;6726 |
| Bacteria;Cyanobacteria;Cyanobacteria;Chloroplasts;Chloroplasts;sf_5;5182 |
| Bacteria;Actinobacteria;Actinobacteria;Actinomycetales;Kineosporiaceae;sf_1;1598 |

Example 11: Clean Room Quality Testing

Traditional clean room testing relies on a wipe method and observing any spore growth in a petri dish. Comparison of the microbial communities detected using the wipe method with those detected by the PhyloChip of Example 7 are shown.

A total of 125 wipes that were applied to various clean rooms and satellite or spacecraft surfaces and their samples were compared. Each sample was about -250 mL each and hence concentrating samples are difficult. The samples were filtered using 0.45 μm filter followed by 0.2 μm filter. The resulting 10 mL fluid was concentrated using Amicon filters. DNA was extracted using a Maxwell extractor.

Table 12. Fourteen pooled samples according to spore count

| Spore Count | Number of Samples pooled and ID no. |
|---|---|
| Sample sets without spores: 7 sets | GI-15, 16, 17, 25, 26, 27, 28 |
| Spore count: 1: | GI-18 (10 samples pooled) |
| Spore count 2 to 4: | GI-19 (15 samples pooled) |
| Spore count 5 to 9: | GI-20 (5 samples pooled) |
| Spore count 10 to 11: | GI-21 (4 samples pooled) |
| Spore count 32: | GI-22 (1 sample) |
| Spore count 59: | GI-23 (1 sample) |
| Spore count 151: | GI-24 (1 sample) |

Referring now to Figure 8, the petri dish method does not predict diversity of the microbial communities found by the PhyloChip. The PhyloChip detects OTUs when even zero spores were detected by the spore count method. As shown, up to 650 OTUs were detected using the methods of testing and analysis described in Examples 1 and 2. No relationship between spore count and PhyloChip OTU counts is observed.

Referring to Figure 9, the PhyloChip is able to detect what microbial families the samples have in common or which are unique. Figure 9 shows a graphical network of the samples to show common or unique families. The dark dots are samples and the lighter dots are the family detected. Two families Pseudomonadaceae and Ralstoniaceae were found in most samples. Families connected to single samples are unique, while families connected to many samples indicate families which are likely cosmopolitan among other similar environments where the sample was found.

Referring now to Figure 10A and 10B, the pair difference score responses on the PhyloChip of Example 7 show that the PhyloChip is more sensitive to 16S amplicons and more sensitive than PCR methods. In Figure 10A, the paired difference score responses are sensitive to 16S PCR products. Frequency of all probe pairs are shown. As shown, the closer the score to zero, the more positive the probe is determined to be. A sample that was not able to be PCR amplified correlated well with our PhyloChip detection results, showing very few responsive probe pairs. Inversely, if the PCR sample was positive, then a greater number of probe pairs responded positively. In Figure 10B, four phyla were detected by the PhyloChip. Proteobacteria, Firmicutes, Bacteroidetes, and Actinobacteria, were detected even when no PCR products were detected.

<u>Example 12: Microbial Community Dynamics at the Rifle IFRC:Influence of Acetate Additions in the Field</u>

Microbial community characterization of the Rifle, CO Integrated Field Research Challenge site began nearly10 years ago. Early methodologies involved analysis of groundwater and sediments using clonal library approaches and demonstrated enrichment of *Geobacter*-like sequences. Recent research efforts at Rifle have focused on three subsequent field-scale acetate amendment experiments (Winchester [2007], Big Rusty [2008], and Buckskin [2009]) and on characterizing a naturally bioreduced area--La Quinta (2009). All of these field-amendments replicated results from earlier experiments, with uranium reduction in groundwater during biostimulation. However, additional molecular approaches have been employed to characterize the bacterial communities including PLFA, qPCR, TRFLP, and microarray analyses (Akonni and Affymetrix-based LBNL G3 PhyloChip). Quantitative PCR demonstrated significant shifts in *Geobacter* species during field amendment. TRFLP profiling also indicated *Geobacter*-like sequences represented nearly 50% of the bacterial community in groundwater at early stages of acetate amendment, with replacement by bacteria distantly related to *Acinetobacteria* and *Desulfobacter* with time. The Akonni microarray detected signals for *Geobacter, Pelobacter,* and *Geothrix,* in addition to *Dechloromonas* and *Dechlorosoma* for Winchester (2007). Furthermore, the 2007 profiles differed from 2008, which is supported by PLFA and qPCR data, indicating a residual biomass/stimulated community going into the Big Rusty experiment. The G3 PhyloChip documented how acetate-stimulated groundwater samples differed from background sediment samples by high amounts of *Geobacter* species and, to a lesser extent, *Desulfobacteraceae.* Both arrays showed a decrease in *Geobacter* species during the amendment as predominantly iron-reducing conditions transitioned to predominantly sulfate-reducing conditions.

Later samples probed by the G3 PhyloChip contained high amounts of sulfate-reducing taxa bacteria, including *Desulfobacteraceae Desulfovibrionales, Desulfitobacterium,* and *Desulfotomaculum.* To ascertain the active bacteria at the Rifle IFRC, stable isotope probing methods were employed in groundwater and sediments during the Winchester experiment. Specifically, $^{13}C$ acetate was used to assess the active microbes on three size fractions of sediments (coarse sand, fines [8-approximately 150 micron], groundwater [0.2- 8 micron]) over a 24-day time frame. Results indicated differences between active bacteria in the planktonic and particle associated phases. with a *Geobacter-like* group (187, 210, 212 bp) active in the groundwater phase, an alpha Proteobacterium (166 bp) growing on the fines/sands, and an *Acinetobacter sp.* (277 bp) utilized much of the $^{13}C$ acetate in both groundwater and particle-associated phases. Analysis of the microbial community in the naturally reduced sediment (La Quinta) indicated *Geobacteraceae* comprised 20% of the natural background community, 4 times greater than more oxidized sediment collected from the Rifle IFRC site. When La Quinta sediment was incubated with acetate, *Geobacteraceae* never became predominant, suggesting that the *Geobacteraceae* found in La Quinta may function differently from other organisms belonging to this family.

Example 13: Complexity and Heterogeneity in Biostimulated Sediment and Groundwater Communities during Iron, Sulfate, and Uranium Reduction

A phylogenetic microarray investigation into biostimulated iron- and sulfate-reducing bacterial (SRB) communities revealed unexpected similarity between sediment and groundwater fractions, variability in key functional groups, and an insight into potentially important low-abundance organisms. Bacterial communities from a range of acetate-amended and unstimulated samples associated with a U(VI) bioremediation experiment in Rifle, CO, were compared using a newly developed LBNL PhyloChip, which is able to detect DNA from tens of thousands of organisms of even extremely low abundance. In contrast, more traditional techniques (e.g. clone libraries) tend to under represent low-abundance community members.

Addition of acetate to Rifle groundwater stimulated the indigenous microbial community to reduce Fe(III) and sulfate consecutively, and U(VI) concomitantly. It is likely that abundant *Geobacter* spp. were responsible for Fe(III) and U(VI) reduction during early stage biostimulation, while sulfate was primarily reduced by *Desulfobacteraceae.* Data also suggest that minor enrichments of non-acetate-oxidizing SRB groups - *Peptococcaceae* and previously undetected *Desulfovibrio* (See Anderson RT et al. (2003) Stimulating the in situ activity of Geobacter species to remove uranium from the groundwater of a uranium-contaminated aquifer. AEM 69: 5884-5891) - included potential competitors to residual *Geobacter* spp. for enzymatic U(VI) reduction during sulfate reduction. Communities were highly similar within specific sample treatments (acetate amended: [a, b] subsurface groundwater/sediment, and [c] laboratory or [d] in-well field column sediment/quartz; [e] naturally reduced subsurface sediment), with the exception that *Geobacter* demonstrated a strong preference for attachment to the Fe(III)-bearing Rifle sediment over quartz sand in column experiments (c). Curiously, a subset of sulfate-reducing sediments (d) displayed greater similarity to Fe(III)- and sulfate-reducing groundwater communities than to other sulfate-reducing sediments (b-e). This is likely due in part to the broad overlap in elevated *Geobacter* with *Desulfobacteraceae* and *Desulfovibrionales,* and to differential increases in *Peptococcaceae,* which were limited to selective sediments (c, e).

Example 14: Uranium Biomineralization by Natural Microbial Phosphatase Activities in the Subsurface

The goal of this example is to examine the role of microbial phosphohydrolases in naturally occurring subsurface microorganisms for the purpose of promoting the immobilization uranium through the production of insoluble uranium phosphate minerals. The results of our prior NABIR-ERSP (SBR) project demonstrate that subsurface microorganisms isolated from radionuclide- and metal-contaminated soils at the DOE Oak Ridge Field Research Center (ORFRC) are acid-tolerant and resistant to numerous toxic heavy metals, including lead. In addition, many of these lead-resistant isolates exhibit phosphatase phenotypes (i.e., in particular those surmised to be phosphate-irrepressible) capable of ameliorating metal toxicity by the liberation of inorganic phosphate during growth on organophosphorus compounds, with the concomitant production of a metal-phosphate precipitate. Liberated phosphate from glycerol-3-phosphate was sufficient to precipitate as much as 95% of U(VI) as low-solubility uranium-phosphate minerals in synthetic groundwater containing either dissolved oxygen or nitrate as terminal electron acceptor in the pH range 5 to 7. In this example, we have developed an experimental approach to determine whether the activities of naturally occurring microbial phosphatases in subsurface microbial communities result in the immobilization of uranium via the formation of phosphate minerals in contaminated soils.

Characterization is being carried out of the subsurface microbial community responses of U(VI) and $NO_3^-$ contaminated ORFRC Area 2 and Area 3 soils, as well as the microbial population responses to exogenous organophosphate additions under oxic and anoxic growth conditions, soil slurry, and flow-through reactor experiments conducted at pH 5.5 and 7.0. Soil slurry and flow-through reactor experiments were conducted for 36 days and 80 days at 25°C with 10 mM G2P and 15 mM $NO_3^-$ as the sole C, P, and N sources, respectively. Under oxic growth conditions, greater than 4 mM soluble $PO_4^{3-}$ was measured at the end of the slurry incubations, and $NO_2^-$ was not detected. Preliminary data obtained for anoxic soil slurry incubations indicated an accumulation of greater than 1mM $PO_4^{3-}$, as well as the accumulation and subsequent removal of $NO_2^-$. Following triplicate incubations, 16S rDNA diversity of slurries were analyzed via high-density 16S oligonucleotide microarrays (PhyloChip). Preliminary results suggest that under oxic conditions, the microbial community structure is enriched in proteobacterial taxa at low pH as compared to the diversity of unamended soils. Analyses of slurries incubated under anoxic conditions are under way to identify bacterial taxa capable of organophosphate hydrolysis under both oxic and anoxic environments. Flowthrough reactor studies of soils with an initial pH of 3.7 demonstrated robust microbial activities once a pore water pH of 5.5 was achieved. Both denitrification and organophosphate hydrolysis were measured within 2 days of pH adjustment. Our soil slurry and column studies demonstrate the potential efficacy of organophosphate-mediated sequestration of U(VI) by the microbial community residing in ORFRC contaminated subsurface soils.

Example 15: Microbial Community Trajectories in Response to Accelerated Remediation of Subsurface Metal Contaminants

Remediation of subsurface metal contaminants at DOE sites involves microbial mechanisms of oxidation/reduction or complexation, which are controlled in large part by the ecology of the microbial community. Recognizing and quantifying the relationships between community structure, function, and key environmental factors may yield quantitative understanding that can inform future decisions on remediation strategies. We have previously found that U bioreduction and maintenance of low aqueous U concentrations is strongly dependent on the organic carbon (OC) supply rate. Our results also showed that OC supply rate had a significant effect on microbial community structure, while the effect of two different OC types was secondary over the duration of the experiment. The differences between communities attributable to different rates of OC supply diminished through time, despite the fact that different rates of OC supply resulted in different environmental conditions within the columns. Together, these data indicate that microbial communities stimulated for bioremediation may follow predictable trajectories.

Based on our prior work, and operating under the premise that microbial communities can be controlled and predicted, as well as the resulting remediation capacity, the objectives of our current project are to: (1) determine if the trajectories of microbial community structure, composition and function following OC amendment can be related to, and predicted by, key environmental determinants; and (2) assess the relative importance of the characteristics of the indigenous microbial community, sediment, groundwater, and OC supply rate as the major determinants of microbial community functional response and bioremediation capacity. We are analyzing three sediments (Oak Ridge, TN; Rifle, CO; Hanford, WA) and their microbial communities using a reciprocal transplant experimental design. Initial characterization of the three sediments show that they vary in mineralogy; particle size distribution; bulk density; base cations; CEC; SAR; iron, manganese, phosphate, and sulfate concentrations; organic and inorganic carbon concentrations; pore-water chemistry; and microbial community size and composition. Flow-through reactors, receiving simulated groundwater at two OC supply rates, are being destructively sampled over a period of 18 months. Microbial community trajectories are being followed using: 16S PhyloChip analysis of community DNA (overall structure) and RNA (active members); GeoChip functional analysis of community DNA (functional potential) and community RNA (active functions); and meta-transcriptome analyses to explore functional capacities not included on extant arrays. Geochemical characteristics of reactor effluents and sediments are being used to model factors influencing microbial community structural and functional trajectories. These analyses will provide a framework for the microbial community ecology underlying subsurface metal remediation at DOE sites.

Example 16: Quantitative Analysis Aids in Ordination

Subsurface sediments were collected from metal-contaminated DOE sites at Oak Ridge, TN, Hanford, WA, and Rifle, CO. Multiple (n=13-15) gDNA extractions using 1-3 g sediment were performed from each site. Extracts were quantified then 10 ng of gDNA was amplified by 8-temperature gradient 16S PCR. From the temperature pools, 500 ng were hybridized to the G3 PhyloChip. Hybridization intensity for each OTU was determined as the trimmed mean of PM-MM differences for each OTU's set of probe pairs. NMDS ordinations were made in R using Bray-Curtis distance for relative abundance and Sorensen for presence/absence data.

Figure 17 is a chart showing PhyloChip results from similar biological communities form ordination clusters. OTUs were called present or absent from samples taken from subsurface sediments from three different locations. A distance matrix between the samples was created based on the Sorrensen distance. The distance matrix was ordinated using NMDS and colored by sample location. Anosim analysis revels that samples within groups are more similar in composition than samples from different groups.

Figure 18 is a chart showing PhyloChip results from similar biological communities form ordination clustersOTUs were quantified from samples taken from subsurface sediments from three different locations. A distance matrix between the samples was created based on the Bray-Curtis distance. The distance matrix was ordinated using NMDS and colored by sample location. Anosim analysis revels that samples within groups are more similar in composition than samples from different groups. The R value is greater compared to previous plot indicating that relationships among similar sample types are closer when utilizing the quantitative PhyloChip data.

Example 17: Quantitative Analysis in Sludge Bioreactors

Activated sludge bioreactors are widely used to remove organics and nutrients from wastewater. However, the role of immigration in structuring activated sludge microbial communities is little understood. Converging lines of evidence from a year-long series of weekly samples at a full-scale wastewater treatment plant indicated a strong link between aeration basin influent $NO_2^-$ and shifts in activated sludge microbial community structure. To further investigate this association, we sampled four locations along a transect within this plant: 1) plant influent; 2) trickling filter biofilm; 3) trickling filter effluent; and 4) the activated sludge bioreactor. Here, we show via a polyphasic approach that influent $NO_2^-$ is a signature of microbial immigration from the upstream biofilm-based trickling filter to the activated sludge bioreactor. High-density phylogenetic microarray (PhyloChip) analyses revealed an overabundance of methanogens and sulfate-reducing bacteria in the trickling filter and suggested microbial transport to the activated sludge via the trickling filter effluent. Furthermore, ammonia-oxidizing bacterial (AOB) *amoA* copy number increased by an order of magnitude between plant influent and trickling filter effluent, indicating accumulation of AOB in the trickling filter and significant immigration to the activated sludge unit. Molecular fingerprinting (T-RFLP) analyses corroborated by clone libraries showed that *Nitrosomonas europaea* dominated the trickling filter, while a '*Nitrosomonas-like*' lineage dominated in activated sludge. *N. europaea* was previously shown to dominate in activated sludge during elevated influent $NO_2^-$ events, suggesting that activated sludge AOB community dynamics are driven in part by immigration via sloughing from the upstream trickling filter.

Figure 19 and 20 illustrate the analysis that was performed using the PhyloChip G3 array. Figure 19 shows an NMS analysis demonstrating that the four sampling sites are quite distinct, and that the biological replicates show quite high levels of similarity. Figure 20 is a heatplot summary of an analysis called the Method of Shrunken Centroids. The basic idea of this analysis is to identify the ~50 or so microbial OTUs that most significantly define the observed differences in overall community structure between sampling locations. As we hypothesized, anaerobes (particularly methanogens) are well represented in this set of 50 microbial types, and we see evidence of transport between sampling locations (namely the trickling filter and the activated sludge aeration basin) of these microbes. In addition, *Nitrospira* (nitrite-oxidizing bacteria) are also fairly well represented in this "minimal" dataset. Notably, we see small levels of nitrite accumulation in one of the sampling locations-- the trickling filter biofilm-- in which the PhyloChip results indicate essentially an absence of *Nitrospira,* and essentially no nitrite accumulation in the downstream activated sludge unit, where *Nitrospira* are much more abundant.

Taken together, our results provide compelling evidence that immigration between coupled process units can significantly influence activated sludge microbial community structure.

Example 18: PhyloChip G3 Analysis on the Impact of Climate Change on Redwood Forests

This project examined the potential impacts of climate change on the composition of soil microbial communities in coastal redwood forests. Understanding their response to climate change is important for predicting changes in ecosystem services and of interest to ecosystem stewards.

A 3-way reciprocal transplant experiment was conducted across the latitudinal gradient of coastal redwood forests. Samples were collected 1 year and 3 years after transplanting. Bacterial community composition was analyzed using a high-density 16S rDNA microarray (PhyloChip). Climatic variables and soil variables (rainfall, soil moisture, soil temperature, soil C and N availability, pH, soil texture) were measured. Changes in community composition were assessed with non-metric multidimensional scaling (for the entire community) and ANOVA (for individual taxa). The relationships between bacterial community composition and climatic and edaphic variables were examined with Mantel tests.

: The change in climate had an intermediate to strong influence on bacterial community composition. The amount of rainfall and its impact on soil moisture were the strongest and most significant correlates with community composition. In addition, the number of bacterial species that responded to the change in climate increased from year 1 to year 3.

The results indicate that climate change has an intermediate to strong influence on bacterial community composition at a regional scale. The amount of rainfall had the most significant correlation with bacterial community composition. While other factors, such as species interactions or other stochastic processes, may also greatly influence changes in community composition over time, it appears that the number of species that respond to the impact of climate change increases with time and 3 years may not be long enough to assess the long-term impact of climate change on microbial community composition.

Table 13 shows significant standardized Mantel statistics (r) for the relationships between the bacterial community composition of transplanted samples and controls and environmental variables, for both one and three years after samples were transplanted.

Table 13.

| Environmental variable | Axis 1 Mantel test r | Mantel test p-value |
|---|---|---|
| **1 year after transplanted** | | |
| Annual rainfall | 0.19 | 0.013 |
| Late spring rain | 0.19 | 0.019 |
| All env. variables | 0.19 | 0.015 |
| | | |
| **3 years after transplanted** | | |
| Annual rainfall | 0.17 | 0.009 |
| Summer rain | 0.19 | 0.007 |
| Gravimetric water content | 0.18 | 0.040 |
| Temperature | 0.13 | 0.047 |
| Maximum temperature | 0.12 | 0.034 |
| Annual rainfall + temperature | 0.17 | 0.011 |

Table 14. Bacteria (OTUs) that respond to transplanting after 1 year and 3 years

| Phylum/Division | Class | After 1 year (no.*) | After 3 years (no.*) |
|---|---|---|---|
| Acidobacteria | | 1 | 17 |
| Actinobacteria | | 0 | 38 |
| Bacteroidetes | | 1 | 8 |
| Chlorflexi | | 0 | 14 |
| Firmicutes | | 16 | 39 |
| Planctomycetlaes | | 0 | 9 |
| Proteobacteria | Alpha- | 11 | 104 |
| | Beta- | 21 | 15 |
| | Delta- | 0 | 11 |
| | Gamma- | 20 | 13 |
| Spirochaetes | | 1 | 18 |
| Other | | 3 (from 2 divisions) | 39 (across 18 divisions) |
| | | | |
| **TOTAL** | | 74 | 325 |

The number of OTUs that have a difference in relative abundance (OTU intensity) between treatments (origin-incubation combinations) by ANOVA at $p<0.10$.

Figure 21 is a representation of differing degrees of change in community composition in response to a change in climate. The open squares represent the position of a Southern-lat. site in an ordination, and the black squares represent the position of a Northen-lat.site. The open triangles represent the community of a Northan-lat. site that experienced the Southern-lat. climate. The length of the arrow shows the degree of change.

Figure 22 is two charts showing NMS ordinations of: a) Fresh samples collected from the North, Mid and South-lat. sites in August 2005 and b) fresh samples and transplant-control samples from the same sited at the same time (1 year after transplanting). The fresh samples depicted in both graphs are the same samples. The bars represent 1 s.d. of 3 replicates.

Figure 23 is four charts showing NMS ordinations of reciprocally transplanted samples and transplanted controls collected 1 year after they were transplanted. Arrows show the trajectory of the change in composition of transplanted samples away from that of their site-of-origin controls.

Figure 24 shows 2 charts showing the NMS ordinations of: a) Fresh samples collected from the North, Mid and South-lat. sites in September 2007 and b) fresh samples and transplant-control samples from the same sites at the same time (3 years after transplanting). The fresh samples depicted in both graphs are the same samples. The bars represent 1 s.d. of 3 replicates.

Figure 25 is four charts showing NMS ordinations of reciprocally transplanted samples and transplanted controls collected 3 years after they were transplanted. Arrows show the trajectory of the change in composition of transplanted samples away from that of their site-of-origin controls.

<u>Example 19: Microbial community analysis of mammalian and avian sources of fecal contamination in coastal California</u>

Wild and domestic animals that inhabit coastal areas deposit fecal microorganisms that impact water quality. The extent to which coastal waters are impaired by various human and animal sources of fecal pollution is hard to determine with single biomarkers and low-resolution profiling methods. High-throughput sequence analysis of gut microbial communities has potential to reliably identify fecal sources and resolve contentious water quality issues. In this study we characterized bacterial communities from a variety animal feces and human wastes to identify taxa that distinguish contamination sources. We then tested the utility of these findings during water pollution events.

Fresh fecal samples were collected from at least four geographically-distinct populations each of gulls, geese, pinnipeds (seals and sea lions), cows, horses and elk. Human sewage and septic waste were gathered from multiple locations. We analyzed bacterial 16S rRNA gene composition using the PhyloChip micrорray, which is capable of quantifying differences in the relative abundance of both rare and abundant bacterial taxa by detecting the entire targeted pool of 16S rRNA gene copies in each sample.

Ambient water samples were collected weekly over two years at nine recreational beaches in N. California and during a major sewage spill in San Francisco Bay. Water samples were measured using common fecal indicator tests and analyzed using the PhyloChip for source identification.

Fecal bacterial communities strongly clustered by animal species/type. We identified thousands of bacterial taxa that distinguished human wastes from animal feces, and different animals from each other. Human waste samples clustered together despite differences in the scale and type of processing. Bacterial communities in cows and elk were nearly indistinguishable, and there was little variation among different populations of these ruminants. In contrast, bacterial communities in birds were much more variable among populations, even within the same species. Horse populations clustered with other grazers but were distinct in composition from the ruminants. Analysis of water samples during pollution events demonstrated that libraries of distinctive taxa developed from our source characterization could successfully identify or exclude causes of contamination.

Cluster analysis of detected bacterial taxa in fecal samples and clean water samples was performed and showed that the PhyloChip G3 array detected 3513 different bacterial subfamilies in fecal samples (passed stage 1 analysis). Strong clustering by species and type of animal (ruminants and grazers, pinnipeds, birds) was shown and displayed in Figure 26. Using the PhyloChip G3 array, human sources (septic tanks, sewage) are distinct from animals and wildlife, and background waters. Source identifier communities were defined for each source. Detected OTUs (pass stage 1) had significantly higher array intensity than background waters (t-test and difference in avg. array intensity >2000) (Figure 27). In Figure 28, indicator communities were compared to polluted water samples for source identification.

Sewage taxa with strong correlations to FIB are shown in Figure 29. Abundances of 4,625 different taxa found in sewage were strongly correlated (r>0.9) with fecal indicators. The most correlated taxa were Bacteroidales and Clostridia.

Not shown is a phylogenetic tree of potential indicator taxa identified in Tomales Bay diffusion chamber experiment. Potential indicator taxa are OTUs that are unique to a particular waste and absent in the receiving waters. There were 165 potential indicator taxa identified for dairy farm waste and 119 indicator taxa identified for septic tank waste. A total of 13,341 different taxa were detected in waste and receiving water samples with the G3 chip.

Figure 30 shows results of cluster analysis which showed the comparison of community composition. Communities can be clustered according to the time in the receiving waters, source, and type of receiving waters.

Figure 31 is a bar chart showing the effect of time in receiving waters on fecal microbial communities. A four day immersion shows differences in persistence among taxonomic groups with similar shifts in cattle and septic communities. Most proteobacteria decrease in relative abundance over time. Clostridia increase in relative abundance over time.

Figure 32 is a bar chart showing the effect of creek versus bay water on waste microbial communities. Similar response of cattle and septage communities to different water types is illustrated. Clostridia, γ-proteobacteria, coliforms favored in creek while β-proteobacteria is favored in Bay. Selection of molecular indicators for monitoring should consider persistence of taxa under relevant conditions

Thus, different animals harbor distinct fecal microbial communities that can be exploited for source tracking in spite of intra-source variability due to diet, location or processing

Example 20: Evaluation of Oil Spill Effects and Clean-up on Ocean Microbiome

The methods, compositions, and systems of the invention can be applied to evaluate the effects of changes in an environment on the microbiome supporting and supported by that environment. In this example, an array of the invention is used to establish a baseline for the microbiome of healthy ocean environments, and this baseline is then used to assess the effects of an oil spill on the microbiome, as well as to assess the progress of recovery efforts.

Microbial DNA is isolated from 150 samples representing the diverse ecosystems affected by the oil spill, as well as ~100 samples from similar, unaffected ecosystems. Samples are collected from a representative range of ocean depths, commercial and recreational fishing areas and coastal areas, e.g., beach and marsh surface water, inlets, and lagoons. Ideally, multiple samples (5-10) are collected per site initially and at each quarterly re-sampling.. DNA is extracted from the sample, amplified, processed, and analyzed as in Example 2. Analysis by probe hybridization is conducted using an array, such as described in Examples 2 and 7. The presence, absence, and/or level is scored for each probe evaluated, and/or for each OTU represented by the probes evaluated. The result is a biosignature for unaffected ocean environments and a biosignature for ocean environments affected by the oil spill. Analysis and bioinformatic data mining of the results produces reports on the status of the microbial populations at each site, as well as an interpretative report indicating the scope of damage to the microbial ecosystem services as compared to undamaged, similar marine ecosystems.

Thereafter, samples are collected from each monitoring site on a quarterly basis, and changes from the initial biosignature of oil spill affected areas as well as continuing ecosystem damages relative to unaffected, similar ecosystem sampling sites, are assessed. The relative success of restoration efforts, measured in terms of degree of improvement in similarity between spill-affected biosignatures and unaffected biosignatures, can be used to inform the most appropriate actions for containment or dispersal of future oil spill disasters. Profiles for each healthy marine microbial ecosystem evaluated are established between 3-5 quarters of sampling and take into account normal seasonal fluctuations in the relative abundance and diversity of particular microbial species. By comparing microbial biosignatures from remediated sites with unaffected sites, including confidence and probability information, site specific restoration is tracked. Once these parameters are established, progress towards remediation from the oil spill damage and restoration of healthy, functioning marine ecosystems is projected and qualified. Degree of restoration is assigned a restoration score, which represents a percentage of similarity between the biosignatures of unaffected and affected ocean environments. High similarity of affected treated areas to unaffected area microbial populations provides evidence that spill areas have recovered and are capable of supporting healthy marine life. Tracking increases in similarity between the biosignatures of unaffected and affected ocean environment provides a projection of time to restoration to the unaffected state, as well as defining an endpoint for remediation efforts, wherein remediation efforts are halted once a threshold of similarity is reached. Thresholds can be higher than about 80%, 85%, 90%, 95%, 97.5%, 98%, 95.5%, 99%, 99.1%, 99.2%, 99.3%, 99.4%, 99.5%, 99.6%, 99.7%, 99.8%, 99.9%, 99.95%, or higher similarity.

Example 21: Effects of deep water oil plume on bacterial community

The oil from the Deepwater Horizon spill in the Gulf of Mexico represents an enormous carbon input to this ecosystem, and hydrocarbon components in the oil could potentially serve as a carbon substrate for the microorganisms present in the water. The impact of the plume on the microbial community and its potential for hydrocarbon degradation was evaluated. This study covers 19 sampling sites on the cruises for two ships from May 25 to June 2, 2010.

Sample Collection

A colored dissolved organic matter (CDOM) WETstar fluorometer (WET Labs, Philomath, OR) was attached to a CTD sampling rosette (Sea-Bird Electronics Inc., Bellevue, WA) and used to detect the presence of oil along depth profiles between the surface and seafloor. Fluorometer results were subsequently confirmed with laboratory hydrocarbon analysis. A total of seventeen samples were analyzed from ten locations.

Niskin bottles attached to the CTD rosette were used to capture water samples at various depths inside and outside waters with detected hydrocarbons. From each sample 800-2000 mL of water were filtered through sterile filter units containing 47 mm diameter polyethylsulfone membranes with 0.22 μm pore size (MO BIO Laboratories, Inc., Carlsbad, CA) and then immediately frozen and stored at -20°C. Filters were shipped on dry ice and stored at -80°C until DNA and phospholipid fatty acid (PLFA) extraction.

100 mL of water was syringe-filtered and injected into pre-evacuated 25 mL serum bottles capped with thick butyl rubber stoppers. 100 mL of water was frozen in 125 mL HDPE bottles for nutrient analyses. For AODC 36 mL water was preserved in 4% formaldehyde (final concentration).

DNA Extraction

One quarter of each filter was cut into small pieces and placed in a Lysing Marix E tube (MP Biomedicals, Solon, OH). 300 μL of Miller phosphate buffer and 300μL of Miller SDS lysis buffer were added and mixed. 600 μL phenol:chloroform:isoamyl alcohol (25:24:1) was then added, and the tubes were bead-beat at 5.5m/s for 45sec in a FastPrep instrument. The tubes were spun at 16,000 x g for 5 min at 4°C. 540 μL of supernatant was transferred to a 2 mL tube and an equal volume of chloroform was added. Tubes were mixed and then spun at 10,000 x g for 5 min 400 μL aqueous phase was transferred to another tube and 2 volumes of Solution S3 (MoBio, Carlsbad, CA) was added and mixed by inversion. The rest of the clean-up procedures followed the instructions in the MoBio Soil DNA extraction kit. Samples were recovered in 60μL Solution S5 and stored at -20°C.

PCR Amplification

The 16S rRNA gene was amplified using PCR with primers 27F and 1492R for bacteria, and 4Fa and 1492R for archaea. Each PCR reaction contained 1× Ex Taq buffer (Takara Bio Inc., Japan), 0.025 units/μl Ex Taq polymerase, 0.8 mM dNTP mixture, 1.0 μg/μl BSA, and 200 pM each primer and 0.15-0.5 ng genomic DNA as template. For the PhyloChip assay (PhyloTech Inc., San Francisco, CA) analysis each sample was amplified in 4 replicate 25 μl reactions spanning a range of annealing temperatures. PCR conditions were 95°C (3 min), followed by 30 cycles 95°C (30 s), 46-56°C (25 s), 72°C (2 min), followed by a final extension 72°C (10 min). Amplicons from each reaction were pooled for each sample, purified with the QIAquick PCR purification kit (Qiagen, Valencia, CA), and eluted in 20 μL elution buffer.

Phylochip Assay Design

The PhyloChip microarray probe design was applied to all known high-quality 16S rRNA gene sequences containing at least 1,300 nucleotides. Sequences (Escherichia coli base pair positions 47 to 1473) were extracted from the NAST multiple sequence alignment available from the16S rRNA gene database, greengenes.lbl.gov. This region was selected because it is flanked by universally conserved segments that can be used as PCR priming sites to amplify bacterial or archaeal genomic material using only 2 to 4 primers. Putative chimeric sequences were identified and removed where Bellerophon divergence ratios >=1.1 with >=90% lane-masked identity to one or both putative parents were encountered. Sequences containing three or greater homo-octomers or longer, or those with >=0.3% ambiguous base calls , were also omitted. From the sub-alignment, putative 25- mer targets were selected with G+C content of 35-75%, secondary structure free energy (ΔG) >= -4 kcal/mol as calculated by RNAfold (17), complimentary melting temperature of 61°C - 80°C, and self-dimerazation melting temperature < 35°C as calculated by Thermalign.

Filtered rRNA gene sequences were clustered to enable selection of perfectly complementary probes representing each sequence of a cluster. Putative amplicons containing 17-mers with sequence identity to a cluster were included in that cluster. The resulting 59,959 clusters, each encapsulating an average of 0.5% sequence divergence were considered operational taxonomic units (OTUs). The OTUs represented 2 domains, 147 phyla, 1,123 classes, and 1, 219 orders demarcated within the archaea and bacteria. Each OTU was assigned to one of 1,464 families according to the placement of its member organisms in the taxonomic outline as maintained by Philip Hugenholtz (Hugenholtz 2002, Genome Biol. 3(2): 1-8). The OTUs comprising each family were clustered into sub-families by transitive (single linkage) sequence identity of 72% common heptamers. Altogether, 10,993 sub-families were found. The taxonomic position of each OTU as well as the accompanying NCBI accession numbers of the sequences composing each OTU are available in the files sequences_by_OTU_G3.gz, taxonomy_by_OTU_G3.gz.

For each OTU, multiple specific 25-mer targets were sought for prevalence in members of a given OTU but dissimilar from sequences outside the given OTU. In the first step of probe selection for a particular OTU, each of the sequences in the OTU was separated into overlapping 25-mers, the potential targets. Then each potential target was matched to as many sequences of the OTU as possible. The multiple sequence alignment provided by Greengenes was used to provide a discrete measurement of group size at each potential probe site. For example, if an OTU containing seven sequences possessed a probe site where one member was missing data, then the site-specific OTU size was only six. In ranking the possible targets, those having data for all members of that OTU were preferred over those found only in a fraction of the OTU members. In the second step, a subset of the prevalent targets was selected and the probe orientation was flipped to the reverse complement to minimize hybridization to unintended amplicons. Probes presumed to be potentially problematic were 25-mers containing a central 17-mer matching sequences in more than one OTU. Thus, probes that were unique to an OTU solely due to a distinctive base in one of four flanking bases were avoided. Also, probes having a common tree node near the root were favored over those with a common node near the terminal branch. Probes complementary to target sequences that were selected for fabrication are termed perfectly matching (PM) probes. As each PM probe was chosen, it was paired with a control 25-mer (mismatching probe, MM), identical in all positions except the thirteenth base. The MM probe did not contain a central 17-mer complimentary to sequences in any OTU. The PM and MM probes constitute a probe pair analyzed together. The average number of probe pairs assigned to each OTU was 37 (s.d. 9.6).

The chosen oligonucleotides were synthesized by a photolithographic method at Affymetrix Inc. (Santa Clara, CA) directly onto a glass surface at an approximate density of 10,000 molecules per $\mu m^2$ and placed into "midi 100 format" hybridization cartridges. The entire array of 1,016,064 probe features was arranged as a grid of 1,008 rows and columns. Additional probes for quality management, processing controls, image orientation, normalization controls, hierarchical taxonomic identification, for pathogen-specific signature detection and some implement additional targeted regions of the chromosome. Furthermore, probes complementary to lower confidence 16S sequences were included to enable broadening the phylogenetic scope of the analysis, when those sequences are validated with unambiguous entries into public repositories. The PhyloChip assay design includes control probes for preanalytic, processing, prelabeled hybridization controls, and negative controls. Preanalytic and hybridization controls can also be used in interpretation of background signal intensity and to support normalization of overall fluorescent intensity for sample to sample comparisons.

Sample Preparation forPhyloChip Assay

From Deep Horizon nucleic acids, 500 ng of bacterial PCR product and 25 ng of archaeal PCR product were prepared for hybridization. PCR products were fragmented to a range of 50-200bp as verified by agarose gels. Commercial kits were utilized for DNA preparation: Affymetrix (Santa Clara, CA) WT Double Stranded DNA Terminal Labeling, and Affymetrix GeneChip Hybridization, Wash, and Stain kits were used for analysis. Briefly, fragmented 16S amplicons and non-16S quantitative amplicon reference controls were labeled with biotin in 40uL reactions containing: 8μL of 5X TDF buffer, 40 units of TDF, 3.32 nanomoles of GeneChip labeling reagent. After incubating at 37°C for 60 min, 2uL of 0.5M EDTA was added to terminate the reaction. Labeled DNA was combined with 65μL of 2X MES hybridization buffer, 20.4μL of DMSO, 2μL of Affymetrix control oligo B2, and 0.4μL nuclease free water. Each reaction mixture was injected into the hybridization chamber of an array cartridge and incubated for 16 hours in an Affymetrix hybridization oven at 48°C and 60 RPM. Hybridization solution was removed and the microarrays were stained and scanned according to the manufacturer's instructions.

PhyloChip Assay Analysis

Fluorescent images were captured with the GeneChip Scanner 3000 7G (Affymetrix, Sanat Clara, CA). An individual array feature occupied approximately 8x8 pixels in the image file corresponding to a single probe 25mer on the surface. The central 9 pixels were ranked by intensity and the 75% percentile was used as the summary intensity for the feature. Probe intensities were background-subtracted and scaled to the Quantitative Standards (non-16S spike-ins) and outliers were identified as previously described(DeSantis et al. 2007, Microb. Ecol. 53: 371). The hybridization score (HybScore) for an OTU was calculated as the mean intensity of the perfectly matching probes exclusive of the maximum and minimum.

Comparison of the PM and corresponding MM intensities is summarized as the pair difference score, d, described above. The d scores are standardized to enable comparison of probe pairs with various nucleotide compositions. The goal in this transformation is determining if a pair's d value is more similar to d values derived from negative controls (NC, probe pairs without potential cross-hybridization to any 16S rRNA sequence nor Quantitative Standards) or to d values from positive controls, the Quantitative Standards (QS, probe pairs with PM's matching the non-16S rRNA genes which are spiked into the experiment). Because the $d_{QS}$ values are dependent on their target's A+T count and T count, the QS pairs are grouped by these attributes into classes and a separate distribution of $d_{QS}$ values are found for each. The dNC values are grouped in the same way. A distribution is estimated for each class from the observations. Each d value from an OTU probe set is compared to the distributions of $d_{QS}$ and $d_{NC}$ from the same class to produce a pair response score, r (described above). The r scores for a set of probe pairs complimentary to an OTU are considered collectively in Stage 1 probe set Presence/Absence scoring. At minimum, 18 probe pairs are considered. The r scores are ranked and the quartiles, $rQ_1$, $rQ_2$ and $rQ_3$ are found. For an OTU to pass Stage 1, all three of the following criteria must be met: $rQ_1 \geq .70$, $rQ_2 \geq 0.95$, and $rQ_3 \geq 0.98$. OTUs which pass Stage 1 are considered in Stage 2 scoring for subfamily detection. In this stage, a cross-hybridization adjusted response score, $r_x$, is calculated for all responsive probes (r > 0.5), described above. After all penalties are considered, the $r_x$ values are ranked and quartiles found as above ($r_xQ_1$, $r_xQ_2$, $r_xQ_3$). Subfamilies having a $r_xQ_3$ values >=0.48 were considered present.

Significantly enriched OTUs within the plume were defined as those achieving a p-value <0.05 with Student's t-test upon $log_2$(HybScores), Stage1 present call in >=4 of 9 plume samples, and an increase in mean HybScores compared to background (outside of plume samples) of >1000 units and >35%.

PhyloChip Assay Performance

Twenty-six 16S rDNA mixtures from different species were prepared as mock communities using a semi-randomized Latin square structure described by Jacobson and Mathews (Jacobson et al. 1995, Journal of Combinatorial Designs 4: 405). A stepwise function was used so that each successive organism was added at a final concentration 37% greater than the previous organism. Each test organism was represented in all mixtures at each possible concentration step. The 26 DNA mixtures were hybridized in triplicate on different days. Also as a control, one hybridization was carried out using only the quantitative reference controls. All 16S probe pairs producing a response score, r, above 0.5 for the reference controls were masked from subsequent analysis.

Background-subtracted probe intensities from 12,202 replicate probes representing 3,548 different 25-mer combinations were used to determine the coefficient of variation (CV) for each assay. Overall, the variations were minor producing a mean CV = 0.097. Additionally, a significant correlation was found between the concentrations of each gene in the Latin Square and the corresponding HybScore generating and average correlation coefficient, r = 0.941).

The ability to detect and classify amplicons within the hybridization mix was evaluated using receiver operating characteristic (ROC) curves. The $rQ_1$, $rQ_2$ and $rQ_3$ probe set summarizations were collected from each of the possible OTUs from all Latin Square results. ROC curves were plotted to evaluate the effect of choosing a single threshold to determine presence. The γ-axis, Expected Positive Rate, is the fraction of OTUs expected to be present that were called present. The x-axis, Unexpected Positive Rate, is the fraction of OTUs not-expected to present that were called present. Presence/Absence thresholds for each quartile were varied from 0, least stringent to 1, most stringent. For example, in the $rQ_1$ plot, a threshold of 0.5 allows 97.5% of the expected detection events to pass. Instead of relying on a single threshold to determine presence, all three quartiles of a probe set are examined to ensure the distribution of response scores are skewed toward 1. Collectively, $rQ_1 \geq .70$, $rQ_2 \geq 0.95$, and $rQ_3 \geq 0.98$ was required to achieve a 0.961 Expected Positive OTU Rate for amplicons >2 and <348 pM with a 0.020 Unexpected Positive OTU Rate. In Stage 2 $r_xQ_3$ subfamily thresholds set at 0.48 allowed a 0.969 Expected Positive Subfamily Rate with a corresponding 0.019 Unexpected Positive Subfamily Rate when applied to the Latin Square data over the same concentration range.

Hybridization results were reduced to a community profile from each PhyloChip assay in a format useful for multivariate statistics. OTUs passing Stage 1 within subfamilies passing Stage 2 constituted the community profile. Replicate community profiles of the Latin Square mock communities were compared by ordination. Inter-profile distance was calculated with either the Bray-Curtis or weighted Unifrac method and resulting distance matrices were ordinated with non-metric multidimentional scaling(NMDS). Profiles from each of the 26 mock communities were clearly distinguishable using either distance method. Analysis of variance using either distance matrix (Adonis) concluded a significant difference among mock-communities (p<0.005).

Results

The plume significantly altered microbial community phylogenetic composition and structure. Using a phylogenetic microarray (PhyloChip assay), a 40% decline in detectable bacterial richness was found and a significant shift in microbial community composition. Ordination of community composition determined by phylogenetic microarray analysis revealed two distinct clusters of samples: one composed entirely of samples with detected oil and the other with samples that had no oil detected. No other physical or chemical factors other than hydrocarbons were significantly different between these groups, indicating that microorganisms are responding directly to the presence of dispersed oil.

Only bacteria in the class γ-proteobacteria were significantly enriched in plume samples (Table 15). In plume samples 951 distinct bacterial taxa in 62 phyla were detected, but only sixteen distinct taxa that were all classified as g-proteobacteria were significantly enriched by the plume relative to deep waters outside the plume (Table 15, Fig. 33). Nearly all of enriched taxa are known to degrade hydrocarbons or are stimulated by the presence of oil in cold environments (Table 15). Plume-enriched bacteria include many psychrophilic and psychrotolerant species that are known from cold ocean waters, sea ice and circum-polar habitats. The results indicate that these γ-proteobacteria dominate the microbial community in the deep-sea plume. While cell densities are higher, taxonomic richness is lower and the diversity of enriched bacteria is restricted to these few γ-proteobacteria. *Oceanospirillales* in the γ-proteobacteria was detected in all 9 oil plume samples analyzed by the PhyloChip assay, and was significantly enriched relative to background deep seawater with no oil.

Table 15. γ-proteobacteria taxa enriched by the oil plume. Taxa that include known hydrocarbon degraders or previously shown in cold waters to become enriched in response to hydrocarbons are indicated.

| Class | Family | Hydrocarbon degraders* | Enriched by crude oil* | Representative sequence |
|---|---|---|---|---|
| Aeromonadaceae | Aeromonadaceae | + | + | DQ816633.1 Zebrafish gut clone |
| Alteromonadales | Colwelliaceae | ND | + | EU491914.1 East Pacific Rise deepwater clone |
| Alteromonadales | Pseudoalteromonadaceae | + | + | AY646431.1 *Pseudoalteromonas* sp. |
| Arctic96B-1 | Unclassified | ND | + | EU544859.1 Arctic seawater clone |
| BPC036 | Unclassified | ND | + | DQ925906.1 Guaymas Basin clone |
| Halomonadaceae | Halomonadaceae | + | + | DQ270747.1 *Halomonas* sp. |
| Marinobacter | Marinobacter | + | + | DQ157009.2 *Marinobacter haloterrigenus* |
| Marinospirillum | Marinospirillum | + | + | AF275713.1 *Marinospirillum alkaliphilum* |
| Moraxellaceae | Moraxellaceae | + | + | A200213.1 Psychrophilic marine isolate |
| Oceanospirillales | Marinobacterium | + | + | AY549003.2 Marine bone clone |
| Oceanospilillales | Marinomonas | + | + | EF673290.1 *Marinomonas* sp. |
| Oceanospirillales | Unclassified | + | + | AM747817.1 *Oceaniserpentilla haliotidis* |
| Pseudomonadaceae | Pseudomonadaceae | + | + | AM111047.1 *Pseudomonas* sp. |
| Shewanellaceae | Shewanellaceae | + | + | DQ665797.1 *Shewanella frigidimarina* |
| Unclassified | Unclassified_sfB | ND | ND | EU491790.1 East Pacific Rise seafloor clone |
| Unclassified | Unclassified_sfC | ND | ND | EU652559.1 Yel Sea sediment clone |
| ND = No Data | | | | |

Figure 33 provides an illustration of enrichment of select bacterial taxa by the oil plume. Phylogenetic microarray analysis was used to calculate average difference in estimated concentration between plume and non-plume samples. Average difference is shown as a percentage of non-plume concentration for representative OTUs in enriched taxonomic subfamilies (Table 15).

## Claims

1. A system for detecting in a single assay a plurality of operational taxonomic units (OTUs) in a sample, wherein each of a plurality of first nucleic acid sequences comprise less than 0.01 percent of the total nucleic acids in a population of nucleic acid sequences from the sample; each of the plurality of first nucleic acid sequences are at least 95% homologous to all of the other first nucleic acid sequences in the population of nucleic acid sequences; the system comprising

   (a) a plurality of probe pairs for each of at least 10,000 OTUs, each probe pair comprising a perfect match (PM) probe and a mismatch (MM) probe;
   (b) a plurality of negative and positive control probes; and
   (c) computer executable logic for deconvoluting signal intensities of the plurality of probe pairs into probability estimates by:

      (i) comparing signal intensities for PM probes, MM probes, positive control probes, and negative control probes;
      (ii) calculating a probability that an individual OTU is present based on the signal intensities of probes for that OTU; and
      (iii) penalizing the probability based on potential for cross-hybridization of probes with sequences from other OTUs.

2. The system of claim 1, wherein said system detects presence, absence, relative abundance, and/or quantity of more than 10,000 different OTUs of a single domain in a single assay with confidence greater than 95%.

3. The system of claim 1, wherein one or more of said first nucleic acid sequences are 16S rRNA gene, 23S rRNA gene, 5S rRNA gene, 5.85 rRNA gene, 12S rRNA 20 gene, 18S rRNA gene, 28S rRNA gene, gyrB gene, rpoB gene, fusA gene, recA gene, coxl gene, nifl3 gene, RNA molecules derived therefrom, or a combination thereof.

4. The system of claim 1, said system comprising at least 20,000 PM probes, and a plurality of MM probes for each PM probe, wherein each PM probe detects a different first nucleic acid sequence, and each MM probe sequence differs from the PM probe to which it corresponds by at least one nucleotide.

5. The system of claim 4, having one or more of the following characteristics:

   (a) a mismatch probe is located on an array at a position adjacent to where the interrogation probe to which it corresponds is attached; and
   (b) no mismatch probe contains a central 15-mer that is identical to the complement of any sequence to which any interrogation probe specifically hybridizes.

6. A method for measuring an increase of a particular microorganism’s percentage of gut microbiome in an individual comprising applying a sample to the system of any of the above claims and identifying the increase.

7. A method for determining the probability of the presence or quantity of a unique sequence or microorganism in a sample comprising:

   (a) contacting said sample with a plurality of different probes;
   (b) determining hybridization signal strength for sample sequences to each of said probes; and
   (c) removing or attenuating from analysis an operational taxonomic unit (OTU) from the possible list based on hybridization signal strength data, thereby increasing the confidence in the remaining hybridization signal strength data;

wherein removing or attenuating is performed by penalizing OTUs present in the sample based on potential cross-hybridization of probes from the OTU with polynucleotides from the other OTUs.

**8.** The method of claim 7, wherein the penalization based on cross-hybridization is performed at each level of a phylogenetic tree starting with the lowest level.

**9.** The method of claim 7, wherein the method comprises determining GC content of each probe in the system and using a d score that compares each probe intensity to a positive control probe intensity and negative probe intensity to determine quantity of positive probe pairs.

**10.** A method for identifying a microbiome signature indicative of a condition comprising:

(a) comparing the presence and optionally abundance of one of more different OTUs in a control sample without said condition and a reference sample with said condition using the system of claims 1-5; and
(b) identifying one or more OTUs that associate with said condition.

**11.** The method of claim 10, wherein said reference and control samples are obtained from the gut, respiratory system, oral cavity, sinuses, nares, urogenital tract, skin, feces, udders, auditory canal, blood, sputum, urine or a combination thereof.

**12.** The method of claim 10, wherein said condition is Crohn's Disease, irritable bowel syndrome, stomach ulcers, colitis, neonatal necrotizing enterocolitis, gastroesophageal reflux disease, cystic fibrosis, chronic obstructive pulmonary disease, rhinitis, atopy, asthma, acne, food allergy, obesity, or periodontal disease.

**13.** The method of claim 10, wherein said condition is any disease or disorder caused by, aggravated by or related to the presence, absence or population change of a microorganism.

**14.** The method of claim 10, wherein changes in the degree of similarity in the presence and optionally abundance of said OTUs in said reference sample with respect to said control sample are provided as a measure of remediation of said condition.

**15.** The method of claim 10, wherein said microbiome signature consists of up to 200 different OTUs that associate with said condition.

**16.** The method of claim 1, wherein the calculating and penalizing steps in (c) are performed for those probes passing a first quantile threshold.

**Patentansprüche**

**1.** System zum Detektieren einer Vielzahl von operativen taxonomischen Einheiten (operational taxonomic units; OTUs) in einer Probe in einem einzelnen Test, wobei jede einer Vielzahl von ersten Nukleinsäuresequenzen weniger als 0,01 Prozent der gesamten Nukleinsäuren in einer Population von Nukleinsäuresequenzen aus der Probe umfasst; jede der Vielzahl von ersten Nukleinsäuresequenzen mindestens 95% homolog zu allen der anderen ersten Nukleinsäuresequenzen in der Population von Nukleinsäuresequenzen sind; wobei das System umfasst

(a) eine Vielzahl von Sondenpaaren für jede von mindestens 10.000 OTUs, wobei jedes Sondenpaar eine perfekt übereinstimmende (perfect match, PM) Sonde und eine nicht übereinstimmende (mismatch, MM) Sonde umfasst;
(b) eine Vielzahl von Negativ- und Positivkontrollsonden; und
(c) Computer-ausführbarer Logik zum Dekonvolutieren von Signalintensitäten der Vielzahl von Sondenpaaren in Wahrscheinlichkeitsabschätzungen durch:

(i) Vergleichen von Signalintensitäten für PM-Sonden, MM-Sonden, Positivkontrollsonden und Negativkontrollsonden;

(ii) Berechnen einer Wahrscheinlichkeit, dass eine individuelle OTU vorhanden ist basierend auf den Signalintensitäten von Sonden für diese OTU; und
(iii) Bestrafen der Wahrscheinlichkeit basierend auf Potential für Kreuzhybridisierung von Sonden mit Sequenzen von andern OTUs.

**2.** System nach Anspruch 1, wobei das System Gegenwart, Abwesenheit, relative Häufigkeit und/oder Quantität von mehr als 10.000 unterschiedlichen OTUs einer einzelnen Domäne in einem einzigen Test mit einer Konfidenz von mehr als 95% detektiert.

**3.** System nach Anspruch 1, wobei eine oder mehrere der ersten Nukleinsäuresequenzen 16S rRNA Gen, 23S rRNA Gen, 5S rRNA Gen, 5,8S rRNA Gen, 12S rRNA 20 Gen, 18S rRNA Gen, 28S rRNA Gen, gyrB Gen, rpoB Gen, fusA Gen, recA Gen, coxl Gen, nifl3 Gen, davon abgeleitete RNA Moleküle, oder eine Kombination davon ist/sind.

**4.** System nach Anspruch 1, wobei das System mindestens 20.000 PM-Sonden und eine Vielzahl von MM-Sonden für jede PM-Sonde umfasst, wobei jede PM-Sonde eine unterschiedliche Nukleinsäuresequenz detektiert, und jede MM-Sondensequenz sich von der PM-Sonde, zu der sie korrespondiert, durch mindestens ein Nukleotid unterscheidet.

**5.** System nach Anspruch 4 mit einer oder mehreren der folgenden Eigenschaften:

(a) eine nicht übereinstimmende Sonde ist auf einer Anordnung an einer Position lokalisiert, angrenzend zu wo die Untersuchungssonde zu der sie korrespondiert angebracht ist; und
(b) keine nicht übereinstimmende Sonde ein zentrales 15-mer enthält, das zu dem Komplementär einer jeden Sequenz, an die eine beliebige Sonde spezifisch hybridisiert, identisch ist.

**6.** Verfahren zum Messen einer Zunahme eines prozentualen Anteils eines besonderen Mikroorganismus der Darmflora in einem Individuum, umfassend das Auftragen einer Probe zu dem System eines beliebigen der vorstehenden Ansprüche und Identifizieren der Zunahme.

**7.** Verfahren zum Bestimmen der Wahrscheinlichkeit der Gegenwart oder Quantität einer einzigartigen Sequenz oder Mikroorganismus in einer Probe, umfassend:

(a) In-Kontakt-Bringen der Probe mit einer Vielzahl von unterschiedlichen Sonden;
(b) Bestimmen der Hybridisierungssignalstärke für Probensequenzen zu jeder der Sonden; und
(c) Entfernen oder Abschwächen aus Analyse einer operativen taxonomischen Einheit (OTU) aus der möglichen Liste basierend auf Hybridisierungssignalstärkendaten, dadurch erhöhen der Konfidenz in den verbleibenden Hybridisierungssignalstärkedaten;

wobei Entfernen oder Abschwächen durchgeführt wird durch Bestrafen von in der Probe vorhandenen OTUs basierend auf potentieller Kreuzhybridisierung von Sonden aus der OTU mit Polynukleotiden aus den anderen OTUs.

**8.** Verfahren nach Anspruch 7, wobei die auf Kreuzhybridisierung basierende Bestrafung auf jeder Ebene eines phylogenetischen Baums startend mit der niedrigsten Ebene durchgeführt wird.

**9.** Verfahren nach Anspruch 7, wobei das Verfahren das Bestimmen des GC-Gehalts von jeder Sonde in dem System und Verwenden eines d-Wert, der jede Sondenintensität zu einer Positivkontrollsondenintensität und Negativsondenintensität vergleicht, umfasst, um die Quantität von positiven Sondenpaaren zu bestimmen.

**10.** Verfahren zum Identifizieren einer Mikrobiomsignatur, die für einen Zustand indikativ ist, umfassend:

(a) Vergleichen der Gegenwart und gegebenenfalls Häufigkeit von einer oder mehreren OTUs in einer Kontrollprobe ohne den Zustand und einer Referenzprobe mit dem Zustand unter Verwendung des Systems gemäß Ansprüchen 1-5; und
(b) Identifizieren einer oder mehrerer OTUs, die mit dem Zustand assoziiert ist/sind.

**11.** Verfahren nach Anspruch 10, wobei die Referenz- und Kontrollproben aus dem Darm, Atmungssystem, Mundhöhle, Sinus, Nasenöffnungen, Urogenitaltrakt, Haut, Faeces, Euter, Gehörgang, Blut, Sputum, Urin oder einer Kombination davon erhalten sind.

12. Verfahren nach Anspruch 10, wobei der Zustand Morbus Crohn, Reizdarmsyndrom, Magengeschwüre, Kolitis, neonatale nekrotisierende Enterokolitis, gastroösophageale Refluxkrankheit, zystische Fibrose, chronisch obstruktive Lungenerkrankung, Rhinitis, Atopie, Asthma, Akne, Lebensmittelallergie, O-besitas oder Paradontose ist.

13. Verfahren nach Anspruch 10, wobei der Zustand eine beliebige Erkrankung oder Störung ist, verursacht wird durch oder mit der Gegenwart, Abwesenheit oder Populationsveränderung eines Mikroorganismus.

14. Verfahren nach Anspruch 10, wobei Änderungen im Ähnlichkeitsgrad in der Gegenwart und gegebenenfalls Abwesenheit der OTUs in der Referenzprobe in Bezug auf die Kontrollprobe als ein Mittel der Heilung des Zustands bereitgestellt wird.

15. Verfahren nach Anspruch 10, wobei die Mikrobiomsignatur aus bis zu 200 unterschiedlichen OTUs, die mit dem Zustand assoziiert sind, besteht.

16. Verfahren nach Anspruch 1, wobei die Berechnungs- und Bestrafungsschritte in (c) für die Sonden durchgeführt werden, die einen ersten Quantilenschwellwert passieren.

## Revendications

1. Système permettant de détecter en un seul essai de multiples unités taxonomiques opérationnelles (UTO) dans un échantillon, dans lequel chacune d'un ensemble de multiples premières séquences d'acides nucléiques représente moins de 0,01 % de tous les acides nucléiques dans une population de séquences d'acides nucléiques provenant de l'échantillon, et chacune de ces multiples premières séquences d'acides nucléiques est homologue, à un degré d'au moins 95 %, à toutes les autres premières séquences d'acides nucléiques dans la population de séquences d'acides nucléiques, lequel système comporte :

    a) plusieurs paires de sondes pour chacune d'au moins 10 000 UTO, chaque paire de sondes comprenant une sonde à parfait appariement (sonde PA) et une sonde à mauvais appariement (sonde MA) ;
    b) plusieurs sondes témoins négatifs et témoins positifs ;
    c) et une logique exécutable sur ordinateur, servant à déconvoluer les intensités des signaux fournis par les multiples paires de sondes en estimations de probabilité, par les opérations suivantes :

        i) comparer les intensités des signaux fournis par les sondes PA, les sondes MA, les sondes témoins positifs et les sondes témoins négatifs,
        ii) calculer la probabilité qu'une UTO individuelle soit présente, en se basant sur les intensités des signaux fournis par les sondes pour cette UTO,
        iii) et affecter cette probabilité d'une pénalité, en se basant sur les possibilités d'hybridation croisée des sondes avec des séquences provenant d'autres UTO.

2. Système conforme à la revendication 1, lequel système permet de détecter en un seul essai, avec un seuil de confiance de plus de 95 %, la présence, l'absence, l'abondance relative et/ou la quantité de plus de 10 000 UTO différentes d'un domaine unique.

3. Système conforme à la revendication 1, dans lequel l'une ou plusieurs desdites premières séquences d'acides nucléiques sont le gène d'ARNr 165, le gène d'ARNr 23S, le gène d'ARNr 5S, le gène d'ARNr 5,8S, le gène d'ARNr 12S, le gène d'ARNr 18S, le gène d'ARNr 28S, le gène gyrB, le gène rpoB, le gène fusA, le gène recA, le gène cox1, le gène nifl3, les molécules d'ARN qui en dérivent, ou une combinaison de ceux-ci.

4. Système conforme à la revendication 1, lequel système comprend au moins 20 000 sondes PA et plusieurs sondes MA pour chaque sonde PA, étant entendu que chaque sonde PA détecte une première séquence d'acide nucléique différente et que la séquence de chaque sonde MA diffère par au moins un nucléotide de celle de la sonde PA à laquelle elle correspond.

5. Système conforme à la revendication 4, doté de l'une ou de plusieurs des caractéristiques suivantes :

    a) une sonde à mauvais appariement est placée, sur un réseau, en une position adjacente à l'endroit où la sonde interrogatoire à laquelle elle correspond est attachée ;

b) aucune sonde à mauvais appariement ne contient un oligonucléotide 15-mère central qui soit identique au complémentaire d'une séquence quelconque à laquelle s'hybride spécifiquement une séquence interrogatoire quelconque.

**6.** Procédé de mesure de l'augmentation du pourcentage d'un microorganisme particulier dans le microbiome intestinal d'un individu, comportant le fait d'appliquer un échantillon à un système conforme à l'une des revendications précédentes et le fait de mettre l'augmentation en évidence.

**7.** Procédé permettant de déterminer la probabilité de présence ou la quantité d'une unique séquence ou d'un unique microorganisme dans un échantillon, lequel procédé comporte les étapes suivantes :

a) mettre ledit échantillon en contact avec un ensemble de multiples sondes différentes ;
b) déterminer l'intensité du signal d'hybridation pour des séquences de l'échantillon vis-à-vis de chacune desdites sondes ;
c) et éliminer de l'analyse une unité taxonomique opérationnelle (UTO) de la liste possible ou en atténuer le poids dans l'analyse, en se basant sur les données d'intensité de signal d'hybridation, ce qui fait qu'on augmente la fiabilité des données d'intensité de signal d'hybridation restantes ;

étant entendu qu'on réalise cette élimination ou atténuation de poids en affectant les UTO présentes dans l'échantillon d'une pénalité, en se basant sur les possibilités d'hybridation croisée des sondes provenant de l'UTO avec des polynucléotides des autres UTO.

**8.** Procédé conforme à la revendication 7, dans lequel c'est à chaque niveau d'un arbre phylogénétique, en partant du niveau le plus bas, qu'on effectue l'application d'une pénalité basée sur l'hybridation croisée.

**9.** Procédé conforme à la revendication 7, lequel procédé comporte le fait de déterminer la teneur en GC de chaque sonde dans le système et le fait d'utiliser une note d qui permet de comparer chaque intensité de sonde à une intensité de sonde témoin positif et à une intensité de sonde témoin négatif pour déterminer la quantités de paires de sondes positives.

**10.** Procédé permettant d'identifier au sein d'un microbiome une signature indicatrice d'un certain état, lequel procédé comporte les étapes suivantes :

a) comparer la présence, et en option l'abondance, d'une ou de plusieurs UTO différente(s), dans un échantillon témoin sans lien avec ledit état et dans un échantillon de référence en liaison avec ledit état, au moyen d'un système conforme à l'une des revendications 1 à 5 ;
b) et identifier une ou plusieurs UTO associée(s) audit état.

**11.** Procédé conforme à la revendication 10, dans lequel lesdits échantillons, témoin et de référence, proviennent de l'intestin, de l'appareil respiratoire, de la cavité buccale, des sinus, des narines, du tractus uro-génital, de la peau, des fèces, des mamelles, du canal auditif, du sang, d'expectorations, de l'urine, ou d'une combinaison de telles origines.

**12.** Procédé conforme à la revendication 10, dans lequel ledit état est la maladie de Crohn, le syndrome de l'intestin irritable, un ulcère de l'estomac, une colite, l'entérocolite ulcéro-nécrosante du nouveau-né, la maladie de reflux gastro-oesophagien, la mucoviscidose, la broncho-pneumopathie chronique obstructive, une rhinite, une atopie, un asthme, une acné, une allergie alimentaire, une obésité ou la parondontolyse.

**13.** Procédé conforme à la revendication 10, dans lequel ledit état est n'importe quelle maladie ou n'importe quel trouble provoqué(e) ou aggravé(e) par, ou en relation avec, la présence, l'absence ou une variation de population d'un microorganisme.

**14.** Procédé conforme à la revendication 10, dans lequel les variations du degré de similitude dans la présence, et en option dans l'abondance, desdites UTO dans ledit échantillon de référence, par rapport audit échantillon témoin, sont prises comme une évaluation du processus de remédiation dudit état.

**15.** Procédé conforme à la revendication 10, dans lequel ladite signature de microbiome consiste en jusqu'à 200 UTO différentes qui sont associées audit état.

**16.** Procédé conforme à la revendication 1, dans lequel, dans l'étape (c), les opérations de calcul et d'application de pénalité sont effectuées pour les sondes qui dépassent un premier quantile seuil.

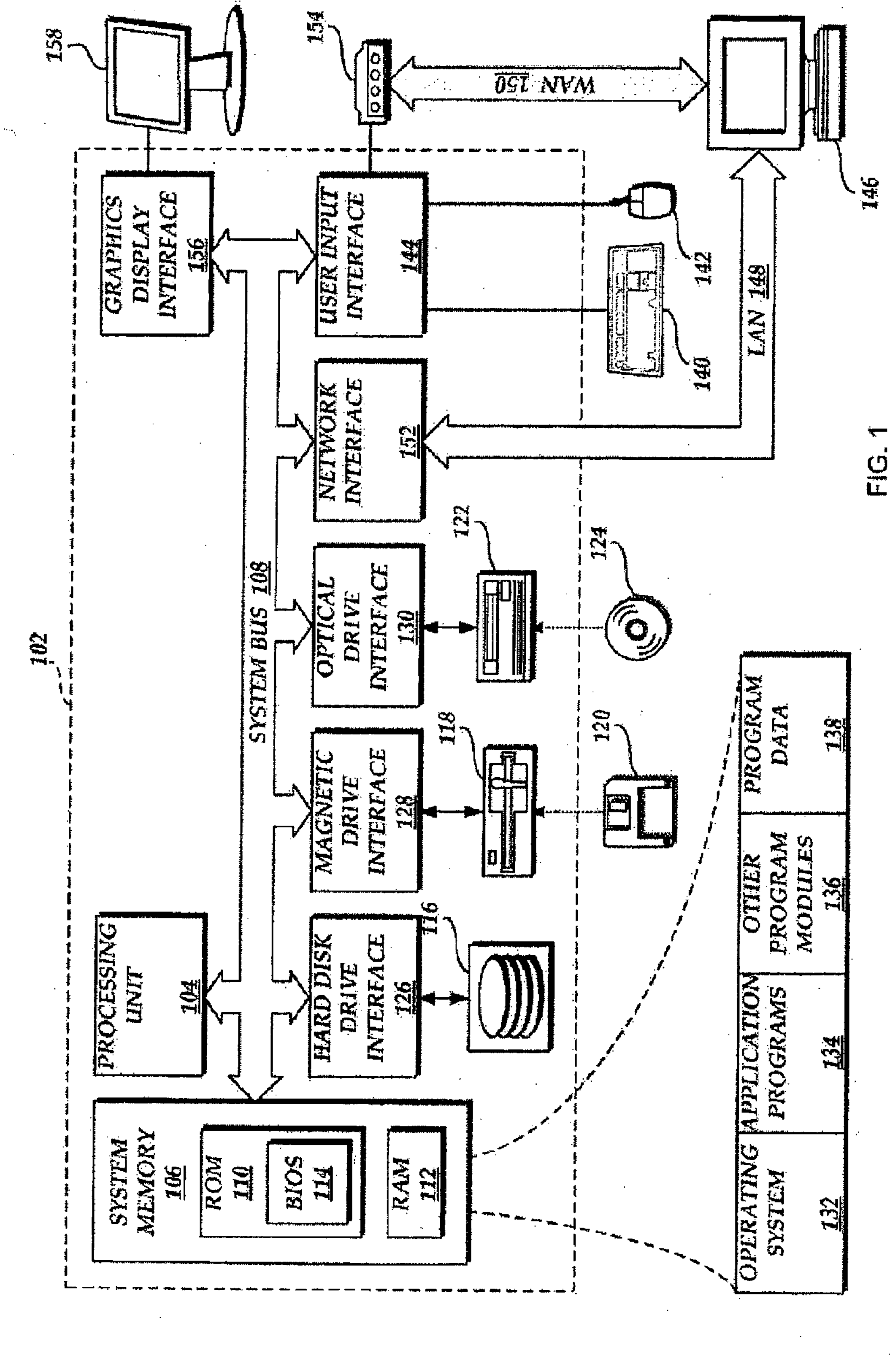
102
SYSTEM MEMORY 106
ROM 110
BIOS 114
RAM 112
PROCESSING UNIT 104
SYSTEM BUS 108
HARD DISK DRIVE INTERFACE 126
MAGNETIC DRIVE INTERFACE 128
OPTICAL DRIVE INTERFACE 130
NETWORK INTERFACE 152
USER INPUT INTERFACE 144
GRAPHICS DISPLAY INTERFACE 156
116
118
120
122
124
140
142
LAN 148
WAN 150
154
158
146
OPERATING SYSTEM 132
APPLICATION PROGRAMS 134
OTHER PROGRAM MODULES 136
PROGRAM DATA 138

FIG. 1

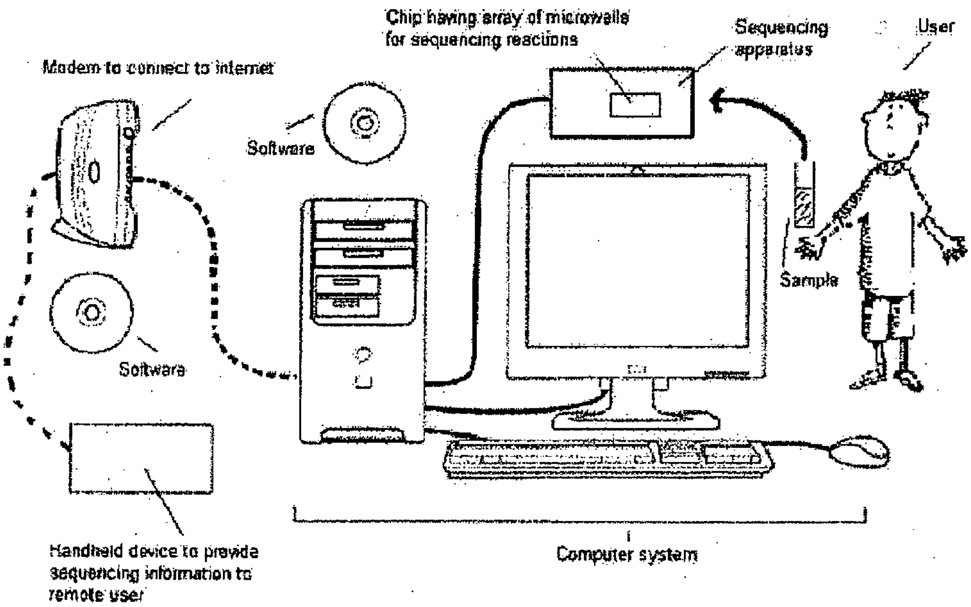
Chip having array of microwells
for sequencing reactions
Sequencing
apparatus
User
Modem to connect to internet
Software
Software
Sample
Handheld device to provide
sequencing information to
remote user
Computer system

FIG. 2

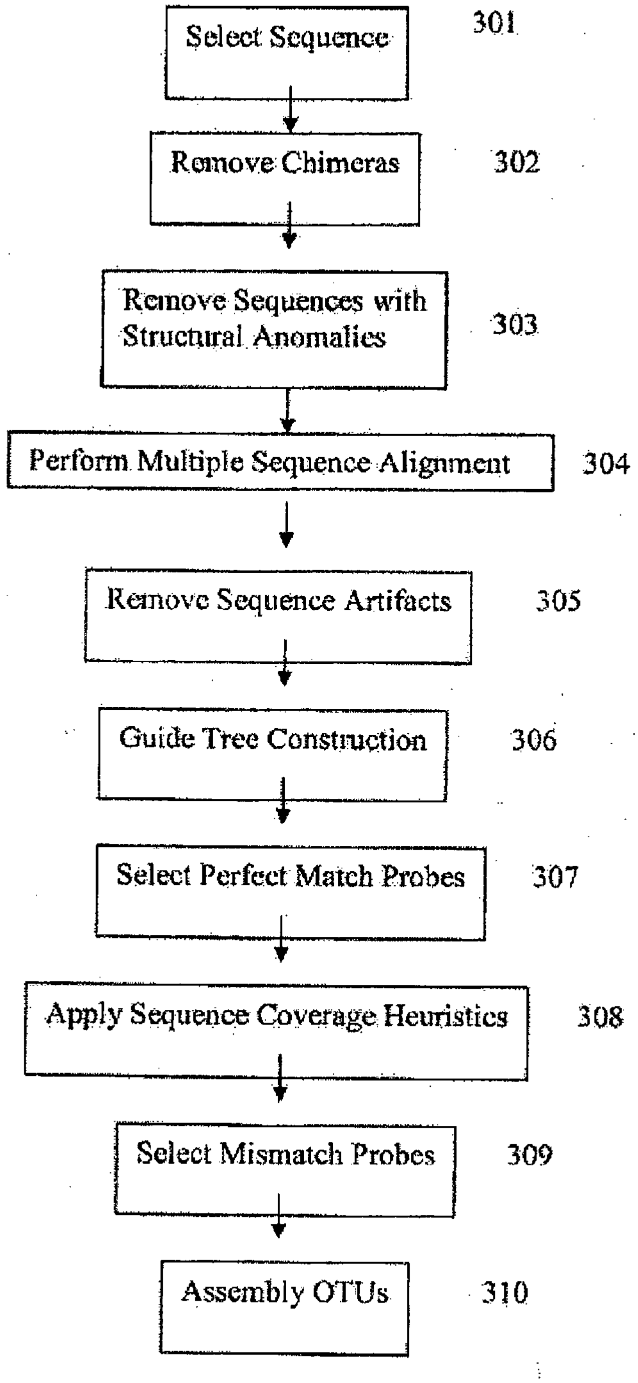
Select Sequence
301
Remove Chimeras
302
Remove Sequences with Structural Anomalies
303
Perform Multiple Sequence Alignment
304
Remove Sequence Artifacts
305
Guide Tree Construction
306
Select Perfect Match Probes
307
Apply Sequence Coverage Heuristics
308
Select Mismatch Probes
309
Assembly OTUs
310

Fig. 3

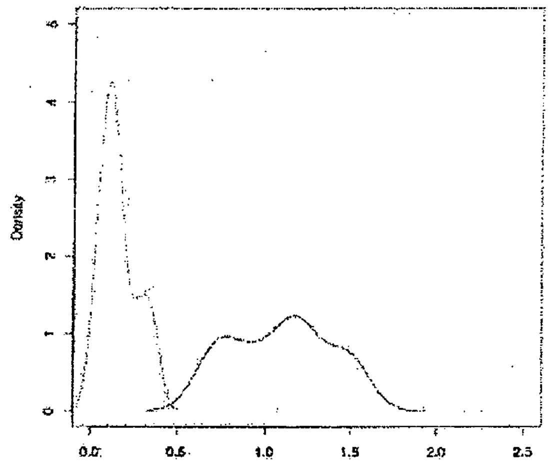


Fig. 4A

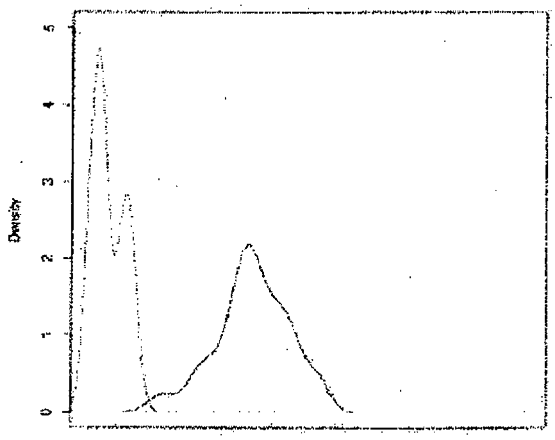


Fig. 4B

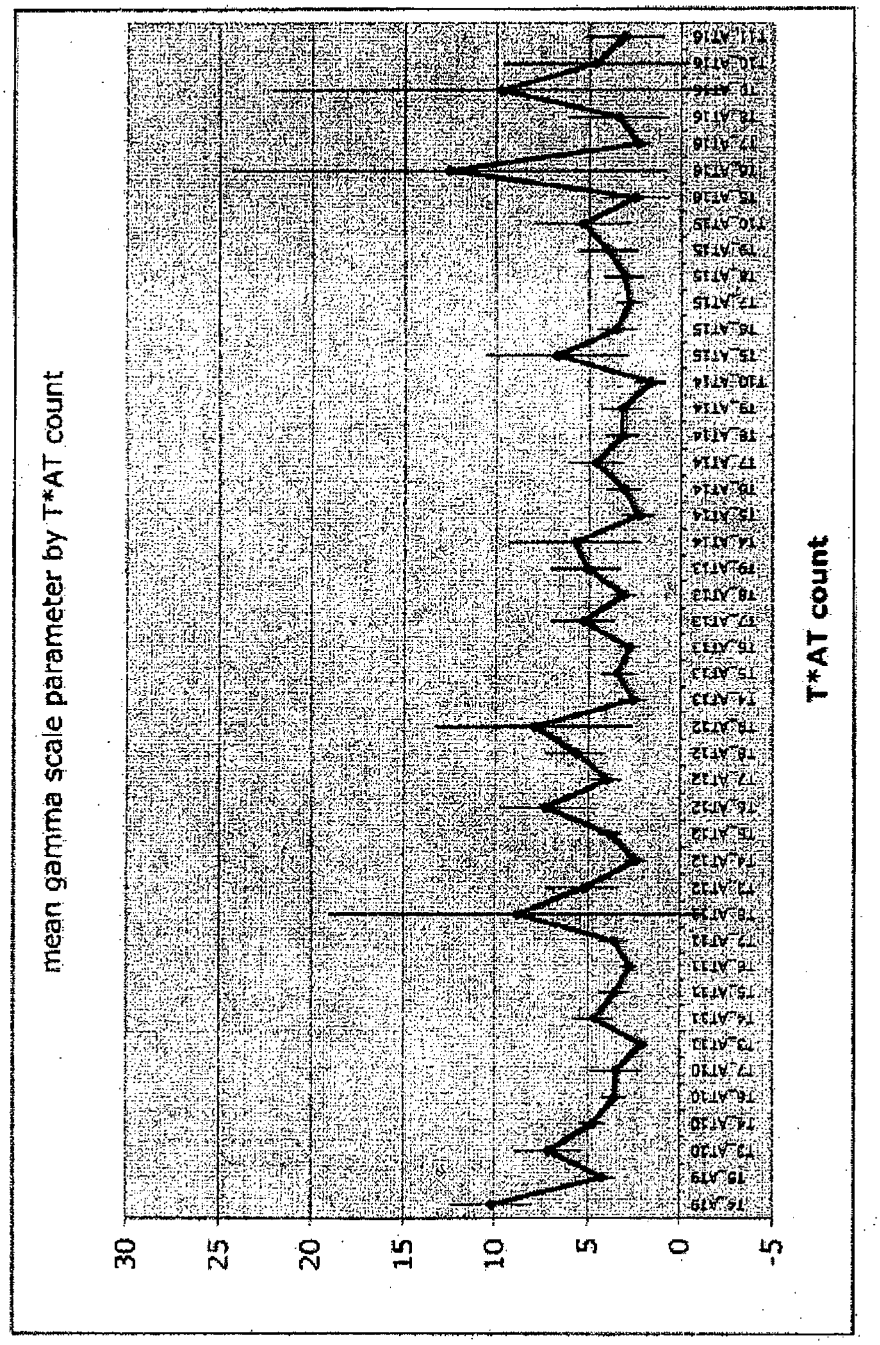
mean gamma scale parameter by T*AT count
T*AT count
30
25
20
15
10
5
0
-5

Fig. 5

Fig. 6

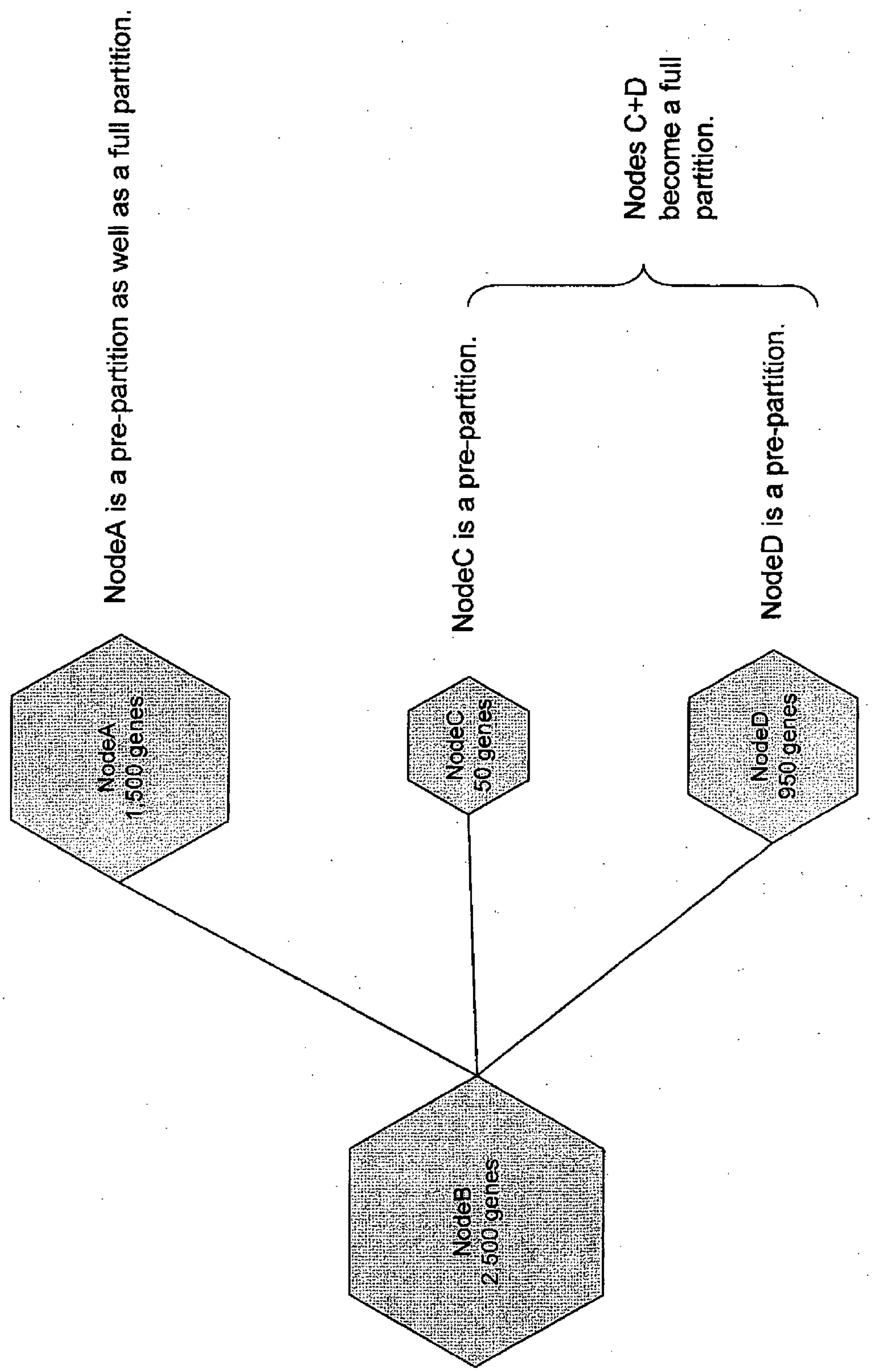
NodeA is a pre-partition as well as a full partition.
NodeC is a pre-partition.
NodeD is a pre-partition.
Nodes C+D become a full partition.
NodeA 1.500 genes
NodeC 50 genes
NodeD 950 genes
NodeB 2.500 genes

Fig. 7

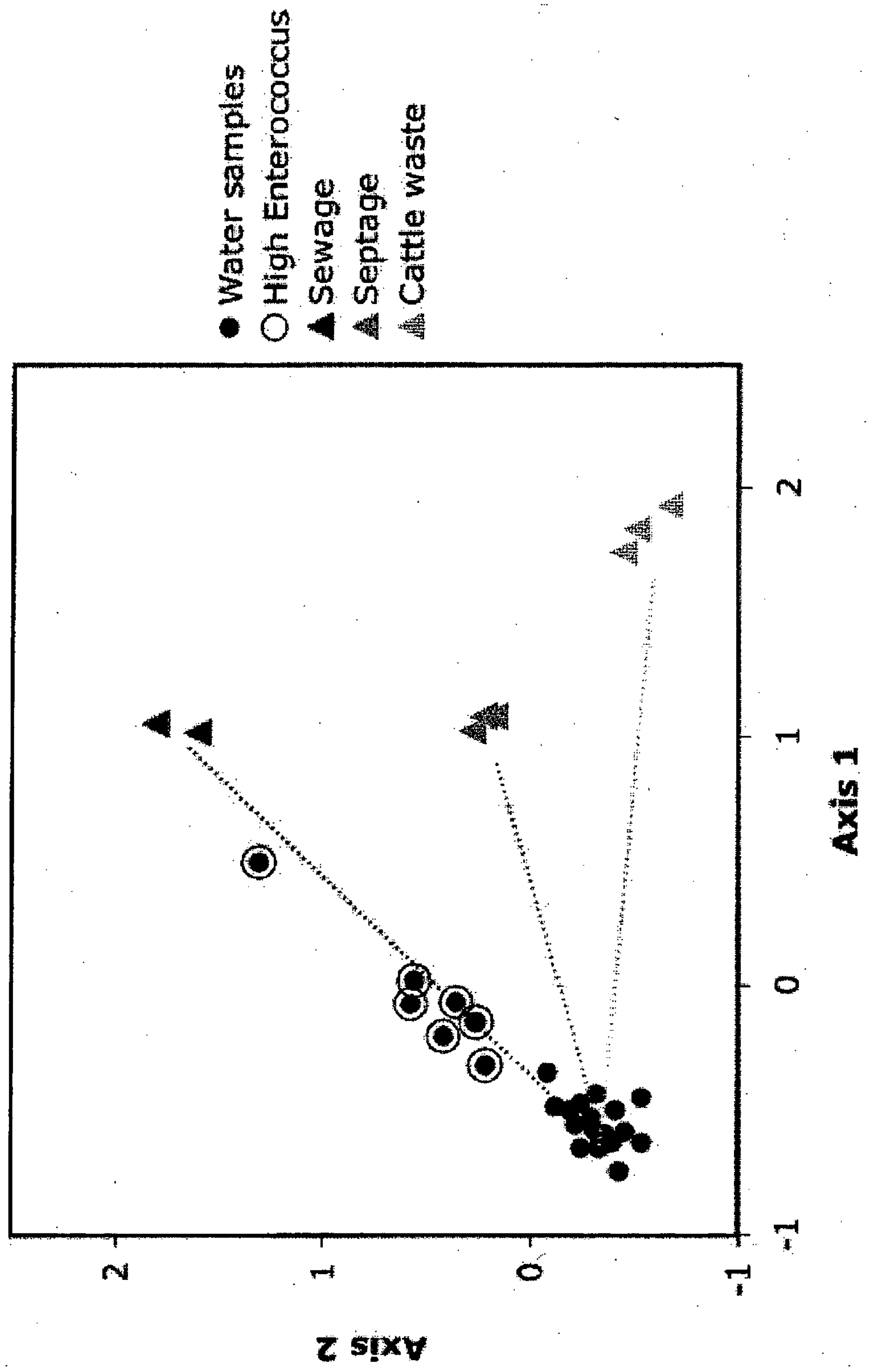
Water samples
High Enterococcus
Sewage
Septage
Cattle waste
Axis 1
Axis 2
-1
0
1
2
-1
0
1
2

Fig. 8

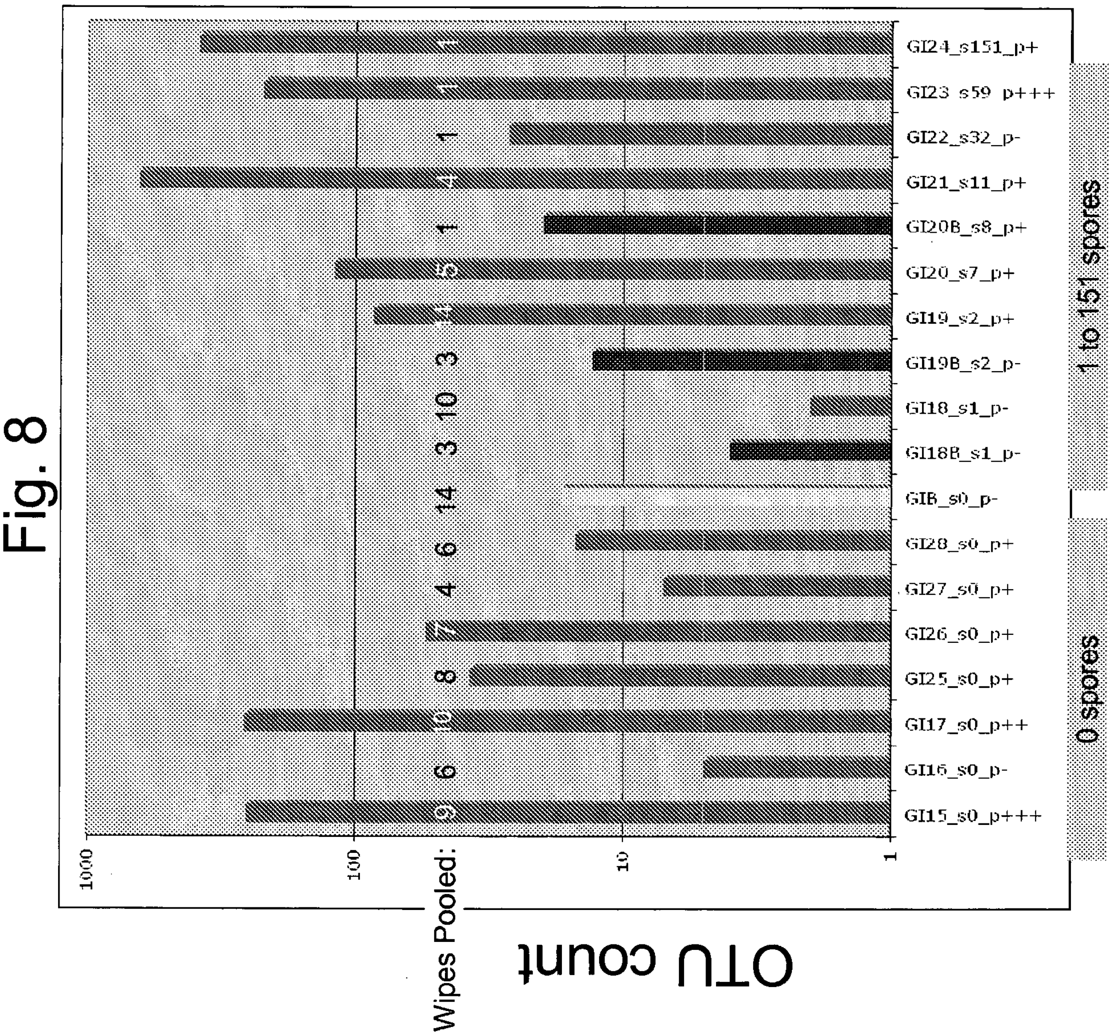
OTU count
Wipes Pooled:
1000
100
10
1
GI24_s151_p+
GI23_s59_p+++
GI22_s32_p-
GI21_s11_p+
GI20B_s8_p+
GI20_s7_p+
GI19_s2_p+
GI19B_s2_p-
GI18_s1_p-
GI18B_s1_p-
GIB_s0_p-
GI28_s0_p+
GI27_s0_p+
GI26_s0_p+
GI25_s0_p+
GI17_s0_p++
GI16_s0_p-
GI15_s0_p+++
1 1 1 4 1 5 4 3 10 3 14 6 4 7 8 10 6 9
1 to 151 spores
0 spores

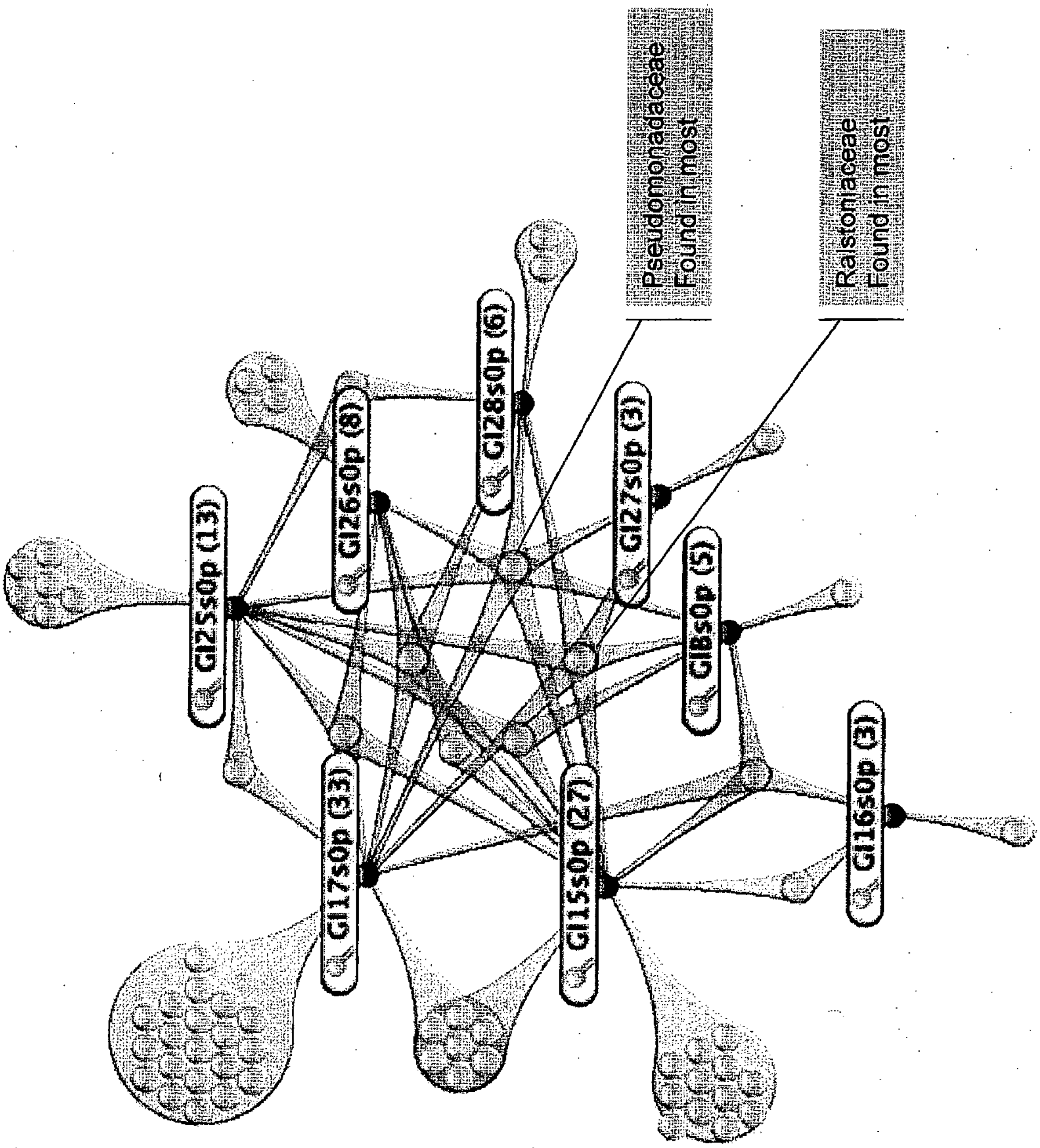
Pseudomonadaceae
Found in most
Ralstoniaceae
Found in most
GI25s0p (13)
GI26s0p (8)
GI28s0p (6)
GI27s0p (3)
GI8s0p (5)
GI16s0p (3)
GI17s0p (33)
GI15s0p (27)

Fig. 9

Fig10A

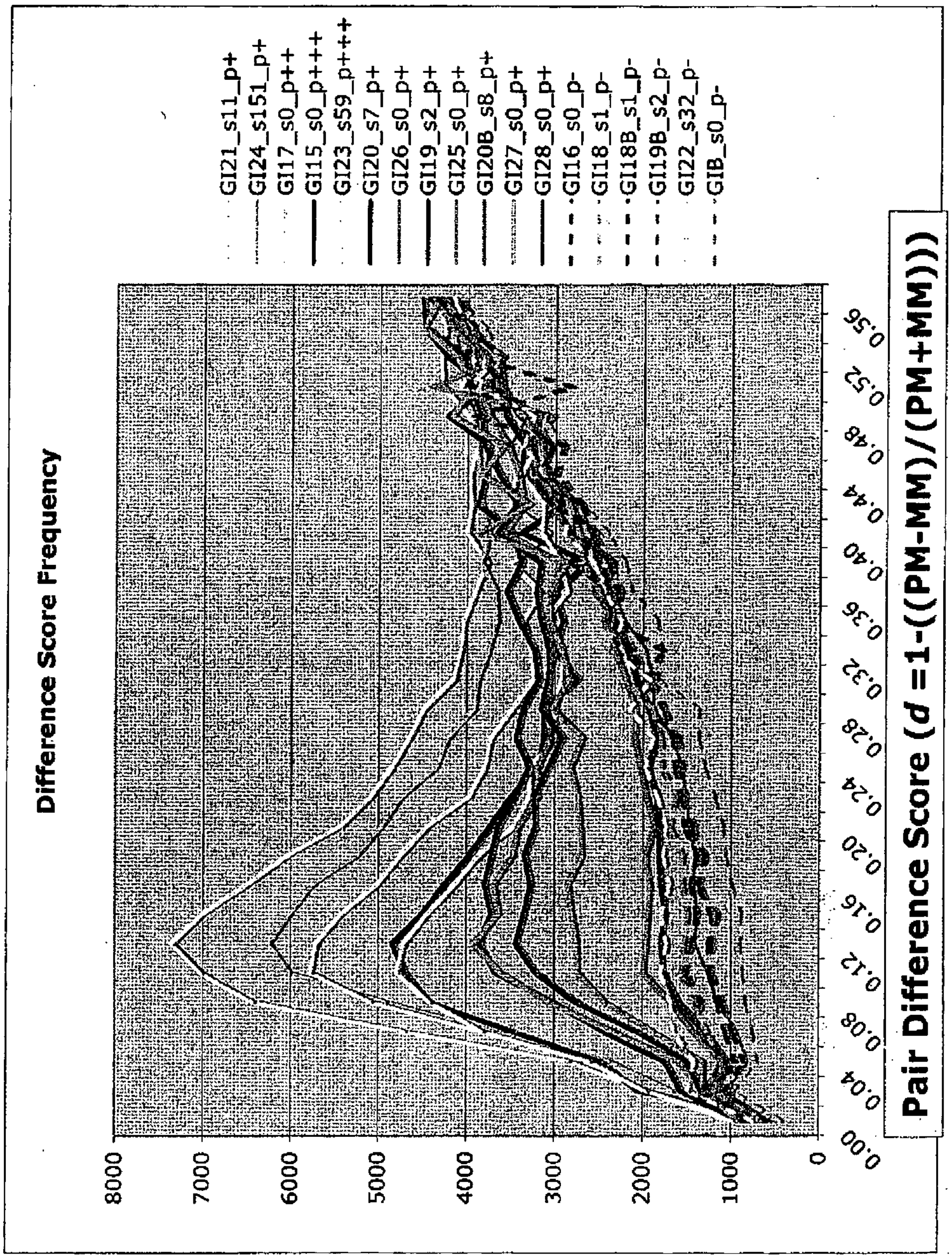
Difference Score Frequency
8000
7000
6000
5000
4000
3000
2000
1000
0
0.00
0.04
0.08
0.12
0.16
0.20
0.24
0.28
0.32
0.36
0.40
0.44
0.48
0.52
0.56
Pair Difference Score (d =1-((PM-MM)/(PM+MM)))
GI21_s11_p+
GI24_s151_p+
GI17_s0_p++
GI15_s0_p+++
GI23_s59_p+++
GI20_s7_p+
GI26_s0_p+
GI19_s2_p+
GI25_s0_p+
GI20B_s8_p+
GI27_s0_p+
GI28_s0_p+
GI16_s0_p-
GI18_s1_p-
GI18B_s1_p-
GI19B_s2_p-
GI22_s32_p-
GIB_s0_p-

Fig. 10B

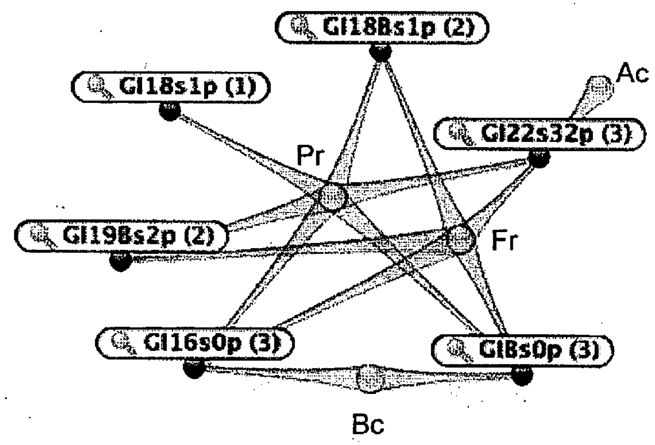


•32 spore sample
•non-detectable PCR products
•Actinobacteria found

4 phyla found in PCR negative samples

Pr: Proteobacteria Fr: Firmicutes Bc: Bacteroidetes AO: ABY1_OD1 Ac: Actinobacteria CyL Cyanobacteria Pl: Plactomycetes 49:49S1_2B_6

# Figure 11 Faecalibacterium OTU 36742

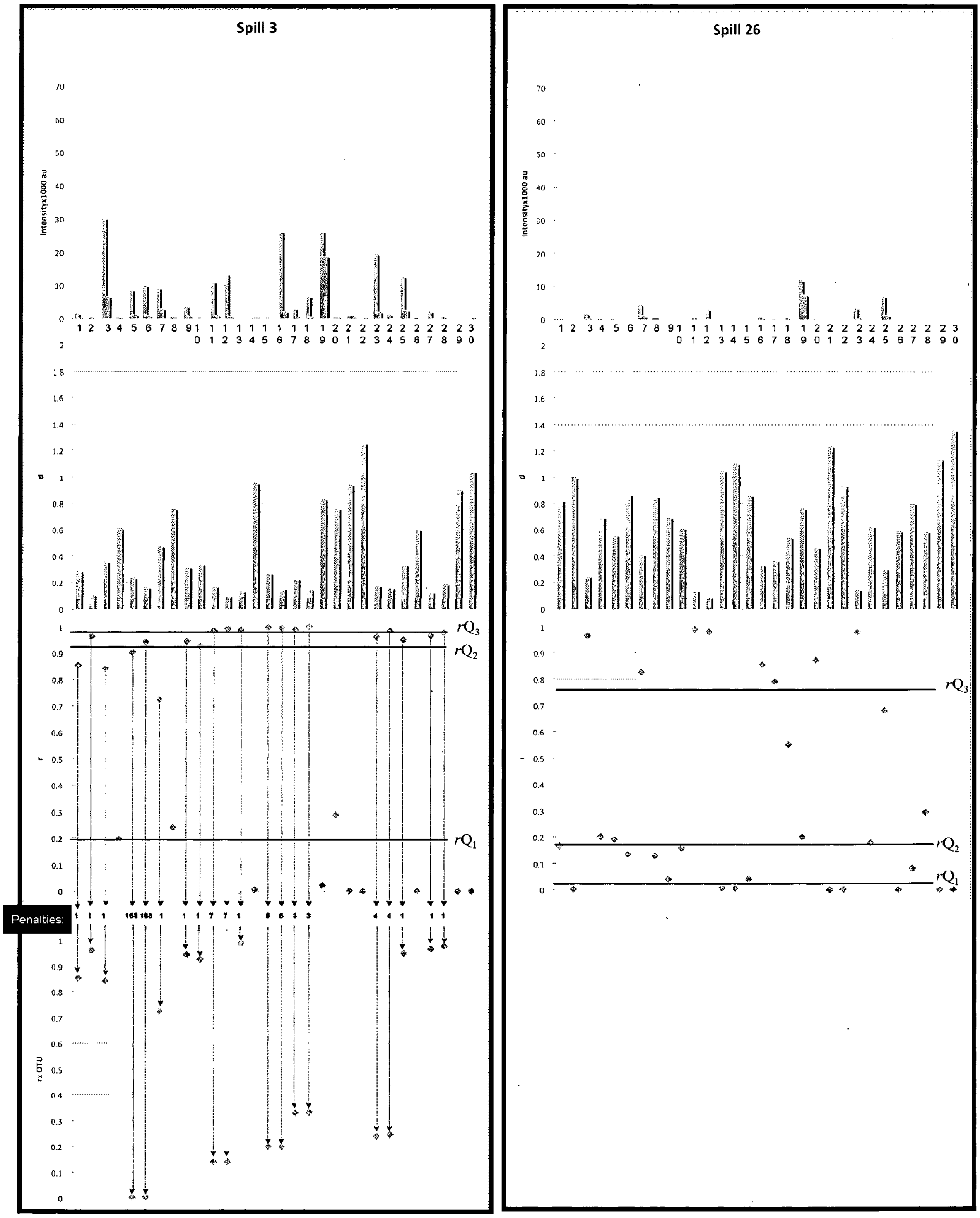

Figure 12 Ruminococcus OTU 38712

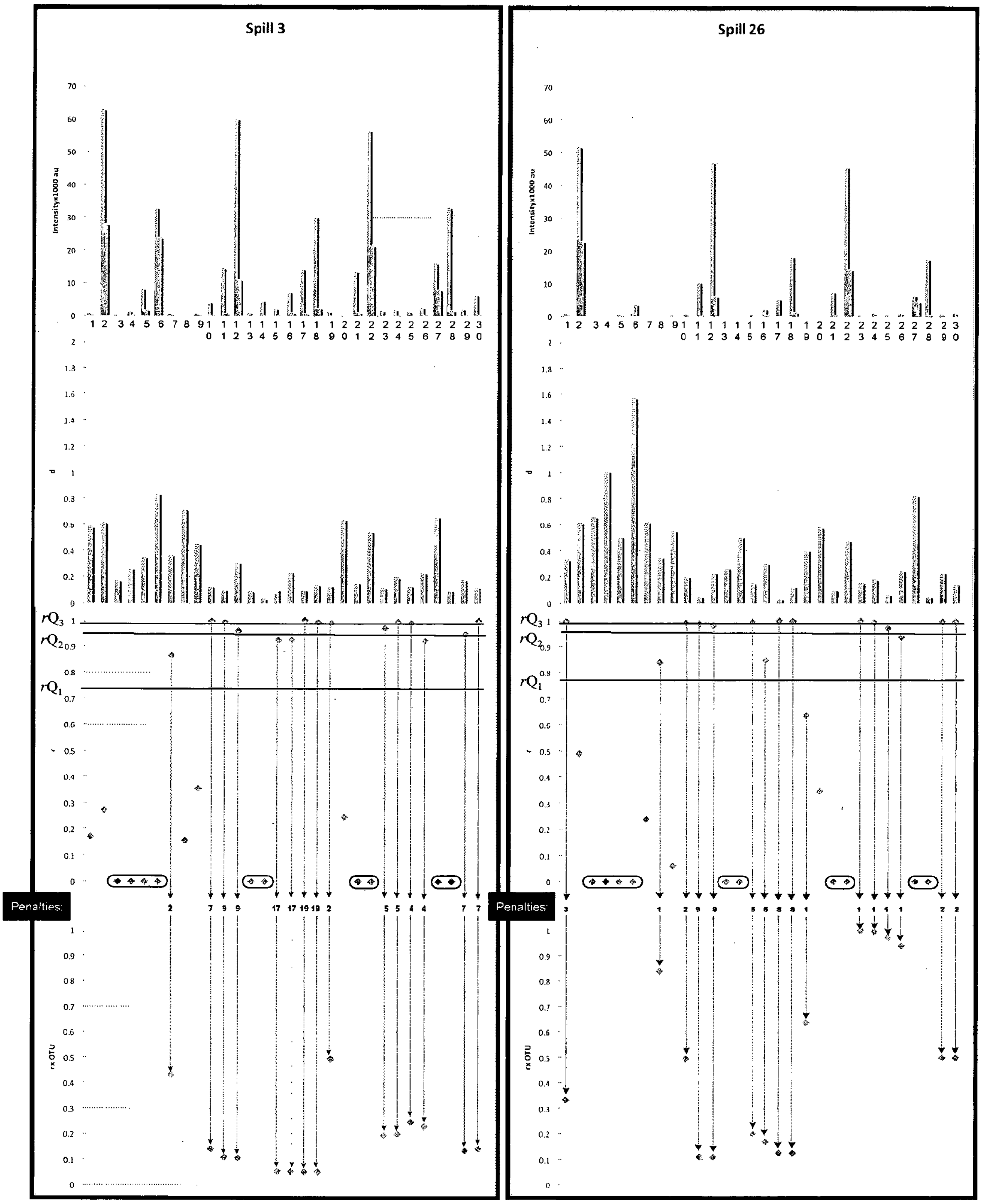

Fig. 13

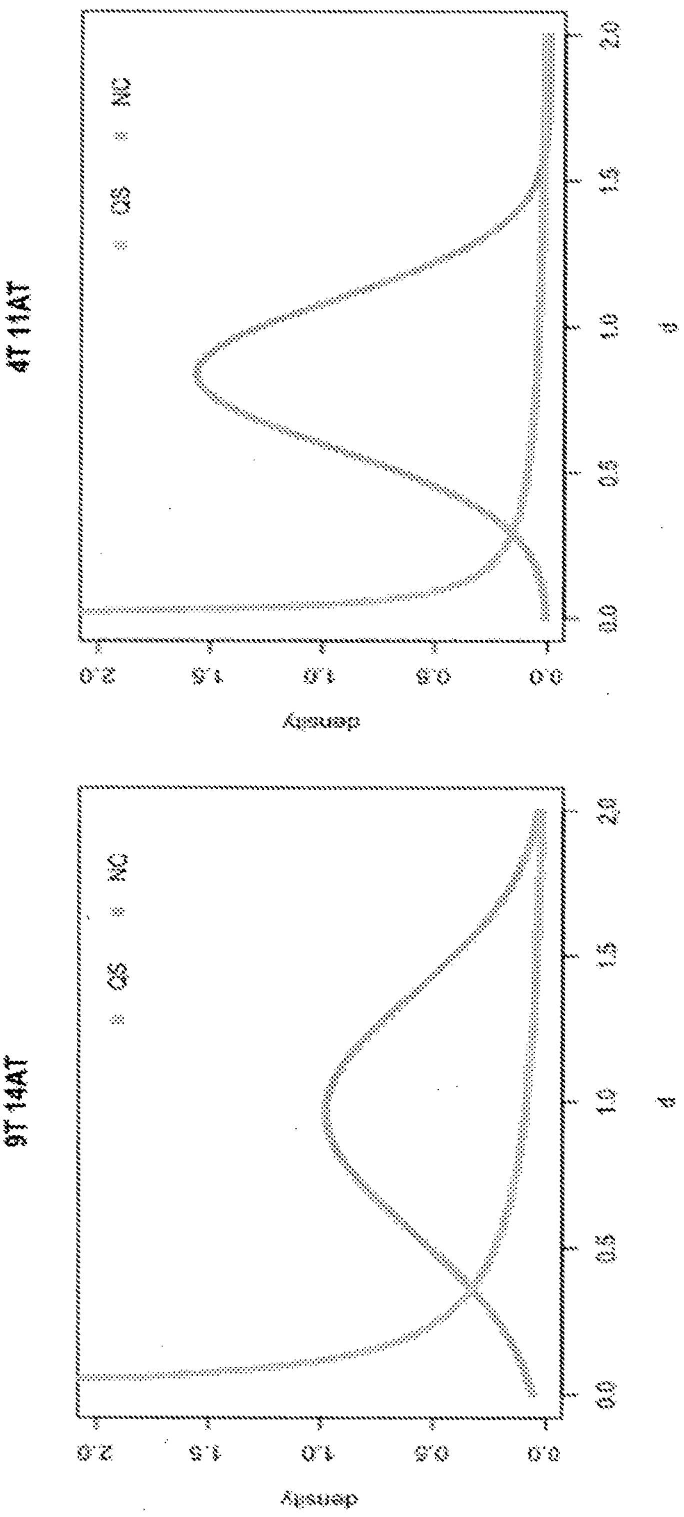

Fig. 14

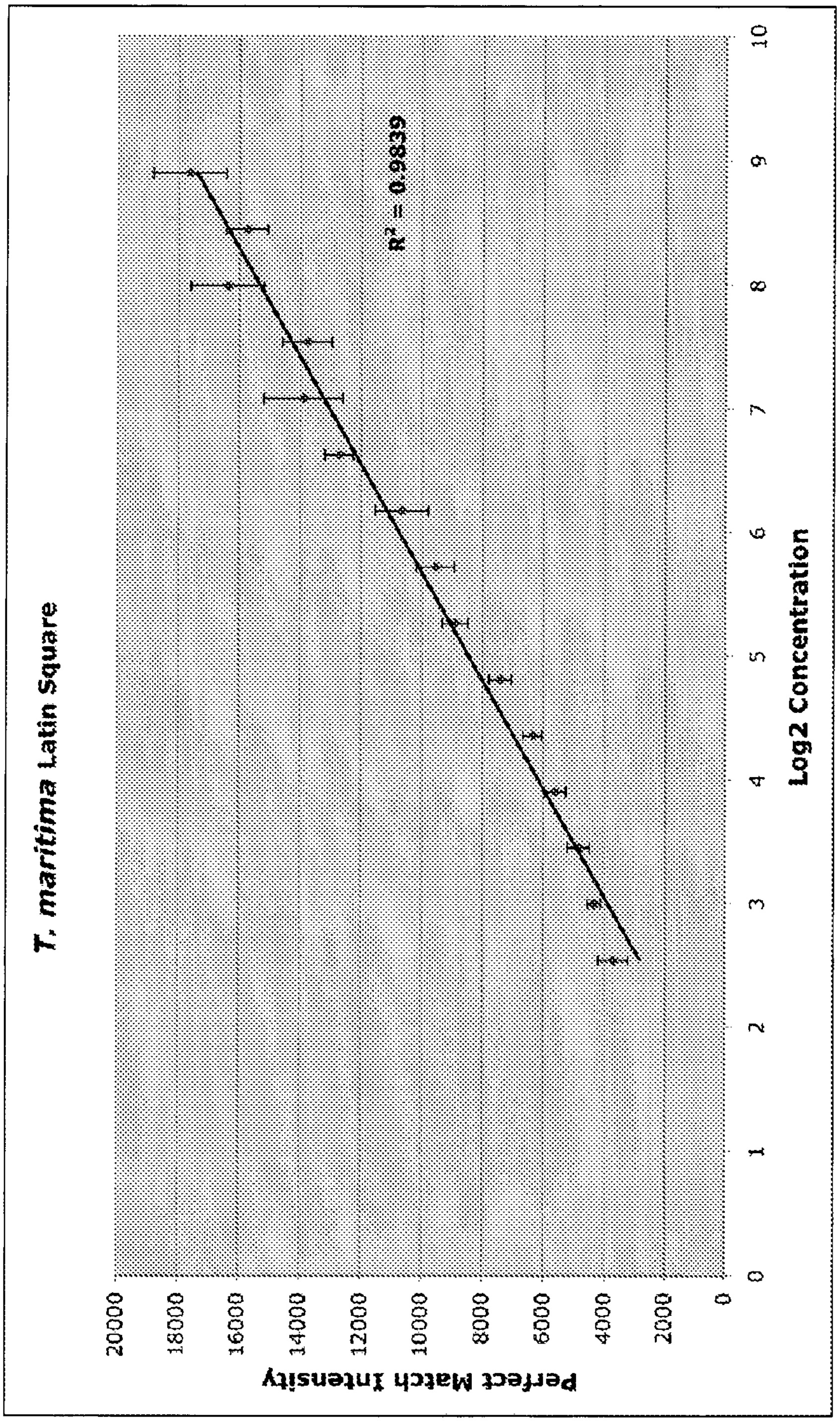
*T. maritima* Latin Square
Perfect Match Intensity
20000
18000
16000
14000
12000
10000
8000
6000
4000
2000
0
0
1
2
3
4
5
6
7
8
9
10
Log2 Concentration
$R^2 = 0.9839$

# Fig. 15

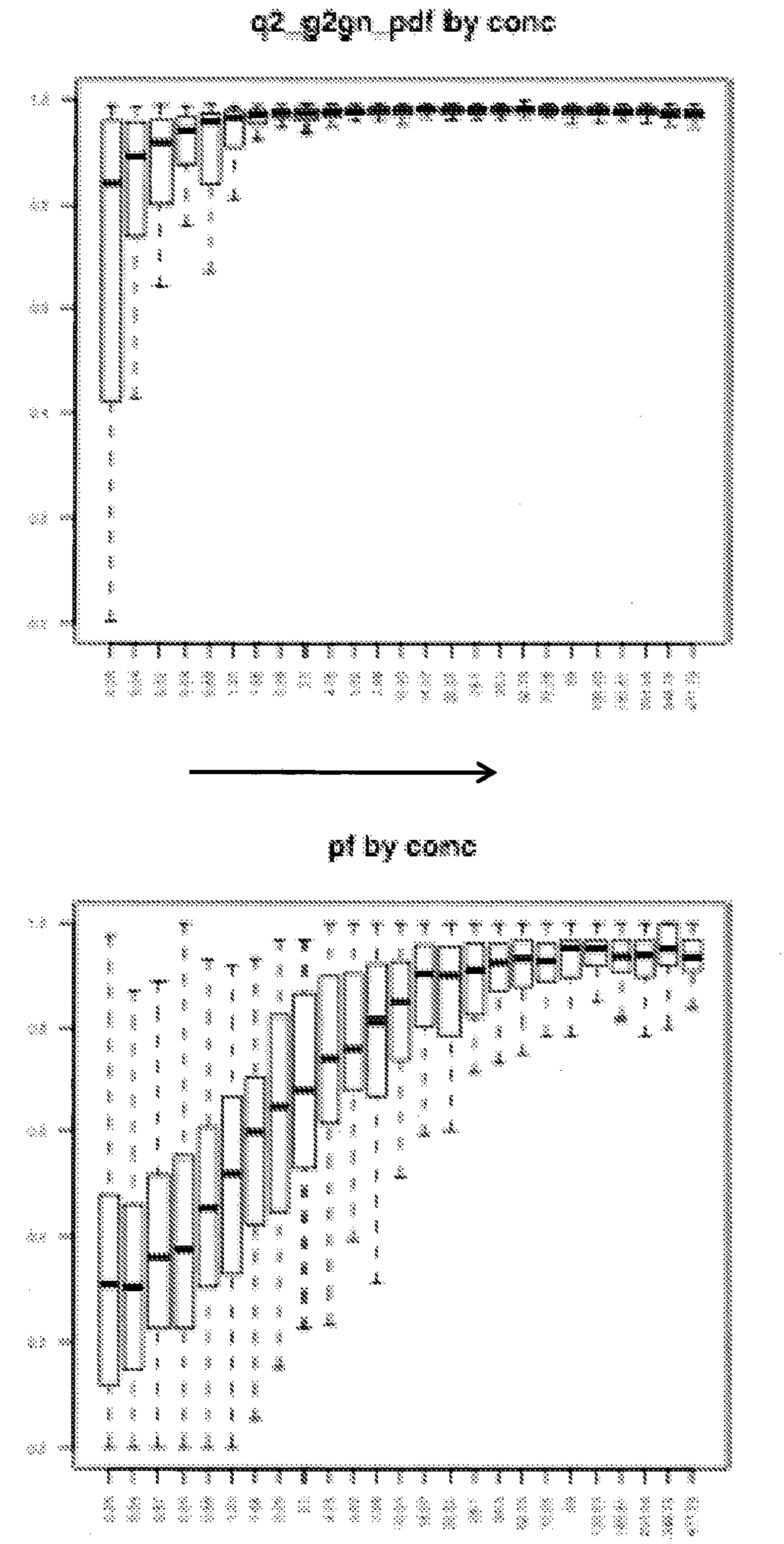

# Fig. 16

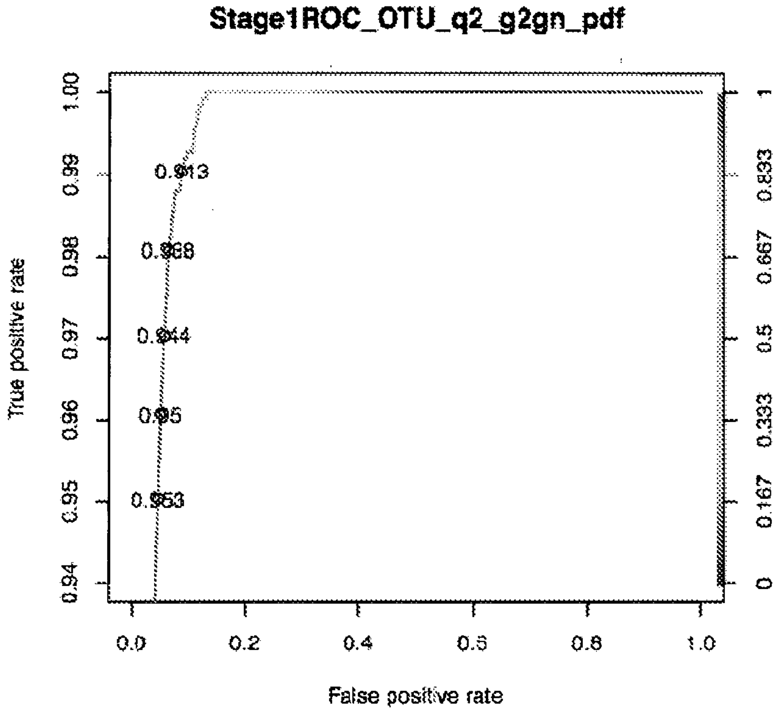
Stage1ROC_OTU_q2_g2gn_pdf
True positive rate
False positive rate
0.94
0.95
0.96
0.97
0.98
0.99
1.00
0.0
0.2
0.4
0.6
0.8
1.0
0
0.167
0.333
0.5
0.667
0.833
1

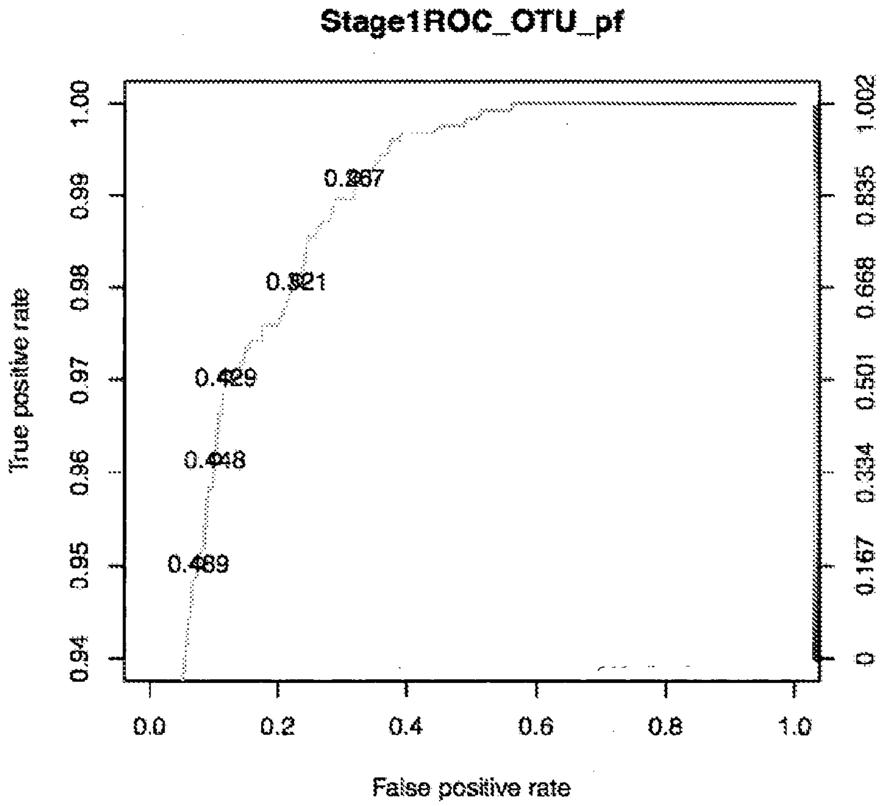
Stage1ROC_OTU_pf
True positive rate
False positive rate
0.94
0.95
0.96
0.97
0.98
0.99
1.00
0.0
0.2
0.4
0.6
0.8
1.0
0
0.167
0.334
0.501
0.668
0.835
1.002

Fig. 17

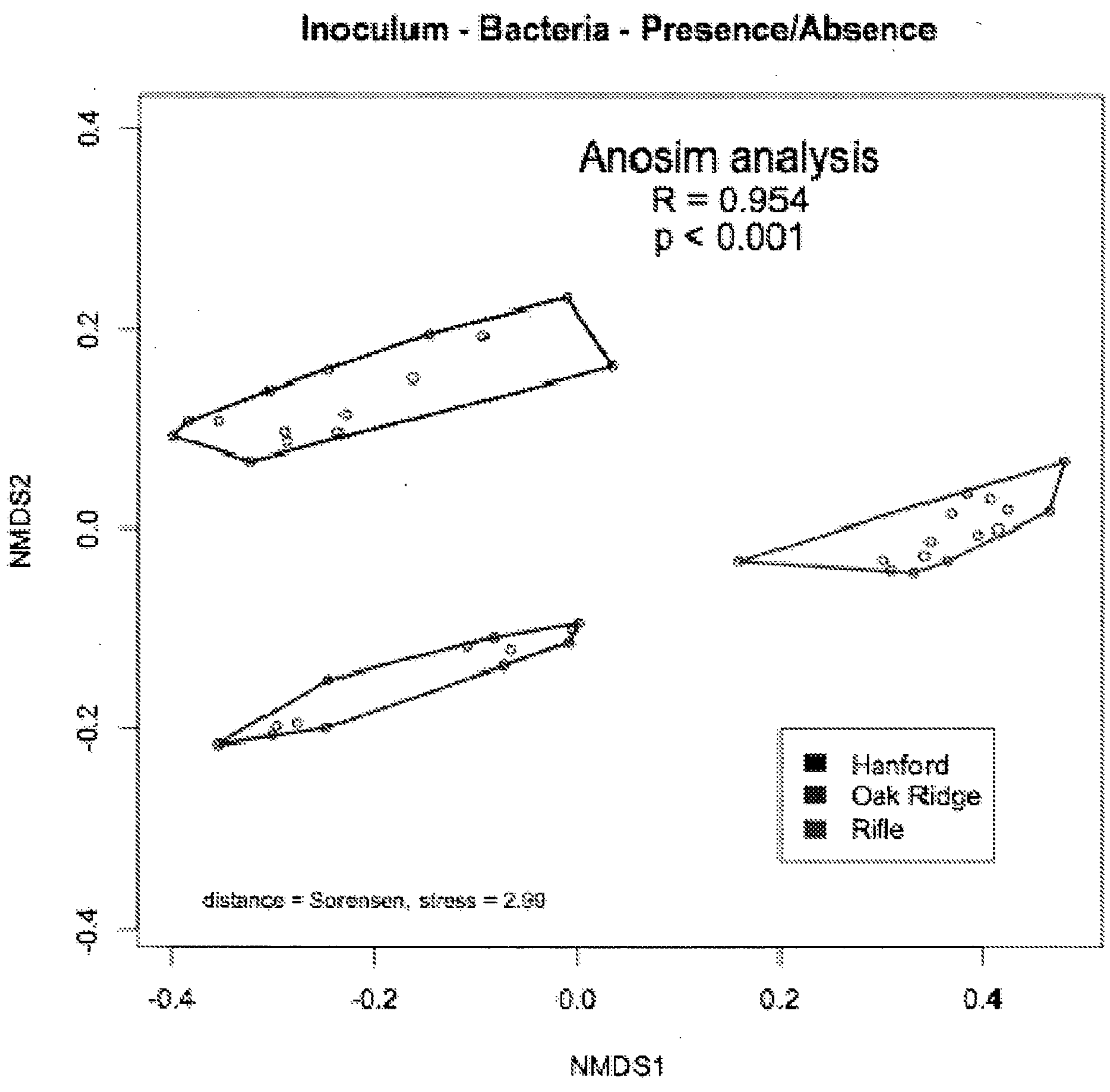
Inoculum - Bacteria - Presence/Absence
Anosim analysis
R = 0.954
p < 0.001
NMDS2
NMDS1
0.4
0.2
0.0
-0.2
-0.4
-0.4
-0.2
0.0
0.2
0.4
Hanford
Oak Ridge
Rifle
distance = Sorensen, stress = 2.99

Fig. 18

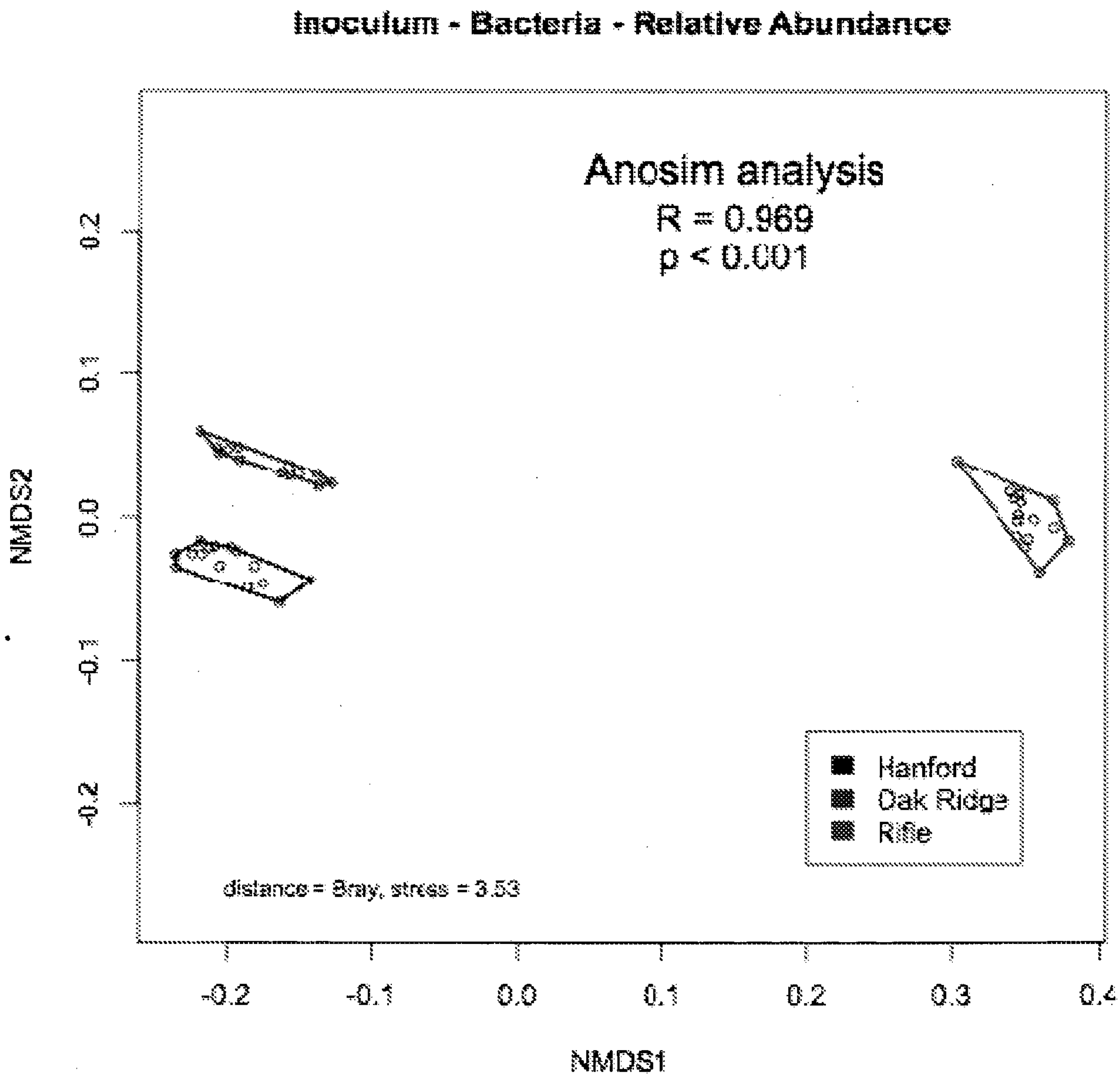
Inoculum - Bacteria - Relative Abundance
Anosim analysis
R = 0.969
p < 0.001
NMDS2
0.2
0.1
0.0
-0.1
-0.2
Hanford
Oak Ridge
Rifle
distance = Bray, stress = 3.53
-0.2
-0.1
0.0
0.1
0.2
0.3
0.4
NMDS1

Figure 19

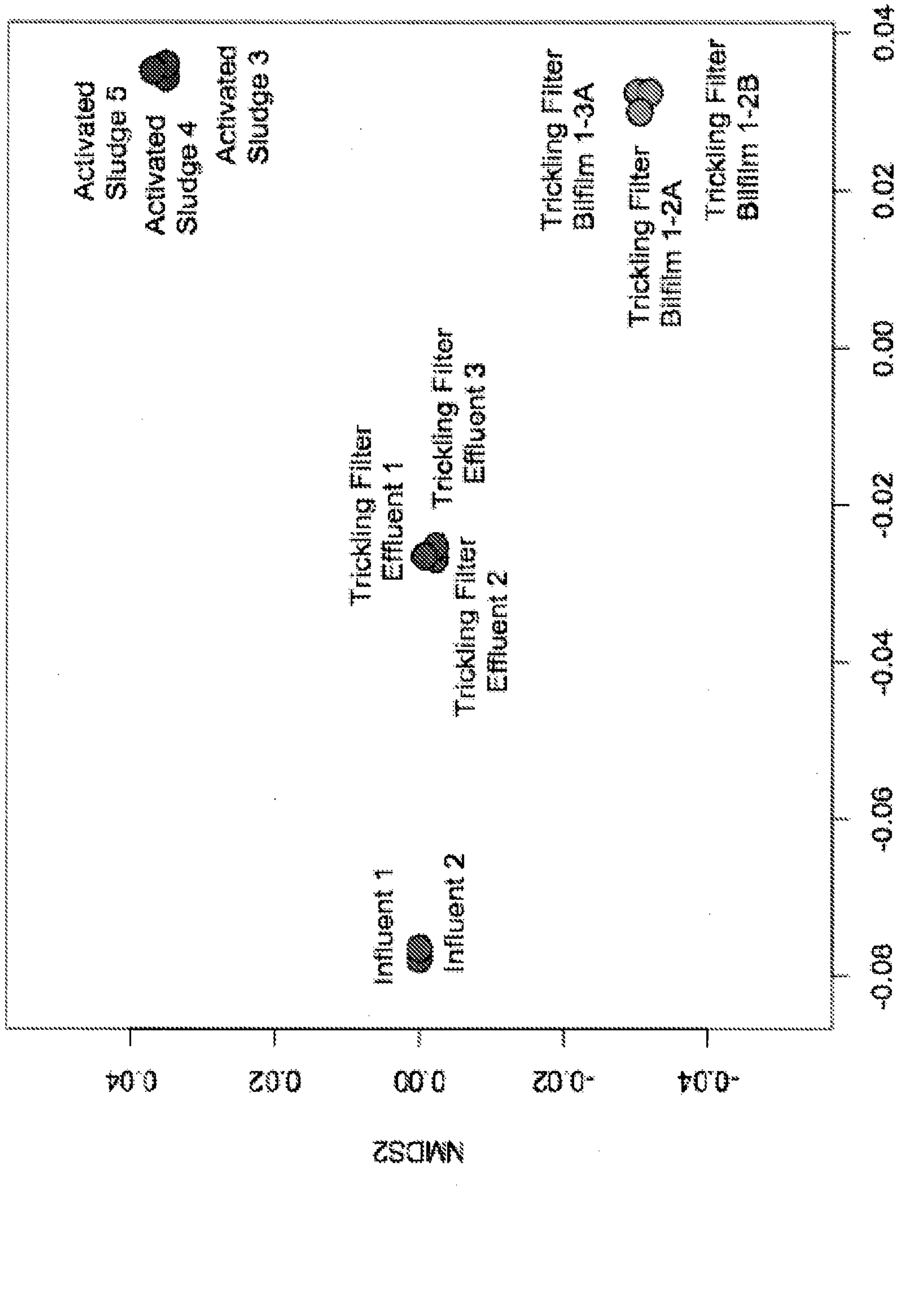
Activated Sludge 5
Activated Sludge 4
Activated Sludge 3
Trickling Filter Bilfilm 1-3A
Trickling Filter Bilfilm 1-2A
Trickling Filter Bilfilm 1-2B
Trickling Filter Effluent 1
Trickling Filter Effluent 2
Trickling Filter Effluent 3
Influent 1
Influent 2
0.04
0.02
0.00
-0.02
-0.04
-0.06
-0.08
NMDS1
NMDS2

Figure 20

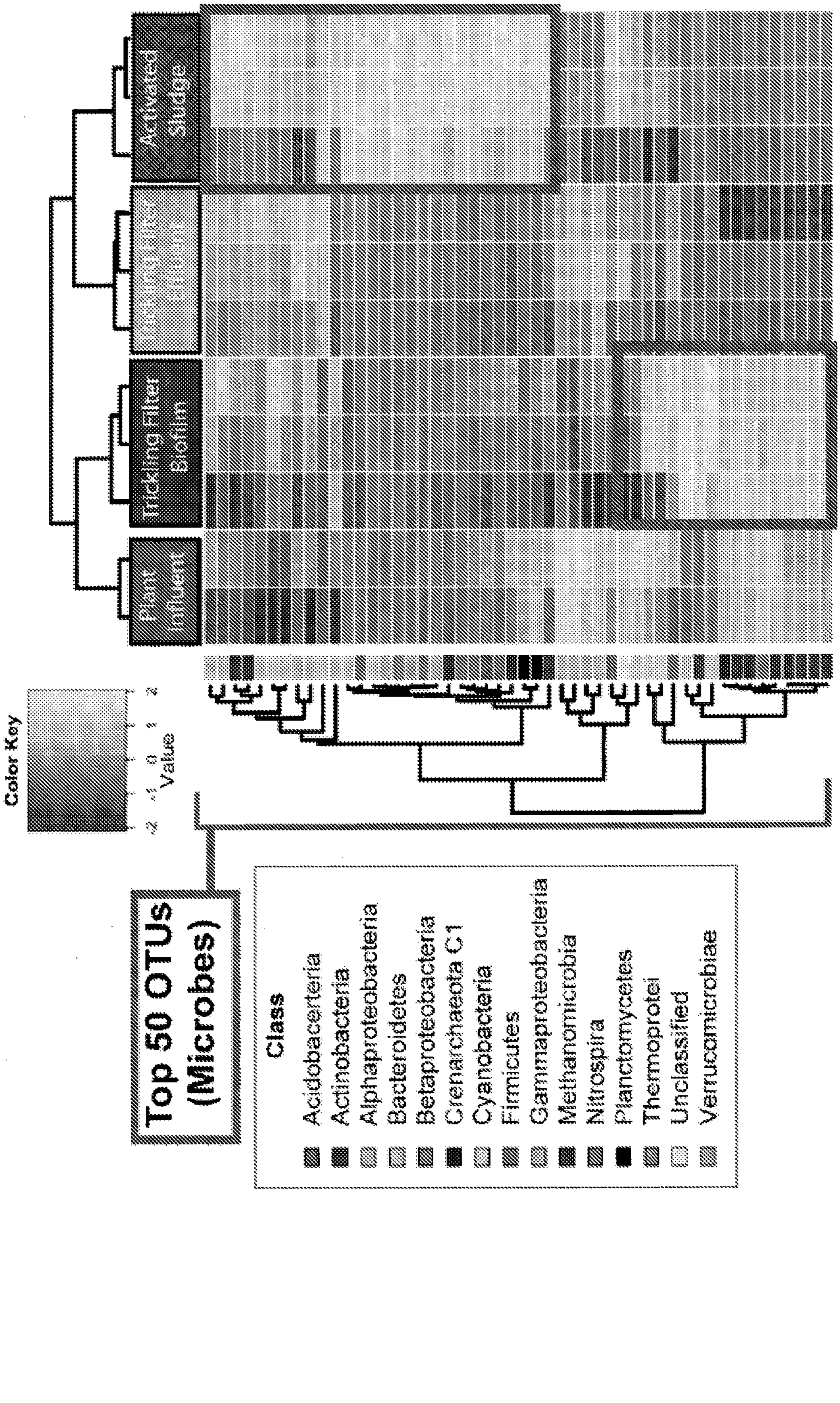

Color Key
-2 -1 0 1 2
Value
Plant Influent
Trickling Filter Biofilm
Activated Sludge
Top 50 OTUs (Microbes)
Class
Acidobacerteria
Actinobacteria
Alphaproteobacteria
Bacteroidetes
Betaproteobacteria
Crenarchaeota C1
Cyanobacteria
Firmicutes
Gammaproteobacteria
Methanomicrobia
Nitrospira
Planctomycetes
Thermoprotei
Unclassified
Verrucomicrobiae

# Figure 21

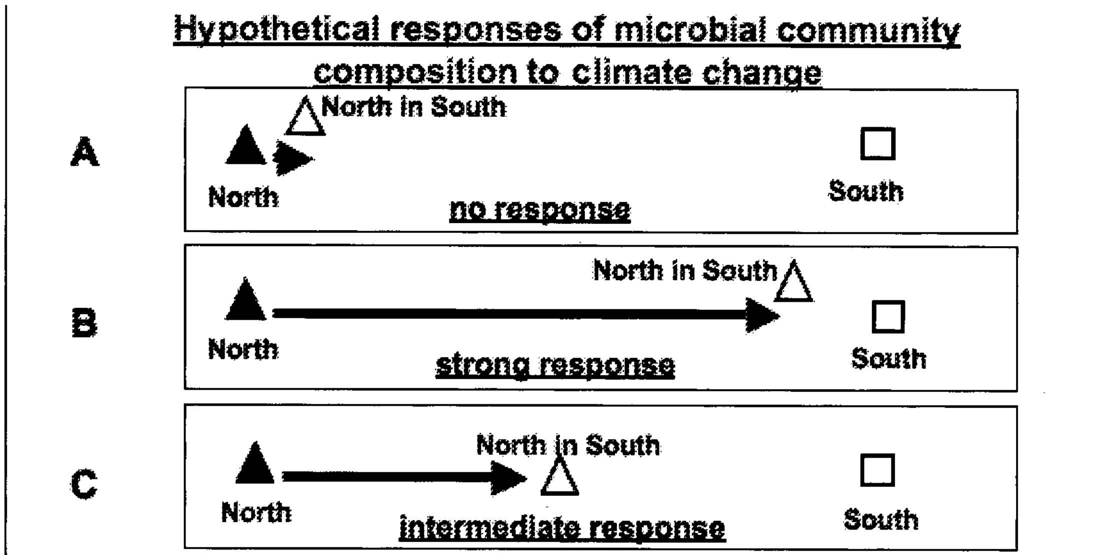


A suggests a strong influence of original edaphic factors, or slow change in response to climate

B indicates a strong influence of climate.

C indicates influence of both edaphic and climatic factors, and/or a longer time for change.

# Figure 22

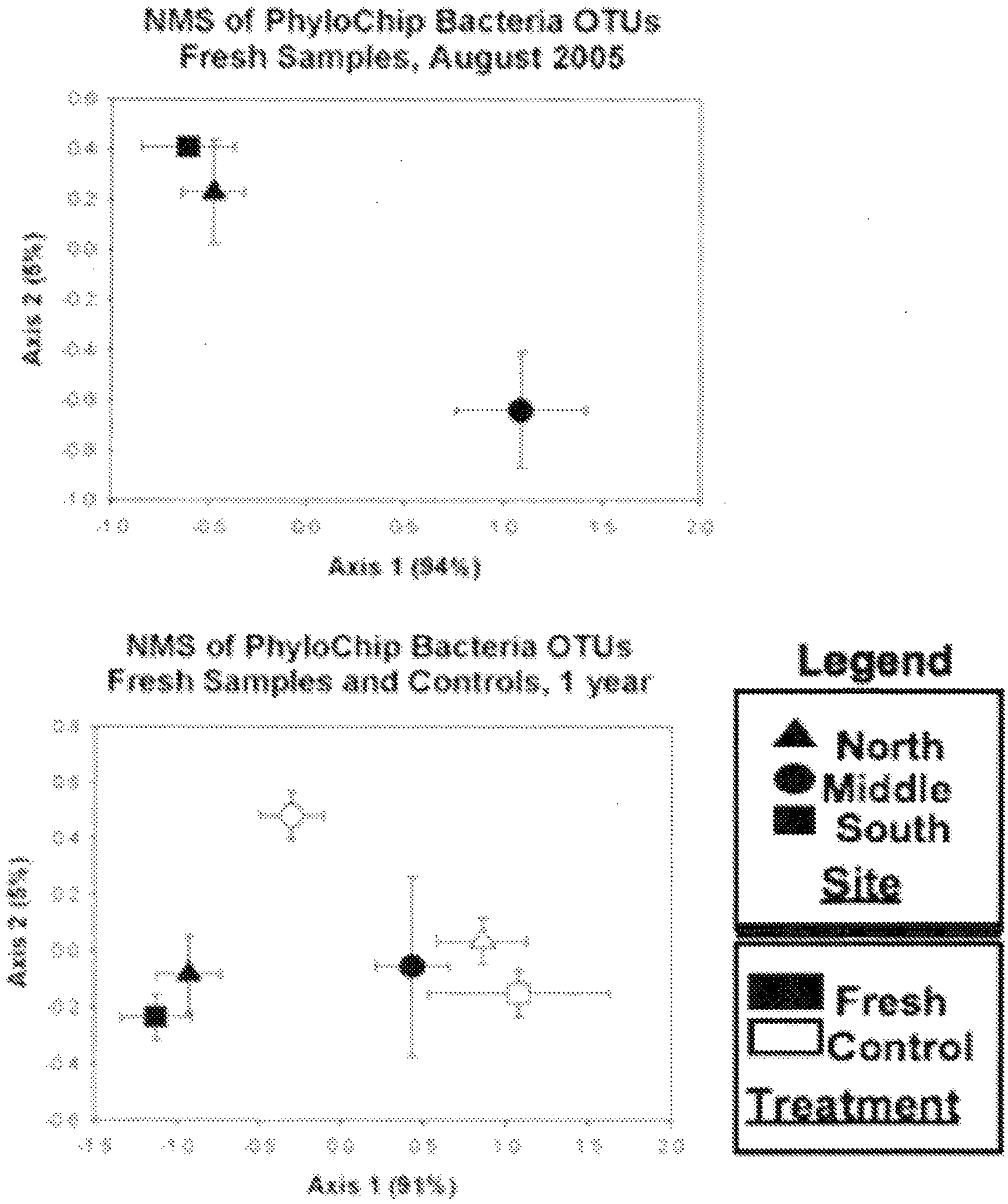
NMS of PhyloChip Bacteria OTUs
Fresh Samples, August 2005
Axis 1 (94%)
NMS of PhyloChip Bacteria OTUs
Fresh Samples and Controls, 1 year
Axis 1 (91%)
Legend
North
Middle
South
Site
Fresh
Control
Treatment

Figure 23

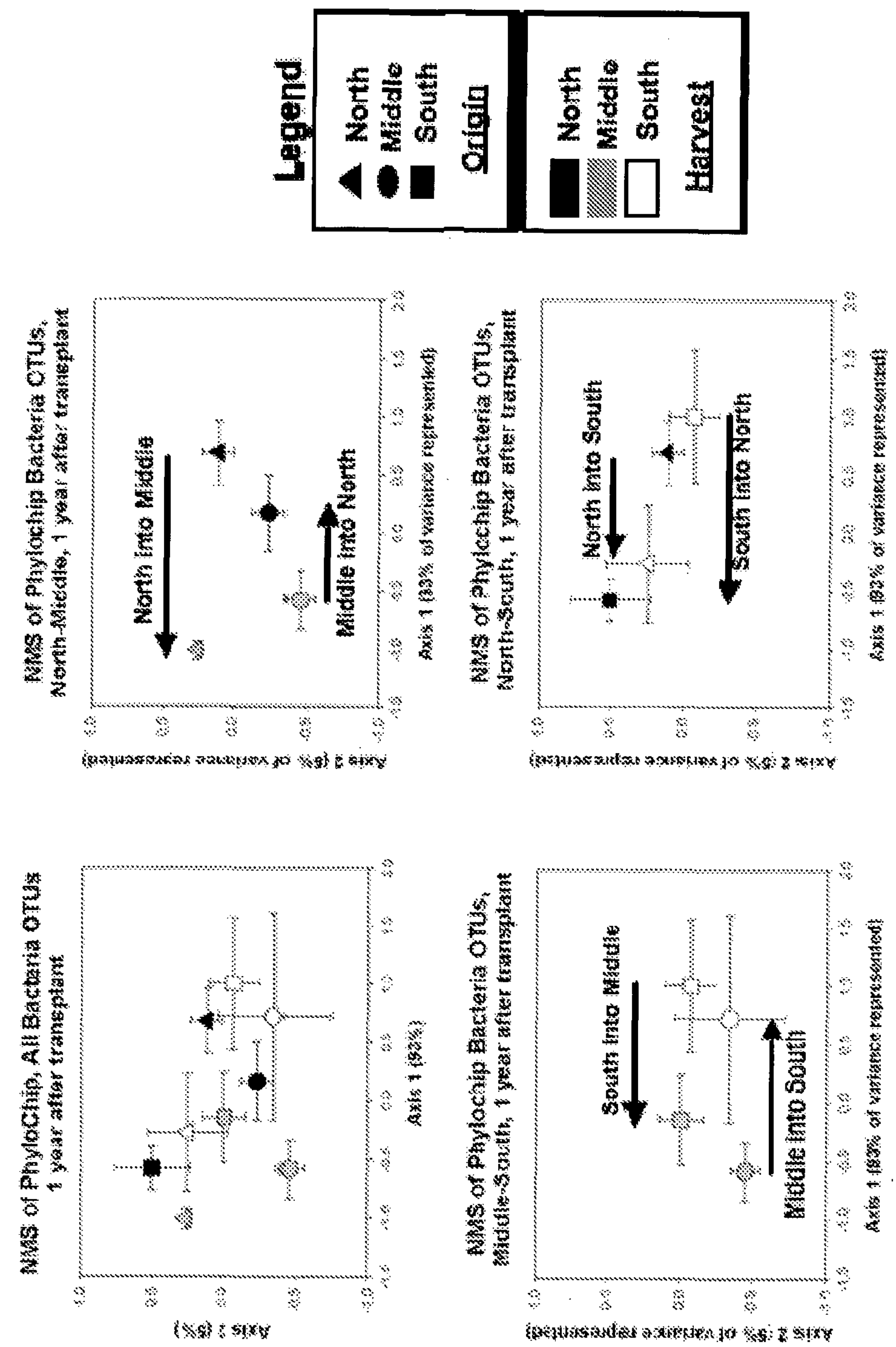
Legend
North
Middle
South
Origin
North
Middle
South
Harvest
NMS of PhyloChip, All Bacteria OTUs
1 year after transplant
NMS of Phylochip Bacteria OTUs,
North-Middle, 1 year after transplant
North into Middle
Middle into North
NMS of Phylochip Bacteria OTUs,
Middle-South, 1 year after transplant
South into Middle
Middle into South
NMS of Phylochip Bacteria OTUs,
North-South, 1 year after transplant
North into South
South into North

Figure 24

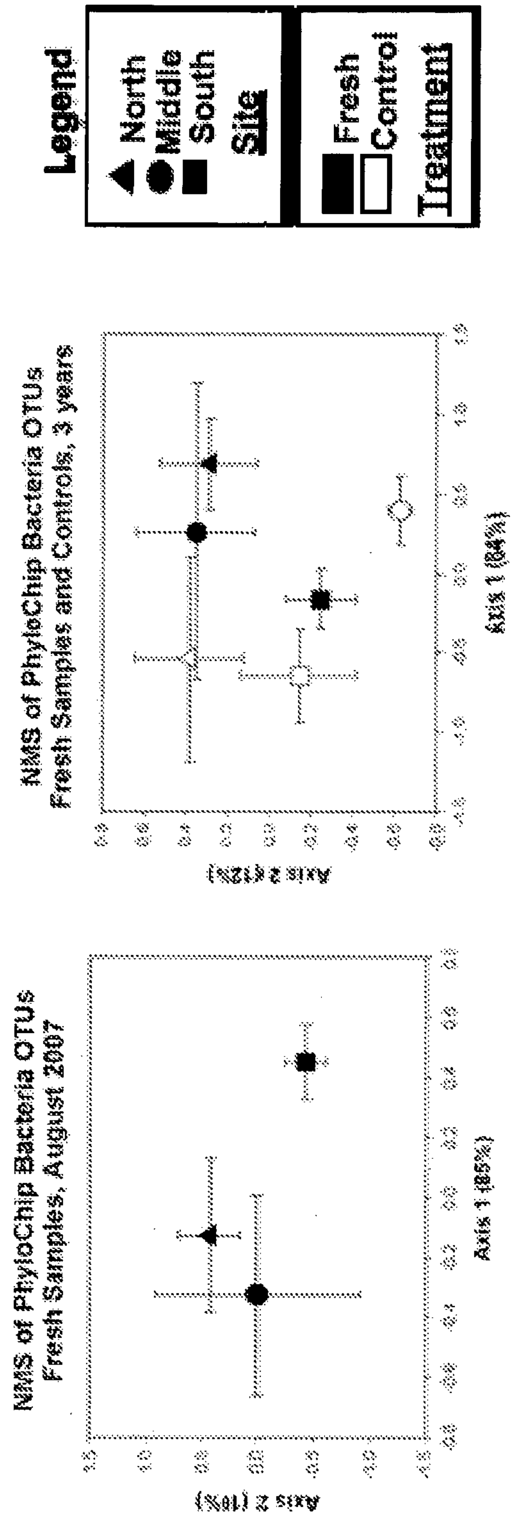

NMS of PhyloChip Bacteria OTUs
Fresh Samples, August 2007
Axis 1 (85%)
Axis 2 (10%)
NMS of PhyloChip Bacteria OTUs
Fresh Samples and Controls, 3 years
Axis 1 (84%)
Axis 2 (12%)
Legend
North
Middle
South
Site
Fresh
Control
Treatment

Figure 25

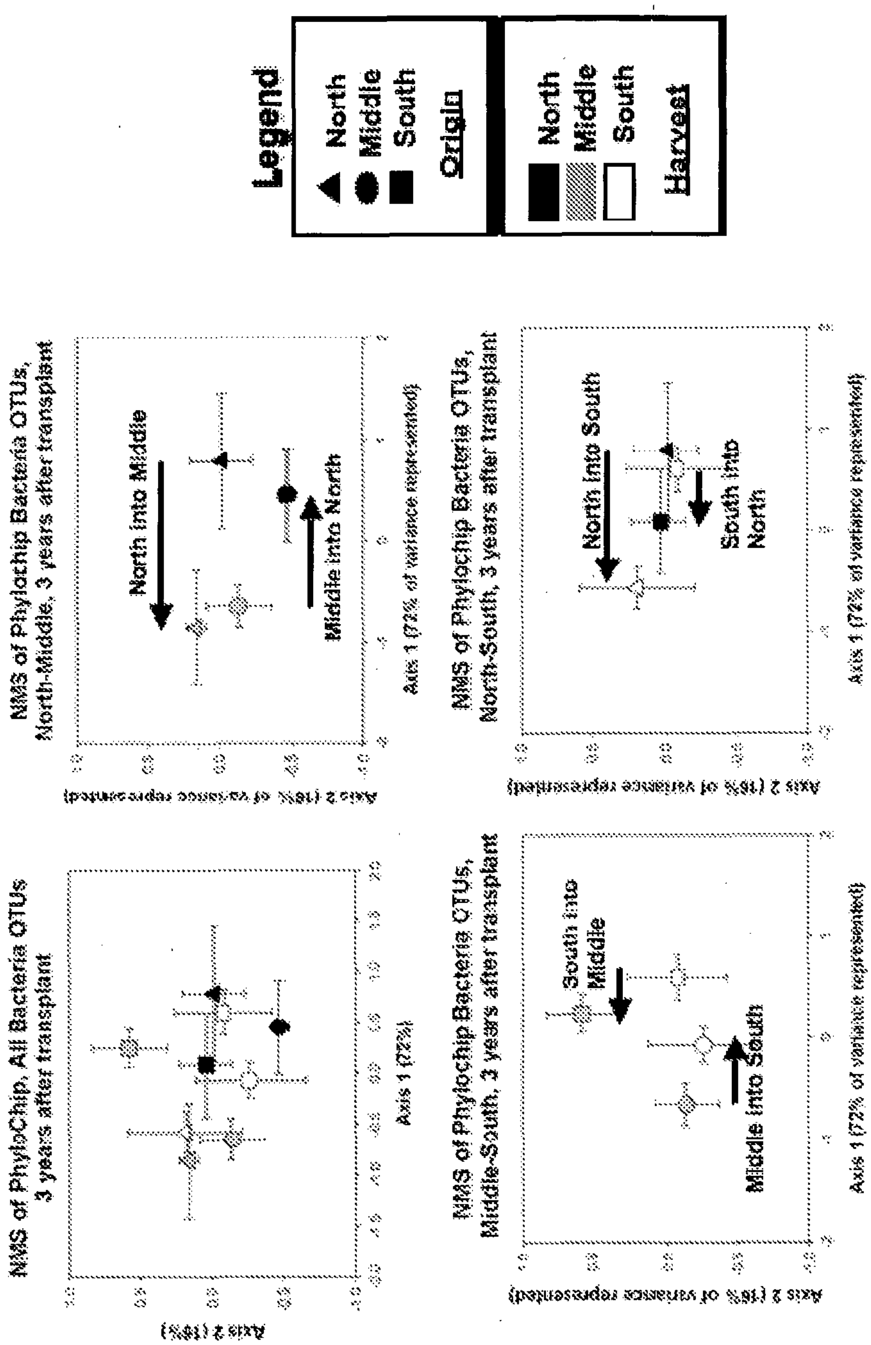
NMS of PhyloChip, All Bacteria OTUs
3 years after transplant
Axis 1 (72%)
Axis 2 (18%)
NMS of Phylochip Bacteria OTUs,
North-Middle, 3 years after transplant
North into Middle
Middle into North
Axis 1 (72% of variance represented)
Axis 2 (18% of variance represented)
NMS of Phylochip Bacteria OTUs,
Middle-South, 3 years after transplant
South into
Middle
Middle into South
NMS of Phylochip Bacteria OTUs,
North-South, 3 years after transplant
North into South
South into
North
Legend
North
Middle
South
Origin
North
Middle
South
Harvest

Figure 26

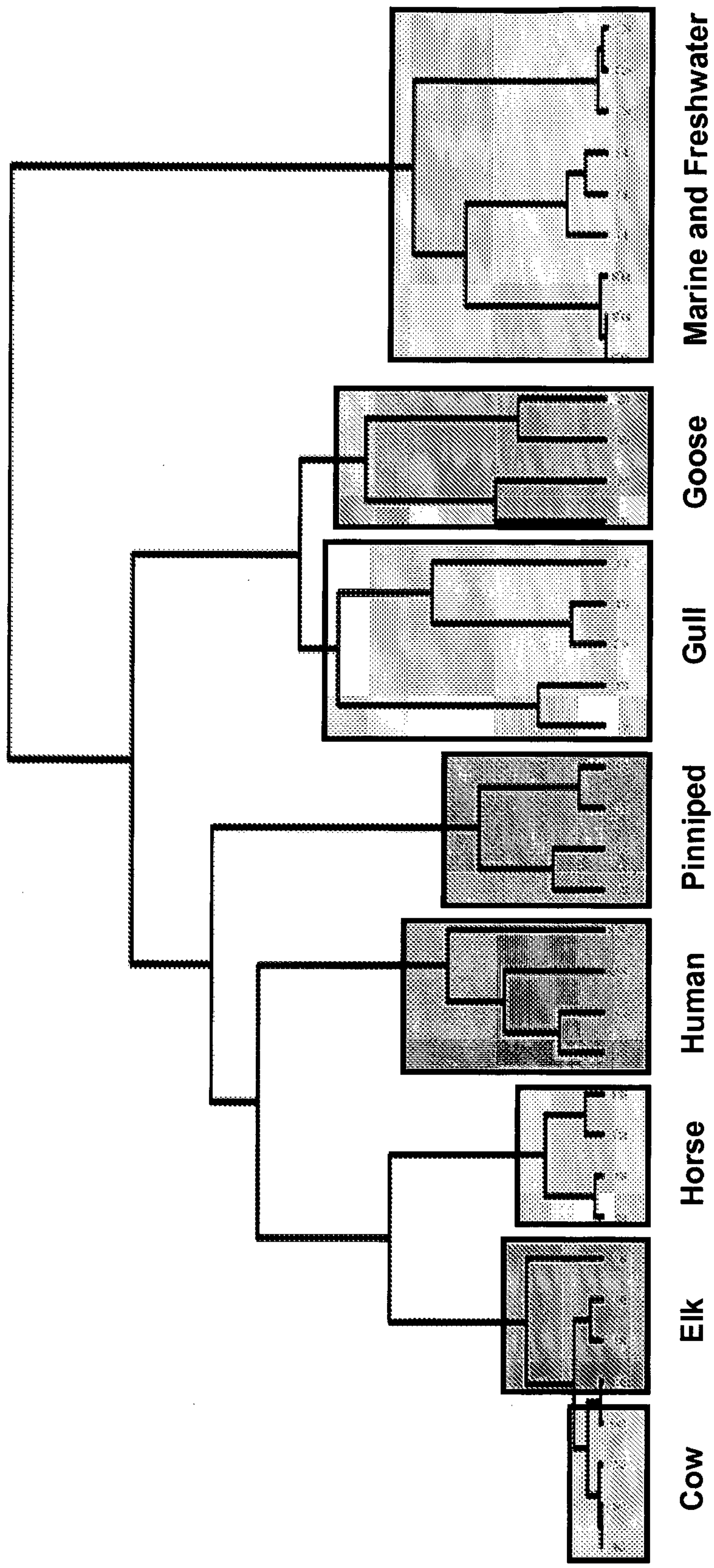
Marine and Freshwater
Goose
Gull
Pinniped
Human
Horse
Elk
Cow

Figure 27

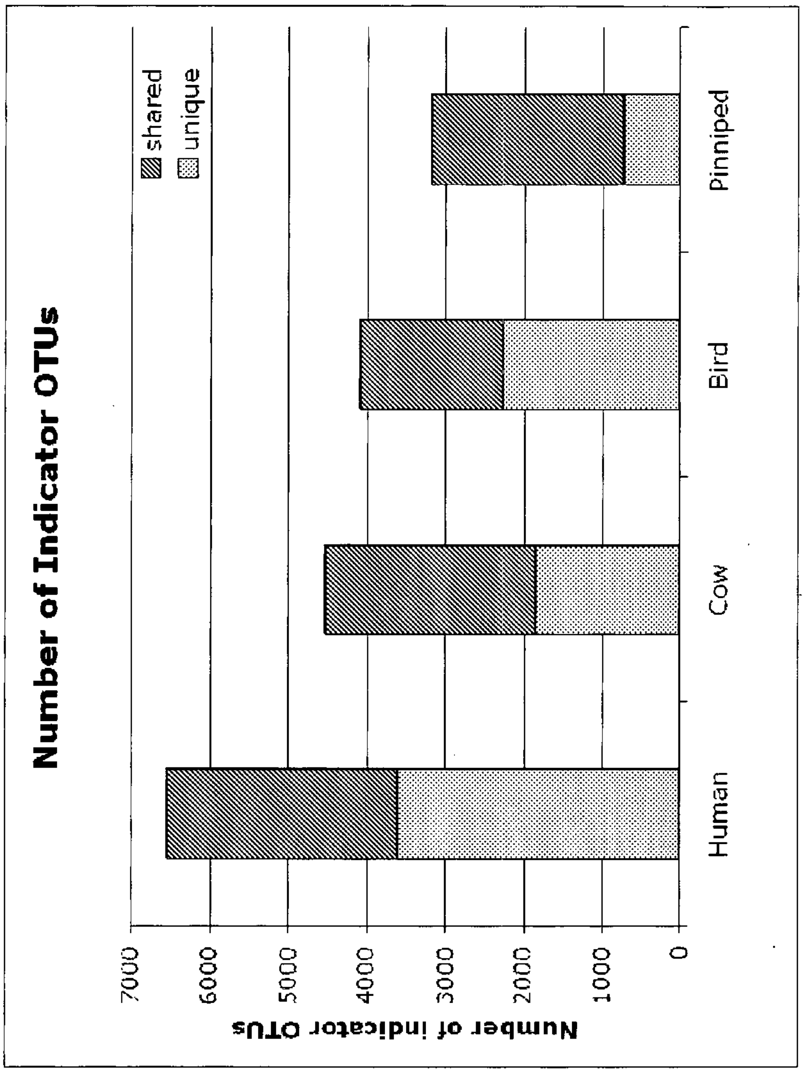
Number of Indicator OTUs
shared
unique
Number of indicator OTUs
7000
6000
5000
4000
3000
2000
1000
0
Human
Cow
Bird
Pinniped

# Figure 28

Compare indicator communities with elevated OTUs from each sample

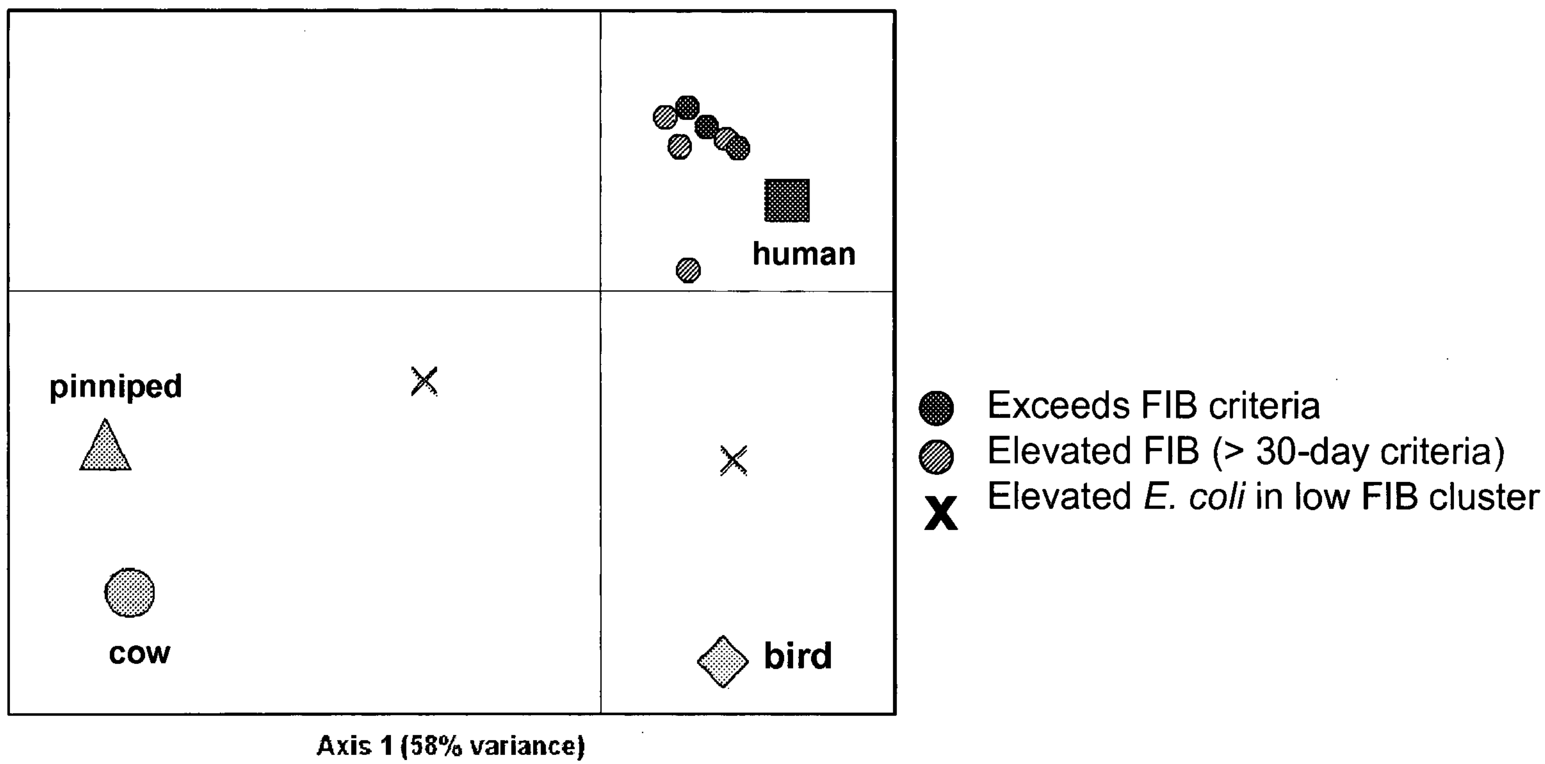

Figure 29

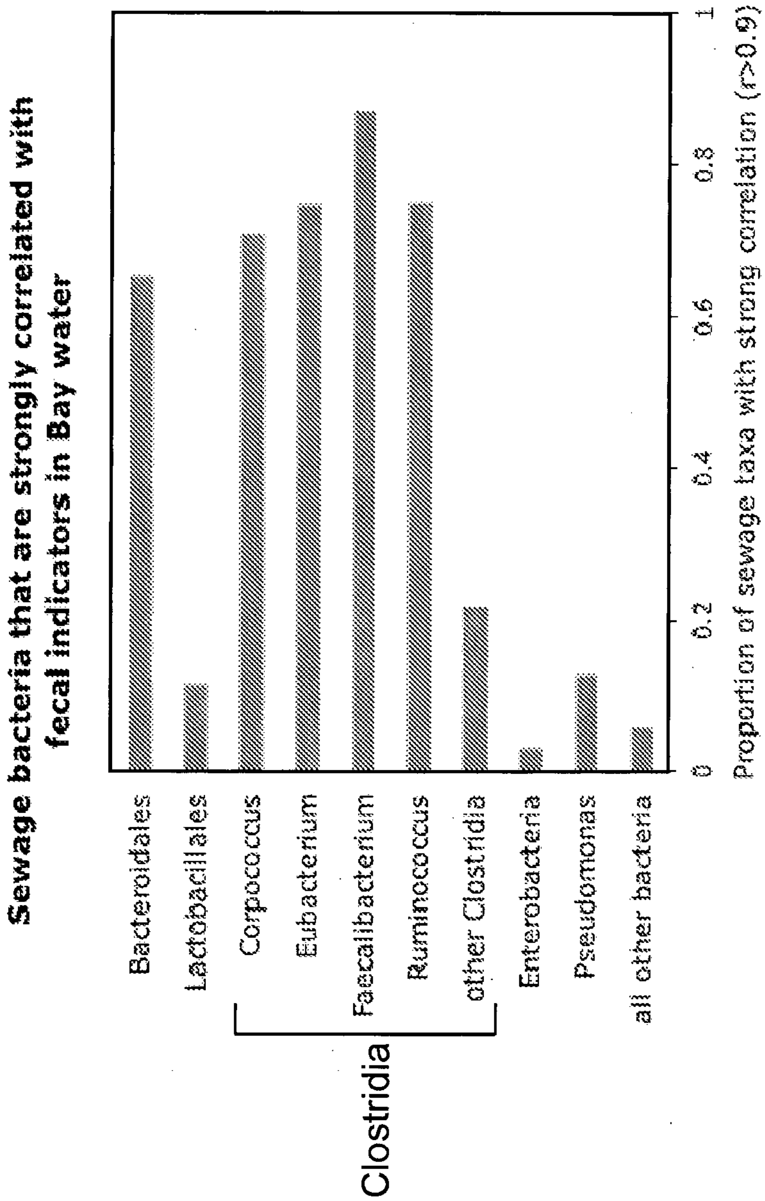
Sewage bacteria that are strongly correlated with fecal indicators in Bay water
Bacteroidales
Lactobacillales
Corpococcus
Eubacterium
Faecalibacterium
Ruminococcus
other Clostridia
Enterobacteria
Pseudomonas
all other bacteria
Clostridia
0
0.2
0.4
0.6
0.8
1
Proportion of sewage taxa with strong correlation (r>0.9)

Figure 30

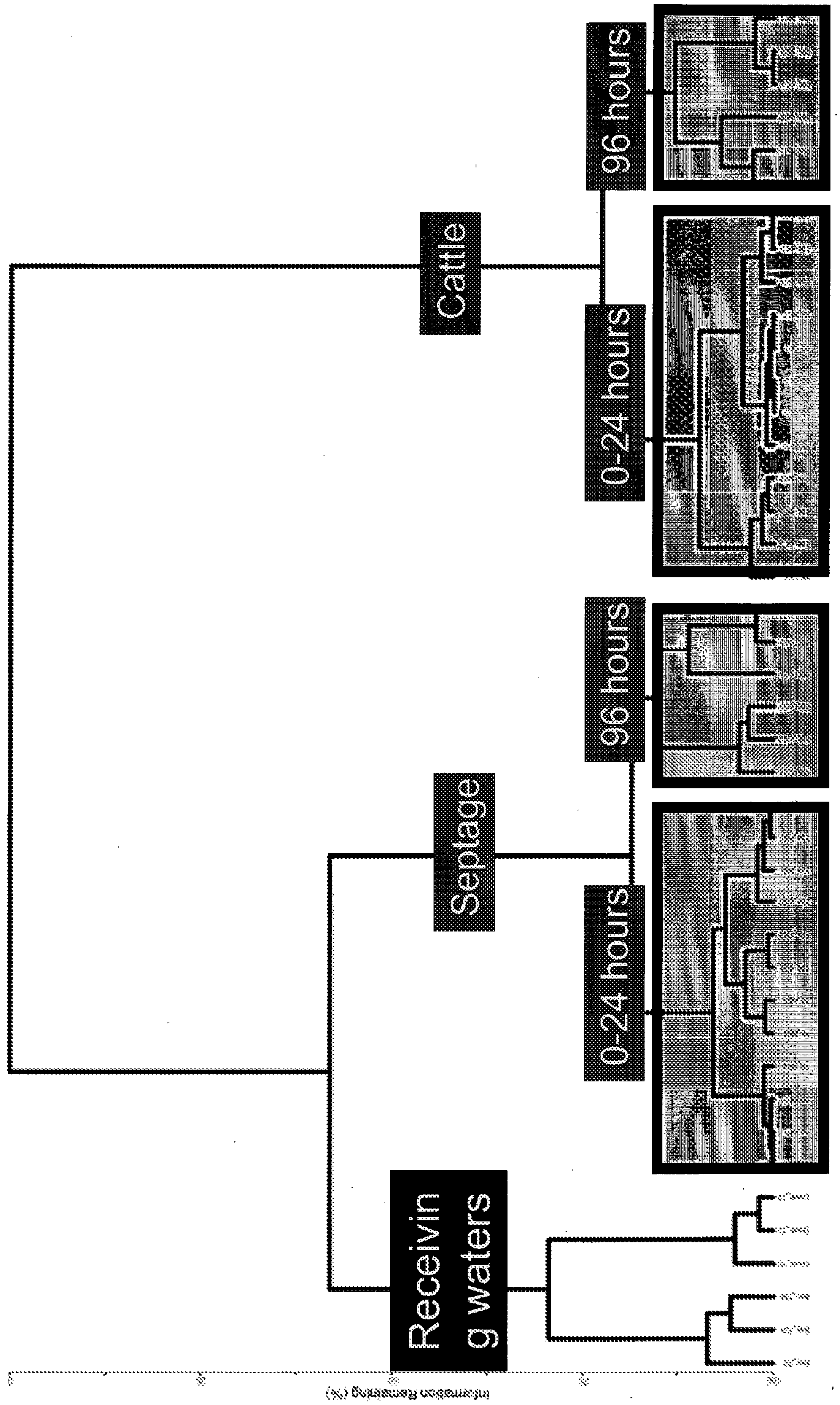

Cattle
96 hours
0-24 hours
Septage
96 hours
0-24 hours
Receivin
g waters

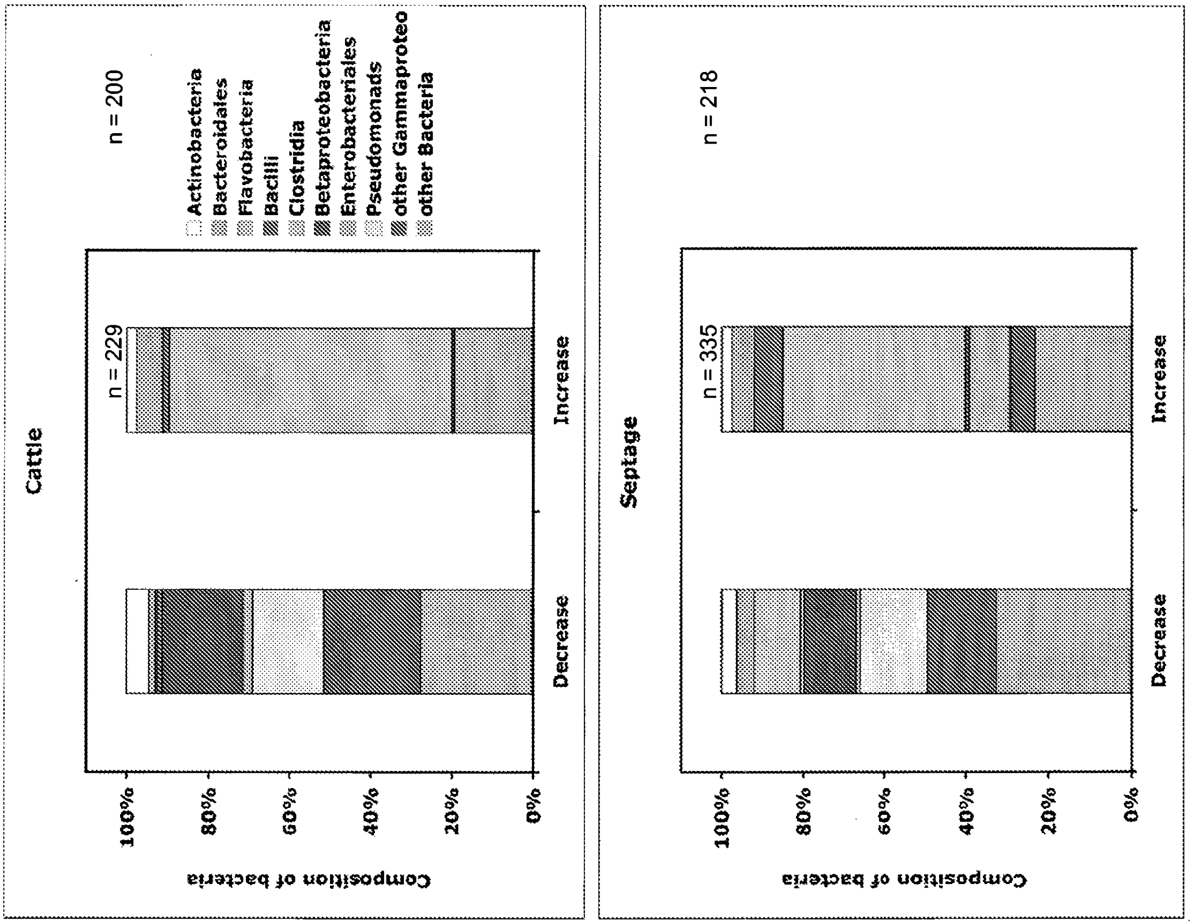
Cattle
Composition of bacteria
100%
80%
60%
40%
20%
0%
Decrease
Increase
n = 229
n = 200
Actinobacteria
Bacteroidales
Flavobacteria
Bacilli
Clostridia
Betaproteobacteria
Enterobacteriales
Pseudomonads
other Gammaproteo
other Bacteria
Septage
n = 335
n = 218

FIGURE 31

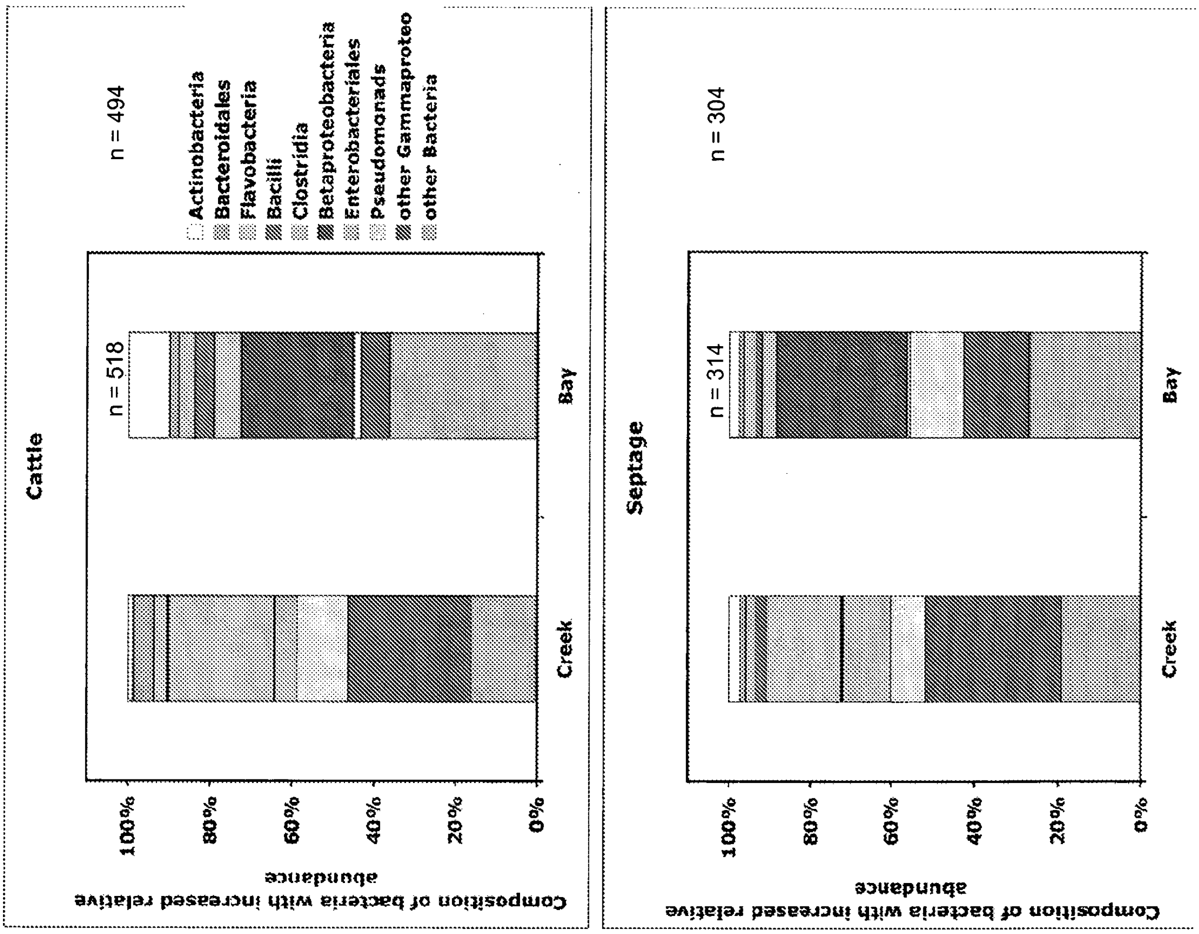
Cattle
Composition of bacteria with increased relative abundance
100%
80%
60%
40%
20%
0%
Creek
Bay
n = 518
n = 494
Actinobacteria
Bacteroidales
Flavobacteria
Bacilli
Clostridia
Betaproteobacteria
Enterobacteriales
Pseudomonads
other Gammaproteo
other Bacteria
Septage
Composition of bacteria with increased relative abundance
100%
80%
60%
40%
20%
0%
Creek
Bay
n = 314
n = 304

FIGURE 32

Figure 33

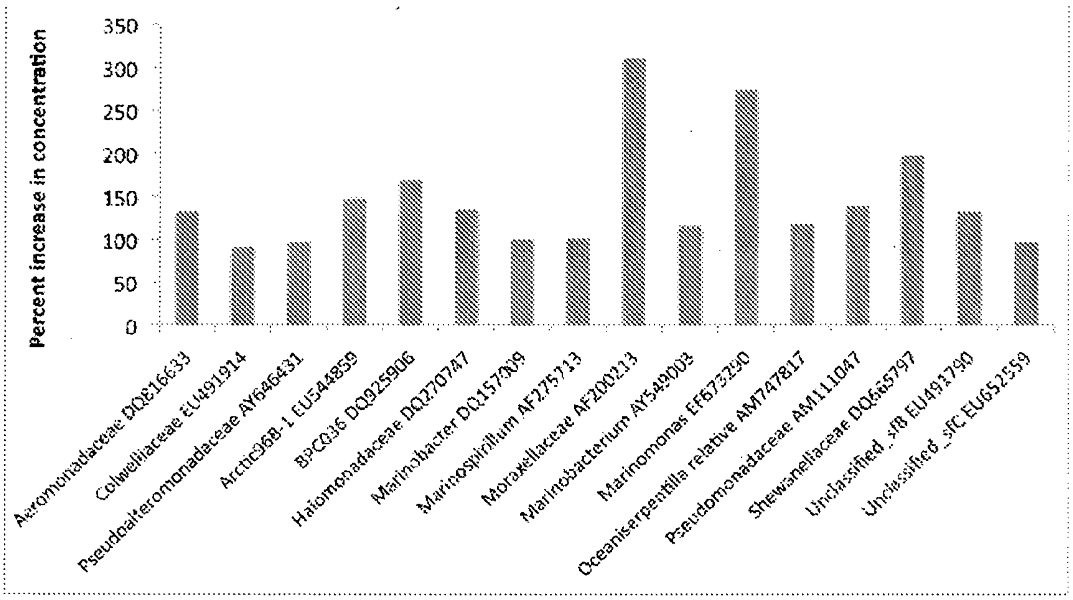
Percent increase in concentration
350
300
250
200
150
100
50
0
Aeromonadaceae DQ816633
Colwelliaceae EU491914
Pseudoalteromonadaceae AY646431
Arctic96B-1 EU544859
BPC036 DQ925906
Halomonadaceae DQ270747
Marinobacter DQ157009
Marinospirillum AF275713
Moraxellaceae AF200213
Marinobacterium AY549003
Marinomonas EF673290
Oceanisarpentilla relative AM747817
Pseudomonadaceae AM111047
Shewanellaceae DQ665797
Unclassified_sfB EU491790
Unclassified_sfC EU652559

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008130394 A
- US 5644048 A
- US 5386023 A
- US 5637684 A
- US 5602240 A
- US 5216141 A
- US 4469863 A
- US 5235033 A
- US 5034506 A
- US 6524793 B
- US 5539083 A
- US 5578832 A
- US 5556752 A
- US 5510270 A
- US 6449562 B
- WO 61316267 A
- WO 61240616 A
- WO 61240598 A
- WO 61221541 A


### Non-patent literature cited in the description


- **HUSE et al.** *PLoS Genetics,* 2008, vol. 4 (11), e1000255
- **HATTORI et al.** *DNA Res.,* 2009, vol. 16, 1-12
- **BEAUCAGE et al.** *Tetrahedron,* 1993, vol. 49 (10), 1925
- **LETSINGER.** *J. Org. Chem.,* 1970, vol. 35, 3800
- **SPRINZL et al.** *Eur. J. Biochem.,* 1977, vol. 81, 579
- **LETSINGER et al.** *Nucl. Acids Res.,* 1986, vol. 14, 3487
- **SAWAI et al.** *Chem. Lett.,* 1984, vol. 805
- **LETSINGER et al.** *J. Am. Chem. Soc.,* 1988, vol. 110, 4470
- **PAUWELS et al.** *Chemica Scripta,* 1986, vol. 26, 141
- **MAG et al.** *Nucleic Acids Res.,* 1991, vol. 19, 1437
- **BRIU et al.** *J. Am. Chem. Soc.,* 1989, vol. 111, 2321
- **ECKSTEIN.** Oligonucleotides and Analogues: A Practical Approach. Oxford University Press
- **EGHOLM.** *J. Am. Chem. Soc.,* 1992, vol. 114, 1895
- **MEIER et al.** *Chem. Int. Ed. Engl.,* 1992, vol. 31, 1008
- **NIELSEN.** *Nature,* 1993, vol. 365, 566
- **CARLSSON et al.** *Nature,* 1996, vol. 380, 207
- **DENPCY et al.** *Proc. Natl. Acad. Sci. USA,* 1995, vol. 92, 6097
- **KIEDROWSHI et al.** *Angew. Chem. Intl. Ed. English,* 1991, vol. 30, 423
- **LETSINGER et al.** Nucleosides & Nucleotides. 1994, vol. 13, 1597
- Carbohydrate Modifications in Antisense Research. ASC Symposium Series 580
- **MESMAEKER et al.** *Bioorganic & Medicinal Chem. Lett.,* 1994, vol. 4, 395
- **JEFFS et al.** *J. Biomolecular NMR,* 1994, vol. 34, 17
- *Tetrahedron Lett.,* 1996, vol. 37, 743
- **JENKINS et al.** *Chem. Soc. Rev.,* 1995, 169-176
- **RAWLS.** *C & E News,* 02 June 1997, 35
- **NG et al.** A spatially addressable bead-based biosensor for simple and rapid DNA detection. *Biosensors & Bioelectronics,* 2008, vol. 23, 803-810
- **GEISS G et al.** Direct multiplexed measurement of gene expression with color-coded probe pairs. *Nature Biotech,* 2008, vol. 26, 317-325
- **DESANTIS et al.** *Nucleic Acids Res.,* 2006, vol. 34, W394-399
- **THOMPSON et al.** *Nucleic Acids Res,* 1994, vol. 22, 4673-4680
- **EDGAR.** *Nucleic Acids Res.,* 2004, vol. 32, 1792-1797
- **ZHOU et al.** *Appl. Environ. Microbiol.,* 1996, vol. 62, 316-322
- **WU et al.** *Appl. Environ. Microbiol.,* 2006, vol. 72, 4931-4941
- **HURT et al.** *Appl. Environ. Microbiol.,* 2001, vol. 67, 4495-4503
- **MCGRATH et al.** *J. Microbiol. Methods,* 2008, vol. 75, 172-176
- **GAO et al.** *Appl. Environ. Microbiol.,* 2007, vol. 73, 563-571
- **DESANTIS et al.** *Microbial Ecology,* 2007, vol. 53 (3), 371-383

- **FROEHLER et al.** *Nucleic Acid Res.,* 1986, vol. 14, 5399-5407
- **MCBRIDE et al.** *Tetrahedron Lett.,* 1983, vol. 24, 246-248
- **EGHOLM et al.** *Nature,* 1993, vol. 363, 566-568
- **SCHENA et al.** *Science,* vol. 270
- **FODOR et al.** *Science,* 1991, vol. 251, 767-773
- **PEASE et al.** *Proc. Natl. Acad. Sci. U.S.A.,* 1994, vol. 91, 5022-5026
- **LOCKHART et al.** *Nature Biotechnology,* 1996, vol. 14, 1675
- **BLANCHARD et al.** *Biosensors & Bioelectronics,* vol. 11, 687-690
- **MASKOS ; SOUTHERN.** *Nuc. Acids. Res.,* 1992, vol. 20
- **CHEE et al.** *Science,* 1996, vol. 274, 610-614
- **RHEE et al.** *Appl. Environ. Microbiol.,* 2004, vol. 70, 4303-4317
- **ZHANG L. et al.** A model of molecular interactions on short oligonucleotide analysis systems. *Nat. Biotechnol.,* 2003, vol. 21 (7), 818-821
- **YERGEAU et al.** Environmental microarray analyses of Antarctic soil microbial communities. *ISME J.,* 2009, vol. 3, 340-351
- **LOZUPONE et al.** UniFrac--an online tool for comparing microbial community diversity in a phylogenetic context. *BMC Bioinformatics,* 2006, vol. 7, 371
- **DE HOON et al.** Open source clustering software. *Bioinformatics,* 2004, vol. 20, 1453-1454
- **BRODIE et al.** Application of a High Density Oligonucleotide Analysis system Approach to Study Bacterial Population Dynamics during Uranium Reduction and Reoxidation. *Applied Environ Microbio.,* 2006, vol. 72
- **GOSLEE SC ; URBAN DL.** The ecodist package for dissimilarity- based analysis of ecological data. *J Stat Softw,* 2007, vol. 22 (7), 1-19
- **EVANS J ; SHENEMAN L ; FOSTER JA.** Relaxed neighbor-joining: a fast distance-based phylogenetic tree. Construction method. *J Mol Evol,* 2006, vol. 62, 785-792
- **LOZUPONE C ; KNIGHT R.** UniFrac: a new phylogenetic method for comparing microbial communities. *Applied and Environmental Microbiology,* 2005, vol. 71 (12
- **DESANTIS TZ et al.** Greengenes, a chimera-checked 16S rRNA gene database and workbench compatible with ARB. *Applied and Environmental Microbiology,* 2006, vol. 72 (7
- **JUTEAU P ; TREMBLAY D ; VILLEMUR R ; BISAILLON JG ; BEAUDET R.** Analysis of the bacterial community inhabiting an aerobic thermophilic sequencing batch reactor (AT-SBR) treating swine waste. *Applied Microbiology and Biotechnology,* 2005, vol. 66, 115-122
- *Sausalito-Marin City Sanitary District,* February 2009
- **ANDERSON RT et al.** Stimulating the in situ activity of Geobacter species to remove uranium from the groundwater of a uranium-contaminated aquifer. *AEM,* 2003, vol. 69, 5884-5891
- **HUGENHOLTZ.** *Genome Biol.,* 2002, vol. 3 (2), 1-8
- **DESANTIS et al.** *Microb. Ecol.,* 2007, vol. 53, 371

SEQUENCE LISTING

<110> GENOCEA BIOSCIENCES INC. CHILDREN’S HOSPITAL BOSTON

<120> VACCINES AND COMPOSITIONS AGAINST STREPTOCOCCUS PNEUMONIAE

<130> 105592-0003-WO1

<140>
<141>

<150> 61/316,267
<151> 2010-03-22

<150> 61/240,616
<151> 2009-09-08

<150> 61/240,598
<151> 2009-09-08

<150> 61/221,541
<151> 2009-06-29

<160> 264

<170> PatentIn version 3.5

<210> 1
<211> 165
<212> PRT
<213> Streptococcus pneumoniae

<400> 1

Met Ser Tyr Phe Glu Gln Phe Met Gln Ala Asn Gln Ala Tyr Val Ala
1 5 10 15

Leu His Gly Gln Leu Asn Leu Pro Leu Lys Pro Lys Thr Arg Val Ala
20 25 30

Ile Val Thr Cys Met Asp Ser Arg Leu His Val Ala Gln Ala Leu Gly
35 40 45

Leu Ala Leu Gly Asp Ala His Ile Leu Arg Asn Ala Gly Gly Arg Val
50 55 60

Thr Glu Asp Met Ile Arg Ser Leu Val Ile Ser Gln Gln Gln Met Gly
65 70 75 80

Thr Arg Glu Ile Val Val Leu His His Thr Asp Cys Gly Ala Gln Thr
85 90 95

Phe Glu Asn Glu Pro Phe Gln Glu Tyr Leu Lys Glu Glu Leu Gly Val
100 105 110

Asp Val Ser Asp Gln Asp Phe Leu Pro Phe Gln Asp Ile Glu Glu Ser
115 120 125

Val Arg Glu Asp Met Gln Leu Leu Ile Glu Ser Pro Leu Ile Pro Asp
130 135 140

Asp Val Ile Ile Ser Gly Ala Ile Tyr Asn Val Asp Thr Gly Ser Met
145 150 155 160

Thr Val Val Glu Leu
165

<210> 2
<211> 274
<212> PRT
<213> Streptococcus pneumoniae

<400> 2

Met Asn Gln Ser Tyr Phe Tyr Leu Lys Met Lys Glu His Lys Leu Lys
1 5 10 15

Val Pro Tyr Thr Gly Lys Glu Arg Arg Val Arg Ile Leu Leu Pro Lys
20 25 30

Asp Tyr Glu Lys Asp Thr Asp Arg Ser Tyr Pro Val Val Tyr Phe His
35 40 45

Asp Gly Gln Asn Val Phe Asn Ser Lys Glu Ser Phe Ile Gly His Ser
50 55 60

Trp Lys Ile Ile Pro Ala Ile Lys Arg Asn Pro Asp Ile Ser Arg Met
65 70 75 80

Ile Val Val Ala Ile Asp Asn Asp Gly Met Gly Arg Met Asn Glu Tyr
85 90 95

Ala Ala Trp Lys Phe Gln Glu Ser Pro Ile Pro Gly Gln Gln Phe Gly
100 105 110

Gly Lys Gly Val Glu Tyr Ala Glu Phe Val Met Glu Val Val Lys Pro
115 120 125

Phe Ile Asp Glu Thr Tyr Arg Thr Lys Ala Asp Cys Gln His Thr Ala
130 135 140

Met Ile Gly Ser Ser Leu Gly Gly Asn Ile Thr Gln Phe Ile Gly Leu
145 150 155 160

Glu Tyr Gln Asp Gln Ile Gly Cys Leu Gly Val Phe Ser Ser Ala Asn
165 170 175

Trp Leu His Gln Glu Ala Phe Asn Arg Tyr Phe Glu Cys Gln Lys Leu
180 185 190

```
Ser Pro Asp Gln Arg Ile Phe Ile Tyr Val Gly Thr Glu Glu Ala Asp
        195                 200                 205

Asp Thr Asp Lys Thr Leu Met Asp Gly Asn Ile Lys Gln Ala Tyr Ile
    210                 215                 220

Asp Ser Ser Leu Cys Tyr Tyr His Asp Leu Ile Ala Gly Gly Val His
225                 230                 235                 240

Leu Asp Asn Leu Val Leu Lys Val Gln Ser Gly Ala Ile His Ser Glu
                245                 250                 255

Ile Pro Trp Ser Glu Asn Leu Pro Asp Cys Leu Arg Phe Phe Ala Glu
            260                 265                 270

Lys Trp
```

<210> 3
<211> 130
<212> PRT
<213> Streptococcus pneumoniae

<400> 3

```
Met Asn Gln Ser Tyr Phe Tyr Leu Lys Met Lys Glu His Lys Leu Lys
1               5                   10                  15

Val Pro Tyr Thr Gly Lys Glu Arg Arg Val Arg Ile Leu Leu Pro Lys
            20                  25                  30

Asp Tyr Glu Lys Asp Thr Asp Arg Ser Tyr Pro Val Val Tyr Phe His
        35                  40                  45

Asp Gly Gln Asn Val Phe Asn Ser Lys Glu Ser Phe Ile Gly His Ser
    50                  55                  60

Trp Lys Ile Ile Pro Ala Ile Lys Arg Asn Pro Asp Ile Ser Arg Met
65                  70                  75                  80

Ile Val Val Ala Ile Asp Asn Asp Gly Met Gly Arg Met Asn Glu Tyr
                85                  90                  95

Ala Ala Trp Lys Phe Gln Glu Ser Pro Ile Pro Gly Gln Gln Phe Gly
            100                 105                 110

Gly Lys Gly Val Glu Tyr Ala Glu Phe Val Met Glu Val Val Lys Pro
        115                 120                 125

Phe Ile

                            130
```

<210> 4
<211> 299
<212> PRT
<213> Streptococcus pneumoniae

<400> 4

```
Met Ser Ser Lys Phe Met Lys Ser Ala Ala Val Leu Gly Thr Ala Thr
1               5                   10                  15

Leu Ala Ser Leu Leu Leu Val Ala Cys Met Asn Gln Ser Tyr Phe Tyr
            20                  25                  30

Leu Lys Met Lys Glu His Lys Leu Lys Val Pro Tyr Thr Gly Lys Glu
        35                  40                  45

Arg Arg Val Arg Ile Leu Leu Pro Lys Asp Tyr Glu Lys Asp Thr Asp
    50                  55                  60

Arg Ser Tyr Pro Val Val Tyr Phe His Asp Gly Gln Asn Val Phe Asn
65                  70                  75                  80

Ser Lys Glu Ser Phe Ile Gly His Ser Trp Lys Ile Ile Pro Ala Ile
                85                  90                  95

Lys Arg Asn Pro Asp Ile Ser Arg Met Ile Val Val Ala Ile Asp Asn
            100                 105                 110

Asp Gly Met Gly Arg Met Asn Glu Tyr Ala Ala Trp Lys Phe Gln Glu
        115                 120                 125

Ser Pro Ile Pro Gly Gln Gln Phe Gly Gly Lys Gly Val Glu Tyr Ala
    130                 135                 140

Glu Phe Val Met Glu Val Val Lys Pro Phe Ile Asp Glu Thr Tyr Arg
145                 150                 155                 160

Thr Lys Ala Asp Cys Gln His Thr Ala Met Ile Gly Ser Ser Leu Gly
                165                 170                 175

Gly Asn Ile Thr Gln Phe Ile Gly Leu Glu Tyr Gln Asp Gln Ile Gly
            180                 185                 190

Cys Leu Gly Val Phe Ser Ser Ala Asn Trp Leu His Gln Glu Ala Phe
        195                 200                 205

Asn Arg Tyr Phe Glu Cys Gln Lys Leu Ser Pro Asp Gln Arg Ile Phe
    210                 215                 220
```

Ile Tyr Val Gly Thr Glu Glu Ala Asp Asp Thr Asp Lys Thr Leu Met
225 230 235 240

Asp Gly Asn Ile Lys Gln Ala Tyr Ile Asp Ser Ser Leu Cys Tyr Tyr
245 250 255

His Asp Leu Ile Ala Gly Gly Val His Leu Asp Asn Leu Val Leu Lys
260 265 270

Val Gln Ser Gly Ala Ile His Ser Glu Ile Pro Trp Ser Glu Asn Leu
275 280 285

Pro Asp Cys Leu Arg Phe Phe Ala Glu Lys Trp
290 295

<210> 5
<211> 155
<212> PRT
<213> Streptococcus pneumoniae

<400> 5

```
Met Ser Ser Lys Phe Met Lys Ser Ala Ala Val Leu Gly Thr Ala Thr
1               5                   10                  15

Leu Ala Ser Leu Leu Leu Val Ala Cys Met Asn Gln Ser Tyr Phe Tyr
            20                  25                  30

Leu Lys Met Lys Glu His Lys Leu Lys Val Pro Tyr Thr Gly Lys Glu
        35                  40                  45

Arg Arg Val Arg Ile Leu Leu Pro Lys Asp Tyr Glu Lys Asp Thr Asp
    50                  55                  60

Arg Ser Tyr Pro Val Val Tyr Phe His Asp Gly Gln Asn Val Phe Asn
65                  70                  75                  80

Ser Lys Glu Ser Phe Ile Gly His Ser Trp Lys Ile Ile Pro Ala Ile
                85                  90                  95

Lys Arg Asn Pro Asp Ile Ser Arg Met Ile Val Val Ala Ile Asp Asn
            100                 105                 110

Asp Gly Met Gly Arg Met Asn Glu Tyr Ala Ala Trp Lys Phe Gln Glu
        115                 120                 125

Ser Pro Ile Pro Gly Gln Gln Phe Gly Gly Lys Gly Val Glu Tyr Ala
    130                 135                 140

Glu Phe Val Met Glu Val Val Lys Pro Phe Ile
145                 150                 155
```

<210> 6
<211> 254
<212> PRT
<213> Streptococcus pneumoniae

<400> 6

```
Met Cys Ser Gly Gly Ala Lys Lys Glu Gly Glu Ala Ala Ser Lys Lys
1               5                   10                  15

Glu Ile Ile Val Ala Thr Asn Gly Ser Pro Lys Pro Phe Ile Tyr Glu
            20                  25                  30

Glu Asn Gly Glu Leu Thr Gly Tyr Glu Ile Glu Val Val Arg Ala Ile
        35                  40                  45

Phe Lys Asp Ser Asp Lys Tyr Asp Val Lys Phe Glu Lys Thr Glu Trp
    50                  55                  60

Ser Gly Val Phe Ala Gly Leu Asp Ala Asp Arg Tyr Asn Met Ala Val
65                  70                  75                  80

Asn Asn Leu Ser Tyr Thr Lys Glu Arg Ala Glu Lys Tyr Leu Tyr Ala
                85                  90                  95

Ala Pro Ile Ala Gln Asn Pro Asn Val Leu Val Val Lys Lys Asp Asp
            100                 105                 110

Ser Ser Ile Lys Ser Leu Asp Asp Ile Gly Gly Lys Ser Thr Glu Val
        115                 120                 125

Val Gln Ala Thr Thr Ser Ala Lys Gln Leu Glu Ala Tyr Asn Ala Glu
    130                 135                 140

His Thr Asp Asn Pro Thr Ile Leu Asn Tyr Thr Lys Ala Asp Phe Gln
145                 150                 155                 160

Gln Ile Met Val Arg Leu Ser Asp Gly Gln Phe Asp Tyr Lys Ile Phe
                165                 170                 175

Asp Lys Ile Gly Val Glu Thr Val Ile Lys Asn Gln Gly Leu Asp Asn
            180                 185                 190

Leu Lys Val Ile Glu Leu Pro Ser Asp Gln Gln Pro Tyr Val Tyr Pro
        195                 200                 205

Leu Leu Ala Gln Gly Gln Asp Glu Leu Lys Ser Phe Val Asp Lys Arg
    210                 215                 220

Ile Lys Glu Leu Tyr Lys Asp Gly Thr Leu Glu Lys Leu Ser Lys Gln
225                 230                 235                 240

    Phe Phe Gly Asp Thr Tyr Leu Pro Ala Glu Ala Asp Ile Lys
                    245                 250
```

<210> 7
<211> 276
<212> PRT
<213> Streptococcus pneumoniae

<400> 7

```
Met Lys Lys Ile Val Lys Tyr Ser Ser Leu Ala Ala Leu Ala Leu Val
1               5                   10                  15

Ala Ala Gly Val Leu Ala Ala Cys Ser Gly Gly Ala Lys Lys Glu Gly
            20                  25                  30

Glu Ala Ala Ser Lys Lys Glu Ile Ile Val Ala Thr Asn Gly Ser Pro
        35                  40                  45

Lys Pro Phe Ile Tyr Glu Glu Asn Gly Glu Leu Thr Gly Tyr Glu Ile
    50                  55                  60

Glu Val Val Arg Ala Ile Phe Lys Asp Ser Asp Lys Tyr Asp Val Lys
65                  70                  75                  80

Phe Glu Lys Thr Glu Trp Ser Gly Val Phe Ala Gly Leu Asp Ala Asp
                85                  90                  95

Arg Tyr Asn Met Ala Val Asn Asn Leu Ser Tyr Thr Lys Glu Arg Ala
            100                 105                 110

Glu Lys Tyr Leu Tyr Ala Ala Pro Ile Ala Gln Asn Pro Asn Val Leu
        115                 120                 125

Val Val Lys Lys Asp Asp Ser Ser Ile Lys Ser Leu Asp Asp Ile Gly
    130                 135                 140

Gly Lys Ser Thr Glu Val Val Gln Ala Thr Thr Ser Ala Lys Gln Leu
145                 150                 155                 160

Glu Ala Tyr Asn Ala Glu His Thr Asp Asn Pro Thr Ile Leu Asn Tyr
                165                 170                 175

Thr Lys Ala Asp Phe Gln Gln Ile Met Val Arg Leu Ser Asp Gly Gln
            180                 185                 190

Phe Asp Tyr Lys Ile Phe Asp Lys Ile Gly Val Glu Thr Val Ile Lys
        195                 200                 205

Asn Gln Gly Leu Asp Asn Leu Lys Val Ile Glu Leu Pro Ser Asp Gln
```

210 215 220

Gln Pro Tyr Val Tyr Pro Leu Leu Ala Gln Gly Gln Asp Glu Leu Lys
225 230 235 240

Ser Phe Val Asp Lys Arg Ile Lys Glu Leu Tyr Lys Asp Gly Thr Leu
245 250 255

Glu Lys Leu Ser Lys Gln Phe Phe Gly Asp Thr Tyr Leu Pro Ala Glu
260 265 270

Ala Asp Ile Lys
275

<210> 8
<211> 586
<212> PRT
<213> Streptococcus pneumoniae

<400> 8

```
Met Val Asp Lys Gln Val Ile Glu Glu Ile Lys Asn Asn Ala Asn Ile
1               5                   10                  15

Val Glu Val Ile Gly Asp Val Ile Ser Leu Gln Lys Ala Gly Arg Asn
            20                  25                  30

Tyr Leu Gly Leu Cys Pro Phe His Gly Glu Lys Thr Pro Ser Phe Asn
        35                  40                  45

Val Val Glu Asp Lys Gln Phe Tyr His Cys Phe Gly Cys Gly Arg Ser
    50                  55                  60

Gly Asp Val Phe Lys Phe Ile Glu Glu Tyr Gln Gly Val Pro Phe Ile
65                  70                  75                  80

Glu Ala Val Gln Ile Leu Gly Gln Arg Val Gly Ile Glu Val Glu Lys
                85                  90                  95

Pro Leu Tyr Ser Glu Gln Lys Ser Ala Ser Pro His Gln Ala Leu Tyr
            100                 105                 110

Asp Met His Glu Asp Ala Ala Lys Phe Tyr His Ala Ile Leu Met Thr
        115                 120                 125

Thr Thr Met Gly Glu Glu Ala Arg Asn Tyr Leu Tyr Gln Arg Gly Leu
    130                 135                 140

Thr Asp Glu Val Leu Lys His Phe Trp Ile Gly Leu Ala Pro Pro Glu
145                 150                 155                 160
```

```
Arg Asn Tyr Leu Tyr Gln Arg Leu Ser Asp Gln Tyr Arg Glu Glu Asp
                165                 170                 175

Leu Leu Asp Ser Gly Leu Phe Tyr Leu Ser Asp Ala Asn Gln Phe Val
            180                 185                 190

Asp Thr Phe His Asn Arg Ile Met Phe Pro Leu Thr Asn Asp Gln Gly
        195                 200                 205

Lys Val Ile Ala Phe Ser Gly Arg Ile Trp Gln Lys Thr Asp Ser Gln
    210                 215                 220

Thr Ser Lys Tyr Lys Asn Ser Arg Ser Thr Ala Ile Phe Asn Lys Ser
225                 230                 235                 240

Tyr Glu Leu Tyr His Met Asp Arg Ala Lys Arg Ser Ser Gly Lys Ala
                245                 250                 255

Ser Glu Ile Tyr Leu Met Glu Gly Phe Met Asp Val Ile Ala Ala Tyr
            260                 265                 270

Arg Ala Gly Ile Glu Asn Ala Val Ala Ser Met Gly Thr Ala Leu Ser
        275                 280                 285

Arg Glu His Val Glu His Leu Lys Arg Leu Thr Lys Lys Leu Val Leu
    290                 295                 300

Val Tyr Asp Gly Asp Lys Ala Gly Gln Ala Ala Thr Leu Lys Ala Leu
305                 310                 315                 320

Asp Glu Ile Gly Asp Met Pro Val Gln Ile Val Ser Met Pro Asp Asn
                325                 330                 335

Leu Asp Pro Asp Glu Tyr Leu Gln Lys Asn Gly Pro Glu Asp Leu Ala
            340                 345                 350

Tyr Leu Leu Thr Lys Thr Arg Ile Ser Pro Ile Glu Phe Tyr Ile His
        355                 360                 365

Gln Tyr Lys Pro Glu Asn Ser Glu Asn Leu Gln Ala Gln Ile Glu Phe
    370                 375                 380

Leu Glu Lys Ile Ala Pro Leu Ile Val Gln Glu Lys Ser Ile Ala Ala
385                 390                 395                 400

Gln Asn Ser Tyr Ile His Ile Leu Ala Asp Ser Leu Ala Ser Phe Asp
                405                 410                 415

Tyr Thr Gln Ile Glu Gln Ile Val Asn Glu Ser Arg Gln Val Gln Arg
```

420 425 430

Gln Asn Arg Met Glu Gly Ile Ser Arg Pro Thr Pro Ile Thr Met Pro
435 440 445

Val Thr Lys Gln Leu Ser Ala Ile Met Arg Ala Glu Ala His Leu Leu
450 455 460

Tyr Arg Met Met Glu Ser Pro Leu Val Leu Asn Asp Tyr Arg Leu Arg
465 470 475 480

Glu Asp Phe Ala Phe Ala Thr Pro Glu Phe Gln Val Leu Tyr Asp Leu
485 490 495

Leu Gly Gln Tyr Gly Asn Leu Pro Pro Glu Val Leu Ala Glu Gln Thr
500 505 510

Glu Glu Val Glu Arg Ala Trp Tyr Gln Val Leu Ala Gln Asp Leu Pro
515 520 525

Ala Glu Ile Ser Pro Gln Glu Leu Ser Glu Val Glu Met Thr Arg Asn
530 535 540

Lys Ala Leu Leu Asn Gln Asp Asn Met Arg Ile Lys Lys Lys Val Gln
545 550 555 560

Glu Ala Ser His Val Gly Asp Thr Asp Thr Ala Leu Glu Glu Leu Glu
565 570 575

Arg Leu Ile Ser Gln Lys Arg Arg Met Glu
580 585

<210> 9
<211> 423
<212> PRT
<213> Streptococcus pneumoniae

<400> 9

```
Met Ser Ser Lys Phe Met Lys Ser Ala Ala Val Leu Gly Thr Ala Thr
1               5                   10                  15

Leu Ala Ser Leu Leu Leu Val Ala Cys Gly Ser Lys Thr Ala Asp Lys
            20                  25                  30

Pro Ala Asp Ser Gly Ser Ser Glu Val Lys Glu Leu Thr Val Tyr Val
        35                  40                  45

Asp Glu Gly Tyr Lys Ser Tyr Ile Glu Glu Val Ala Lys Ala Tyr Glu
    50                  55                  60
```

Lys Glu Ala Gly Val Lys Val Thr Leu Lys Thr Gly Asp Ala Leu Gly
65 70 75 80

Gly Leu Asp Lys Leu Ser Leu Asp Asn Gln Ser Gly Asn Val Pro Asp
85 90 95

Val Met Met Ala Pro Tyr Asp Arg Val Gly Ser Leu Gly Ser Asp Gly
100 105 110

Gln Leu Ser Glu Val Lys Leu Ser Asp Gly Ala Lys Thr Asp Asp Thr
115 120 125

Thr Lys Ser Leu Val Thr Ala Ala Asn Gly Lys Val Tyr Gly Ala Pro
130 135 140

Ala Val Ile Glu Ser Leu Val Met Tyr Tyr Asn Lys Asp Leu Val Lys
145 150 155 160

Asp Ala Pro Lys Thr Phe Ala Asp Leu Glu Asn Leu Ala Lys Asp Ser
165 170 175

Lys Tyr Ala Phe Ala Gly Glu Asp Gly Lys Thr Thr Ala Phe Leu Ala
180 185 190

Asp Trp Thr Asn Phe Tyr Tyr Thr Tyr Gly Leu Leu Ala Gly Asn Gly
195 200 205

Ala Tyr Val Phe Gly Gln Asn Gly Lys Asp Ala Lys Asp Ile Gly Leu
210 215 220

Ala Asn Asp Gly Ser Ile Val Gly Ile Asn Tyr Ala Lys Ser Trp Tyr
225 230 235 240

Glu Lys Trp Pro Lys Gly Met Gln Asp Thr Glu Gly Ala Gly Asn Leu
245 250 255

Ile Gln Thr Gln Phe Gln Glu Gly Lys Thr Ala Ala Ile Ile Asp Gly
260 265 270

Pro Trp Lys Ala Gln Ala Phe Lys Asp Ala Lys Val Asn Tyr Gly Val
275 280 285

Ala Thr Ile Pro Thr Leu Pro Asn Gly Lys Glu Tyr Ala Ala Phe Gly
290 295 300

Gly Gly Lys Ala Trp Val Ile Pro Gln Ala Val Lys Asn Leu Glu Ala
305 310 315 320

Ser Gln Lys Phe Val Asp Phe Leu Val Ala Thr Glu Gln Gln Lys Val

```
                325                     330                     335

Leu Tyr Asp Lys Thr Asn Glu Ile Pro Ala Asn Thr Glu Ala Arg Ser
            340                     345                     350

Tyr Ala Glu Gly Lys Asn Asp Glu Leu Thr Thr Ala Val Ile Lys Gln
        355                     360                     365

Phe Lys Asn Thr Gln Pro Leu Pro Asn Ile Ser Gln Met Ser Ala Val
    370                     375                     380

Trp Asp Pro Ala Lys Asn Met Leu Phe Asp Ala Val Ser Gly Gln Lys
385                     390                     395                     400

Asp Ala Lys Thr Ala Ala Asn Asp Ala Val Thr Leu Ile Lys Glu Thr
                405                     410                     415

Ile Lys Gln Lys Phe Gly Glu
            420
```

<210> 10
<211> 400
<212> PRT
<213> Streptococcus pneumoniae

<400> 10

```
Met Cys Gly Ser Lys Thr Ala Asp Lys Pro Ala Asp Ser Gly Ser Ser
1               5                   10                  15

Glu Val Lys Glu Leu Thr Val Tyr Val Asp Glu Gly Tyr Lys Ser Tyr
            20                  25                  30

Ile Glu Glu Val Ala Lys Ala Tyr Glu Lys Glu Ala Gly Val Lys Val
        35                  40                  45

Thr Leu Lys Thr Gly Asp Ala Leu Gly Gly Leu Asp Lys Leu Ser Leu
    50                  55                  60

Asp Asn Gln Ser Gly Asn Val Pro Asp Val Met Met Ala Pro Tyr Asp
65                  70                  75                  80

Arg Val Gly Ser Leu Gly Ser Asp Gly Gln Leu Ser Glu Val Lys Leu
                85                  90                  95

Ser Asp Gly Ala Lys Thr Asp Asp Thr Thr Lys Ser Leu Val Thr Ala
            100                 105                 110

Ala Asn Gly Lys Val Tyr Gly Ala Pro Ala Val Ile Glu Ser Leu Val
        115                 120                 125
```

Met Tyr Tyr Asn Lys Asp Leu Val Lys Asp Ala Pro Lys Thr Phe Ala
130 135 140

Asp Leu Glu Asn Leu Ala Lys Asp Ser Lys Tyr Ala Phe Ala Gly Glu
145 150 155 160

Asp Gly Lys Thr Thr Ala Phe Leu Ala Asp Trp Thr Asn Phe Tyr Tyr
165 170 175

Thr Tyr Gly Leu Leu Ala Gly Asn Gly Ala Tyr Val Phe Gly Gln Asn
180 185 190

Gly Lys Asp Ala Lys Asp Ile Gly Leu Ala Asn Asp Gly Ser Ile Val
195 200 205

Gly Ile Asn Tyr Ala Lys Ser Trp Tyr Glu Lys Trp Pro Lys Gly Met
210 215 220

Gln Asp Thr Glu Gly Ala Gly Asn Leu Ile Gln Thr Gln Phe Gln Glu
225 230 235 240

Gly Lys Thr Ala Ala Ile Ile Asp Gly Pro Trp Lys Ala Gln Ala Phe
245 250 255

Lys Asp Ala Lys Val Asn Tyr Gly Val Ala Thr Ile Pro Thr Leu Pro
260 265 270

Asn Gly Lys Glu Tyr Ala Ala Phe Gly Gly Gly Lys Ala Trp Val Ile
275 280 285

Pro Gln Ala Val Lys Asn Leu Glu Ala Ser Gln Lys Phe Val Asp Phe
290 295 300

Leu Val Ala Thr Glu Gln Gln Lys Val Leu Tyr Asp Lys Thr Asn Glu
305 310 315 320

Ile Pro Ala Asn Thr Glu Ala Arg Ser Tyr Ala Glu Gly Lys Asn Asp
325 330 335

Glu Leu Thr Thr Ala Val Ile Lys Gln Phe Lys Asn Thr Gln Pro Leu
340 345 350

Pro Asn Ile Ser Gln Met Ser Ala Val Trp Asp Pro Ala Lys Asn Met
355 360 365

Leu Phe Asp Ala Val Ser Gly Gln Lys Asp Ala Lys Thr Ala Ala Asn
370 375 380

Asp Ala Val Thr Leu Ile Lys Glu Thr Ile Lys Gln Lys Phe Gly Glu

385 390 395 400

<210> 11
<211> 648
<212> PRT
<213> Streptococcus pneumoniae

<400> 11

Met Ser Gly Thr Ser Met Ala Thr Pro Ile Val Ala Ala Ser Thr Val
1               5                   10                  15

Leu Ile Arg Pro Lys Leu Lys Glu Met Leu Glu Arg Pro Val Leu Lys
            20                  25                  30

Asn Leu Lys Gly Asp Asp Lys Ile Asp Leu Thr Ser Leu Thr Lys Ile
        35                  40                  45

Ala Leu Gln Asn Thr Ala Arg Pro Met Met Asp Ala Thr Ser Trp Lys
    50                  55                  60

Glu Lys Ser Gln Tyr Phe Ala Ser Pro Arg Gln Gln Gly Ala Gly Leu
65                  70                  75                  80

Ile Asn Val Ala Asn Ala Leu Arg Asn Glu Val Val Ala Thr Phe Lys
                85                  90                  95

Asn Thr Asp Ser Lys Gly Leu Val Asn Ser Tyr Gly Ser Ile Ser Leu
            100                 105                 110

Lys Glu Ile Lys Gly Asp Lys Lys Tyr Phe Thr Ile Lys Leu His Asn
        115                 120                 125

Thr Ser Asn Arg Pro Leu Thr Phe Lys Val Ser Ala Ser Ala Ile Thr
    130                 135                 140

Thr Asp Ser Leu Thr Asp Arg Leu Lys Leu Asp Glu Thr Tyr Lys Asp
145                 150                 155                 160

Glu Lys Ser Pro Asp Gly Lys Gln Ile Val Pro Glu Ile His Pro Glu
                165                 170                 175

Lys Val Lys Gly Ala Asn Ile Thr Phe Glu His Asp Thr Phe Thr Ile
            180                 185                 190

Gly Ala Asn Ser Ser Phe Asp Leu Asn Ala Val Ile Asn Val Gly Glu
        195                 200                 205

Ala Lys Asn Lys Asn Lys Phe Val Glu Ser Phe Ile His Phe Glu Ser
    210                 215                 220

Val Glu Glu Met Glu Ala Leu Asn Ser Asn Gly Lys Lys Ile Asn Phe
225 230 235 240

Gln Pro Ser Leu Ser Met Pro Leu Met Gly Phe Ala Gly Asn Trp Asn
245 250 255

His Glu Pro Ile Leu Asp Lys Trp Ala Trp Glu Glu Gly Ser Arg Ser
260 265 270

Lys Thr Leu Gly Gly Tyr Asp Asp Asp Gly Lys Pro Lys Ile Pro Gly
275 280 285

Thr Leu Asn Lys Gly Ile Gly Gly Glu His Gly Ile Asp Lys Phe Asn
290 295 300

Pro Ala Gly Val Ile Gln Asn Arg Lys Asp Lys Asn Thr Thr Ser Leu
305 310 315 320

Asp Gln Asn Pro Glu Leu Phe Ala Phe Asn Asn Glu Gly Ile Asn Ala
325 330 335

Pro Ser Ser Ser Gly Ser Lys Ile Ala Asn Ile Tyr Pro Leu Asp Ser
340 345 350

Asn Gly Asn Pro Gln Asp Ala Gln Leu Glu Arg Gly Leu Thr Pro Ser
355 360 365

Pro Leu Val Leu Arg Ser Ala Glu Glu Gly Leu Ile Ser Ile Val Asn
370 375 380

Thr Asn Lys Glu Gly Glu Asn Gln Arg Asp Leu Lys Val Ile Ser Arg
385 390 395 400

Glu His Phe Ile Arg Gly Ile Leu Asn Ser Lys Ser Asn Asp Ala Lys
405 410 415

Gly Ile Lys Ser Ser Lys Leu Lys Val Trp Gly Asp Leu Lys Trp Asp
420 425 430

Gly Leu Ile Tyr Asn Pro Arg Gly Arg Glu Glu Asn Ala Pro Glu Ser
435 440 445

Lys Asp Asn Gln Asp Pro Ala Thr Lys Ile Arg Gly Gln Phe Glu Pro
450 455 460

Ile Ala Glu Gly Gln Tyr Phe Tyr Lys Phe Lys Tyr Arg Leu Thr Lys
465 470 475 480

Asp Tyr Pro Trp Gln Val Ser Tyr Ile Pro Val Lys Ile Asp Asn Thr

485 490 495

Ala Pro Lys Ile Val Ser Val Asp Phe Ser Asn Pro Glu Lys Ile Lys
500 505 510

Leu Ile Thr Lys Asp Thr Tyr His Lys Val Lys Asp Gln Tyr Lys Asn
515 520 525

Glu Thr Leu Phe Ala Arg Asp Gln Lys Glu His Pro Glu Lys Phe Asp
530 535 540

Glu Ile Ala Asn Glu Val Trp Tyr Ala Gly Ala Ala Leu Val Asn Glu
545 550 555 560

Asp Gly Glu Val Glu Lys Asn Leu Glu Val Thr Tyr Ala Gly Glu Gly
565 570 575

Gln Gly Arg Asn Arg Lys Leu Asp Lys Asp Gly Asn Thr Ile Tyr Glu
580 585 590

Ile Lys Gly Ala Gly Asp Leu Arg Gly Lys Ile Ile Glu Val Ile Ala
595 600 605

Leu Asp Gly Ser Ser Asn Phe Thr Lys Ile His Arg Ile Lys Phe Ala
610 615 620

Asn Gln Ala Asp Glu Lys Gly Met Ile Ser Tyr Tyr Leu Val Asp Pro
625 630 635 640

Asp Gln Asp Ser Ser Lys Tyr Gln
645

<210> 12
<211> 2140
<212> PRT
<213> Streptococcus pneumoniae

<400> 12

```
Met Lys Lys Ser Thr Val Leu Ser Leu Thr Thr Ala Ala Val Ile Leu
1               5                   10                  15

Ala Ala Tyr Ala Pro Asn Glu Val Val Leu Ala Asp Thr Ser Ser Ser
            20                  25                  30

Glu Asp Ala Leu Asn Ile Ser Asp Lys Glu Lys Val Ala Glu Asn Lys
        35                  40                  45

Glu Lys His Glu Asn Ile His Ser Ala Met Glu Thr Ser Gln Asp Phe
    50                  55                  60
```

```
Lys Glu Lys Lys Thr Ala Val Ile Lys Glu Lys Glu Val Val Ser Lys
65                  70                  75                  80

Asn Pro Val Ile Asp Asn Asn Thr Ser Asn Glu Glu Ala Lys Ile Lys
                85                  90                  95

Glu Glu Asn Ser Asn Lys Ser Gln Gly Asp Tyr Thr Asp Ser Phe Val
            100                 105                 110

Asn Lys Asn Thr Glu Asn Pro Lys Lys Glu Asp Lys Val Val Tyr Ile
        115                 120                 125

Ala Glu Phe Lys Asp Lys Glu Ser Gly Glu Lys Ala Ile Lys Glu Leu
    130                 135                 140

Ser Ser Leu Lys Asn Thr Lys Val Leu Tyr Thr Tyr Asp Arg Ile Phe
145                 150                 155                 160

Asn Gly Ser Ala Ile Glu Thr Thr Pro Asp Asn Leu Asp Lys Ile Lys
                165                 170                 175

Gln Ile Glu Gly Ile Ser Ser Val Glu Arg Ala Gln Lys Val Gln Pro
            180                 185                 190

Met Met Asn His Ala Arg Lys Glu Ile Gly Val Glu Glu Ala Ile Asp
        195                 200                 205

Tyr Leu Lys Ser Ile Asn Ala Pro Phe Gly Lys Asn Phe Asp Gly Arg
    210                 215                 220

Gly Met Val Ile Ser Asn Ile Asp Thr Gly Thr Asp Tyr Arg His Lys
225                 230                 235                 240

Ala Met Arg Ile Asp Asp Asp Ala Lys Ala Ser Met Arg Phe Lys Lys
                245                 250                 255

Glu Asp Leu Lys Gly Thr Asp Lys Asn Tyr Trp Leu Ser Asp Lys Ile
            260                 265                 270

Pro His Ala Phe Asn Tyr Tyr Asn Gly Gly Lys Ile Thr Val Glu Lys
        275                 280                 285

Tyr Asp Asp Gly Arg Asp Tyr Phe Asp Pro His Gly Met His Ile Ala
    290                 295                 300

Gly Ile Leu Ala Gly Asn Asp Thr Glu Gln Asp Ile Lys Asn Phe Asn
305                 310                 315                 320

Gly Ile Asp Gly Ile Ala Pro Asn Ala Gln Ile Phe Ser Tyr Lys Met
```

325 330 335

Tyr Ser Asp Ala Gly Ser Gly Phe Ala Gly Asp Glu Thr Met Phe His
340 345 350

Ala Ile Glu Asp Ser Ile Lys His Asn Val Asp Val Val Ser Val Ser
355 360 365

Ser Gly Phe Thr Gly Thr Gly Leu Val Gly Glu Lys Tyr Trp Gln Ala
370 375 380

Ile Arg Ala Leu Arg Lys Ala Gly Ile Pro Met Val Val Ala Thr Gly
385 390 395 400

Asn Tyr Ala Thr Ser Ala Ser Ser Ser Ser Trp Asp Leu Val Ala Asn
405 410 415

Asn His Leu Lys Met Thr Asp Thr Gly Asn Val Thr Arg Thr Ala Ala
420 425 430

His Glu Asp Ala Ile Ala Val Ala Ser Ala Lys Asn Gln Thr Val Glu
435 440 445

Phe Asp Lys Val Asn Ile Gly Gly Glu Ser Phe Lys Tyr Arg Asn Ile
450 455 460

Gly Ala Phe Phe Asp Lys Ser Lys Ile Thr Thr Asn Glu Asp Gly Thr
465 470 475 480

Lys Ala Pro Ser Lys Leu Lys Phe Val Tyr Ile Gly Lys Gly Gln Asp
485 490 495

Gln Asp Leu Ile Gly Leu Asp Leu Arg Gly Lys Ile Ala Val Met Asp
500 505 510

Arg Ile Tyr Thr Lys Asp Leu Lys Asn Ala Phe Lys Lys Ala Met Asp
515 520 525

Lys Gly Ala Arg Ala Ile Met Val Val Asn Thr Val Asn Tyr Tyr Asn
530 535 540

Arg Asp Asn Trp Thr Glu Leu Pro Ala Met Gly Tyr Glu Ala Asp Glu
545 550 555 560

Gly Thr Lys Ser Gln Val Phe Ser Ile Ser Gly Asp Asp Gly Val Lys
565 570 575

Leu Trp Asn Met Ile Asn Pro Asp Lys Lys Thr Glu Val Lys Arg Asn
580 585 590

```
Asn Lys Glu Asp Phe Lys Asp Lys Leu Glu Gln Tyr Tyr Pro Ile Asp
        595                 600                 605

Met Glu Ser Phe Asn Ser Asn Lys Pro Asn Val Gly Asp Glu Lys Glu
    610                 615                 620

Ile Asp Phe Lys Phe Ala Pro Asp Thr Asp Lys Glu Leu Tyr Lys Glu
625                 630                 635                 640

Asp Ile Ile Val Pro Ala Gly Ser Thr Ser Trp Gly Pro Arg Ile Asp
                645                 650                 655

Leu Leu Leu Lys Pro Asp Val Ser Ala Pro Gly Lys Asn Ile Lys Ser
            660                 665                 670

Thr Leu Asn Val Ile Asn Gly Lys Ser Thr Tyr Gly Tyr Met Ser Gly
        675                 680                 685

Thr Ser Met Ala Thr Pro Ile Val Ala Ala Ser Thr Val Leu Ile Arg
    690                 695                 700

Pro Lys Leu Lys Glu Met Leu Glu Arg Pro Val Leu Lys Asn Leu Lys
705                 710                 715                 720

Gly Asp Asp Lys Ile Asp Leu Thr Ser Leu Thr Lys Ile Ala Leu Gln
                725                 730                 735

Asn Thr Ala Arg Pro Met Met Asp Ala Thr Ser Trp Lys Glu Lys Ser
            740                 745                 750

Gln Tyr Phe Ala Ser Pro Arg Gln Gln Gly Ala Gly Leu Ile Asn Val
        755                 760                 765

Ala Asn Ala Leu Arg Asn Glu Val Val Ala Thr Phe Lys Asn Thr Asp
    770                 775                 780

Ser Lys Gly Leu Val Asn Ser Tyr Gly Ser Ile Ser Leu Lys Glu Ile
785                 790                 795                 800

Lys Gly Asp Lys Lys Tyr Phe Thr Ile Lys Leu His Asn Thr Ser Asn
                805                 810                 815

Arg Pro Leu Thr Phe Lys Val Ser Ala Ser Ala Ile Thr Thr Asp Ser
            820                 825                 830

Leu Thr Asp Arg Leu Lys Leu Asp Glu Thr Tyr Lys Asp Glu Lys Ser
        835                 840                 845
```

Pro Asp Gly Lys Gln Ile Val Pro Glu Ile His Pro Glu Lys Val Lys
850 855 860

Gly Ala Asn Ile Thr Phe Glu His Asp Thr Phe Thr Ile Gly Ala Asn
865 870 875 880

Ser Ser Phe Asp Leu Asn Ala Val Ile Asn Val Gly Glu Ala Lys Asn
885 890 895

Lys Asn Lys Phe Val Glu Ser Phe Ile His Phe Glu Ser Val Glu Glu
900 905 910

Met Glu Ala Leu Asn Ser Asn Gly Lys Lys Ile Asn Phe Gln Pro Ser
915 920 925

Leu Ser Met Pro Leu Met Gly Phe Ala Gly Asn Trp Asn His Glu Pro
930 935 940

Ile Leu Asp Lys Trp Ala Trp Glu Glu Gly Ser Arg Ser Lys Thr Leu
945 950 955 960

Gly Gly Tyr Asp Asp Asp Gly Lys Pro Lys Ile Pro Gly Thr Leu Asn
965 970 975

Lys Gly Ile Gly Gly Glu His Gly Ile Asp Lys Phe Asn Pro Ala Gly
980 985 990

Val Ile Gln Asn Arg Lys Asp Lys Asn Thr Thr Ser Leu Asp Gln Asn
995 1000 1005

Pro Glu Leu Phe Ala Phe Asn Asn Glu Gly Ile Asn Ala Pro Ser
1010 1015 1020

Ser Ser Gly Ser Lys Ile Ala Asn Ile Tyr Pro Leu Asp Ser Asn
1025 1030 1035

Gly Asn Pro Gln Asp Ala Gln Leu Glu Arg Gly Leu Thr Pro Ser
1040 1045 1050

Pro Leu Val Leu Arg Ser Ala Glu Glu Gly Leu Ile Ser Ile Val
1055 1060 1065

Asn Thr Asn Lys Glu Gly Glu Asn Gln Arg Asp Leu Lys Val Ile
1070 1075 1080

Ser Arg Glu His Phe Ile Arg Gly Ile Leu Asn Ser Lys Ser Asn
1085 1090 1095

Asp Ala Lys Gly Ile Lys Ser Ser Lys Leu Lys Val Trp Gly Asp
1100 1105 1110

Leu Lys Trp Asp Gly Leu Ile Tyr Asn Pro Arg Gly Arg Glu Glu
1115 1120 1125

Asn Ala Pro Glu Ser Lys Asp Asn Gln Asp Pro Ala Thr Lys Ile
1130 1135 1140

Arg Gly Gln Phe Glu Pro Ile Ala Glu Gly Gln Tyr Phe Tyr Lys
1145 1150 1155

Phe Lys Tyr Arg Leu Thr Lys Asp Tyr Pro Trp Gln Val Ser Tyr
1160 1165 1170

Ile Pro Val Lys Ile Asp Asn Thr Ala Pro Lys Ile Val Ser Val
1175 1180 1185

Asp Phe Ser Asn Pro Glu Lys Ile Lys Leu Ile Thr Lys Asp Thr
1190 1195 1200

Tyr His Lys Val Lys Asp Gln Tyr Lys Asn Glu Thr Leu Phe Ala
1205 1210 1215

Arg Asp Gln Lys Glu His Pro Glu Lys Phe Asp Glu Ile Ala Asn
1220 1225 1230

Glu Val Trp Tyr Ala Gly Ala Ala Leu Val Asn Glu Asp Gly Glu
1235 1240 1245

Val Glu Lys Asn Leu Glu Val Thr Tyr Ala Gly Glu Gly Gln Gly
1250 1255 1260

Arg Asn Arg Lys Leu Asp Lys Asp Gly Asn Thr Ile Tyr Glu Ile
1265 1270 1275

Lys Gly Ala Gly Asp Leu Arg Gly Lys Ile Ile Glu Val Ile Ala
1280 1285 1290

Leu Asp Gly Ser Ser Asn Phe Thr Lys Ile His Arg Ile Lys Phe
1295 1300 1305

Ala Asn Gln Ala Asp Glu Lys Gly Met Ile Ser Tyr Tyr Leu Val
1310 1315 1320

Asp Pro Asp Gln Asp Ser Ser Lys Tyr Gln Lys Leu Gly Glu Ile
1325 1330 1335

Ala Glu Ser Lys Phe Lys Asn Leu Gly Asn Gly Lys Glu Gly Ser

1340 1345 1350

Leu Lys Lys Asp Thr Thr Gly Val Glu His His His Gln Glu Asn
1355 1360 1365

Glu Glu Ser Ile Lys Glu Lys Ser Ser Phe Thr Ile Asp Arg Asn
1370 1375 1380

Ile Ser Thr Ile Arg Asp Phe Glu Asn Lys Asp Leu Lys Lys Leu
1385 1390 1395

Ile Lys Lys Lys Phe Arg Glu Val Asp Asp Phe Thr Ser Glu Thr
1400 1405 1410

Gly Lys Arg Met Glu Glu Tyr Asp Tyr Lys Tyr Asp Asp Lys Gly
1415 1420 1425

Asn Ile Ile Ala Tyr Asp Asp Gly Thr Asp Leu Glu Tyr Glu Thr
1430 1435 1440

Glu Lys Leu Asp Glu Ile Lys Ser Lys Ile Tyr Gly Val Leu Ser
1445 1450 1455

Pro Ser Lys Asp Gly His Phe Glu Ile Leu Gly Lys Ile Ser Asn
1460 1465 1470

Val Ser Lys Asn Ala Lys Val Tyr Tyr Gly Asn Asn Tyr Lys Ser
1475 1480 1485

Ile Glu Ile Lys Ala Thr Lys Tyr Asp Phe His Ser Lys Thr Met
1490 1495 1500

Thr Phe Asp Leu Tyr Ala Asn Ile Asn Asp Ile Val Asp Gly Leu
1505 1510 1515

Ala Phe Ala Gly Asp Met Arg Leu Phe Val Lys Asp Asn Asp Gln
1520 1525 1530

Lys Lys Ala Glu Ile Lys Ile Arg Met Pro Glu Lys Ile Lys Glu
1535 1540 1545

Thr Lys Ser Glu Tyr Pro Tyr Val Ser Ser Tyr Gly Asn Val Ile
1550 1555 1560

Glu Leu Gly Glu Gly Asp Leu Ser Lys Asn Lys Pro Asp Asn Leu
1565 1570 1575

Thr Lys Met Glu Ser Gly Lys Ile Tyr Ser Asp Ser Glu Lys Gln
1580 1585 1590

```
Gln Tyr  Leu Leu Lys Asp Asn  Ile Ile Leu Arg Lys  Gly Tyr Ala
    1595                 1600                 1605

Leu Lys  Val Thr Thr Tyr Asn  Pro Gly Lys Thr Asp  Met Leu Glu
    1610                 1615                 1620

Gly Asn  Gly Val Tyr Ser Lys  Glu Asp Ile Ala Lys  Ile Gln Lys
    1625                 1630                 1635

Ala Asn  Pro Asn Leu Arg Ala  Leu Ser Glu Thr Thr  Ile Tyr Ala
    1640                 1645                 1650

Asp Ser  Arg Asn Val Glu Asp  Gly Arg Ser Thr Gln  Ser Val Leu
    1655                 1660                 1665

Met Ser  Ala Leu Asp Gly Phe  Asn Ile Ile Arg Tyr  Gln Val Phe
    1670                 1675                 1680

Thr Phe  Lys Met Asn Asp Lys  Gly Glu Ala Ile Asp  Lys Asp Gly
    1685                 1690                 1695

Asn Leu  Val Thr Asp Ser Ser  Lys Leu Val Leu Phe  Gly Lys Asp
    1700                 1705                 1710

Asp Lys  Glu Tyr Thr Gly Glu  Asp Lys Phe Asn Val  Glu Ala Ile
    1715                 1720                 1725

Lys Glu  Asp Gly Ser Met Leu  Phe Ile Asp Thr Lys  Pro Val Asn
    1730                 1735                 1740

Leu Ser  Met Asp Lys Asn Tyr  Phe Asn Pro Ser Lys  Ser Asn Lys
    1745                 1750                 1755

Ile Tyr  Val Arg Asn Pro Glu  Phe Tyr Leu Arg Gly  Lys Ile Ser
    1760                 1765                 1770

Asp Lys  Gly Gly Phe Asn Trp  Glu Leu Arg Val Asn  Glu Ser Val
    1775                 1780                 1785

Val Asp  Asn Tyr Leu Ile Tyr  Gly Asp Leu His Ile  Asp Asn Thr
    1790                 1795                 1800

Arg Asp  Phe Asn Ile Lys Leu  Asn Val Lys Asp Gly  Asp Ile Met
    1805                 1810                 1815

Asp Trp  Gly Met Lys Asp Tyr  Lys Ala Asn Gly Phe  Pro Asp Lys
    1820                 1825                 1830
```

Val Thr Asp Met Asp Gly Asn Val Tyr Leu Gln Thr Gly Tyr Ser
1835 1840 1845

Asp Leu Asn Ala Lys Ala Val Gly Val His Tyr Gln Phe Leu Tyr
1850 1855 1860

Asp Asn Val Lys Pro Glu Val Asn Ile Asp Pro Lys Gly Asn Thr
1865 1870 1875

Ser Ile Glu Tyr Ala Asp Gly Lys Ser Val Val Phe Asn Ile Asn
1880 1885 1890

Asp Lys Arg Asn Asn Gly Phe Asp Gly Glu Ile Gln Glu Gln His
1895 1900 1905

Ile Tyr Ile Asn Gly Lys Glu Tyr Thr Ser Phe Asn Asp Ile Lys
1910 1915 1920

Gln Ile Ile Asp Lys Thr Leu Asn Ile Lys Ile Val Val Lys Asp
1925 1930 1935

Phe Ala Arg Asn Thr Thr Val Lys Glu Phe Ile Leu Asn Lys Asp
1940 1945 1950

Thr Gly Glu Val Ser Glu Leu Lys Pro His Arg Val Thr Val Thr
1955 1960 1965

Ile Gln Asn Gly Lys Glu Met Ser Ser Thr Ile Val Ser Glu Glu
1970 1975 1980

Asp Phe Ile Leu Pro Val Tyr Lys Gly Glu Leu Glu Lys Gly Tyr
1985 1990 1995

Gln Phe Asp Gly Trp Glu Ile Ser Gly Phe Glu Gly Lys Lys Asp
2000 2005 2010

Ala Gly Tyr Val Ile Asn Leu Ser Lys Asp Thr Phe Ile Lys Pro
2015 2020 2025

Val Phe Lys Lys Ile Glu Glu Lys Lys Glu Glu Glu Asn Lys Pro
2030 2035 2040

Thr Phe Asp Val Ser Lys Lys Lys Asp Asn Pro Gln Val Asn His
2045 2050 2055

Ser Gln Leu Asn Glu Ser His Arg Lys Glu Asp Leu Gln Arg Glu
2060 2065 2070

Glu His Ser Gln Lys Ser Asp Ser Thr Lys Asp Val Thr Ala Thr
2075 2080 2085

Val Leu Asp Lys Asn Asn Ile Ser Ser Lys Ser Thr Thr Asn Asn
2090 2095 2100

Pro Asn Lys Leu Pro Lys Thr Gly Thr Ala Ser Gly Ala Gln Thr
2105 2110 2115

Leu Leu Ala Ala Gly Ile Met Phe Ile Val Gly Ile Phe Leu Gly
2120 2125 2130

Leu Lys Lys Lys Asn Gln Asp
2135 2140

<210> 13
<211> 662
<212> PRT
<213> Streptococcus pneumoniae

<400> 13

Met Val Val Leu Ala Asp Thr Ser Ser Ser Glu Asp Ala Leu Asn Ile
1 5 10 15

Ser Asp Lys Glu Lys Val Ala Glu Asn Lys Glu Lys His Glu Asn Ile
20 25 30

His Ser Ala Met Glu Thr Ser Gln Asp Phe Lys Glu Lys Lys Thr Ala
35 40 45

Val Ile Lys Glu Lys Glu Val Val Ser Lys Asn Pro Val Ile Asp Asn
50 55 60

Asn Thr Ser Asn Glu Glu Ala Lys Ile Lys Glu Glu Asn Ser Asn Lys
65 70 75 80

Ser Gln Gly Asp Tyr Thr Asp Ser Phe Val Asn Lys Asn Thr Glu Asn
85 90 95

Pro Lys Lys Glu Asp Lys Val Val Tyr Ile Ala Glu Phe Lys Asp Lys
100 105 110

Glu Ser Gly Glu Lys Ala Ile Lys Glu Leu Ser Ser Leu Lys Asn Thr
115 120 125

Lys Val Leu Tyr Thr Tyr Asp Arg Ile Phe Asn Gly Ser Ala Ile Glu
130 135 140

Thr Thr Pro Asp Asn Leu Asp Lys Ile Lys Gln Ile Glu Gly Ile Ser
145 150 155 160

```
Ser Val Glu Arg Ala Gln Lys Val Gln Pro Met Met Asn His Ala Arg
                165                 170                 175

Lys Glu Ile Gly Val Glu Glu Ala Ile Asp Tyr Leu Lys Ser Ile Asn
            180                 185                 190

Ala Pro Phe Gly Lys Asn Phe Asp Gly Arg Gly Met Val Ile Ser Asn
        195                 200                 205

Ile Asp Thr Gly Thr Asp Tyr Arg His Lys Ala Met Arg Ile Asp Asp
    210                 215                 220

Asp Ala Lys Ala Ser Met Arg Phe Lys Lys Glu Asp Leu Lys Gly Thr
225                 230                 235                 240

Asp Lys Asn Tyr Trp Leu Ser Asp Lys Ile Pro His Ala Phe Asn Tyr
                245                 250                 255

Tyr Asn Gly Gly Lys Ile Thr Val Glu Lys Tyr Asp Asp Gly Arg Asp
            260                 265                 270

Tyr Phe Asp Pro His Gly Met His Ile Ala Gly Ile Leu Ala Gly Asn
        275                 280                 285

Asp Thr Glu Gln Asp Ile Lys Asn Phe Asn Gly Ile Asp Gly Ile Ala
    290                 295                 300

Pro Asn Ala Gln Ile Phe Ser Tyr Lys Met Tyr Ser Asp Ala Gly Ser
305                 310                 315                 320

Gly Phe Ala Gly Asp Glu Thr Met Phe His Ala Ile Glu Asp Ser Ile
                325                 330                 335

Lys His Asn Val Asp Val Val Ser Val Ser Ser Gly Phe Thr Gly Thr
            340                 345                 350

Gly Leu Val Gly Glu Lys Tyr Trp Gln Ala Ile Arg Ala Leu Arg Lys
        355                 360                 365

Ala Gly Ile Pro Met Val Val Ala Thr Gly Asn Tyr Ala Thr Ser Ala
    370                 375                 380

Ser Ser Ser Ser Trp Asp Leu Val Ala Asn Asn His Leu Lys Met Thr
385                 390                 395                 400

Asp Thr Gly Asn Val Thr Arg Thr Ala Ala His Glu Asp Ala Ile Ala
                405                 410                 415
```

Val Ala Ser Ala Lys Asn Gln Thr Val Glu Phe Asp Lys Val Asn Ile
420 425 430

Gly Gly Glu Ser Phe Lys Tyr Arg Asn Ile Gly Ala Phe Phe Asp Lys
435 440 445

Ser Lys Ile Thr Thr Asn Glu Asp Gly Thr Lys Ala Pro Ser Lys Leu
450 455 460

Lys Phe Val Tyr Ile Gly Lys Gly Gln Asp Gln Asp Leu Ile Gly Leu
465 470 475 480

Asp Leu Arg Gly Lys Ile Ala Val Met Asp Arg Ile Tyr Thr Lys Asp
485 490 495

Leu Lys Asn Ala Phe Lys Lys Ala Met Asp Lys Gly Ala Arg Ala Ile
500 505 510

Met Val Val Asn Thr Val Asn Tyr Tyr Asn Arg Asp Asn Trp Thr Glu
515 520 525

Leu Pro Ala Met Gly Tyr Glu Ala Asp Glu Gly Thr Lys Ser Gln Val
530 535 540

Phe Ser Ile Ser Gly Asp Asp Gly Val Lys Leu Trp Asn Met Ile Asn
545 550 555 560

Pro Asp Lys Lys Thr Glu Val Lys Arg Asn Asn Lys Glu Asp Phe Lys
565 570 575

Asp Lys Leu Glu Gln Tyr Tyr Pro Ile Asp Met Glu Ser Phe Asn Ser
580 585 590

Asn Lys Pro Asn Val Gly Asp Glu Lys Glu Ile Asp Phe Lys Phe Ala
595 600 605

Pro Asp Thr Asp Lys Glu Leu Tyr Lys Glu Asp Ile Ile Val Pro Ala
610 615 620

Gly Ser Thr Ser Trp Gly Pro Arg Ile Asp Leu Leu Leu Lys Pro Asp
625 630 635 640

Val Ser Ala Pro Gly Lys Asn Ile Lys Ser Thr Leu Asn Val Ile Asn
645 650 655

Gly Lys Ser Thr Tyr Gly
660

<210> 14
<211> 274
<212> PRT
<213> Artificial Sequence

<220>
<223> Description of Artificial Sequence: Synthetic polypeptide

<220>
<221> MOD_RES
<222> (1)..(1)
<223> Met or Ile

<220>
<221> MOD_RES
<222> (55)..(55)
<223> Asn or Tyr

<220>
<221> MOD_RES
<222> (63)..(63)
<223> His or Tyr

<220>
<221> MOD_RES
<222> (79)..(79)
<223> Arg or His

<220>
<221> MOD_RES
<222> (97)..(97)
<223> Ala or Glu

<220>
<221> MOD_RES
<222> (108)..(108)
<223> Gly or Glu

<220>
<221> MOD_RES
<222> (164)..(164)
<223> Asp or Glu

<220>
<221> MOD_RES
<222> (165)..(165)
<223> Gln or Lys

<220>
<221> MOD_RES
<222> (187)..(187)
<223> Phe or Ile

<220>
<221> MOD_RES
<222> (197)..(197)
<223> Arg or His

<220>
<221> MOD_RES
<222> (238)..(238)
<223> Gly or Arg

<400> 14

Xaa Asn Gln Ser Tyr Phe Tyr Leu Lys Met Lys Glu His Lys Leu Lys
1 5 10 15

Val Pro Tyr Thr Gly Lys Glu Arg Arg Val Arg Ile Leu Leu Pro Lys
20 25 30

Asp Tyr Glu Lys Asp Thr Asp Arg Ser Tyr Pro Val Val Tyr Phe His
35 40 45

Asp Gly Gln Asn Val Phe Xaa Ser Lys Glu Ser Phe Ile Gly Xaa Ser
50 55 60

Trp Lys Ile Ile Pro Ala Ile Lys Arg Asn Pro Asp Ile Ser Xaa Met
65 70 75 80

Ile Val Val Ala Ile Asp Asn Asp Gly Met Gly Arg Met Asn Glu Tyr
85 90 95

Xaa Ala Trp Lys Phe Gln Glu Ser Pro Ile Pro Xaa Gln Gln Phe Gly
100 105 110

Gly Lys Gly Val Glu Tyr Ala Glu Phe Val Met Glu Val Val Lys Pro
115 120 125

Phe Ile Asp Glu Thr Tyr Arg Thr Lys Ala Asp Cys Gln His Thr Ala
130 135 140

Met Ile Gly Ser Ser Leu Gly Gly Asn Ile Thr Gln Phe Ile Gly Leu
145 150 155 160

Glu Tyr Gln Xaa Xaa Ile Gly Cys Leu Gly Val Phe Ser Ser Ala Asn
165 170 175

Trp Leu His Gln Glu Ala Phe Asn Arg Tyr Xaa Glu Cys Gln Lys Leu
180 185 190

Ser Pro Asp Gln Xaa Ile Phe Ile Tyr Val Gly Thr Glu Glu Ala Asp
195 200 205

Asp Thr Asp Lys Thr Leu Met Asp Gly Asn Ile Lys Gln Ala Tyr Ile
210 215 220

Asp Ser Ser Leu Cys Tyr Tyr His Asp Leu Ile Ala Gly Xaa Val His
225 230 235 240

Leu Asp Asn Leu Val Leu Lys Val Gln Ser Gly Ala Ile His Ser Glu
245 250 255

Ile Pro Trp Ser Glu Asn Leu Pro Asp Cys Leu Arg Phe Phe Ala Glu

260 265 270

Lys Trp

<210> 15
<211> 130
<212> PRT
<213> Artificial Sequence

<220>
<223> Description of Artificial Sequence: Synthetic polypeptide

<220>
<221> MOD_RES
<222> (1)..(1)
<223> Met or Ile

<220>
<221> MOD_RES
<222> (55)..(55)
<223> Asn or Tyr

<220>
<221> MOD_RES
<222> (63)..(63)
<223> His or Tyr

<220>
<221> MOD_RES
<222> (79)..(79)
<223> Arg or His

<220>
<221> MOD_RES
<222> (97)..(97)
<223> Ala or Glu

<220>
<221> MOD_RES
<222> (108)..(108)
<223> Gly or Glu

<400> 15

```
Xaa Asn Gln Ser Tyr Phe Tyr Leu Lys Met Lys Glu His Lys Leu Lys
1               5                   10                  15

Val Pro Tyr Thr Gly Lys Glu Arg Arg Val Arg Ile Leu Leu Pro Lys
            20                  25                  30

Asp Tyr Glu Lys Asp Thr Asp Arg Ser Tyr Pro Val Val Tyr Phe His
        35                  40                  45

Asp Gly Gln Asn Val Phe Xaa Ser Lys Glu Ser Phe Ile Gly Xaa Ser
    50                  55                  60

Trp Lys Ile Ile Pro Ala Ile Lys Arg Asn Pro Asp Ile Ser Xaa Met

65                  70                  75                  80

Ile Val Val Ala Ile Asp Asn Asp Gly Met Gly Arg Met Asn Glu Tyr
                85                  90                  95

Xaa Ala Trp Lys Phe Gln Glu Ser Pro Ile Pro Xaa Gln Gln Phe Gly
            100                 105                 110

Gly Lys Gly Val Glu Tyr Ala Glu Phe Val Met Glu Val Val Lys Pro
        115                 120                 125

Phe Ile
    130
```

<210> 16
<211> 299
<212> PRT
<213> Artificial Sequence

<220>
<223> Description of Artificial Sequence: Synthetic polypeptide

<220>
<221> MOD_RES
<222> (6)..(6)
<223> Met or Thr

<220>
<221> MOD_RES
<222> (9)..(9)
<223> Ala or Thr

<220>
<221> MOD_RES
<222> (15)..(15)
<223> Ala or Val

<220>
<221> MOD_RES
<222> (26)..(26)
<223> Met or Ile

<220>
<221> MOD_RES
<222> (80)..(80)
<223> Asn or Tyr

<220>
<221> MOD_RES
<222> (88)..(88)
<223> His or Tyr

<220>
<221> MOD_RES
<222> (104)..(104)
<223> Arg or His

<220>
<221> MOD_RES
<222> (122)..(122)
<223> Ala or Glu

<220>
<221> MOD_RES
<222> (133)..(133)
<223> Gly or Glu

<220>
<221> MOD_RES
<222> (189)..(189)
<223> Asp or Glu

<220>
<221> MOD_RES
<222> (190)..(190)
<223> Gln or Lys

<220>
<221> MOD_RES
<222> (212)..(212)
<223> Phe or Ile

<220>
<221> MOD_RES
<222> (222)..(222)
<223> Arg or His

<220>
<221> MOD_RES
<222> (263)..(263)
<223> Arg or His

<400> 16

```
Met Ser Ser Lys Phe Xaa Lys Ser Xaa Ala Val Leu Gly Thr Xaa Thr
1               5                   10                  15

Leu Ala Ser Leu Leu Leu Val Ala Cys Xaa Asn Gln Ser Tyr Phe Tyr
            20                  25                  30

Leu Lys Met Lys Glu His Lys Leu Lys Val Pro Tyr Thr Gly Lys Glu
        35                  40                  45

Arg Arg Val Arg Ile Leu Leu Pro Lys Asp Tyr Glu Lys Asp Thr Asp
    50                  55                  60

Arg Ser Tyr Pro Val Val Tyr Phe His Asp Gly Gln Asn Val Phe Xaa
65                  70                  75                  80

Ser Lys Glu Ser Phe Ile Gly Xaa Ser Trp Lys Ile Ile Pro Ala Ile
                85                  90                  95

Lys Arg Asn Pro Asp Ile Ser Xaa Met Ile Val Val Ala Ile Asp Asn
            100                 105                 110

Asp Gly Met Gly Arg Met Asn Glu Tyr Xaa Ala Trp Lys Phe Gln Glu
        115                 120                 125
```

Ser Pro Ile Pro Xaa Gln Gln Phe Gly Gly Lys Gly Val Glu Tyr Ala
130 135 140

Glu Phe Val Met Glu Val Val Lys Pro Phe Ile Asp Glu Thr Tyr Arg
145 150 155 160

Thr Lys Ala Asp Cys Gln His Thr Ala Met Ile Gly Ser Ser Leu Gly
165 170 175

Gly Asn Ile Thr Gln Phe Ile Gly Leu Glu Tyr Gln Xaa Xaa Ile Gly
180 185 190

Cys Leu Gly Val Phe Ser Ser Ala Asn Trp Leu His Gln Glu Ala Phe
195 200 205

Asn Arg Tyr Xaa Glu Cys Gln Lys Leu Ser Pro Asp Gln Xaa Ile Phe
210 215 220

Ile Tyr Val Gly Thr Glu Glu Ala Asp Asp Thr Asp Lys Thr Leu Met
225 230 235 240

Asp Gly Asn Ile Lys Gln Ala Tyr Ile Asp Ser Ser Leu Cys Tyr Tyr
245 250 255

His Asp Leu Ile Ala Gly Xaa Val His Leu Asp Asn Leu Val Leu Lys
260 265 270

Val Gln Ser Gly Ala Ile His Ser Glu Ile Pro Trp Ser Glu Asn Leu
275 280 285

Pro Asp Cys Leu Arg Phe Phe Ala Glu Lys Trp
290 295

<210> 17
<211> 155
<212> PRT
<213> Artificial Sequence

<220>
<223> Description of Artificial Sequence: Synthetic polypeptide

<220>
<221> MOD_RES
<222> (6)..(6)
<223> Met or Thr

<220>
<221> MOD_RES
<222> (9)..(9)
<223> Ala or Thr

<220>
<221> MOD_RES
<222> (15)..(15)
<223> Ala or Val

<220>
<221> MOD_RES
<222> (26)..(26)
<223> Met or Ile

<220>
<221> MOD_RES
<222> (80)..(80)
<223> Asn or Tyr

<220>
<221> MOD_RES
<222> (88)..(88)
<223> His or Tyr

<220>
<221> MOD_RES
<222> (104)..(104)
<223> Arg or His

<220>
<221> MOD_RES
<222> (122)..(122)
<223> Ala or Glu

<220>
<221> MOD_RES
<222> (133)..(133)
<223> Gly or Glu

<400> 17

Met Ser Ser Lys Phe Xaa Lys Ser Xaa Ala Val Leu Gly Thr Xaa Thr
1 5 10 15

Leu Ala Ser Leu Leu Leu Val Ala Cys Xaa Asn Gln Ser Tyr Phe Tyr
20 25 30

Leu Lys Met Lys Glu His Lys Leu Lys Val Pro Tyr Thr Gly Lys Glu
35 40 45

Arg Arg Val Arg Ile Leu Leu Pro Lys Asp Tyr Glu Lys Asp Thr Asp
50 55 60

Arg Ser Tyr Pro Val Val Tyr Phe His Asp Gly Gln Asn Val Phe Xaa
65 70 75 80

Ser Lys Glu Ser Phe Ile Gly Xaa Ser Trp Lys Ile Ile Pro Ala Ile
85 90 95

Lys Arg Asn Pro Asp Ile Ser Xaa Met Ile Val Val Ala Ile Asp Asn
100 105 110

Asp Gly Met Gly Arg Met Asn Glu Tyr Xaa Ala Trp Lys Phe Gln Glu
115 120 125

Ser Pro Ile Pro Xaa Gln Gln Phe Gly Gly Lys Gly Val Glu Tyr Ala
130 135 140

Glu Phe Val Met Glu Val Val Lys Pro Phe Ile
145 150 155

<210> 18
<211> 254
<212> PRT
<213> Artificial Sequence

<220>
<223> Description of Artificial Sequence: Synthetic polypeptide

<220>
<221> MOD_RES
<222> (11)..(11)
<223> Gly or Glu

<220>
<221> MOD_RES
<222> (24)..(24)
<223> Gly or Ser

<220>
<221> MOD_RES
<222> (27)..(27)

<223> Lys or Arg

<220>
<221> MOD_RES
<222> (30)..(30)
<223> Ile or Asn

<220>
<221> MOD_RES
<222> (56)..(56)
<223> Asp or Asn

<220>
<221> MOD_RES
<222> (58)..(58)
<223> Any amino acid

<220>
<221> MOD_RES
<222> (83)..(83)
<223> Leu or Ile

<220>
<221> MOD_RES
<222> (111)..(111)
<223> Asp or Glu

<220>
<221> MOD_RES
<222> (159)..(159)
<223> Leu or Phe

<220>
<221> MOD_RES
<222> (192)..(192)
<223> Asn or Tyr

<220>
<221> MOD_RES
<222> (199)..(199)
<223> Pro or Ser

<400> 18

```
Met Cys Ser Gly Gly Ala Lys Lys Glu Gly Xaa Ala Ala Ser Lys Lys
1               5                   10                  15

Glu Ile Ile Val Ala Thr Asn Xaa Ser Pro Xaa Pro Phe Xaa Tyr Glu
            20                  25                  30

Glu Asn Gly Glu Leu Thr Gly Tyr Glu Ile Glu Val Val Arg Ala Ile
        35                  40                  45

Phe Lys Asp Ser Asp Lys Tyr Xaa Val Xaa Phe Glu Lys Thr Glu Trp
    50                  55                  60

Ser Gly Val Phe Ala Gly Leu Asp Ala Asp Arg Tyr Asn Met Ala Val
65                  70                  75                  80

Asn Asn Xaa Ser Tyr Thr Lys Glu Arg Ala Glu Lys Tyr Leu Tyr Ala
                85                  90                  95

Ala Pro Ile Ala Gln Asn Pro Asn Val Leu Val Val Lys Lys Xaa Asp
            100                 105                 110

Ser Ser Ile Lys Ser Leu Asp Asp Ile Gly Gly Lys Ser Thr Glu Val
        115                 120                 125

Val Gln Ala Thr Thr Ser Ala Lys Gln Leu Glu Ala Tyr Asn Ala Glu
    130                 135                 140

His Thr Asp Asn Pro Thr Ile Leu Asn Tyr Thr Lys Ala Asp Xaa Gln
145                 150                 155                 160

Gln Ile Met Val Arg Leu Ser Asp Gly Gln Phe Asp Tyr Lys Ile Phe
                165                 170                 175

Asp Lys Ile Gly Val Glu Thr Val Ile Lys Asn Gln Gly Leu Asp Xaa
            180                 185                 190

Leu Lys Val Ile Glu Leu Xaa Ser Asp Gln Gln Pro Tyr Val Tyr Pro
        195                 200                 205

Leu Leu Ala Gln Gly Gln Asp Glu Leu Lys Ser Phe Val Asp Lys Arg
    210                 215                 220

Ile Lys Glu Leu Tyr Lys Asp Gly Thr Leu Glu Lys Leu Ser Lys Gln
225                 230                 235                 240

Phe Phe Gly Asp Thr Tyr Leu Pro Ala Glu Ala Asp Ile Lys
                245                 250
```

<210> 19
<211> 276
<212> PRT
<213> Artificial Sequence

<220>
<223> Description of Artificial Sequence: Synthetic polypeptide

<220>
<221> MOD_RES
<222> (14)..(14)
<223> Ala or Gly

<220>
<221> MOD_RES
<222> (20).. (20)
<223> Val or Leu

<220>
<221> MOD_RES
<222> (33)..(33)
<223> Glu or Gln

<220>
<221> MOD_RES
<222> (46)..(46)
<223> Gly or Ser

<220>
<221> MOD_RES
<222> (49)..(49)
<223> Lys or Arg

<220>
<221> MOD_RES
<222> (52)..(52)
<223> Ile or Asn

<220>
<221> MOD_RES
<222> (78)..(78)
<223> Asp or Asn

<220>
<221> MOD_RES
<222> (80)..(80)
<223> Any amino acid

<220>
<221> MOD_RES
<222> (105)..(105)
<223> Leu or Ile

<220>
<221> MOD_RES
<222> (133)..(133)
<223> Asp or Glu

<220>
<221> MOD_RES
<222> (181)..(181)
<223> Asp or Glu

<220>
<221> MOD_RES
<222> (214)..(214)
<223> Asn or Tyr

<220>
<221> MOD_RES
<222> (221)..(221)
<223> Pro or Ser

<400> 19

```
Met Lys Lys Ile Val Lys Tyr Ser Ser Leu Ala Ala Leu Xaa Leu Val
1               5                   10                  15

Ala Ala Gly Xaa Leu Ala Ala Cys Ser Gly Gly Ala Lys Lys Glu Gly
            20                  25                  30

Xaa Ala Ala Ser Lys Lys Glu Ile Ile Val Ala Thr Asn Xaa Ser Pro
        35                  40                  45

Xaa Pro Phe Xaa Tyr Glu Glu Asn Gly Glu Leu Thr Gly Tyr Glu Ile
    50                  55                  60

Glu Val Val Arg Ala Ile Phe Lys Asp Ser Asp Lys Tyr Xaa Val Xaa
65                  70                  75                  80

Phe Glu Lys Thr Glu Trp Ser Gly Val Phe Ala Gly Leu Asp Ala Asp
                85                  90                  95

Arg Tyr Asn Met Ala Val Asn Asn Xaa Ser Tyr Thr Lys Glu Arg Ala
            100                 105                 110

Glu Lys Tyr Leu Tyr Ala Ala Pro Ile Ala Gln Asn Pro Asn Val Leu
        115                 120                 125

Val Val Lys Lys Xaa Asp Ser Ser Ile Lys Ser Leu Asp Asp Ile Gly
    130                 135                 140

Gly Lys Ser Thr Glu Val Val Gln Ala Thr Thr Ser Ala Lys Gln Leu
145                 150                 155                 160

Glu Ala Tyr Asn Ala Glu His Thr Asp Asn Pro Thr Ile Leu Asn Tyr
                165                 170                 175
```

Thr Lys Ala Asp Xaa Gln Gln Ile Met Val Arg Leu Ser Asp Gly Gln
180 185 190

Phe Asp Tyr Lys Ile Phe Asp Lys Ile Gly Val Glu Thr Val Ile Lys
195 200 205

Asn Gln Gly Leu Asp Xaa Leu Lys Val Ile Glu Leu Xaa Ser Asp Gln
210 215 220

Gln Pro Tyr Val Tyr Pro Leu Leu Ala Gln Gly Gln Asp Glu Leu Lys
225 230 235 240

Ser Phe Val Asp Lys Arg Ile Lys Glu Leu Tyr Lys Asp Gly Thr Leu
245 250 255

Glu Lys Leu Ser Lys Gln Phe Phe Gly Asp Thr Tyr Leu Pro Ala Glu
260 265 270

Ala Asp Ile Lys
275

<210> 20
<211> 400
<212> PRT
<213> Artificial Sequence

<220>
<223> Description of Artificial Sequence: Synthetic polypeptide

<220>
<221> MOD_RES
<222> (18)..(18)
<223> Val or Ala

<220>
<221> MOD_RES
<222> (48)..(48)
<223> Val or Ile

<220>
<221> MOD_RES
<222> (74)..(74)
<223> Val or Ile

<220>
<221> MOD_RES
<222> (83)..(83)
<223> Any amino acid

<220>
<221> MOD_RES
<222> (100)..(100)
<223> Ala or Thr

<220>
<221> MOD_RES
<222> (177)..(177)
<223> Thr or Ala

<220>
<221> MOD_RES
<222> (186)..(186)
<223> Ala or Gly

<220>
<221> MOD_RES
<222> (196)..(196)
<223> Ala or Pro

<220>
<221> MOD_RES
<222> (208)..(208)
<223> Val or Ala

<220>
<221> MOD_RES
<222> (214)..(214)
<223> Any amino acid

<220>
<221> MOD_RES
<222> (237)..(237)
<223> Gln or His

<220>
<221> MOD_RES
<222> (298)..(298)
<223> Ser or Ala

<220>
<221> MOD_RES
<222> (307)..(307)
<223> Ala or Ser

<220>
<221> MOD_RES
<222> (313)..(313)
<223> Val or Ala

<220>
<221> MOD_RES
<222> (348)..(348)
<223> Asn or Ser

<400> 20

```
Met Cys Gly Ser Lys Thr Ala Asp Lys Pro Ala Asp Ser Gly Ser Ser
1               5                   10                  15

Glu Xaa Lys Glu Leu Thr Val Tyr Val Asp Glu Gly Tyr Lys Ser Tyr
            20                  25                  30

Ile Glu Glu Val Ala Lys Ala Tyr Glu Lys Glu Ala Gly Val Lys Xaa
        35                  40                  45

Thr Leu Lys Thr Gly Asp Ala Leu Gly Gly Leu Asp Lys Leu Ser Leu
    50                  55                  60
```

Asp Asn Gln Ser Gly Asn Val Pro Asp Xaa Met Met Ala Pro Tyr Asp
65 70 75 80

Arg Val Xaa Ser Leu Gly Ser Asp Gly Gln Leu Ser Glu Val Lys Leu
85 90 95

Ser Asp Gly Xaa Lys Thr Asp Asp Thr Thr Lys Ser Leu Val Thr Ala
100 105 110

Ala Asn Gly Lys Val Tyr Gly Ala Pro Ala Val Ile Glu Ser Leu Val
115 120 125

Met Tyr Tyr Asn Lys Asp Leu Val Lys Asp Ala Pro Lys Thr Phe Ala
130 135 140

Asp Leu Glu Asn Leu Ala Lys Asp Ser Lys Tyr Ala Phe Ala Gly Glu
145 150 155 160

Asp Gly Lys Thr Thr Ala Phe Leu Ala Asp Trp Thr Asn Phe Tyr Tyr
165 170 175

Xaa Tyr Gly Leu Leu Ala Gly Asn Gly Xaa Tyr Val Phe Gly Gln Asn
180 185 190

Gly Lys Asp Xaa Lys Asp Ile Gly Leu Ala Asn Asp Gly Ser Ile Xaa
195 200 205

Gly Ile Asn Tyr Ala Xaa Ser Trp Tyr Glu Lys Trp Pro Lys Gly Met
210 215 220

Gln Asp Thr Glu Gly Ala Gly Asn Leu Ile Gln Thr Xaa Phe Gln Glu
225 230 235 240

Gly Lys Thr Ala Ala Ile Ile Asp Gly Pro Trp Lys Ala Gln Ala Phe
245 250 255

Lys Asp Ala Lys Val Asn Tyr Gly Val Ala Thr Ile Pro Thr Leu Pro
260 265 270

Asn Gly Lys Glu Tyr Ala Ala Phe Gly Gly Gly Lys Ala Trp Val Ile
275 280 285

Pro Gln Ala Val Lys Asn Leu Glu Ala Xaa Gln Lys Phe Val Asp Phe
290 295 300

Leu Val Xaa Thr Glu Gln Gln Lys Xaa Leu Tyr Asp Lys Thr Asn Glu
305 310 315 320

Ile Pro Ala Asn Thr Glu Ala Arg Ser Tyr Ala Glu Gly Lys Asn Asp
325 330 335

Glu Leu Thr Thr Ala Val Ile Lys Gln Phe Lys Xaa Thr Gln Pro Leu
340 345 350

Pro Asn Ile Ser Gln Met Ser Ala Val Trp Asp Pro Ala Lys Asn Met
355 360 365

Leu Phe Asp Ala Val Ser Gly Gln Lys Asp Ala Lys Thr Ala Ala Asn
370 375 380

Asp Ala Val Thr Leu Ile Lys Glu Thr Ile Lys Gln Lys Phe Gly Glu
385 390 395 400

<210> 21
<211> 423
<212> PRT
<213> Artificial Sequence

<220>
<223> Description of Artificial Sequence: Synthetic polypeptide

<220>
<221> MOD_RES
<222> (6)..(6)
<223> Met or Thr

<220>
<221> MOD_RES
<222> (9)..(9)
<223> Ala or Thr

<220>
<221> MOD_RES
<222> (15)..(15)
<223> Ala or Val

<220>
<221> MOD_RES
<222> (41)..(41)
<223> Val or Ala

<220>
<221> MOD_RES
<222> (71)..(71)
<223> Val or Ile

<220>
<221> MOD_RES
<222> (97)..(97)
<223> Val or Ile

<220>
<221> MOD_RES

<222> (106)..(106)
<223> Any amino acid

<220>
<221> MOD_RES
<222> (123)..(123)
<223> Ala or Thr

<220>
<221> MOD_RES
<222> (200)..(200)
<223> Thr or Ala

<220>
<221> MOD_RES
<222> (209)..(209)
<223> Ala or Gly

<220>
<221> MOD_RES
<222> (219)..(219)
<223> Ala or Pro

<220>
<221> MOD_RES
<222> (231)..(231)
<223> Val or Ala

<220>
<221> MOD_RES
<222> (237)..(237)
<223> Any amino acid

<220>
<221> MOD_RES
<222> (260)..(260)
<223> Gln or His

<220>
<221> MOD_RES
<222> (321)..(321)
<223> Ser or Ala

<220>
<221> MOD_RES
<222> (330)..(330)
<223> Ala or Ser

<220>
<221> MOD_RES
<222> (336)..(336)
<223> Val or Ala

<220>
<221> MOD_RES
<222> (371)..(371)
<223> Asn or Ser

<400> 21

```
Met Ser Ser Lys Phe Xaa Lys Ser Xaa Ala Val Leu Gly Thr Xaa Thr
1               5                   10                  15

Leu Ala Ser Leu Leu Leu Val Ala Cys Gly Ser Lys Thr Ala Asp Lys
            20                  25                  30

Pro Ala Asp Ser Gly Ser Ser Glu Xaa Lys Glu Leu Thr Val Tyr Val
```

35 40 45

Asp Glu Gly Tyr Lys Ser Tyr Ile Glu Glu Val Ala Lys Ala Tyr Glu
50 55 60

Lys Glu Ala Gly Val Lys Xaa Thr Leu Lys Thr Gly Asp Ala Leu Gly
65 70 75 80

Gly Leu Asp Lys Leu Ser Leu Asp Asn Gln Ser Gly Asn Val Pro Asp
85 90 95

Xaa Met Met Ala Pro Tyr Asp Arg Val Xaa Ser Leu Gly Ser Asp Gly
100 105 110

Gln Leu Ser Glu Val Lys Leu Ser Asp Gly Xaa Lys Thr Asp Asp Thr
115 120 125

Thr Lys Ser Leu Val Thr Ala Ala Asn Gly Lys Val Tyr Gly Ala Pro
130 135 140

Ala Val Ile Glu Ser Leu Val Met Tyr Tyr Asn Lys Asp Leu Val Lys
145 150 155 160

Asp Ala Pro Lys Thr Phe Ala Asp Leu Glu Asn Leu Ala Lys Asp Ser
165 170 175

Lys Tyr Ala Phe Ala Gly Glu Asp Gly Lys Thr Thr Ala Phe Leu Ala
180 185 190

Asp Trp Thr Asn Phe Tyr Tyr Xaa Tyr Gly Leu Leu Ala Gly Asn Gly
195 200 205

Xaa Tyr Val Phe Gly Gln Asn Gly Lys Asp Xaa Lys Asp Ile Gly Leu
210 215 220

Ala Asn Asp Gly Ser Ile Xaa Gly Ile Asn Tyr Ala Xaa Ser Trp Tyr
225 230 235 240

Glu Lys Trp Pro Lys Gly Met Gln Asp Thr Glu Gly Ala Gly Asn Leu
245 250 255

Ile Gln Thr Xaa Phe Gln Glu Gly Lys Thr Ala Ala Ile Ile Asp Gly
260 265 270

Pro Trp Lys Ala Gln Ala Phe Lys Asp Ala Lys Val Asn Tyr Gly Val
275 280 285

Ala Thr Ile Pro Thr Leu Pro Asn Gly Lys Glu Tyr Ala Ala Phe Gly
290 295 300

```
Gly Gly Lys Ala Trp Val Ile Pro Gln Ala Val Lys Asn Leu Glu Ala
305                 310                 315                 320

Xaa Gln Lys Phe Val Asp Phe Leu Val Xaa Thr Glu Gln Gln Lys Xaa
                325                 330                 335

Leu Tyr Asp Lys Thr Asn Glu Ile Pro Ala Asn Thr Glu Ala Arg Ser
            340                 345                 350

Tyr Ala Glu Gly Lys Asn Asp Glu Leu Thr Thr Ala Val Ile Lys Gln
        355                 360                 365

Phe Lys Xaa Thr Gln Pro Leu Pro Asn Ile Ser Gln Met Ser Ala Val
    370                 375                 380

Trp Asp Pro Ala Lys Asn Met Leu Phe Asp Ala Val Ser Gly Gln Lys
385                 390                 395                 400

Asp Ala Lys Thr Ala Ala Asn Asp Ala Val Thr Leu Ile Lys Glu Thr
                405                 410                 415

Ile Lys Gln Lys Phe Gly Glu
            420
```

<210> 22
<211> 357
<212> PRT
<213> Streptococcus pneumoniae

<400> 22

```
Met Ala Asn Ile Phe Asp Tyr Leu Lys Asp Val Ala Tyr Asp Ser Tyr
1               5                   10                  15

Tyr Asp Leu Pro Leu Asn Glu Leu Asp Ile Leu Thr Leu Ile Glu Ile
            20                  25                  30

Thr Tyr Leu Ser Phe Asp Asn Leu Val Ser Thr Leu Pro Gln Arg Leu
        35                  40                  45

Leu Asp Leu Ala Pro Gln Val Pro Arg Asp Pro Thr Met Leu Thr Ser
    50                  55                  60

Lys Asn Arg Leu Gln Leu Leu Asp Glu Leu Ala Gln His Lys Arg Phe
65                  70                  75                  80

Lys Asn Cys Lys Leu Ser His Phe Ile Asn Asp Ile Asp Pro Glu Leu
                85                  90                  95

Gln Lys Gln Phe Ala Ala Met Thr Tyr Arg Val Ser Leu Asp Thr Tyr
```

100 105 110

Leu Ile Val Phe Arg Gly Thr Asp Asp Ser Ile Ile Gly Trp Lys Glu
115 120 125

Asp Phe His Leu Thr Tyr Met Lys Glu Ile Pro Ala Gln Lys His Ala
130 135 140

Leu Arg Tyr Leu Lys Asn Phe Phe Ala His His Pro Lys Gln Lys Val
145 150 155 160

Ile Leu Ala Gly His Ser Lys Gly Gly Asn Leu Ala Ile Tyr Ala Ala
165 170 175

Ser Gln Ile Glu Gln Ser Leu Gln Asn Gln Ile Thr Ala Val Tyr Thr
180 185 190

Phe Asp Ala Pro Gly Leu His Gln Glu Leu Thr Gln Thr Ala Gly Tyr
195 200 205

Gln Arg Ile Met Asp Arg Ser Lys Ile Phe Ile Pro Gln Gly Ser Ile
210 215 220

Ile Gly Met Met Leu Glu Ile Pro Ala His Gln Ile Ile Val Gln Ser
225 230 235 240

Thr Ala Leu Gly Gly Ile Ala Gln His Asp Thr Phe Ser Trp Gln Ile
245 250 255

Glu Asp Lys His Phe Val Gln Leu Asp Lys Thr Asn Ser Asp Ser Gln
260 265 270

Gln Val Asp Thr Thr Phe Lys Glu Trp Val Ala Thr Val Pro Asp Glu
275 280 285

Glu Leu Gln Leu Tyr Phe Asp Leu Phe Phe Gly Thr Ile Leu Asp Ala
290 295 300

Gly Ile Ser Ser Ile Asn Asp Leu Ala Ser Leu Lys Ala Leu Glu Tyr
305 310 315 320

Ile His His Leu Phe Val Gln Ala Gln Ser Leu Thr Pro Glu Glu Arg
325 330 335

Glu Thr Leu Gly Arg Leu Thr Gln Leu Leu Ile Asp Thr Arg Tyr Gln
340 345 350

Ala Trp Lys Asn Arg
355

<210> 23
<211> 1066
<212> PRT
<213> Streptococcus pneumoniae

<400> 23

Met Gln Thr Lys Thr Lys Lys Leu Ile Val Ser Leu Ser Ser Leu Val
1               5                   10                  15

Leu Ser Gly Phe Leu Leu Asn His Tyr Met Thr Ile Gly Ala Glu Glu
            20                  25                  30

Thr Thr Thr Asn Thr Ile Gln Gln Ser Gln Lys Glu Val Gln Tyr Gln
        35                  40                  45

Gln Arg Asp Thr Lys Asn Leu Val Glu Asn Gly Asp Phe Gly Gln Thr
    50                  55                  60

Glu Asp Gly Ser Ser Pro Trp Thr Gly Ser Lys Ala Gln Gly Trp Ser
65                  70                  75                  80

Ala Trp Val Asp Gln Lys Asn Ser Ala Asp Ala Ser Thr Arg Val Ile
                85                  90                  95

Glu Ala Lys Asp Gly Ala Ile Thr Ile Ser Ser His Glu Lys Leu Arg
            100                 105                 110

Ala Ala Leu His Arg Met Val Pro Ile Glu Ala Lys Lys Lys Tyr Lys
        115                 120                 125

Leu Arg Phe Lys Ile Lys Thr Asp Asn Lys Ile Gly Ile Ala Lys Val
    130                 135                 140

Arg Ile Ile Glu Glu Ser Gly Lys Asp Lys Arg Leu Trp Asn Ser Ala
145                 150                 155                 160

Thr Thr Ser Gly Thr Lys Asp Trp Gln Thr Ile Glu Ala Asp Tyr Ser
                165                 170                 175

Pro Thr Leu Asp Val Asp Lys Ile Lys Leu Glu Leu Phe Tyr Glu Thr
            180                 185                 190

Gly Thr Gly Thr Val Ser Phe Lys Asp Ile Glu Leu Val Glu Val Ala
        195                 200                 205

Asp Gln Leu Ser Glu Asp Ser Gln Thr Asp Lys Gln Leu Glu Glu Lys
    210                 215                 220

Ile Asp Leu Pro Ile Gly Lys Lys His Val Phe Ser Leu Ala Asp Tyr

225 230 235 240

Thr Tyr Lys Val Glu Asn Pro Asp Val Ala Ser Val Lys Asn Gly Ile
245 250 255

Leu Glu Pro Leu Lys Glu Gly Thr Thr Asn Val Ile Val Ser Lys Asp
260 265 270

Gly Lys Glu Val Lys Lys Ile Pro Leu Lys Ile Leu Ala Ser Val Lys
275 280 285

Asp Ala Tyr Thr Asp Arg Leu Asp Asp Trp Asn Gly Ile Ile Ala Gly
290 295 300

Asn Gln Tyr Tyr Asp Ser Lys Asn Glu Gln Met Ala Lys Leu Asn Gln
305 310 315 320

Glu Leu Glu Gly Lys Val Ala Asp Ser Leu Ser Ser Ile Ser Ser Gln
325 330 335

Ala Asp Arg Thr Tyr Leu Trp Glu Lys Phe Ser Asn Tyr Lys Thr Ser
340 345 350

Ala Asn Leu Thr Ala Thr Tyr Arg Lys Leu Glu Glu Met Ala Lys Gln
355 360 365

Val Thr Asn Pro Ser Ser Arg Tyr Tyr Gln Asp Glu Thr Val Val Arg
370 375 380

Thr Val Arg Asp Ser Met Glu Trp Met His Lys His Val Tyr Asn Ser
385 390 395 400

Glu Lys Ser Ile Val Gly Asn Trp Trp Asp Tyr Glu Ile Gly Thr Pro
405 410 415

Arg Ala Ile Asn Asn Thr Leu Ser Leu Met Lys Glu Tyr Phe Ser Asp
420 425 430

Glu Glu Ile Lys Lys Tyr Thr Asp Val Ile Glu Lys Phe Val Pro Asp
435 440 445

Pro Glu His Phe Arg Lys Thr Thr Asp Asn Pro Phe Lys Ala Leu Gly
450 455 460

Gly Asn Leu Val Asp Met Gly Arg Val Lys Val Ile Ala Gly Leu Leu
465 470 475 480

Arg Lys Asp Asp Gln Glu Ile Ser Ser Thr Ile Arg Ser Ile Glu Gln
485 490 495

```
Val Phe Lys Leu Val Asp Gln Gly Glu Gly Phe Tyr Gln Asp Gly Ser
            500                 505                 510

Tyr Ile Asp His Thr Asn Val Ala Tyr Thr Gly Ala Tyr Gly Asn Val
        515                 520                 525

Leu Ile Asp Gly Leu Ser Gln Leu Leu Pro Val Ile Gln Lys Thr Lys
    530                 535                 540

Asn Pro Ile Asp Lys Asp Lys Met Gln Thr Met Tyr His Trp Ile Asp
545                 550                 555                 560

Lys Ser Phe Ala Pro Leu Leu Val Asn Gly Glu Leu Met Asp Met Ser
                565                 570                 575

Arg Gly Arg Ser Ile Ser Arg Ala Asn Ser Glu Gly His Val Ala Ala
            580                 585                 590

Val Glu Val Leu Arg Gly Ile His Arg Ile Ala Asp Met Ser Glu Gly
        595                 600                 605

Glu Thr Lys Gln Cys Leu Gln Ser Leu Val Lys Thr Ile Val Gln Ser
    610                 615                 620

Asp Ser Tyr Tyr Asp Val Phe Lys Asn Leu Lys Thr Tyr Lys Asp Ile
625                 630                 635                 640

Ser Leu Met Gln Ser Leu Leu Ser Asp Ala Gly Val Ala Ser Val Pro
                645                 650                 655

Arg Pro Ser Tyr Leu Ser Ala Phe Asn Lys Met Asp Lys Thr Ala Met
            660                 665                 670

Tyr Asn Ala Glu Lys Gly Phe Gly Phe Gly Leu Ser Leu Phe Ser Ser
        675                 680                 685

Arg Thr Leu Asn Tyr Glu His Met Asn Lys Glu Asn Lys Arg Gly Trp
    690                 695                 700

Tyr Thr Ser Asp Gly Met Phe Tyr Leu Tyr Asn Gly Asp Leu Ser His
705                 710                 715                 720

Tyr Ser Asp Gly Tyr Trp Pro Thr Val Asn Pro Tyr Lys Met Pro Gly
                725                 730                 735

Thr Thr Glu Thr Asp Ala Lys Arg Ala Asp Ser Asp Thr Gly Lys Val
            740                 745                 750
```

```
Leu Pro Ser Ala Phe Val Gly Thr Ser Lys Leu Asp Asp Ala Asn Ala
        755                 760                 765

Thr Ala Thr Met Asp Phe Thr Asn Trp Asn Gln Thr Leu Thr Ala His
    770                 775                 780

Lys Ser Trp Phe Met Leu Lys Asp Lys Ile Ala Phe Leu Gly Ser Asn
785                 790                 795                 800

Ile Gln Asn Thr Ser Thr Asp Thr Ala Ala Thr Thr Ile Asp Gln Arg
                805                 810                 815

Lys Leu Glu Ser Gly Asn Pro Tyr Lys Val Tyr Val Asn Asp Lys Glu
            820                 825                 830

Ala Ser Leu Thr Glu Gln Glu Lys Asp Tyr Pro Glu Thr Gln Ser Val
        835                 840                 845

Phe Leu Glu Ser Phe Asp Ser Lys Lys Asn Ile Gly Tyr Phe Phe Phe
    850                 855                 860

Lys Lys Ser Ser Ile Ser Met Ser Lys Ala Leu Gln Lys Gly Ala Trp
865                 870                 875                 880

Lys Asp Ile Asn Glu Gly Gln Ser Asp Lys Glu Val Glu Asn Glu Phe
                885                 890                 895

Leu Thr Ile Ser Gln Ala His Lys Gln Asn Arg Asp Ser Tyr Gly Tyr
            900                 905                 910

Met Leu Ile Pro Asn Val Asp Arg Ala Thr Phe Asn Gln Met Ile Lys
        915                 920                 925

Glu Leu Glu Ser Ser Leu Ile Glu Asn Asn Glu Thr Leu Gln Ser Val
    930                 935                 940

Tyr Asp Ala Lys Gln Gly Val Trp Gly Ile Val Lys Tyr Asp Asp Ser
945                 950                 955                 960

Val Ser Thr Ile Ser Asn Gln Phe Gln Val Leu Lys Arg Gly Val Tyr
                965                 970                 975

Thr Ile Arg Lys Glu Gly Asp Glu Tyr Lys Ile Ala Tyr Tyr Asn Pro
            980                 985                 990

Glu Thr Gln Glu Ser Ala Pro Asp  Gln Glu Val Phe Lys  Lys Leu Glu
        995                 1000                 1005
```

Gln Ala Ala Gln Pro Gln Val Gln Asn Ser Lys Glu Lys Glu Lys
1010 1015 1020

Ser Glu Glu Glu Lys Asn His Ser Asp Gln Lys Asn Leu Pro Gln
1025 1030 1035

Thr Gly Glu Gly Gln Ser Ile Leu Ala Ser Leu Gly Phe Leu Leu
1040 1045 1050

Leu Gly Ala Phe Tyr Leu Phe Arg Arg Gly Lys Asn Asn
1055 1060 1065

<210> 24
<211> 390
<212> DNA
<213> Streptococcus pneumoniae

<400> 24

atgaatcaat cctactttta tctaaaaatg aaagaacaca aactcaaggt tccttataca 60

ggtaaggagc gccgtgtacg tattcttctt cctaaagatt atgagaaaga tacagaccgt 120

tcctatcctg ttgtatactt tcatgacggg caaaatgttt ttaatagcaa agagtctttc 180

attggacatt catggaagat tatcccagct atcaaacgaa atccggatat cagtcgcatg 240

attgtcgttg ctattgacaa tgatggtatg ggcggatga atgagtatgc ggcttggaag 300

ttccaagaat ctcctatccc agggcagcag tttggtggta agggtgtgga gtatgctgag 360

tttgtcatgg aggtggtcaa gccttttatc 390

<210> 25
<211> 900
<212> DNA
<213> Streptococcus pneumoniae

<400> 25

atgtcatcta aatttatgaa gagcgctgcg gtgcttggaa ctgctacact tgctagcttg 60
cttttggtag cttgcatgaa tcaatcctac ttttatctaa aaatgaaaga acacaaactc 120
aaggttcctt atacaggtaa ggagcgccgt gtacgtattc ttcttcctaa agattatgag 180
aaagatacag accgttccta tcctgttgta tactttcatg acgggcaaaa tgtttttaat 240
agcaaagagt ctttcattgg acattcatgg aagattatcc cagctatcaa acgaaatccg 300
gatatcagtc gcatgattgt cgttgctatt gacaatgatg gtatggggcg gatgaatgag 360
tatgcggctt ggaagttcca agaatctcct atcccagggc agcagtttgg tggtaagggt 420
gtggagtatg ctgagtttgt catggaggtg gtcaagcctt ttatcgatga gacctatcgt 480
acaaaagcag actgccagca tacggctatg attggttcct cactaggagg caatattacc 540
cagtttatcg gtttggaata ccaagaccaa attggttgct tgggcgtttt ttcatctgca 600
aactggctcc accaagaagc ctttaaccgc tatttcgagt gccagaaact atcgcctgac 660
cagcgcatct tcatctatgt aggaacagaa gaagcagatg atacagacaa gaccttgatg 720
gatggcaata tcaaacaagc ctatatcgac tcgtcgcttt gctattacca tgatttgata 780
gcaggggggag tacatctgga taatcttgtg ctaaaagttc agtctggtgc catccatagt 840
gaaatccctt ggtcagaaaa tctaccagat tgtctgagat tttttgcaga aaaatggtaa 900

<210> 26
<211> 465
<212> DNA
<213> Streptococcus pneumoniae

<400> 26

atgtcatcta aatttatgaa gagcgctgcg gtgcttggaa ctgctacact tgctagcttg 60
cttttggtag cttgcatgaa tcaatcctac ttttatctaa aaatgaaaga acacaaactc 120
aaggttcctt atacaggtaa ggagcgccgt gtacgtattc ttcttcctaa agattatgag 180
aaagatacag accgttccta tcctgttgta tactttcatg acgggcaaaa tgtttttaat 240
agcaaagagt ctttcattgg acattcatgg aagattatcc cagctatcaa acgaaatccg 300
gatatcagtc gcatgattgt cgttgctatt gacaatgatg gtatggggcg gatgaatgag 360
tatgcggctt ggaagttcca agaatctcct atcccagggc agcagtttgg tggtaagggt 420
gtggagtatg ctgagtttgt catggaggtg gtcaagcctt ttatc 465

<210> 27
<211> 765
<212> DNA
<213> Streptococcus pneumoniae

<400> 27

atgtgctcag ggggtgctaa gaaagaagga gaagcagcta gcaagaaaga aatcatcgtt 60
gcaaccaatg gatcaccaaa gccatttatc tatgaagaaa atggcgaatt gactggttac 120
gagattgaag tcgttcgcgc tatctttaaa gattctgaca aatatgatgt caagtttgaa 180
aagacagaat ggtcaggtgt ctttgctggt cttgacgctg atcgttacaa tatggctgtc 240
aacaatctta gctacactaa agaacgtgcg gagaaatacc tctatgccgc accaattgcc 300
caaaatccta atgtccttgt cgtgaagaaa gatgactcta gtatcaagtc tctcgatgat 360
atcggtggaa aatcgacgga agtcgttcaa gccactacat cagctaagca gttagaagca 420
tacaatgctg aacacacgga caacccaact atccttaact atactaaggc agacttccaa 480
caaatcatgg tacgtttgag cgatggacaa tttgactata agatttttga taaaatcggt 540
gttgaaacag tgatcaagaa ccaaggtttg gacaacttga aagttatcga acttccaagc 600
gaccaacaac cgtacgttta cccacttctt gctcagggtc aagatgagtt gaaatcgttt 660
gtagacaaac gcatcaaaga actttataaa gatggaactc ttgaaaaatt gtctaaacaa 720
ttcttcggag acacttatct accggcagaa gctgatatta aataa 765

<210> 28
<211> 831
<212> DNA
<213> Streptococcus pneumoniae

<400> 28

atgaaaaaaa tcgttaaata ctcatctctt gcagcccttg ctcttgttgc tgcaggtgtg 60
cttgcggctt gctcaggggg tgctaagaaa gaaggagaag cagctagcaa gaaagaaatc 120
atcgttgcaa ccaatggatc accaaagcca tttatctatg aagaaaatgg cgaattgact 180
ggttacgaga ttgaagtcgt tcgcgctatc tttaaagatt ctgacaaata tgatgtcaag 240
tttgaaaaga cagaatggtc aggtgtcttt gctggtcttg acgctgatcg ttacaatatg 300
gctgtcaaca atcttagcta cactaaagaa cgtgcggaga aatacctcta tgccgcacca 360
attgcccaaa atcctaatgt ccttgtcgtg aagaaagatg actctagtat caagtctctc 420
gatgatatcg gtggaaaatc gacggaagtc gttcaagcca ctacatcagc taagcagtta 480
gaagcataca atgctgaaca cacggacaac ccaactatcc ttaactatac taaggcagac 540
ttccaacaaa tcatggtacg tttgagcgat ggacaatttg actataagat tttgataaa 600
atcggtgttg aaacagtgat caagaaccaa ggtttggaca acttgaaagt tatcgaactt 660
ccaagcgacc aacaaccgta cgtttaccca cttcttgctc agggtcaaga tgagttgaaa 720
tcgtttgtag acaaacgcat caaagaactt tataaagatg gaactcttga aaaattgtct 780
aaacaattct tcggagacac ttatctaccg gcagaagctg atattaaata a 831

<210> 29
<211> 1203
<212> DNA
<213> Streptococcus pneumoniae

<400> 29

```
atgtgcggaa gcaaaactgc tgataagcct gctgattctg gttcatctga agtcaaagaa     60
ctcactgtat atgtagacga gggatataag agctatattg aagaggttgc taaagcttat    120
gaaaaagaag ctggagtaaa agtcactctt aaaactggtg atgctctagg aggtcttgat    180
aaactttctc ttgacaacca atctggtaat gtccctgatg ttatgatggc tccatacgac    240
cgtgtaggta gccttggttc tgacggacaa ctttcagaag tgaaattgag cgatggtgct    300
aaaacagacg acacaactaa atctcttgta acagctgcta atggtaaagt ttacggtgct    360
cctgccgtta tcgagtcact tgttatgtac tacaacaaag acttggtgaa agatgctcca    420
aaaacatttg ctgacttgga aaaccttgct aaagatagca aatacgcatt cgctggtgaa    480
gatggtaaaa ctactgcctt cctagctgac tggacaaact tctactatac atatggactt    540
cttgccggta acggtgctta cgtctttggc caaaacggta aagacgctaa agacatcggt    600
cttgcaaacg acggttctat cgtaggtatc aactacgcta aatcttggta cgaaaaatgg    660
cctaaaggta tgcaagatac agaaggtgct ggaaacttaa tccaaactca attccaagaa    720

ggtaaaacag ctgctatcat cgacggacct tggaaagctc aagcctttaa agatgctaaa    780
gtaaactacg gagttgcaac tatcccaact cttccaaatg gaaaagaata tgctgcattc    840
ggtggtggta aagcttgggt cattcctcaa gccgttaaga accttgaagc ttctcaaaaa    900
tttgtagact tccttgttgc aactgaacaa caaaaagtat tatatgataa gactaacgaa    960
atcccagcta atactgaggc tcgttcatac gctgaaggta aaaacgatga gttgacaaca   1020
gctgttatca aacagttcaa gaacactcaa ccactgccaa acatctctca aatgtctgca   1080
gtttgggatc cagcgaaaaa tatgctcttt gatgctgtaa gtggtcaaaa agatgctaaa   1140
acagctgcta acgatgctgt aacattgatc aaagaaacaa tcaaacaaaa atttggtgaa   1200
taa                                                                 1203
```

<210> 30
<211> 1944
<212> DNA
<213> Streptococcus pneumoniae

<400> 30

```
atgtcaggaa ctagtatggc gactccaatc gtggcagctt ctactgtttt gattagaccg      60
aaattaaagg aaatgcttga aagacctgta ttgaaaaatc ttaagggaga tgacaaaata     120
gatcttacaa gtcttacaaa aattgcccta caaaatactg cgcgacctat gatggatgca     180
acttcttgga aagaaaaaag tcaatacttt gcatcaccta gacaacaggg agcaggccta     240
attaatgtgg ccaatgcttt gagaaatgaa gttgtagcaa ctttcaaaaa cactgattct     300
aaaggtttgg taaactcata tggttccatt tctcttaaag aaataaaagg tgataaaaaa     360
tactttacaa tcaagcttca caatacatca aacagacctt tgacttttaa agtttcagca     420
tcagcgataa ctacagattc tctaactgac agattaaaac ttgatgaaac atataaagat     480
gaaaaatctc cagatggtaa gcaaattgtt ccagaaattc acccagaaaa agtcaaagga     540
gcaaatatca catttgagca tgatactttc actataggcg caaattctag ctttgatttg     600
aatgcggtta taaatgttgg agaggccaaa aacaaaaata aatttgtaga atcatttatt     660
cattttgagt cagtggaaga aatggaagct ctaaactcca acgggaagaa aataaacttc     720
caaccttctt tgtcgatgcc tctaatggga tttgctggga attggaacca cgaaccaatc     780
cttgataaat gggcttggga agaagggtca agatcaaaaa cactgggagg ttatgatgat     840
gatggtaaac cgaaaattcc aggaacctta aataagggaa ttggtggaga acatggtata     900
gataaattta atccagcagg agttatacaa aatagaaaag ataaaaatac aacatccctg     960
gatcaaaatc cagaattatt tgctttcaat aacgaaggga tcaacgctcc atcatcaagt    1020
ggttctaaga ttgctaacat ttatccttta gattcaaatg gaaatcctca agatgctcaa    1080
cttgaaagag gattaacacc ttctccactt gtattaagaa gtgcagaaga aggattgatt    1140
tcaatagtaa atacaaataa agagggagaa aatcaaagag acttaaaagt catttcgaga    1200
```

```
gaacacttta ttagaggaat tttaaattct aaaagcaatg atgcaaaggg aatcaaatca      1260
tctaaactaa aagtttgggg tgacttgaag tgggatggac tcatctataa tcctagaggt      1320
agagaagaaa atgcaccaga aagtaaggat aatcaagatc ctgctactaa gataagaggt      1380
caatttgaac cgattgcgga aggtcaatat ttctataaat ttaaatatag attaactaaa      1440
gattacccat ggcaggtttc ctatattcct gtaaaaattg ataacaccgc ccctaagatt      1500
gtttcggttg atttttcaaa tcctgaaaaa attaagttga ttacaaagga tacttatcat      1560
aaggtaaaag atcagtataa gaatgaaacg ctatttgcga gagatcaaaa agaacatcct      1620
gaaaaatttg acgagattgc gaacgaagtt tggtatgctg gcgccgctct tgttaatgaa      1680
gatggagagg ttgaaaaaaa tcttgaagta acttacgcag gtgagggtca aggaagaaat      1740
agaaaacttg ataaagacgg aaataccatt tatgaaatta aaggtgcggg agatttaagg      1800
ggaaaaatca ttgaagtcat tgcattagat ggttctagca atttcacaaa gattcataga      1860
attaaatttg ctaatcaggc tgatgaaaag gggatgattt cctattatct agtagatcct      1920
gatcaagatt catctaaata tcaa                                            1944
```

<210> 31
<211> 1986
<212> DNA
<213> Streptococcus pneumoniae

<400> 31

```
atggtagtct tagcagacac atctagctct gaagatgctt taaacatctc tgataaagaa      60
aaagtagcag aaaataaaga gaaacatgaa aatatccata gtgctatgga aacttcacag     120
gattttaaag agaagaaaac agcagtcatt aaggaaaaag aagttgttag taaaaatcct     180
gtgatagaca ataacactag caatgaagaa gcaaaaatca aagaagaaaa ttccaataaa     240
tcccaaggag attatacgga ctcatttgtg aataaaaaca cagaaaatcc caaaaaagaa     300
gataaagttg tctatattgc tgaatttaaa gataaagaat ctggagaaaa agcaatcaag     360
gaactatcca gtcttaagaa tacaaaagtt ttatatactt atgatagaat ttttaacggt     420
agtgccatag aaacaactcc agataacttg gacaaaatta aacaaataga aggtatttca     480
tcggttgaaa gggcacaaaa agtccaaccc atgatgaatc atgccagaaa ggaaattgga     540
gttgaggaag ctattgatta cctaaagtct atcaatgctc cgtttgggaa aaattttgat     600
ggtagaggta tggtcatttc aaatatcgat actggaacag attatagaca taaggctatg     660
agaatcgatg atgatgccaa agcctcaatg agatttaaaa aagaagactt aaaaggcact     720
gataaaaatt attggttgag tgataaaatc cctcatgcgt tcaattatta taatggtggc     780
aaaatcactg tagaaaaata tgatgatgga agggattatt ttgacccaca tgggatgcat     840
attgcaggga ttcttgctgg aaatgatact gaacaagaca tcaaaaactt taacggcata     900
gatggaattg cacctaatgc acaaattttc tcttacaaaa tgtattctga cgcaggatct     960
```

gggtttgcgg gtgatgaaac aatgtttcat gctattgaag attctatcaa acacaacgtt 1020

gatgttgttt cggtatcatc tggttttaca ggaacaggtc ttgtaggtga gaaatattgg 1080

caagctattc gggcattaag aaaagcaggc attccaatgg ttgtcgctac gggtaactat 1140

gcgacttctg cttcaagttc ttcatgggat ttagtagcaa ataatcatct gaaaatgacc 1200

gacactggaa atgtaacacg aactgcagca catgaagatg cgatagcggt cgcttctgct 1260

aaaaatcaaa cagttgagtt tgataaagtt aacataggtg gagaaagttt taaatacaga 1320

aatatagggg ccttttttcga taagagtaaa atcacaacaa atgaagatgg aacaaaagct 1380

cctagtaaat taaaatttgt atatataggc aaggggcaag accaagattt gataggtttg 1440

gatcttaggg gcaaaattgc agtaatggat agaatttata caaaggattt aaaaaatgct 1500

tttaaaaaag ctatggataa gggtgcacgc gccattatgg ttgtaaatac tgtaaattac 1560

tacaatagag ataattggac agagcttcca gctatgggat atgaagcgga tgaaggtact 1620

aaaagtcaag tgttttcaat ttcaggagat gatggtgtaa agctatggaa catgattaat 1680

cctgataaaa aaactgaagt caaaagaaat aataaagaag attttaaaga taaattggag 1740

caatactatc caattgatat ggaaagtttt aattccaaca aaccgaatgt aggtgacgaa 1800

aaagagattg actttaagtt tgcacctgac acagacaaag aactctataa agaagatatc 1860

atcgttccag caggatctac atcttggggg ccaagaatag atttactttt aaaacccgat 1920

gtttcagcac ctggtaaaaa tattaaatcc acgcttaatg ttattaatgg caaatcaact 1980

tatggc 1986

<210> 32
<211> 6
<212> PRT
<213> Artificial Sequence

<220>
<223> Description of Artificial Sequence: Synthetic 6xHis tag

<400> 32

His His His His His His
1 5

<210> 33
<211> 10
<212> PRT
<213> Artificial Sequence

<220>
<223> Description of Artificial Sequence: Synthetic peptide

<400> 33

Met Ser Tyr Tyr His His His His His His
1 5 10

<210> 34
<211> 9
<212> PRT
<213> Streptococcus pneumoniae

<400> 34

Ala Ile Ile Asp Gly Pro Trp Lys Ala
1 5

<210> 35
<211> 9
<212> PRT
<213> Streptococcus pneumoniae

<400> 35

Val Met Met Ala Pro Tyr Asp Arg Val
1 5

<210> 36
<211> 9
<212> PRT
<213> Streptococcus pneumoniae

<400> 36

Ser Ile Ala Gly Ile Asn Tyr Ala Lys
1 5

<210> 37
<211> 9
<212> PRT
<213> Streptococcus pneumoniae

<400> 37

Val Trp Asp Pro Ala Lys Asn Met Leu
1 5

<210> 38
<211> 9
<212> PRT
<213> Streptococcus pneumoniae

<400> 38

Gln Pro Leu Pro Asn Ile Ser Gln Met
1 5

<210> 39
<211> 9
<212> PRT
<213> Streptococcus pneumoniae

<400> 39

Ala Pro Tyr Asp Arg Val Gly Ser Leu
1               5

<210> 40
<211> 9
<212> PRT
<213> Streptococcus pneumoniae

<400> 40

Ala Pro Ala Val Ile Glu Ser Leu Val
1               5

<210> 41
<211> 9
<212> PRT
<213> Streptococcus pneumoniae

<400> 41

Phe Tyr Tyr Thr Tyr Gly Leu Leu Ala
1               5

<210> 42
<211> 8
<212> PRT
<213> Streptococcus pneumoniae

<400> 42

Ser Lys Tyr Ala Phe Ala Gly Glu
1               5

<210> 43
<211> 8
<212> PRT
<213> Streptococcus pneumoniae

<400> 43

Thr Glu Gly Ala Gly Asn Leu Ile
1               5

<210> 44
<211> 9
<212> PRT
<213> Streptococcus pneumoniae

<400> 44

Leu Ala Asp Trp Thr Asn Phe Tyr Tyr
1               5

<210> 45
<211> 9
<212> PRT

<213> Streptococcus pneumoniae

<400> 45

Ser Leu Val Met Tyr Tyr Asn Lys Asp
1 5

<210> 46
<211> 9
<212> PRT
<213> Streptococcus pneumoniae

<400> 46

Lys Glu Ala Gly Val Lys Val Thr Leu
1 5

<210> 47
<211> 9
<212> PRT
<213> Streptococcus pneumoniae

<400> 47

Lys Ser Thr Ala Val Leu Gly Thr Val
1 5

<210> 48
<211> 9
<212> PRT
<213> Streptococcus pneumoniae

<400> 48

Gly Ala Lys Thr Asp Asp Thr Thr Lys
1 5

<210> 49
<211> 9
<212> PRT
<213> Streptococcus pneumoniae

<400> 49

Ser Gln Lys Phe Val Asp Phe Leu Val
1 5

<210> 50
<211> 9
<212> PRT
<213> Streptococcus pneumoniae

<400> 50

Gln Ala Phe Lys Asp Ala Lys Val Asn
1 5

<210> 51

<211> 9
<212> PRT
<213> Streptococcus pneumoniae

<400> 51

Ala Val Ile Glu Ser Leu Val Met Tyr
1               5

<210> 52
<211> 9
<212> PRT
<213> Streptococcus pneumoniae

<400> 52

Asp Ala Lys Thr Ala Ala Asn Asp Ala
1               5

<210> 53
<211> 9
<212> PRT
<213> Streptococcus pneumoniae

<400> 53

Tyr Gly Val Ala Thr Ile Pro Thr Leu
1               5

<210> 54
<211> 9
<212> PRT
<213> Streptococcus pneumoniae

<400> 54

Lys Thr Ala Ala Ile Ile Asp Gly Pro
1               5

<210> 55
<211> 9
<212> PRT
<213> Streptococcus pneumoniae

<400> 55

Lys Ala Tyr Glu Lys Glu Ala Gly Val
1               5

<210> 56
<211> 9
<212> PRT
<213> Streptococcus pneumoniae

<400> 56

Ala Gly Asn Gly Ala Tyr Val Phe Gly
1               5

<210> 57
<211> 9
<212> PRT
<213> Streptococcus pneumoniae

<400> 57

Ala Trp Val Ile Pro Gln Ala Val Lys
1 5

<210> 58
<211> 9
<212> PRT
<213> Streptococcus pneumoniae

<400> 58

Ala Leu Gly Leu Val Ala Ala Gly Val
1 5

<210> 59
<211> 9
<212> PRT
<213> Streptococcus pneumoniae

<400> 59

Glu Leu Thr Gly Tyr Glu Ile Glu Val
1 5

<210> 60
<211> 9
<212> PRT
<213> Streptococcus pneumoniae

<400> 60

Ala Val Asn Asn Leu Ser Tyr Thr Lys
1 5

<210> 61
<211> 9
<212> PRT
<213> Streptococcus pneumoniae

<400> 61

Thr Tyr Leu Pro Ala Glu Ala Asp Ile
1 5

<210> 62
<211> 9
<212> PRT
<213> Streptococcus pneumoniae

<400> 62

Arg Tyr Asn Met Ala Val Asn Asn Leu
1 5

<210> 63
<211> 9
<212> PRT
<213> Streptococcus pneumoniae

<400> 63

Asp Phe Gln Gln Ile Met Val Arg Leu
1 5

<210> 64
<211> 9
<212> PRT
<213> Streptococcus pneumoniae

<400> 64

Glu His Thr Asp Asn Pro Thr Ile Leu
1 5

<210> 65
<211> 9
<212> PRT
<213> Streptococcus pneumoniae

<400> 65

Ala Pro Ile Ala Gln Asn Pro Asn Val
1 5

<210> 66
<211> 9
<212> PRT
<213> Streptococcus pneumoniae

<400> 66

Leu Pro Ser Asp Gln Gln Pro Tyr Val
1 5

<210> 67
<211> 9
<212> PRT
<213> Streptococcus pneumoniae

<400> 67

Tyr Val Tyr Pro Leu Leu Ala Gln Gly
1 5

<210> 68
<211> 9
<212> PRT
<213> Streptococcus pneumoniae

<400> 68

Gln Gly Leu Asp Asn Leu Lys Val Ile
1               5

<210> 69
<211> 8
<212> PRT
<213> Streptococcus pneumoniae

<400> 69

Lys Tyr Leu Tyr Ala Ala Pro Ile
1               5

<210> 70
<211> 8
<212> PRT
<213> Streptococcus pneumoniae

<400> 70

Gly Glu Leu Thr Gly Tyr Glu Ile
1               5

<210> 71
<211> 9
<212> PRT
<213> Streptococcus pneumoniae

<400> 71

Asn Pro Asn Val Leu Val Val Lys Lys
1               5

<210> 72
<211> 9
<212> PRT
<213> Streptococcus pneumoniae

<400> 72

Lys Leu Ser Lys Gln Phe Phe Gly Asp
1               5

<210> 73
<211> 9
<212> PRT
<213> Streptococcus pneumoniae

<400> 73

Gly Ser Pro Arg Pro Phe Ile Tyr Glu
1               5

<210> 74
<211> 9
<212> PRT

<213> Streptococcus pneumoniae

<400> 74

Ala Val Asn Asn Leu Ser Tyr Thr Lys
1 5

<210> 75
<211> 9
<212> PRT
<213> Streptococcus pneumoniae

<400> 75

Lys Ile Phe Asp Lys Ile Gly Val Glu
1 5

<210> 76
<211> 9
<212> PRT
<213> Streptococcus pneumoniae

<400> 76

Met Val Arg Leu Ser Asp Gly Gln Phe
1 5

<210> 77
<211> 9
<212> PRT
<213> Streptococcus pneumoniae

<400> 77

Tyr Val Tyr Pro Leu Leu Ala Gln Gly
1 5

<210> 78
<211> 9
<212> PRT
<213> Streptococcus pneumoniae

<400> 78

Val Val Gln Ala Thr Thr Ser Ala Lys
1 5

<210> 79
<211> 9
<212> PRT
<213> Streptococcus pneumoniae

<400> 79

Thr Leu Glu Lys Leu Ser Lys Gln Phe
1 5

<210> 80
<211> 9
<212> PRT
<213> Streptococcus pneumoniae

<400> 80

Val Ala Ala Gly Val Leu Ala Ala Cys
1 5

<210> 81
<211> 9
<212> PRT
<213> Streptococcus pneumoniae

<400> 81

Leu Asp Asn Leu Lys Val Ile Glu Leu
1 5

<210> 82
<211> 9
<212> PRT
<213> Streptococcus pneumoniae

<400> 82

Asn Met Ala Val Asn Asn Leu Ser Tyr
1 5

<210> 83
<211> 9
<212> PRT
<213> Streptococcus pneumoniae

<400> 83

Arg Leu Leu Asp Leu Ala Pro Gln Val
1 5

<210> 84
<211> 9
<212> PRT
<213> Streptococcus pneumoniae

<400> 84

Met Leu Glu Ile Pro Ala His Gln Ile
1 5

<210> 85
<211> 9
<212> PRT
<213> Streptococcus pneumoniae

<400> 85

Lys Asn Phe Phe Ala His His Pro Lys
1 5

<210> 86
<211> 9
<212> PRT
<213> Streptococcus pneumoniae

<400> 86

Lys Val Ile Leu Ala Gly His Ser Lys
1 5

<210> 87
<211> 9
<212> PRT
<213> Streptococcus pneumoniae

<400> 87

Ser Phe Asp Asn Leu Val Ser Thr Leu
1 5

<210> 88
<211> 9
<212> PRT
<213> Streptococcus pneumoniae

<400> 88

Tyr Tyr Asp Leu Pro Leu Asn Glu Leu
1 5

<210> 89
<211> 9
<212> PRT
<213> Streptococcus pneumoniae

<400> 89

Tyr Phe Asp Leu Phe Phe Gly Thr Ile
1 5

<210> 90
<211> 9
<212> PRT
<213> Streptococcus pneumoniae

<400> 90

Ala Leu Glu Tyr Ile His His Leu Phe
1 5

<210> 91
<211> 9
<212> PRT
<213> Streptococcus pneumoniae

<400> 91

Leu Pro Leu Asn Glu Leu Asp Ile Leu
1 5

<210> 92
<211> 9
<212> PRT
<213> Streptococcus pneumoniae

<400> 92

Ile Pro Gln Gly Ser Ile Ile Gly Met
1 5

<210> 93
<211> 9
<212> PRT
<213> Streptococcus pneumoniae

<400> 93

Asp Pro Glu Leu Gln Lys Gln Phe Ala
1 5

<210> 94
<211> 9
<212> PRT
<213> Streptococcus pneumoniae

<400> 94

Ala Val Tyr Thr Phe Asp Ala Pro Gly
1 5

<210> 95
<211> 9
<212> PRT
<213> Streptococcus pneumoniae

<400> 95

Gln Ser Leu Thr Pro Glu Glu Arg Glu
1 5

<210> 96
<211> 8
<212> PRT
<213> Streptococcus pneumoniae

<400> 96

Ala Ile Tyr Ala Ala Ser Gln Ile
1 5

<210> 97
<211> 8
<212> PRT

<213> Streptococcus pneumoniae

<400> 97

Leu Glu Ile Pro Ala His Gln Ile
1 5

<210> 98
<211> 9
<212> PRT
<213> Streptococcus pneumoniae

<400> 98

Leu Leu Asp Leu Ala Pro Gln Val Pro
1 5

<210> 99
<211> 9
<212> PRT
<213> Streptococcus pneumoniae

<400> 99

Trp Gln Ile Glu Asp Lys His Phe Val
1 5

<210> 100
<211> 9
<212> PRT
<213> Streptococcus pneumoniae

<400> 100

Thr Leu Gly Arg Leu Thr Gln Leu Leu
1 5

<210> 101
<211> 9
<212> PRT
<213> Streptococcus pneumoniae

<400> 101

Leu Tyr Phe Asp Leu Phe Phe Gly Thr
1 5

<210> 102
<211> 9
<212> PRT
<213> Streptococcus pneumoniae

<400> 102

Ser Ile Asn Asp Leu Ala Ser Leu Lys
1 5

<210> 103

<211> 9
<212> PRT
<213> Streptococcus pneumoniae

<400> 103

Ser Ile Asn Asp Leu Ala Ser Leu Lys
1 5

<210> 104
<211> 9
<212> PRT
<213> Streptococcus pneumoniae

<400> 104

Tyr Tyr Asp Leu Pro Leu Asn Glu Leu
1 5

<210> 105
<211> 9
<212> PRT
<213> Streptococcus pneumoniae

<400> 105

Gln Lys Val Ile Leu Ala Gly His Ser
1 5

<210> 106
<211> 9
<212> PRT
<213> Streptococcus pneumoniae

<400> 106

Gly Thr Asp Asp Ser Ile Ile Gly Trp
1 5

<210> 107
<211> 9
<212> PRT
<213> Streptococcus pneumoniae

<400> 107

Thr Tyr Leu Ser Phe Asp Asn Leu Val
1 5

<210> 108
<211> 9
<212> PRT
<213> Streptococcus pneumoniae

<400> 108

Phe Gly Thr Ile Leu Asp Ala Gly Ile
1 5

<210> 109
<211> 9
<212> PRT
<213> Streptococcus pneumoniae

<400> 109

Asn Gln Ile Thr Ala Val Tyr Thr Phe
1 5

<210> 110
<211> 9
<212> PRT
<213> Streptococcus pneumoniae

<400> 110

His Leu Asp Asn Leu Val Leu Lys Val
1 5

<210> 111
<211> 9
<212> PRT
<213> Streptococcus pneumoniae

<400> 111

Asp Leu Ile Ala Gly Arg Val His Leu
1 5

<210> 112
<211> 9
<212> PRT
<213> Streptococcus pneumoniae

<400> 112

Ile Leu Leu Pro Lys Asp Tyr Glu Lys
1 5

<210> 113
<211> 9
<212> PRT
<213> Streptococcus pneumoniae

<400> 113

Glu Tyr Gln Asp Gln Ile Gly Cys Leu
1 5

<210> 114
<211> 9
<212> PRT
<213> Streptococcus pneumoniae

<400> 114

Tyr Phe His Asp Gly Gln Asn Val Phe
1 5

<210> 115
<211> 9
<212> PRT
<213> Streptococcus pneumoniae

<400> 115

Asn Pro Asp Ile Ser Arg Met Ile Val
1 5

<210> 116
<211> 9
<212> PRT
<213> Streptococcus pneumoniae

<400> 116

Ile Pro Trp Ser Glu Asn Leu Pro Asp
1 5

<210> 117
<211> 9
<212> PRT
<213> Streptococcus pneumoniae

<400> 117

Gln Phe Gly Gly Lys Gly Val Glu Tyr
1 5

<210> 118
<211> 9
<212> PRT
<213> Streptococcus pneumoniae

<400> 118

Ile Gly Leu Glu Tyr Gln Asp Gln Ile
1 5

<210> 119
<211> 8
<212> PRT
<213> Streptococcus pneumoniae

<400> 119

Val Tyr Phe His Asp Gly Gln Asn
1 5

<210> 120
<211> 8
<212> PRT

<213> Streptococcus pneumoniae

<400> 120

Met Glu Val Val Lys Pro Phe Ile
1 5

<210> 121
<211> 9
<212> PRT
<213> Streptococcus pneumoniae

<400> 121

Tyr Leu Lys Met Lys Glu His Lys Leu
1 5

<210> 122
<211> 9
<212> PRT
<213> Streptococcus pneumoniae

<400> 122

Lys Leu Ser Pro Asp Gln Arg Ile Phe
1 5

<210> 123
<211> 9
<212> PRT
<213> Streptococcus pneumoniae

<400> 123

Arg Ile Phe Ile Tyr Val Gly Thr Glu
1 5

<210> 124
<211> 9
<212> PRT
<213> Streptococcus pneumoniae

<400> 124

Phe Ile Asp Glu Thr Tyr Arg Thr Lys
1 5

<210> 125
<211> 9
<212> PRT
<213> Streptococcus pneumoniae

<400> 125

Asp Thr Asp Arg Ser Tyr Pro Val Val
1 5

<210> 126
<211> 9
<212> PRT
<213> Streptococcus pneumoniae

<400> 126

Tyr Ile Asp Ser Ser Leu Cys Tyr Tyr
1 5

<210> 127
<211> 9
<212> PRT
<213> Streptococcus pneumoniae

<400> 127

Thr Gln Phe Ile Gly Leu Glu Tyr Gln
1 5

<210> 128
<211> 9
<212> PRT
<213> Streptococcus pneumoniae

<400> 128

Lys Asp Thr Asp Arg Ser Tyr Pro Val
1 5

<210> 129
<211> 9
<212> PRT
<213> Streptococcus pneumoniae

<400> 129

Leu Cys Tyr Tyr His Asp Leu Ile Ala
1 5

<210> 130
<211> 9
<212> PRT
<213> Streptococcus pneumoniae

<400> 130

Asn Val Phe Asn Ser Lys Glu Ser Phe
1 5

<210> 131
<211> 9
<212> PRT
<213> Streptococcus pneumoniae

<400> 131

Met Leu Lys Asp Lys Ile Ala Phe Leu
1 5

<210> 132
<211> 9
<212> PRT
<213> Streptococcus pneumoniae

<400> 132

Ser Leu Ala Asp Tyr Thr Tyr Lys Val
1 5

<210> 133
<211> 9
<212> PRT
<213> Streptococcus pneumoniae

<400> 133

Phe Leu Leu Leu Gly Ala Phe Tyr Leu
1 5

<210> 134
<211> 9
<212> PRT
<213> Streptococcus pneumoniae

<400> 134

Val Leu Ile Asp Gly Leu Ser Gln Leu
1 5

<210> 135
<211> 9
<212> PRT
<213> Streptococcus pneumoniae

<400> 135

Ile Leu Ala Ser Leu Gly Phe Leu Leu
1 5

<210> 136
<211> 9
<212> PRT
<213> Streptococcus pneumoniae

<400> 136

Gly Leu Ser Gln Leu Leu Pro Val Ile
1 5

<210> 137
<211> 9
<212> PRT
<213> Streptococcus pneumoniae

<400> 137

Phe Leu Leu Asn His Tyr Met Thr Val
1 5

<210> 138
<211> 9
<212> PRT
<213> Streptococcus pneumoniae

<400> 138

Met Leu Ile Pro Asn Val Asp Arg Ala
1 5

<210> 139
<211> 9
<212> PRT
<213> Streptococcus pneumoniae

<400> 139

Lys Leu Glu Glu Met Ala Lys Gln Val
1 5

<210> 140
<211> 9
<212> PRT
<213> Streptococcus pneumoniae

<400> 140

Val Leu Lys Arg Gly Val Tyr Thr Ile
1 5

<210> 141
<211> 9
<212> PRT
<213> Streptococcus pneumoniae

<400> 141

Lys Val Ile Ala Gly Leu Leu Arg Lys
1 5

<210> 142
<211> 9
<212> PRT
<213> Streptococcus pneumoniae

<400> 142

Thr Leu Asn Tyr Glu His Met Asn Lys
1 5

<210> 143
<211> 9
<212> PRT

<213> Streptococcus pneumoniae

<400> 143

Asn Ile Gly Tyr Phe Phe Phe Lys Lys
1 5

<210> 144
<211> 9
<212> PRT
<213> Streptococcus pneumoniae

<400> 144

Lys Tyr Thr Asp Val Ile Glu Lys Phe
1 5

<210> 145
<211> 9
<212> PRT
<213> Streptococcus pneumoniae

<400> 145

Lys Tyr Asp Asp Ser Val Ser Thr Ile
1 5

<210> 146
<211> 9
<212> PRT
<213> Streptococcus pneumoniae

<400> 146

Thr Phe Asn Gln Met Ile Lys Glu Leu
1 5

<210> 147
<211> 9
<212> PRT
<213> Streptococcus pneumoniae

<400> 147

Asp Tyr Pro Glu Thr Gln Ser Val Phe
1 5

<210> 148
<211> 9
<212> PRT
<213> Streptococcus pneumoniae

<400> 148

Thr Pro Arg Ala Ile Asn Asn Thr Leu
1 5

<210> 149

<211> 9
<212> PRT
<213> Streptococcus pneumoniae

<400> 149

Ala Pro Leu Leu Val Asn Gly Glu Leu
1 5

<210> 150
<211> 9
<212> PRT
<213> Streptococcus pneumoniae

<400> 150

Tyr Ile Asp His Thr Asn Val Ala Tyr
1 5

<210> 151
<211> 9
<212> PRT
<213> Streptococcus pneumoniae

<400> 151

Lys Gln Asn Gly Asp Ser Tyr Gly Tyr
1 5

<210> 152
<211> 9
<212> PRT
<213> Streptococcus pneumoniae

<400> 152

Phe Leu Leu Asn His Tyr Met Thr Val
1 5

<210> 153
<211> 9
<212> PRT
<213> Streptococcus pneumoniae

<400> 153

Phe Tyr Leu Tyr Asn Gly Asp Leu Ser
1 5

<210> 154
<211> 8
<212> PRT
<213> Streptococcus pneumoniae

<400> 154

Lys Ser Phe Ala Pro Leu Leu Val
1 5

<210> 155
<211> 8
<212> PRT
<213> Streptococcus pneumoniae

<400> 155

Asp Glu Thr Val Val Arg Thr Val
1 5

<210> 156
<211> 9
<212> PRT
<213> Streptococcus pneumoniae

<400> 156

Tyr Ile Asp His Thr Asn Val Ala Tyr
1 5

<210> 157
<211> 9
<212> PRT
<213> Streptococcus pneumoniae

<400> 157

Met Leu Lys Asp Lys Ile Ala Phe Leu
1 5

<210> 158
<211> 9
<212> PRT
<213> Streptococcus pneumoniae

<400> 158

Lys Leu Arg Phe Lys Ile Lys Thr Asp
1 5

<210> 159
<211> 9
<212> PRT
<213> Streptococcus pneumoniae

<400> 159

Lys Leu Glu Leu Phe Tyr Glu Thr Gly
1 5

<210> 160
<211> 9
<212> PRT
<213> Streptococcus pneumoniae

<400> 160

Lys Ile Ala Phe Leu Gly Ser Asn Ile
1 5

<210> 161
<211> 9
<212> PRT
<213> Streptococcus pneumoniae

<400> 161

Ser Val Pro Arg Thr Ser Tyr Leu Ser
1 5

<210> 162
<211> 9
<212> PRT
<213> Streptococcus pneumoniae

<400> 162

Phe Gly Phe Gly Leu Ser Leu Phe Ser
1 5

<210> 163
<211> 9
<212> PRT
<213> Streptococcus pneumoniae

<400> 163

Ser Thr Ile Arg Ser Ile Glu Gln Val
1 5

<210> 164
<211> 9
<212> PRT
<213> Streptococcus pneumoniae

<400> 164

Phe Arg Lys Thr Thr Asp Asn Pro Phe
1 5

<210> 165
<211> 9
<212> PRT
<213> Streptococcus pneumoniae

<400> 165

Thr Val Val Arg Thr Val Arg Asp Ser
1 5

<210> 166
<211> 9
<212> PRT
<213> Streptococcus pneumoniae

<400> 166

Ser Thr Ile Arg Ser Ile Glu Gln Val
1 5

<210> 167
<211> 9
<212> PRT
<213> Streptococcus pneumoniae

<400> 167

Asp Gly Leu Ser Gln Leu Leu Pro Val
1 5

<210> 168
<211> 9
<212> PRT
<213> Streptococcus pneumoniae

<400> 168

Phe Gly Phe Gly Leu Ser Leu Phe Ser
1 5

<210> 169
<211> 9
<212> PRT
<213> Streptococcus pneumoniae

<400> 169

Lys Leu Val Asp Gln Gly Glu Gly Phe
1 5

<210> 170
<211> 9
<212> PRT
<213> Streptococcus pneumoniae

<400> 170

Ala Ile Val Thr Cys Met Asp Ser Arg
1 5

<210> 171
<211> 9
<212> PRT
<213> Streptococcus pneumoniae

<400> 171

Ala Gln Thr Phe Glu Asn Glu Pro Phe
1 5

<210> 172
<211> 9
<212> PRT

<213> Streptococcus pneumoniae

<400> 172

Ala Tyr Val Ala Leu His Gly Gln Leu
1 5

<210> 173
<211> 9
<212> PRT
<213> Streptococcus pneumoniae

<400> 173

Asp Asp Val Ile Ile Ser Gly Ala Ile
1 5

<210> 174
<211> 9
<212> PRT
<213> Streptococcus pneumoniae

<400> 174

Phe Glu Asn Glu Pro Phe Gln Glu Tyr
1 5

<210> 175
<211> 9
<212> PRT
<213> Streptococcus pneumoniae

<400> 175

Phe Met Gln Ala Asn Gln Ala Tyr Val
1 5

<210> 176
<211> 9
<212> PRT
<213> Streptococcus pneumoniae

<400> 176

Ile Ser Gln Gln Gln Met Gly Thr Arg
1 5

<210> 177
<211> 9
<212> PRT
<213> Streptococcus pneumoniae

<400> 177

Lys Pro Lys Thr Arg Val Ala Ile Val
1 5

<210> 178

<211> 9
<212> PRT
<213> Streptococcus pneumoniae

<400> 178

Leu His Gly Gln Leu Asn Leu Pro Leu
1 5

<210> 179
<211> 9
<212> PRT
<213> Streptococcus pneumoniae

<400> 179

Leu His Val Ala Gln Ala Leu Gly Leu
1 5

<210> 180
<211> 9
<212> PRT
<213> Streptococcus pneumoniae

<400> 180

Leu Pro Leu Lys Pro Lys Thr Arg Val
1 5

<210> 181
<211> 9
<212> PRT
<213> Streptococcus pneumoniae

<400> 181

Met Gly Thr Arg Glu Ile Val Val Leu
1 5

<210> 182
<211> 9
<212> PRT
<213> Streptococcus pneumoniae

<400> 182

Met Gln Leu Leu Ile Glu Ser Pro Leu
1 5

<210> 183
<211> 9
<212> PRT
<213> Streptococcus pneumoniae

<400> 183

Gln Ala Asn Gln Ala Tyr Val Ala Leu
1 5

<210> 184
<211> 9
<212> PRT
<213> Streptococcus pneumoniae

<400> 184

Gln Phe Met Gln Ala Asn Gln Ala Tyr
1 5

<210> 185
<211> 9
<212> PRT
<213> Streptococcus pneumoniae

<400> 185

Gln Leu Asn Leu Pro Leu Lys Pro Lys
1 5

<210> 186
<211> 9
<212> PRT
<213> Streptococcus pneumoniae

<400> 186

Gln Gln Met Gly Thr Arg Glu Ile Val
1 5

<210> 187
<211> 9
<212> PRT
<213> Streptococcus pneumoniae

<400> 187

Arg Glu Ile Val Val Leu His His Thr
1 5

<210> 188
<211> 9
<212> PRT
<213> Streptococcus pneumoniae

<400> 188

Ser Pro Leu Ile Pro Asp Asp Val Ile
1 5

<210> 189
<211> 9
<212> PRT

<213> Streptococcus pneumoniae

<400> 189

**Ser Arg Leu His Val Ala Gln Ala Leu**
**1 5**

<210> 190
<211> 9
<212> PRT
<213> Streptococcus pneumoniae

<400> 190

**Thr Glu Asp Met Ile Arg Ser Leu Val**
**1 5**

<210> 191
<211> 9
<212> PRT
<213> Streptococcus pneumoniae

<400> 191

**Val Asp Val Ser Asp Gln Asp Phe Leu**
**1 5**

<210> 192
<211> 9
<212> PRT
<213> Streptococcus pneumoniae

<400> 192

**Val Ser Asp Gln Asp Phe Leu Pro Phe**
**1 5**

<210> 193
<211> 9
<212> PRT
<213> Streptococcus pneumoniae

<400> 193

**Val Thr Glu Asp Met Ile Arg Ser Leu**
**1 5**

<210> 194
<211> 9
<212> PRT
<213> Streptococcus pneumoniae

<400> 194

**Gly Ile Glu Val Glu Lys Pro Leu Tyr**
**1 5**

<210> 195
<211> 9
<212> PRT
<213> Streptococcus pneumoniae

<400> 195

Ala Glu Ala His Leu Leu Tyr Arg Met
1               5

<210> 196
<211> 9
<212> PRT
<213> Streptococcus pneumoniae

<400> 196

Ala Leu Leu Asn Gln Asp Asn Met Arg
1               5

<210> 197
<211> 9
<212> PRT
<213> Streptococcus pneumoniae

<400> 197

Ala Pro Pro Glu Arg Asn Tyr Leu Tyr
1               5

<210> 198
<211> 9
<212> PRT
<213> Streptococcus pneumoniae

<400> 198

Ala Gln Asn Ser Tyr Ile His Ile Leu
1               5

<210> 199
<211> 9
<212> PRT
<213> Streptococcus pneumoniae

<400> 199

Ala Val Ala Ser Met Gly Thr Ala Leu
1               5

<210> 200
<211> 9
<212> PRT
<213> Streptococcus pneumoniae

<400> 200

Ala Tyr Leu Leu Thr Lys Thr Arg Ile
1               5

<210> 201
<211> 9
<212> PRT
<213> Streptococcus pneumoniae

<400> 201

Asp Ala Ala Lys Phe Tyr His Ala Ile
1               5

<210> 202
<211> 9
<212> PRT
<213> Streptococcus pneumoniae

<400> 202

Asp Thr Ala Leu Glu Glu Leu Glu Arg
1               5

<210> 203
<211> 9
<212> PRT
<213> Streptococcus pneumoniae

<400> 203

Glu Glu Tyr Gln Gly Val Pro Phe Ile
1               5

<210> 204
<211> 9
<212> PRT
<213> Streptococcus pneumoniae

<400> 204

Glu Phe Leu Glu Lys Ile Ala Pro Leu
1               5

<210> 205
<211> 9
<212> PRT
<213> Streptococcus pneumoniae

<400> 205

Glu Phe Gln Val Leu Tyr Asp Leu Leu
1               5

<210> 206
<211> 9
<212> PRT
<213> Streptococcus pneumoniae

<400> 206

Glu His Val Glu His Leu Lys Arg Leu
1 5

<210> 207
<211> 9
<212> PRT
<213> Streptococcus pneumoniae

<400> 207

Glu Leu Ser Glu Val Glu Met Thr Arg
1 5

<210> 208
<211> 9
<212> PRT
<213> Streptococcus pneumoniae

<400> 208

Glu Ser Pro Leu Val Leu Asn Asp Tyr
1 5

<210> 209
<211> 9
<212> PRT
<213> Streptococcus pneumoniae

<400> 209

Gly Glu Lys Thr Pro Ser Phe Asn Val
1 5

<210> 210
<211> 9
<212> PRT
<213> Streptococcus pneumoniae

<400> 210

Gly Leu Cys Pro Phe His Gly Glu Lys
1 5

<210> 211
<211> 9
<212> PRT
<213> Streptococcus pneumoniae

<400> 211

Ile Gly Asp Met Pro Val Gln Ile Val
1 5

<210> 212
<211> 9

<212> PRT
<213> Streptococcus pneumoniae

<400> 212

**Ile Thr Met Pro Val Thr Lys Gln Leu**
**1 5**

<210> 213
<211> 9
<212> PRT
<213> Streptococcus pneumoniae

<400> 213

**Lys Ala Leu Leu Asn Gln Asp Asn Met**
**1 5**

<210> 214
<211> 9
<212> PRT
<213> Streptococcus pneumoniae

<400> 214

**Lys Arg Leu Thr Lys Lys Leu Val Leu**
**1 5**

<210> 215
<211> 9
<212> PRT
<213> Streptococcus pneumoniae

<400> 215

**Leu Thr Lys Thr Arg Ile Ser Pro Ile**
**1 5**

<210> 216
<211> 9
<212> PRT
<213> Streptococcus pneumoniae

<400> 216

**Leu Val Leu Val Tyr Asp Gly Asp Lys**
**1 5**

<210> 217
<211> 9
<212> PRT
<213> Streptococcus pneumoniae

<400> 217

**Met Arg Ala Glu Ala His Leu Leu Tyr**
**1 5**

<210> 218
<211> 9
<212> PRT
<213> Streptococcus pneumoniae

<400> 218

Asn Gly Pro Glu Asp Leu Ala Tyr Leu
1               5

<210> 219
<211> 9
<212> PRT
<213> Streptococcus pneumoniae

<400> 219

Gln Thr Glu Glu Val Glu Arg Ala Trp
1               5

<210> 220
<211> 9
<212> PRT
<213> Streptococcus pneumoniae

<400> 220

Ser Glu Ile Tyr Leu Met Glu Gly Phe
1               5

<210> 221
<211> 9
<212> PRT
<213> Streptococcus pneumoniae

<400> 221

Ser Pro His Gln Ala Leu Tyr Asp Met
1               5

<210> 222
<211> 9
<212> PRT
<213> Streptococcus pneumoniae

<400> 222

Val Asp Lys Gln Val Ile Glu Glu Ile
1               5

<210> 223
<211> 9
<212> PRT
<213> Streptococcus pneumoniae

<400> 223

Val Glu Met Thr Arg Asn Lys Ala Leu
1 5

<210> 224
<211> 9
<212> PRT
<213> Streptococcus pneumoniae

<400> 224

Val Leu Tyr Asp Leu Leu Gly Gln Tyr
1 5

<210> 225
<211> 9
<212> PRT
<213> Streptococcus pneumoniae

<400> 225

Val Pro Phe Ile Glu Ala Val Gln Ile
1 5

<210> 226
<211> 9
<212> PRT
<213> Streptococcus pneumoniae

<400> 226

Trp Tyr Gln Val Leu Ala Gln Asp Leu
1 5

<210> 227
<211> 9
<212> PRT
<213> Streptococcus pneumoniae

<400> 227

Tyr Leu Met Glu Gly Phe Met Asp Val
1 5

<210> 228
<211> 9
<212> PRT
<213> Streptococcus pneumoniae

<400> 228

Ala Ala Tyr Ala Pro Asn Glu Val Val
1 5

<210> 229
<211> 9
<212> PRT
<213> Streptococcus pneumoniae

<400> 229

Ala Gly Asp Leu Arg Gly Lys Ile Ile
1 5

<210> 230
<211> 9
<212> PRT
<213> Streptococcus pneumoniae

<400> 230

Asp Glu Ile Ala Asn Glu Val Trp Tyr
1 5

<210> 231
<211> 9
<212> PRT
<213> Streptococcus pneumoniae

<400> 231

Asp Asn Tyr Leu Ile Tyr Gly Asp Leu
1 5

<210> 232
<211> 9
<212> PRT
<213> Streptococcus pneumoniae

<400> 232

Asp Gln Lys Glu His Pro Glu Lys Phe
1 5

<210> 233
<211> 9
<212> PRT
<213> Streptococcus pneumoniae

<400> 233

Asp Ser Leu Thr Asp Arg Leu Lys Leu
1 5

<210> 234
<211> 9
<212> PRT
<213> Streptococcus pneumoniae

<400> 234

Glu Ala Lys Asn Lys Asn Lys Phe Val
1 5

<210> 235
<211> 9
<212> PRT

<213> Streptococcus pneumoniae

<400> 235

**Glu Gly Gln Gly Arg Asn Arg Lys Leu**
**1 5**

<210> 236
<211> 9
<212> PRT
<213> Streptococcus pneumoniae

<400> 236

**Glu Ile Lys Gly Ala Gly Asp Leu Arg**
**1 5**

<210> 237
<211> 9
<212> PRT
<213> Streptococcus pneumoniae.

<400> 237

**Glu Pro Ile Ala Glu Gly Gln Tyr Phe**
**1 5**

<210> 238
<211> 9
<212> PRT
<213> Streptococcus pneumoniae

<400> 238

**Glu Val Ser Glu Leu Lys Pro His Arg**
**1 5**

<210> 239
<211> 9
<212> PRT
<213> Streptococcus pneumoniae

<400> 239

**Gly Ala Phe Phe Asp Lys Ser Lys Ile**
**1 5**

<210> 240
<211> 9
<212> PRT
<213> Streptococcus pneumoniae

<400> 240

**Gly Asp Leu Lys Trp Asp Gly Leu Ile**
**1 5**

<210> 241

<211> 9
<212> PRT
<213> Streptococcus pneumoniae

<400> 241

Gly Glu Val Glu Lys Asn Leu Glu Val
1 5

<210> 242
<211> 9
<212> PRT
<213> Streptococcus pneumoniae

<400> 242

Ile His Phe Glu Ser Val Glu Glu Met
1 5

<210> 243
<211> 9
<212> PRT
<213> Streptococcus pneumoniae

<400> 243

Ile Met Phe Ile Val Gly Ile Phe Leu
1 5

<210> 244
<211> 9
<212> PRT
<213> Streptococcus pneumoniae

<400> 244

Ile Pro Gly Thr Leu Asn Lys Gly Ile
1 5

<210> 245
<211> 9
<212> PRT
<213> Streptococcus pneumoniae

<400> 245

Ile Arg Tyr Gln Val Phe Thr Phe Lys
1 5

<210> 246
<211> 9
<212> PRT
<213> Streptococcus pneumoniae

<400> 246

Ile Ser Asp Lys Gly Gly Phe Asn Trp
1 5

<210> 247
<211> 9
<212> PRT
<213> Streptococcus pneumoniae

<400> 247

Ile Val Ser Glu Glu Asp Phe Ile Leu
1 5

<210> 248
<211> 9
<212> PRT
<213> Streptococcus pneumoniae

<400> 248

Lys Glu Ile Gly Val Glu Glu Ala Ile
1 5

<210> 249
<211> 9
<212> PRT
<213> Streptococcus pneumoniae

<400> 249

Lys Ile Val Val Lys Asp Phe Ala Arg
1 5

<210> 250
<211> 9
<212> PRT
<213> Streptococcus pneumoniae

<400> 250

Lys Lys Ile Asn Phe Gln Pro Ser Leu
1 5

<210> 251
<211> 9
<212> PRT
<213> Streptococcus pneumoniae

<400> 251

Lys Leu Lys Phe Val Tyr Ile Gly Lys
1 5

<210> 252
<211> 9
<212> PRT
<213> Streptococcus pneumoniae

<400> 252

Lys Val Tyr Tyr Gly Asn Asn Tyr Lys
1               5

<210> 253
<211> 9
<212> PRT
<213> Streptococcus pneumoniae

<400> 253

Lys Tyr Trp Gln Ala Ile Arg Ala Leu
1               5

<210> 254
<211> 9
<212> PRT
<213> Streptococcus pneumoniae

<400> 254

Leu His Ile Asp Asn Thr Arg Asp Phe
1               5

<210> 255
<211> 9
<212> PRT
<213> Streptococcus pneumoniae

<400> 255

Met Arg Phe Lys Lys Glu Asp Leu Lys
1               5

<210> 256
<211> 9
<212> PRT
<213> Streptococcus pneumoniae

<400> 256

Asn Glu Ser Val Val Asp Asn Tyr Leu
1               5

<210> 257
<211> 9
<212> PRT
<213> Streptococcus pneumoniae

<400> 257

Asn Glu Val Trp Tyr Ala Gly Ala Ala
1               5

<210> 258
<211> 9
<212> PRT

<213> Streptococcus pneumoniae

<400> 258

Asn Ile Asn Asp Ile Val Asp Gly Leu
1 5

<210> 259
<211> 9
<212> PRT
<213> Streptococcus pneumoniae

<400> 259

Gln Tyr Leu Leu Lys Asp Asn Ile Ile
1 5

<210> 260
<211> 9
<212> PRT
<213> Streptococcus pneumoniae

<400> 260

Ser Pro Arg Gln Gln Gly Ala Gly Leu
1 5

<210> 261
<211> 9
<212> PRT
<213> Streptococcus pneumoniae

<400> 261

Ser Arg Ser Lys Thr Leu Gly Gly Tyr
1 5

<210> 262
<211> 9
<212> PRT
<213> Streptococcus pneumoniae

<400> 262

Ser Ser Leu Lys Asn Thr Lys Val Leu
1 5

<210> 263
<211> 9
<212> PRT
<213> Streptococcus pneumoniae

<400> 263

Thr Ala Ala Val Ile Leu Ala Ala Tyr
1 5

<210> 264

<211> 9
<212> PRT
<213> Streptococcus pneumoniae

<400> 264

```
Trp Thr Glu Leu Pro Ala Met Gly Tyr
1               5
```